(19) Europäisches Patentamt
European Patent Office
Office européen des brevets

(11) EP 3 544 962 B1

(12) **EUROPEAN PATENT SPECIFICATION**

(45) Date of publication and mention of the grant of the patent:
**28.07.2021 Bulletin 2021/30**

(21) Application number: **17811209.0**

(22) Date of filing: **17.11.2017**

(51) Int Cl.:
***C07D 251/48*** *(2006.01)* ***C07D 401/04*** *(2006.01)*
***C07D 403/04*** *(2006.01)*

(86) International application number:
**PCT/EP2017/079570**

(87) International publication number:
**WO 2018/095811 (31.05.2018 Gazette 2018/22)**

(54) **DIAMINOTRIAZINE COMPOUNDS**

ALS HERBICIDE GEEIGNETE HETEROCYCLYLAMINO- ODER HETEROCYCLYLOXYAMINOTRIAZINVERBINDUNGEN

COMPOSES DE HETEROCYCLYLAMINO- OU HETEROCYCLYLOXY-AMINOTRIAZINE UTILES COMME HERBICIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.11.2016 EP 16200863**

(43) Date of publication of application:
**02.10.2019 Bulletin 2019/40**

(73) Proprietor: **BASF SE**
**67056 Ludwigshafen am Rhein (DE)**

(72) Inventors:
- **VOGT, Florian**
  **67056 Ludwigshafen (DE)**
- **GEERDINK, Danny**
  **67056 Ludwigshafen (DE)**
- **ZIERKE, Thomas**
  **67056 Ludwigshafen (DE)**
- **SEITZ, Thomas**
  **67056 Ludwigshafen (DE)**
- **SCHACHTSCHABEL, Doreen**
  **67117 Limburgerhof (DE)**
- **NEWTON, Trevor William**
  **67117 Limburgerhof (DE)**
- **HANZLIK, Kristin**
  **67117 Limburgerhof (DE)**
- **TRESCH, Stefan**
  **67056 Ludwigshafen (DE)**
- **KREUZ, Klaus**
  **67117 Limburgerhof (DE)**

(74) Representative: **BASF IP Association**
**BASF SE**
**G-FLP-C006**
**67056 Ludwigshafen (DE)**

(56) References cited:
**WO-A1-2015/155273**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 544 962 B1

## Description

The present invention relates to diaminotriazine compounds and to their use as herbicides. The present invention also relates to agrochemical compositions for crop protection and to a method for controlling unwanted vegetation.

Diaminotriazines and their use as herbicides are known from, for example, US 3,816,419; US 3,932,167, DE 197 44 711 and from earlier filed WO 2014/064094 and WO 2015/007711.

WO2015/155273 relates to diaminotriazines bearing $C(OR^3)R^4R^5$ attached to the triazine moiety.

Nevertheless, there is still room for improvement, e.g. regarding activity, scope of activity and compatibility with useful plants of the known herbicidal compounds.

It is therefore an object of the present invention to provide compounds having improved herbicidal action, in particular good herbicide activity at low application rates. Moreover, the herbicides should be sufficiently compatible with crop plants for commercial utilization.

These and further objects are achieved by diaminotriazine compounds of formula (I), defined below, and by their agriculturally suitable salts.

Accordingly, the present invention relates to diaminotriazine compounds of formula (I)

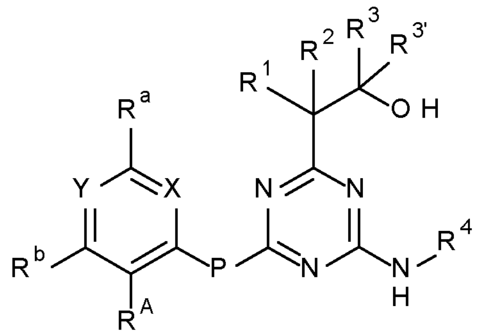

(I)

wherein

P is $NR^5$ or O;
X is $CR^c$ or N;
Y is $CR^d$ or N;
$R^A$ is halogen or CN;
$R^a$, $R^b$, $R^c$ and $R^d$ independently of one another are selected from hydrogen, halogen, OH, CN, amino, $NO_2$, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl, $C_1$-$C_6$-alkylthio, ($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino, ($C_1$-$C_6$-alkyl)-carbonyl, ($C_1$-$C_6$-alkoxy)-carbonyl, ($C_1$-$C_6$-alkyl)-carbonyloxy, $C_3$-$C_6$-cycloalkyl, $C_3$-$C_6$-cycloalkoxy, ($C_3$-$C_6$-cycloalkyl)-$C_1$-$C_4$-alkyl, ($C_3$-$C_6$-cycloalkyl)-$C_1$-$C_4$-alkoxy,
where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups,
or
$R^a$, $R^b$ and $R^d$ are as defined above and $R^c$ is -Q-Ar
Q is a chemical bond, O, $S(O)_m$, $CR^{q1}R^{q2}$, $NR^{q3}$, C(O), C(O)O, $CR^{q1}R^{q2}$-O, $S(O)_mNR^{q3}$ or $C(O)NR^{q3}$,
wherein

m is 0, 1 or 2;
$R^{q1}$, $R^{q2}$ independently of one another are selected from hydrogen, halogen or $C_1$-$C_4$-alkyl;
$R^{q3}$ is H, CN, $C_1$-$C_6$-alkyl, ($C_1$-$C_6$-alkoxy)-$C_1$-$C_6$-alkyl, ($C_1$-$C_6$-alkyl)-carbonyl, ($C_1$-$C_6$-alkoxy)carbonyl, ($C_1$-$C_6$-alkyl)sulfonyl, where the aliphatic parts of the 5 aforementioned radicals are unsubstituted, partly or completely halogenated;

Ar is phenyl, which is unsubstituted or carries 1, 2, 3, 4 or 5 radicals $R^e$ that are identical or different and independently of one another are selected from halogen, OH, CN, amino, $NO_2$, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl, $C_1$-$C_6$-alkylthio, ($C_1$-$C_6$-alkyl)sulfinyl, ($C_1$-$C_6$-alkyl)sul-

fonyl, ($C_1$-$C_6$-alkyl)amino, di($C_1$-$C_6$-alkyl)amino, ($C_1$-$C_6$-alkyl)-carbonyl, ($C_1$-$C_6$-alkoxy)-carbonyl, ($C_1$-$C_6$-alkyl)-carbonyloxy, $C_3$-$C_6$-cycloalkyl, $C_3$-$C_6$-cycloalkoxy, ($C_3$-$C_6$-cycloalkyl)-$C_1$-$C_4$-alkyl, ($C_3$-$C_6$-cycloalkyl)-$C_1$-$C_4$-alkoxy, where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups;

or

$R^b$ and $R^d$ are as defined above and $R^a$ and $R^c$ together with the carbon atoms to which they are attached form a saturated or unsaturated, 5- or 6-membered carbocycle or saturated or unsaturated, 5- or 6-membered heterocycle having 1 or 2 heteroatoms or heteroatom moieties, selected from O, S, S(O), $S(O)_2$, N or $NR^z$ as ring members, where the carbocycle or the heterocycle are unsubstituted or carry 1, 2, 3 or 4 radicals $R^f$ that are identical or different and independently of one another are selected from halogen, OH, CN, amino, $NO_2$, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl, $C_1$-$C_6$-alkylthio,($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino,($C_1$-$C_6$-alkyl)-carbonyl, ($C_1$-$C_6$-alkoxy)-carbonyl, ($C_1$-$C_6$-alkyl)-carbonyloxy, $C_3$-$C_6$-cycloalkyl, $C_3$-$C_6$-cycloalkoxy, ($C_3$-$C_6$-cycloalkyl)-$C_1$-$C_4$-alkyl, ($C_3$-$C_6$-cycloalkyl)-$C_1$-$C_4$-alkoxy,

where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups;

$R^z$ is selected from H, OH, $S(O)_2NH_2$, CN, $C_1$-$C_6$-alkyl, $C_2$-$C_6$-alkenyl, $C_2$-$C_6$-alkynyl, ($C_3$-$C_6$-cycloalkyl)-$C_1$-$C_4$-alkyl, $C_1$-$C_6$-alkoxy, ($C_1$-$C_6$-alkoxy)-$C_1$-$C_6$-alkyl, ($C_1$-$C_6$-alkyl)-carbonyl, ($C_1$-$C_6$-alkoxy)carbonyl, ($C_1$-$C_6$-alkyl)sulfonyl, ($C_1$-$C_6$-alkylamino)carbonyl, di($C_1$-$C_6$-alkyl)aminocarbonyl, ($C_1$-$C_6$-alkylamino)sulfonyl, di($C_1$-$C_6$-alkyl)aminosulfonyl and ($C_1$-$C_6$-alkoxy)sulfonyl,

where the aliphatic and cycloaliphatic parts of the 14 aforementioned radicals are unsubstituted, partly or completely halogenated,

$R^1$ is selected from H, halogen, OH, CN, $C_1$-$C_6$-alkyl, ($C_1$-$C_6$-alkoxy)-$C_1$-$C_6$-alkyl, $C_3$-$C_6$-cycloalkyl, ($C_3$-$C_6$-cycloalkyl)-$C_1$-$C_4$-alkyl, $C_1$-$C_6$-alkoxy, $C_2$-$C_6$-alkenyloxy, $C_2$-$C_6$-alkynyloxy, $C_3$-$C_6$-cycloalkoxy, ($C_3$-$C_6$-cycloalkyl)-$C_1$-$C_4$-alkoxy,

where the aliphatic and cycloaliphatic parts of the 9 aforementioned radicals are unsubstituted, partly or completely halogenated;

$R^2$ is selected from H, halogen, CN, $C_1$-$C_6$-alkyl, $C_1$-$C_6$-haloalkyl, $C_1$-$C_6$-alkoxy and $C_1$-$C_6$-haloalkoxy;

$R^3$ and $R^{3'}$ independently of one another are selected from H, $C_1$-$C_6$-alkyl, $C_3$-$C_6$-cycloalkyl, ($C_3$-$C_6$-cycloalkyl)-$C_1$-$C_4$-alkyl,

where the aliphatic and cycloaliphatic parts of the 3 aforementioned radicals are unsubstituted, partly or completely halogenated; or

$R^3$ and $R^{3'}$ together with the carbon atom to which they are attached form a moiety selected from $C_3$-$C_6$-cycloalkyl or $C_3$-$C_6$-cycloalkenyl where the carbocycle is unsubstituted, partly or completely halogenated or carry from 1 to 6 $C_1$-$C_6$-alkyl groups;

or

$R^1$ and $R^2$ together with the carbon atom to which they are attached form a moiety selected from carbonyl, $C_3$-$C_6$-cycloalkyl, $C_3$-$C_6$-cycloalkenyl, three- to six-membered saturated or partially unsaturated heterocyclyl, where the carbocycle and the heterocycle are unsubstituted, partly or completely halogenated or carry from 1 to 6 $C_1$-$C_6$-alkyl groups;

or

$R^2$ and $R^3$ together with the carbon atom to which they are attached form a moiety selected from $C_3$-$C_6$-cycloalkyl, $C_3$-$C_6$-cycloalkenyl, three- to six-membered saturated or partially unsaturated heterocyclyl, where the carbocycle and the heterocycle are unsubstituted, partly or completely halogenated or carry from 1 to 6 $C_1$-$C_6$-alkyl groups;

$R^4$ and $R^5$ independently of one another are selected from H, OH, $S(O)_2NH_2$, CN, $C_1$-$C_6$-alkyl, $C_2$-$C_6$-alkenyl, $C_2$-$C_6$-alkynyl, ($C_3$-$C_6$-cycloalkyl)-$C_1$-$C_4$-alkyl, $C_3$-$C_6$-cycloalkyl)-carbonyl, $C_1$-$C_6$-alkoxy, ($C_1$-$C_6$-alkoxy)-$C_1$-$C_6$-alkyl, ($C_1$-$C_6$-alkyl)carbonyl, ($C_1$-$C_6$-alkoxy)carbonyl, ($C_1$-$C_6$-alkyl)sulfonyl, ($C_1$-$C_6$-alkylamino)carbonyl, di($C_1$-$C_6$-alkyl)aminocarbonyl, ($C_1$-$C_6$-alkylamino)sulfonyl, di($C_1$-$C_6$-alkyl)aminosulfonyl and ($C_1$-$C_6$-alkoxy)sulfonyl,

where the aliphatic and cycloaliphatic parts of the 15 aforementioned radicals are unsubstituted, partly or completely halogenated,

phenyl, phenyl-$C_1$-$C_6$ alkyl, phenylsulfonyl, phenylaminosulfonyl, phenylaminocarbonyl, phenyl($C_1$-$C_6$-alkyl)aminocarbonyl, phenylcarbonyl and phenoxycarbonyl,

where phenyl in the last 8 mentioned radicals is unsubstituted or substituted by 1, 2, 3, 4 or 5 identical or different

substituents selected from halogen, CN, $NO_2$, $C_1$-$C_6$-alkyl, $C_1$-$C_6$-haloalkyl, $C_1$-$C_6$-alkoxy and $C_1$-$C_6$-haloalkoxy

including their agriculturally acceptable salts.

The present invention also relates to agrochemical compositions comprising at least one diaminotriazine compounds of formula (I) and at least one auxiliary customary for formulating crop protection agents, in particular at least one inert liquid and/or solid carrier and, if appropriate, at least one surface-active substance.

The present invention also relates to the use of diaminotriazine compounds of formula (I) as herbicides, i.e. for controlling unwanted and/or harmful vegetation or plants, or for the desiccation/defoliation of plants.

The present invention furthermore provides a method for controlling unwanted plants. The method includes allowing a herbicidally effective amount of at least one diaminotriazine compound of the formula (I) to act on the unwanted plants or vegetation, their seeds and/or their habitat. Application can be done before, during and/or after the emergence of the unwanted plants.

Moreover, the invention relates to processes for preparing diaminotriazine compound of formula (I) and to intermediates.

Further embodiments of the present invention are evident from the claims, the description and the examples. It is to be understood that the features mentioned above and still to be illustrated below of the subject matter of the invention can be applied not only in the combination given in each particular case but also in other combinations, without leaving the scope of the invention.

As used herein, the terms "undesirable vegetation", "unwanted vegetation", unwanted plants" and "harmful plants" are synonyms.

In the context of substituents, the term "one or more substitutents" means that the number of substituents is e.g. from 1 to 10, in particular 1, 2, 3, 4, 5, 6, 7, 8, 9 or 10.

If the diaminotriazine compounds of formula (I) as described herein is capable of forming geometrical isomers, for example E/Z isomers, the invention relates to both the pure isomers and mixtures thereof. The invention relates to the use of the pure isomers and to the use of their mixtures and to compositions containing the pure isomers or mixtures thereof.

If the diaminotriazine compounds of formula (I) as described herein have one or more centres of chirality and, as a consequence, are present as enantiomers or diastereomers, the invention relates to both the pure enantiomers or diastereomers, and mixtures thereof. The invention relates to the use of the pure enantiomers or diasteremers and to the use of the mixtures thereof and to compositions containing the pure enantiomers or diastereomers or mixtures thereof.

If the diaminotriazine compounds of formula (I) as described herein have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by $C_1$-$C_4$-alkyl, hydroxy-$C_1$-$C_4$-alkyl, ($C_1$-$C_4$-alkoxy)-$C_1$-$C_4$-alkyl, hydroxy-($C_1$-$C_4$-alkoxy)-$C_1$-$C_4$-alkyl, phenyl or benzyl, preferably ammonium, methylammonium, isopropylammonium, dimethylammonium, diisopropylammonium, trimethylammonium, heptylammonium, dodecylammonium, tetradecylammonium, tetramethylammonium, tetraethylammonium, tetrabutylammonium, 2-hydroxyethyl-ammonium (olamine salt), 2-(2-hydroxyeth-1-oxy)eth-1-ylammonium (diglycolamine salt), di(2-hydroxyeth-1-yl)-ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri($C_1$-$C_4$-alkyl)sulfonium, such as trimethylsulfonium, and sulfoxonium ions, preferably tri($C_1$-$C_4$-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogensulfate, methylsulfate, sulfate, dihydrogenphosphate, hydrogenphosphate, nitrate, bicarbonate, carbonate, hexafluorosilicate, hexafluorophosphate, benzoate and also the anions of $C_1$-$C_4$-alkanoic acids, preferably formate, acetate, propionate and butyrate.

The organic moieties mentioned in the definition of the variables, e.g. $R^A$, $R^a$, $R^b$, $R^c$, $R^d$, $R^e$, $R^{q1}$, $R^{q2}$, $R^{q3}$, $R^z$, $R^1$, $R^2$, $R^3$, $R^{3'}$, $R^4$, $R^5$, Ar, P, Q, X, Y are - like the term halogen - collective terms for individual enumerations of the individual group members. The term halogen denotes in each case fluorine, chlorine, bromine or iodine. All hydrocarbon chains, i.e. all alkyl, haloalkyl, alkenyl, alkynyl, alkoxy, alkenyloxy, alkynyloxy, alkylthio, alkylsulfinyl, alkylsulfonyl, (alkyl)amino, di(alkyl)amino, alkoxyalkyl, alkoxyalkoxy, (alky)carbonyl, (alkoxy)carbonyl chains can be straight-chain or branched, the prefix $C_n$-$C_m$ denoting in each case the possible number of carbon atoms in the group. The same applies to composed radicals, such as cycloalkylalkyl and phenylalkyl.

Examples of such meanings are:

- $C_1$-$C_4$-alkyl and also the $C_1$-$C_4$-alkyl moieties of $C_1$-$C_4$-alkoxy, $C_1$-$C_4$-alkylthio, $C_1$-$C_4$-alkylsulfonyl, ($C_1$-$C_4$-alkyl)carbonyl, ($C_1$-$C_4$-alkyl)carbonyl, ($C_1$-$C_4$-alkoxy)carbonyl, ($C_1$-$C_4$-alkyl)carbonyloxy, $C_1$-$C_4$-alkyoxy-$C_1$-$C_4$-alkyl, $C_3$-$C_6$-cycloalkyl-$C_1$-$C_4$-alkyl, ($C_1$-$C_4$-alkylamino)carbonyl, di($C_1$-$C_4$-alkyl)aminocarbonyl, ($C_1$-$C_4$-alkylamino)sulfonyl, di($C_1$-$C_4$-alkyl)aminosulfonyl or phenyl-$C_1$-$C_4$-alkyl: for example $CH_3$, $C_2H_5$, n-propyl, $CH(CH_3)_2$, n-butyl, $CH(CH_3)$-$C_2H_5$, $CH_2$-$CH(CH_3)_2$ and $C(CH_3)_3$;
- $C_1$-$C_6$-alkyl and also the $C_1$-$C_6$-alkyl moieties of $C_1$-$C_6$-alkoxy, $C_1$-$C_6$-alkylthio,$C_1$-$C_6$-alkylsulfonyl, ($C_1$-$C_6$-alkyl)carbonyl, ($C_1$-$C_6$-alkyl)carbonyl, ($C_1$-$C_6$-alkoxy)carbonyl, ($C_1$-$C_6$-alkyl)carbonyloxy, $C_1$-$C_6$-alkyoxy-$C_1$-$C_6$-alkyl, $C_3$-$C_6$-cycloalkyl-$C_1$-$C_6$-alkyl, phenyl($C_1$-$C_6$-alkyl)aminocarbonyl, ($C_1$-$C_6$-alkylamino)carbonyl, di($C_1$-$C_6$-alkyl)aminocarbonyl, ($C_1$-$C_6$-alkylamino)sulfonyl, di($C_1$-$C_6$-alkyl)aminosulfonyl or phenyl-$C_1$-$C_6$-alkyl: $C_1$-$C_4$-alkyl as mentioned above, and also, for example, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl, preferably methyl, ethyl, n-propyl, 1-methylethyl, n-butyl, 1,1-dimethylethyl, n-pentyl or n-hexyl;
- $C_2$-$C_6$-alkenyl and also the $C_2$-$C_6$-alkenyl moieties of ($C_1$-$C_6$-alkoxy)-$C_2$-$C_6$-alkenyl: a linear or branched ethylenically unsaturated hydrocarbon group having 2 to 6 carbon atoms and a C=C-double bond in any position, such as ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;
- $C_2$-$C_6$-alkynyl and also the $C_2$-$C_6$-alkynyl moieties of ($C_1$-$C_6$-alkoxy)-$C_2$-$C_6$-alkynyl: linear or branched unsaturated hydrocarbon group having 2 to 6 carbon atoms and containing at least one C-C-triple bond, such as ethynyl, 1-propynyl, 2-propynyl (propargyl), 1-butynyl, 2-butynyl, 3-butynyl, 1-methyl-2-propynyl;
- $C_1$-$C_4$-haloalkyl: a $C_1$-$C_4$-alkyl radical as mentioned above which is partially or fully substituted by fluorine, chlorine, bromine and/or iodine, for example, chloro-methyl, dichloromethyl, trichloromethyl, fluoromethyl, difluoromethyl, trifluoromethyl, chlorofluoromethyl, dichlorofluoromethyl, chlorodifluoromethyl, bromomethyl, iodomethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl, nonafluorobutyl, 1,1,2,2,-tetrafluoroethyl and 1-trifluoromethyl-1,2,2,2-tetrafluoroethyl;
- $C_1$-$C_6$-haloalkyl: $C_1$-$C_4$-haloalkyl as mentioned above, and also, for example, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl and dodecafluorohexyl;
- $C_3$-$C_6$-cycloalkyl: monocyclic saturated hydrocarbons having 3 to 6 ring members, such as cyclopropyl, cyclobutyl, cyclopentyl and cyclohexyl;
- $C_1$-$C_4$-alkoxy: for example methoxy, ethoxy, propoxy, 1-methylethoxy butoxy, 1-methylpropoxy, 2-methylpropoxy and 1,1-dimethylethoxy;
- $C_1$-$C_6$-alkoxy and also the $C_1$-$C_6$-alkoxy moieties of ($C_1$-$C_6$-alkoxy)carbonyl, ($C_1$-$C_6$-alkoxy)sulfonyl, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl: $C_1$-$C_4$-alkoxy as mentioned above, and also, for example, pentoxy, 1-methylbutoxy, 2-methylbutoxy, 3-methoxylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy;
- $C_1$-$C_4$-haloalkoxy: a $C_1$-$C_4$-alkoxy radical as mentioned above which is partially or fully substituted by fluorine, chlorine, bromine and/or iodine, for example, chloro-methoxy, dichloromethoxy, trichloromethoxy, fluoromethoxy, difluoromethoxy, trifluoromethoxy, chlorofluoromethoxy, dichlorofluoromethoxy, chlorodifluoromethoxy2-fluoroethoxy, 2-chloroethoxy, 2-bromoethxoy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 3,3,3-trifluoropropoxy, 3,3,3-trichloropropoxy, 2,2,3,3,3-pentafluoropropoxy, heptafluoropropoxy, 1-(fluoromethyl)-2-fluoroethoxy, 4-fluorobutoxy, nonafluorobutoxy, 1,1,2,2,-tetrafluoroethoxy and 1-trifluoromethyl-1,2,2,2-tetrafluoroethoxy;

- $C_1$-$C_6$-haloalkoxy: $C_1$-$C_4$-alkoxy as mentioned above: $C_1$-$C_4$-haloalkoxy as mentioned above, and also, for example, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl and dodecafluorohexyl;
- $C_2$-$C_6$-alkenyloxy: $C_2$-$C_6$-alkenyl as defined above, which is bound via an oxygen atom, such as ethenyloxy (vinyloxy), 1-propenyloxy, 2-propenyloxy (allyloxy), 1-butenyloxy, 2-butenyloxy, 3-butenyloxy 1-methyl-2-propenyloxy;
- $C_2$-$C_6$-alkynyloxy: $C_2$-$C_6$-alkynyl as defined above, which is bound via an oxygen atom, such as ethynyloxy, 1-propynyl, 2-propynyloxy (propargyloxy), 1-butynyloxy, 2-butynyloxy, 3-butynyloxy 1-methyl-2-propynyloxy;
- $C_1$-$C_4$-alkylthio: for example methylthio, ethylthio, propylthio, 1-methylethylthio, butylthio, 1-methylpropylthio, 2-methylpropylthio and 1,1-dimethylethylthio;
- $C_1$-$C_6$-alkylthio: $C_1$-$C_4$-alkylthio as mentioned above, and also, for example, pentylthio, 1-methylbutylthio, 2-methylbutylthio, 3-methylbutylthio, 2,2-dimethylpropylthio, 1-ethylpropylthio, hexylthio, 1,1-dimethylpropylthio, 1,2-dimethylpropylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio and 1- ethyl-2-methylpropylthio;
- $C_1$-$C_6$-alkylsulfinyl ($C_1$-$C_6$-alkyl-S(=O)-): z.B. methylsulfinyl, ethylsulfinyl, propylsulfinyl, 1-methylethylsulfinyl, butylsulfinyl, 1-methylpropylsulfinyl, 2-methylpropylsulfinyl, 1,1-dimethylethylsulfinyl, pentylsulfinyl, 1-methylbutylsulfinyl, 2-methylbutylsulfinyl, 3-methylbutylsulfinyl, 2,2-dimethylpropylsulfinyl, 1-ethylpropylsulfinyl, 1,1-dimethylpropylsulfinyl, 1,2-dimethylpropylsulfinyl, hexylsulfinyl, 1-methylpentylsulfinyl, 2-methylpentylsulfinyl, 3-methylpentylsulfinyl, 4-methylpentyl-sulfinyl, 1,1-dimethylbutylsulfinyl, 1,2-dimethylbutylsulfinyl, 1,3-dimethylbutyl-sulfinyl, 2,2-dimethylbutylsulfinyl, 2,3-dimethylbutylsulfinyl, 3,3-dimethylbutyl-sulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl and 1-ethyl-2-methylpropylsulfinyl;
- $C_1$-$C_6$-alkylsulfonyl ($C_1$-$C_6$-alkyl-$S(O)_2$-): for example methylsulfonyl, ethylsulfonyl, propylsulfonyl, 1-methylethylsulfonyl, butylsulfonyl, 1-methylpropylsulfonyl, 2-methylpropylsulfonyl, 1,1-dimethylethylsulfonyl, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethyl-propylsulfonyl, 1,2,2-trimethylpropylsulfonyl, 1-ethyl-1-methylpropylsulfonyl and 1-ethyl-2-methylpropylsulfonyl;
- ($C_1$-$C_4$-alkyl)amino and also the ($C_1$-$C_4$-alkylamino) moieties of ($C_1$-$C_4$-alkylamino)carbonyl or ($C_1$-$C_4$-alkylamino)sulfonyl: for example methylamino, ethylamino, propylamino, 1-methylethylamino, butylamino, 1-methylpropylamino, 2-methylpropylamino or 1,1-dimethylethylamino;
- ($C_1$-$C_6$-alkyl)amino and also the ($C_1$-$C_6$-alkylamino) moieties of ($C_1$-$C_6$-alkylamino)carbonyl, phenyl($C_1$-$C_6$-alkyl)aminocarbonyl or ($C_1$-$C_6$-alkylamino)sulfonyl: ($C_1$-$C_4$-alkyl)amino as mentioned above, and also, for example, pentylamino, 1-methylbutylamino, 2-methylbutylamino, 3-methylbutylamino, 2,2-dimethylpropylamino, 1-ethylpropylamino, hexylamino, 1,1-dimethylpropylamino, 1,2-dimethylpropylamino, 1-methylpentylamino, 2-methylpentylamino, 3-methylpentylamino, 4-methylpentylamino, 1,1-dimethylbutylamino, 1,2-dimethylbutylamino, 1,3-dimethylbutylamino, 2,2-dimethylbutylamino, 2,3-dimethylbutyl-amino 3,3-dimethylbutylamino, 1-ethylbutylamino, 2-ethylbutylamino, 1,1,2-trimethylpropylamino, 1,2,2-trimethyl-propylamino, 1-ethyl-1-methylpropylamino or 1-ethyl-2-methylpropylamino;
- di($C_1$-$C_4$-alkyl)amino and also the di($C_1$-$C_4$-alkylamino) moieties of di($C_1$-$C_4$-alkylamino)carbonyl or di($C_1$-$C_4$-alkylamino)sulfonyl: for example N,N-dimethylamino, N,N-diethylamino, N,N-di(1-methylethyl)amino, N,N-dipropylamino, N,N-dibutylamino, N,N-di(1-methylpropyl)amino, N,N-di(2-methylpropyl)amino, N,N-di(1,1-dimethylethyl)amino, N-ethyl-N-methylamino, N-methyl-N-propylamino, N-methyl-N-(1-methylethyl)amino, N-butyl-N-methylamino, N-methyl-N-(1-methylpropyl)amino, N-methyl-N-(2-methylpropyl)amino, N-(1,1-dimethylethyl)-N-methylamino, N-ethyl-N-propylamino, N-ethyl-N-(1-methylethyl)amino, N-butyl-N-ethylamino, N-ethyl-N-(1-methylpropyl)amino, N-ethyl-N-(2-methylpropyl)amino, N-ethyl-N-(1,1-dimethylethyl)amino, N-(1-methylethyl)-N-propylamino, N-butyl-N-propylamino, N-(1-methylpropyl)-N-propylamino, N-(2-methylpropyl)-N-propylamino, N-(1,1-dimethylethyl)-N-propylamino, N-butyl-N-(1-methylethyl)amino, N-(1-methylethyl)-N-(1-methylpropyl)amino, N-(1-methylethyl)-N-(2-methylpropyl)amino, N-(1,1-dimethylethyl)-N-(1-methylethyl)amino, N-butyl-N-(1-methylpropyl)amino, N-butyl-N-(2-methylpropyl)amino, N-butyl-N-(1,1-dimethylethyl)amino, N-(1-methylpropyl)-N-(2-methylpropyl)amino, N-(1,1-dimethylethyl)-N-(1-methylpropyl)amino or N-(1,1-dimethylethyl)-N-(2-methylpropyl)amino;

- di($C_1$-$C_6$-alkyl)amino and also the di($C_1$-$C_6$-alkylamino) moieties of di($C_1$-$C_6$-alkylamino)carbonyl or di($C_1$-$C_6$-alkylamino)sulfonyl: di($C_1$-$C_4$-alkyl)amino as mentioned above, and also, for example, N-methyl-N-pentylamino, N-methyl-N-(1-methylbutyl)amino, N-methyl-N-(2-methylbutyl)amino, N-methyl-N-(3-methylbutyl)amino, N-methyl-N-(2,2-dimethylpropyl)amino, N-methyl-N-(1-ethylpropyl)amino, N-methyl-N-hexylamino, N-methyl-N-(1,1-dimethylpropyl)amino, N-methyl-N-(1,2-dimethylpropyl)amino, N-methyl-N-(1-methylpentyl)amino, N-methyl-N-(2-methylpentyl)amino, N-methyl-N-(3-methylpentyl)amino, N-methyl-N-(4-methylpentyl)amino, N-methyl-N-(1,1-dimethylbutyl)amino, N-methyl-N-(1,2-dimethylbutyl)amino, N-methyl-N-(1,3-dimethylbutyl)amino, N-methyl-N-(2,2-dimethylbutyl)amino, N-methyl-N-(2,3-dimethylbutyl)amino, N-methyl-N-(3,3-dimethylbutyl)amino, N-methyl-N- (1-ethylbutyl)amino, N-methyl-N-(2-ethylbutyl)amino, N-methyl-N-(1,1,2-trimethylpropyl)amino, N-methyl-N- (1,2,2-trimethylpropyl)amino, N-methyl-N-(1-ethyl-1-methylpropyl)amino, N-methyl-N- (1-ethyl-2-methylpropyl)amino, N-ethyl-N-pentylamino, N-ethyl-N-(1-methylbutyl)amino, N-ethyl-N-(2-methylbutyl)amino, N-ethyl-N-(3-methylbutyl)amino, N-ethyl-N-(2,2-dimethylpropyl)amino, N-ethyl-N-(1-ethylpropyl)amino, N-ethyl-N-hexylamino, N-ethyl-N-(1,1-dimethylpropyl)amino, N-ethyl-N-(1,2-dimethylpropyl)amino, N-ethyl-N-(1-methylpentyl)amino, N-ethyl-N-(2-methylpentyl)amino, N-ethyl-N-(3-methylpentyl)amino, N-ethyl-N-(4-methylpentyl)amino, N-ethyl-N-(1,1-dimethylbutyl)amino, N-ethyl-N-(1,2-dimethylbutyl)amino, N-ethyl-N-(1,3-dimethylbutyl)amino, N-ethyl-N-(2,2-dimethylbutyl)amino, N-ethyl-N-(2,3-dimethylbutyl)amino, N-ethyl-N-(3,3-dimethylbutyl)amino, N-ethyl-N-(1-ethylbutyl)amino, N-ethyl-N-(2-ethylbutyl)amino, N-ethyl-N-(1,1,2-trimethylpropyl)amino, N-ethyl-N-(1,2,2-trimethylpropyl)amino, N-ethyl-N-(1-ethyl-1-methylpropyl)amino, N-ethyl-N-(1-ethyl-2-methylpropyl)amino, N-propyl-N-pentylamino, N-butyl-N-pentylamino, N,N-dipentylamino, N-propyl-N-hexylamino, N-butyl-N-hexylamino, N-pentyl-N-hexylamino or N,N-dihexylamino;
- $C_3$-$C_6$-cyclolalkyl and also the $C_3$-$C_6$-cyclolalkyl moieties of ($C_3$-$C_6$-cyclolalkyl)-carbonyl, ($C_3$-$C_6$-cyclolalkyl)-$C_1$-$C_6$-alkyl, ($C_3$-$C_6$-cycloalkyl)carbonyl and ($C_3$-$C_6$-cyclolalkyl)-$C_1$-$C_6$-alkoxy: a cycloaliphatic radical having 3 to 6 carbon atoms, such as cyclopropyl, cyclobutyl, cyclopentyl and cyclohexyl;
- $C_3$-$C_6$-cyclolalkoxy: a cycloaliphatic radical having 3 to 6 carbon atoms and bound via an oxygen atom, such as cyclopropyloxy, cyclobutyloxy, cyclopentyloxy and cyclohexyloxy;
- ($C_3$-$C_6$-cyclolalkyl)-$C_1$-$C_6$-alkyl: $C_1$-$C_6$-alkyl, in particular $C_1$-$C_4$-alkyl as defined above, such as methyl or ethyl, wherein 1 hydrogen atom is replaced by $C_3$-$C_6$-cyclolalkyl as defined above, examples including cyclopropylmethyl ($CH_2$-cyclopropyl), cyclobutylmethyl, cyclopentylmethyl, cycloexylmethyl, 1-cyclopropylethyl ($CH(CH_3)$-cyclopropyl), 1-cyclobutylethyl, 1-cyclopentylethyl, 1-cycloexylethyl, 2-cyclopropylethyl ($CH_2CH_2$-cyclopropyl), 2-cyclobutylethyl, 2-cyclopentylethyl or 2-cycloexylethyl;
- ($C_3$-$C_6$-cyclolalkyl)-$C_1$-$C_6$-alkoxy: $C_1$-$C_6$-alkoxy, in particular $C_1$-$C_4$-alkoxy as defined above, such as methoxy or ethoxy, wherein 1 hydrogen atom is replaced by $C_3$-$C_6$-cyclolalkyl as defined above, examples including cyclopropylmethoxy ($OCH_2$-cyclopropyl), cyclobutylmethoxy, cyclopentylmethoxy, cycloexylmethoxy, 1-cyclopropylethoxy (O-$CH(CH_3)$-cyclopropyl), 1-cyclobutylethoxy, 1-cyclopentylethoxy, 1-cycloexylethoxy, 2-cyclopropylethoxy ($OCH_2CH_2$)-cyclopropyl), 2-cyclobutylethoxy, 2-cyclopentylethoxy and 2-cycloexylethoxy;
- ($C_1$-$C_6$-alkoxy)-$C_1$-$C_6$-alkyl: $C_1$-$C_6$-alkyl, in particular $C_1$-$C_4$-alkyl as defined above, such as methyl, ethyl or isopropyl, wherein 1 hydrogen atom is replaced by $C_1$-$C_6$-alkoxy as defined above, examples including methoxymethyl, ethoxymethyl, n-propoxymethyl, butoxymethyl, 1-methoxyethyl, 1-ethoxyethyl, 1-(n-propoxy)ethyl, 1-butoxyethyl, 2-methoxyethyl, 2-ethoxyethyl, 2-(n-propoxy)ethyl, 2-butoxyethyl, 2-methoxypropyl, 2-ethoxypropyl, 2-(n-propoxy)propyl, 2-butoxypropyl;
- ($C_1$-$C_6$-alkoxy)-$C_1$-$C_6$-alkoxy: $C_1$-$C_6$-alkoxy, in particular $C_1$-$C_4$-alkoxy as defined above, such as methoxy or ethoxy, wherein 1 hydrogen atom is replaced by $C_1$-$C_6$-alkoxy as defined above, examples including methoxymethoxy, ethoxymethoxy, n-propoxymethoxy, butoxymethoxy, 2-methoxyethoxy, 2-ethoxyethoxy, 2-(n-propoxy)ethoxy and 2-butoxyethoxy;
- ($C_1$-$C_6$-alkoxy)-$C_2$-$C_6$-alkenyl: $C_2$-$C_6$-alkenyl, in particular $C_2$-$C_4$-alkenyl as defined above, such as ethenyl, propenyl, 1-butenyl or 2-butenyl, wherein 1 hydrogen atom is replaced by $C_1$-$C_6$-alkoxy as defined above;
- ($C_1$-$C_6$-alkoxy)-$C_2$-$C_6$-alkynyl: $C_2$-$C_6$-alkynyl, in particular $C_2$-$C_4$-alkynyl as defined above, such as ethynyl, propynyl or 2-butynyl, wherein 1 hydrogen atom is replaced by $C_1$-$C_6$-alkoxy as defined above;
- ($C_1$-$C_6$-alkyl)carbonyl: $C_1$-$C_6$-alkyl as mentioned above, which is bound to the remainder of the molecule by a carbonyl group;
- ($C_1$-$C_6$-alkoxy)carbonyl: $C_1$-$C_6$-alkyloxy as mentioned above, which is bound to the remainder of the molecule by a carbonyl group;
- ($C_1$-$C_6$-alkylamino)carbonyl: ($C_1$-$C_6$-alkyl)amino as mentioned above, which is bound to the remainder of the molecule by a carbonyl group;
- ($C_1$-$C_6$-alkylamino)sulfonyl: ($C_1$-$C_6$-alkyl)amino as mentioned above, which is bound to the remainder of the molecule by a sulfonyl group;
- di($C_1$-$C_6$-alkylamino)carbonyl: di($C_1$-$C_6$-alkyl)amino as mentioned above, which is bound to the remainder of the

molecule by a carbonyl group;

- di($C_1$-$C_6$-alkylamino)sulfonyl: di($C_1$-$C_6$-alkyl)amino as mentioned above, which is bound to the remainder of the molecule by a sulfonyl group;
- phenyl-$C_1$-$C_6$-alkyl: $C_1$-$C_6$-alkyl, in particular $C_1$-$C_4$-alkyl as defined above, such as methyl or ethyl, wherein 1 hydrogen atom is replaced by phenyl, examples including benzyl, 1-phenylethyl, 2-phenylethyl, 1-phenylpropyl, 2-phenylpropyl, 1-phenyl-1-methylethyl;
- three- to six-membered heterocyclyl: monocyclic saturated or partially unsaturated hydrocarbon having three to six ring members as mentioned above which, in addition to carbon atoms, contains one or two heteroatoms selected from O, S and N;
  for example
  saturated heterocycles such as 2-oxiranyl, 2-oxetanyl, 3-oxetanyl, 2-aziridinyl, 3-thietanyl, 1-azetidinyl, 2-azetidinyl, 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 2-piperidinyl, 3-piperidinyl, 4-piperidinyl, 1,3-dioxan-2-yl, 1,3-dioxan-4-yl, 1,3-dioxan-5-yl, 1,4-dioxan-2-yl, 1,3-dithian-2-yl, 1,3-dithian-4-yl, 1,4-dithian-2-yl, 1,3-dithian-5-yl, 2-tetrahydropyranyl, 3-tetrahydropyranyl, 4-tetrahydropyranyl, 2-tetrahydrothiopyranyl, 3-tetrahydrothiopyranyl, 4-tetrahydro-thiopyranyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, tetrahydro-1,3-oxazin-2-yl, tetrahydro-1,3-oxazin-6-yl, 2-morpholinyl, 3-morpholinyl or 4-morpholinyl, for example 2H-pyran-2-yl, 2H-pyran-3-yl, 2H-pyran-4-yl, 2H-pyran-5-yl, 2H-pyran-6-yl, 2H-thiopyran-2-yl, 2H-thiopyran-3-yl, 2H-thiopyran-4-yl, 2H-thiopyran-5-yl, 2H-thiopyran-6-yl;

  - partially unsaturated heterocycles such as 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 4,5-dihydropyrrol-2-yl, 4,5-dihydropyrrol-3-yl, 2,5-dihydropyrrol-2-yl, 2,5-dihydropyrrol-3-yl, 4,5-dihydroisoxazol-3-yl, 2,5-dihydroisoxazol-3-yl, 2,3-dihydroisoxazol-3-yl, 4,5-dihydroisoxazol-4-yl, 2,5-dihydroisoxazol-4-yl, 2,3-dihydroisoxazol-4-yl, 4,5-dihydroisoxazol-5-yl, 2,5-dihydroisoxazol-5-yl, 2,3-dihydroisoxazol-5-yl, 4,5-dihydroisothiazol-3-yl, 2,5-dihydroisothiazol-3-yl, 2,3-dihydroisothiazol-3-yl, 4,5-dihydroisothiazol-4-yl, 2,5-dihydroisothiazol-4-yl, 2,3-dihydroisothiazol-4-yl, 4,5-dihydroisothiazol-5-yl, 2,5-dihydroisothiazol-5-yl, 2,3-dihydroisothiazol-5-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-3-yl, 3,4-dihydropyrazol-4-yl, 3,4-dihydropyrazol-5-yl, 4,5-dihydropyrazol-3-yl, 4,5-dihydropyrazol-4-yl, 4,5-dihydropyrazol-5-yl, 2,3-dihydroimidazol-2-yl, 2,3-dihydroimidazol-3-yl, 2,3-dihydroimidazol-4-yl, 2,3-dihydroimidazol-5-yl, 4,5-dihydroimidazol-2-yl, 4,5-dihydroimidazol-4-yl, 4,5-dihydroimidazol-5-yl, 2,5-dihydroimidazol-2-yl, 2,5-dihydroimidazol-4-yl, 2,5-dihydroimidazol-5-yl, 2,3-dihydrooxazol-3-yl, 2,3-dihydrooxazol-4-yl, 2,3-dihydrooxazol-5-yl, 3,4-dihydrooxazol-3-yl, 3,4-dihydrooxazol-4-yl, 3,4-dihydrooxazol-5-yl, 2,3-dihydrothiazol-3-yl, 2,3-dihydrothiazol-4-yl, 2,3-dihydrothiazol-5-yl, 3,4-dihydrothiazol-3-yl, 3,4-dihydrothiazol-4-yl, 3,4-dihydrothiazol-5-yl, 3,4-dihydrothiazol-2-yl, 3,4-dihydrothiazol-3-yl, 3,4-dihydrothiazol-4-yl, 3,6-dihydro-2H-pyran-2-yl, 3,6-dihydro-2H-pyran-3-yl, 3,6-dihydro-2H-pyran-4-yl, 3,6-dihydro-2H-pyran-5-yl, 3,6-dihydro-2H-pyran-6-yl, 3,4-dihydro-2H-pyran-3-yl, 3,4-dihydro-2H-pyran-4-yl, 3,4-dihydro-2H-pyran-6-yl, 5,6-dihydro-4H-1,3-oxazin-2-yl;
  - 5- and 6-membered hetaryl which conatins 1, 2 or 3 heteroatoms selected from O, S and N:
  - 5-membered or 6-membered heteroaromatic radical, which besides carbon atoms contains 1, 2 or 3 heteroatoms as ring members, which are selected from O, S and N e.g. 1, 2 or 3 nitrogen atoms or 1 oxygen or sulfur atom and optionally 1 or 2 nitogen atoms:

in particular:

- five-membered monocyclic heteroaryl which contains one to three heteroatoms selected from O, S and N:
  for example 2-furyl, 3-furyl, 2-thienyl, 3-thienyl, 1-pyrrolyl, 2-pyrrolyl, 3-pyrrolyl, 1-pyrazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 1-imidazolyl, 2-imidazolyl, 4-imidazolyl, 1,3,4-triazol-1-yl, 1,3,4-triazol-2-yl;
- six-membered monocyclic heteroaryl contains one to three nitrogen atoms as ring memebers:
  for example 2-pyridinyl (2-pyridyl), 3-pyridinyl (3-pyridyl), 4-pyridinyl (4-pyridyl), 1-oxopyridin-2-yl, 1-oxopyridin-3-yl, 1-oxopyridin-4-yl,3-pyridazinyl, 4-pyridazinyl, 2-pyrimidinyl, 4-pyrimidinyl, 5-pyrimidinyl, 2-pyrazinyl and 1,2,3-triazinyl, 1,2,4-triazinyl and 1,3,5-triazinyl.

The preferred embodiments of the invention mentioned herein below have to be understood as being preferred either independently from each other or in combination with one another. Particular groups of embodiments of the

invention relate to those diaminotriazines of formula (I), wherein the variables $R^1$, $R^2$, $R^3$, $R^{3'}$, $R^4$, $R^a$, $R^b$, $R^c$, P, X and Y either independently of one another or in combination with one another, have the meanings listed below.

Preference is given to those diaminotriazine compounds of formula (I), wherein the variables, either independently of one another or in combination with one another, have the following meanings:

P is $NR^5$ or O;
X is $CR^c$ or N;
Y is $CR^d$ or N;
$R^A$ is halogen or CN; preferably halogen, more preferably fluorine;
$R^a$, $R^b$, $R^c$ and $R^d$ independently of one another are selected from hydrogen, halogen, OH, CN, amino, $NO_2$, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl, $C_1$-$C_6$-alkylthio,($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino,($C_1$-$C_6$-alkyl)-carbonyl, ($C_1$-$C_6$-alkoxy)-carbonyl, ($C_1$-$C_6$-alkyl)-carbonyloxy, $C_3$-$C_6$-cycloalkyl, $C_3$-$C_6$-cycloalkoxy, ($C_3$-$C_6$-cycloalkyl)-$C_1$-$C_4$-alkyl, ($C_3$-$C_6$-cycloalkyl)-$C_1$-$C_4$-alkoxy,
where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups,

preferably $R^a$, $R^b$, $R^c$ and $R^d$ independently of one another are selected from hydrogen, halogen, CN, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy,
where the aliphatic parts of the 6 aforementioned radicals are unsubstituted, partly or completely halogenated
more preferably $R^a$, $R^b$, $R^c$ and $R^d$ independently of one another are selected from hydrogen, halogen, CN, $C_1$-$C_4$-alkyl,$C_1$-$C_4$-haloalkyl, $C_2$-$C_4$-alkenyl, $C_2$-$C_4$-alkynyl, $C_1$-$C_4$-alkoxy, $C_1$-$C_4$-haloalkoxy, $C_2$-$C_4$-alkenyloxy, $C_2$-$C_4$-alkynyloxy;
most preferably $R^a$, $R^b$, $R^c$ and $R^d$ independently of one another are selected from hydrogen, halogen, CN or $C_1$-$C_4$-alkyl;
especially preferably $R^a$, $R^b$, $R^c$ and $R^d$ independently of one another are selected from hydrogen or halogen, in particular fluorine

or
$R^a$, $R^b$ and $R^d$ are as defined above and $R^c$ is -Q-Ar, wherein
Q is a chemical bond, O, $S(O)_m$, $CR^{q1}R^{q2}$, $NR^{q3}$, C(O), C(O)O, $CR^{q1}R^{q2}$-O, $S(O)_mNR^{q3}$ or $C(O)NR^{q3}$, wherein

m is 0, 1 or 2;
$R^{q1}$, $R^{q2}$ independently of one another are selected from hydrogen, halogen or $C_1$-$C_4$-alkyl;
$R^{q3}$ is H, CN, $C_1$-$C_6$-alkyl, ($C_1$-$C_6$-alkoxy)-$C_1$-$C_6$-alkyl, ($C_1$-$C_6$-alkyl)-carbonyl, ($C_1$-$C_6$-alkoxy)carbonyl, ($C_1$-$C_6$-alkyl)sulfonyl, where the aliphatic parts of the 6 aforementioned radicals are unsubstituted, partly or completely halogenated;
preferably Q is a chemical bond, O, $CR^{q1}R^{q2}$, S, S(O) or $S(O)_2$,
wherein
$R^{q1}$ and $R^{q2}$ independently of one another are selected from hydrogen, F or Cl or $C_1$-$C_4$-alkyl; preferably selected from hydrogen or $C_1$-$C_4$-alkyl,
more preferably Q is a chemical bond, O, $CH_2$, S, S(O) or $S(O)_2$;
most preferably Q is a chemical bond, O or $CH_2$;

Ar is phenyl, which is unsubstituted or carries 1, 2, 3, 4 or 5 radicals $R^e$ that are identical or different and independently of one another are selected from halogen, OH, CN, amino, $NO_2$, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl, $C_1$-$C_6$-alkylthio,($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino,($C_1$-$C_6$-alkyl)-carbonyl, ($C_1$-$C_6$-alkoxy)-carbonyl, ($C_1$-$C_6$-alkyl)-carbonyloxy, $C_3$-$C_6$-cycloalkyl, $C_3$-$C_6$-cycloalkoxy, ($C_3$-$C_6$-cycloalkyl)-$C_1$-$C_4$-alkyl, ($C_3$-$C_6$-cycloalkyl)-$C_1$-$C_4$-alkoxy,

where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups;
preferably $R^e$ are identical or different and independently of one another are selected from halogen, CN,

$C_1$-$C_6$-alkyl, $C_1$-$C_6$-haloalkyl, $C_1$-$C_6$-alkoxy, $C_1$-$C_6$-haloalkoxy;
more preferably $R^e$ are identical or different and independently of one another are selected from halogen, CN, $C_1$-$C_4$-alkyl,$C_1$-$C_4$-haloalkyl, $C_1$-$C_4$-alkoxy, $C_1$-$C_4$-haloalkoxy;
most preferably $R^e$ are identical or different and independently of one another are selected from halogen, CN and $C_1$-$C_4$-alkyl;
especially preferably $R^e$ are identical or different and independently of one another are selected from F, Cl, CN and $CH_3$;

preferably Ar is phenyl, which carries 1, 2 or 3 radicals $R^e$ as defined above;
more preferably Ar is phenyl, which carries 1 or 2 radicals $R^e$ as defined above;
most preferably Ar is phenyl, which carries 1 radical $R^e$ as defined above;
especially preferably Ar is phenyl, which carries 2 radicals $R^e$ as defined above;

Particularly preferred examples of the moiety Ar-Q are selected from phenyl, 4-methoxyphenyl, 4-cyanophenyl, 3-methylphenyl, 3,5-dimethylphenyl, 3,5-difluorophenyl, 3,5-dichloropheynl, 3,5-dibromopheynl, 3,5-dimethoxyphenyl, 3,5-diethoxyphenyl, 3,5-Bis-(trifluoromethoxy)phenyl, 3,5-Bis-(trifluoromethyl)phenyl, 3,5-dicyanophenyl, 3-fluoro-5-methylphenyl, 3-chloro-5-methylphenyl, 3-bromo-5-methylphenyl, 3-methoxy-5-methylphenyl, 3-ethoxy-5-methylphenyl, 3-(trifluoromethoxy)-5-methylphenyl, 3-(trifluoromethyl)-5-methylphenyl, 3-(*tert*-butyl)-5-methylphenyl, 3-cyano-5-methylphenyl, 3-chloro-5-fluorophenyl, 3-bromo-5-fluorophenyl, 3-fluoro-5-methoxyphenyl, 3-fluoro-5-ethoxyphenyl, 3-fluoro-5-(trifluoromethoxy)phenyl, 3-fluoro-5-(trifluoromethyl)phenyl, 3-fluoro-5-(*tert*-butyl)phenyl, 3-fluoro-5-cyanophenyl, 3-chloro-5-methoxyphenyl, 3-chloro-5-ethoxyphenyl, 3-chloro-5-(trifluoromethoxy)phenyl, 3-chloro-5-(trifluoromethyl)phenyl, 3-chloro-5-(*tert*-butyl)phenyl, 3-chloro-5-cyanophenyl, 3-bromo-5-methoxyphenyl, 3-bromo-5-ethoxyphenyl, 3-bromo-5-(trifluoromethoxy)phenyl, 3-bromo-5-(trifluoromethyl)phenyl, 3-bromo-5-(*tert*-butyl)phenyl, 3-bromo-5-cyanophenyl, 3-ethoxy-5-methoxyphenyl, 3-(trifluoromethoxy)-5-methoxyphenyl, 3-methoxy-5-(trifluoromethyl)phenyl, 5-cyano-3-methoxyphenyl, 5-(*tert*-butyl)-3-methoxyphenyl, 3-(trifluoromethoxy)-5-ethoxyphenyl, 3-ethoxy-5-(trifluoromethyl)phenyl, 5-cyano-3-ethoxyphenyl, 5-(*tert*-butyl)-3-ethoxyphenyl, 3-(trifluoromethoxy)-5-(trifluoromethyl)phenyl, 3-(trifluoromethoxy)-5-(*tert*-butyl)phenyl, 3-(trifluoromethoxy)-5-cyanophenyl, 3-(trifluoromethyl)-5-(*tert*-butyl)phenyl, 3-(trifluoromethyl)-5-cyanophenyl, 3-cyano-5-(*tert*-butyl)phenyl and 3,5-bis(*tert*-butyl)phenyl;

or
$R^b$ and $R^d$ are as defined above and $R^a$ and $R^c$ together with the carbon atoms to which they are attached are selected form a saturated or unsaturated, 5- or 6-membered carbocycle or saturated or unsaturated, 5- or 6-membered heterocycle having 1 or 2 heteroatoms or heteroatom moieties, selected from O, S, S(O), $S(O)_2$, N or $NR^z$ as ring members, preferably from O, S, or $NR^z$ as ring members

where the carbocycle and the heterocycle are unsubstituted or carry 1, 2, 3, 4, 5, 6, 7 or 8 radicals $R^f$ that are identical or different and independently of one another are selected from halogen, OH, CN, amino, $NO_2$, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl, $C_1$-$C_6$-alkylthio,($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino,($C_1$-$C_6$-alkyl)-carbonyl, ($C_1$-$C_6$-alkoxy)-carbonyl, ($C_1$-$C_6$-alkyl)-carbonyloxy, $C_3$-$C_6$-cycloalkyl, $C_3$-$C_6$-cycloalkoxy, ($C_3$-$C_6$-cycloalkyl)-$C_1$-$C_4$-alkyl,($C_3$-$C_6$-cycloalkyl)-$C_1$-$C_4$-alkoxy,
where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups;
preferably $R^f$ are identical or different and independently of one another are selected from halogen, $C_1$-$C_6$-alkyl, $C_1$-$C_6$-haloalkyl, $C_1$-$C_6$-alkoxy, $C_1$-$C_6$-haloalkoxy;
more preferably $R^f$ are identical or different and independently of one another are selected from halogen, $C_1$-$C_4$-alkyl,$C_1$-$C_4$-haloalkyl, $C_1$-$C_4$-alkoxy, $C_1$-$C_4$-haloalkoxy;
most preferably $R^f$ are identical or different and independently of one another are selected from halogen, $C_1$-$C_4$-alkyl and $C_1$-$C_4$-haloalkyl;
especially preferably $R^f$ are identical or different and independently of one another are selected from F, Cl, $CH_3$ and $C_2H_5$.

preferably $R^a$ and $R^c$ together with the carbon atoms to which they are attached are selected form a saturated or unsaturated, 5- or 6-membered carbocycle or a saturated or unsaturated, 5- or 6-membered heterocycle

having 1 or 2 heteroatoms or heteroatom moieties, selected from O, S, S(O), $S(O)_2$, N or $NR^z$ as ring members, preferably from O, S, or $NR^z$ as ring members
where the carbocycle or heterocycle are unsubstituted or carry 1, 2, 3 or 4 radicals $R^f$ as defined above;
more preferably $R^a$ and $R^c$ together with the carbon atoms to which they are attached are selected from a saturated 5- or 6-membered carbocycle or a saturated 5- or 6-membered heterocycle having 1 or 2 heteroatoms or heteroatom moieties, selected from O, S, S(O), $S(O)_2$, N or $NR^z$ as ring members, preferably from O, S, or $NR^z$ as ring members
where the carbocycle or heterocycle is unsubstituted or carry 1, 2, 3 or 4 radicals $R^f$ as defined above
most preferably $R^a$ and $R^c$ together with the carbon atoms to which they are attached are selected form a saturated 5- or 6-membered carbocycle or a saturated 5-membered heterocycle having 1 or 2 heteroatoms or heteroatom moieties, selected from O, S, or $NR^z$ as ring members
where the carbocycle or heterocycle is unsubstituted or carry 1, 2, 3 or 4 radicals $R^f$ as defined above
especially preferably $R^a$ and $R^c$ together with the carbon atoms to which they are attached are selected form a saturated 5- or 6-membered carbocycle or a saturated 5-membered heterocycle having 1 or 2 heteroatoms or heteroatom moieties, selected from O or S as ring members
where the carbocycle or heterocycle is unsubstituted or carry 1, 2, 3 or 4 radicals $R^f$ as defined above

$R^z$ is selected from H, OH, $S(O)_2NH_2$, CN, $C_1$-$C_6$-alkyl, $C_2$-$C_6$-alkenyl, $C_2$-$C_6$-alkynyl, ($C_3$-$C_6$-cycloalkyl)-$C_1$-$C_4$-alkyl, $C_1$-$C_6$-alkoxy, ($C_1$-$C_6$-alkoxy)-$C_1$-$C_6$-alkyl, ($C_1$-$C_6$-alkyl)-carbonyl, ($C_1$-$C_6$-alkoxy)carbonyl, ($C_1$-$C_6$-alkyl)sulfonyl, ($C_1$-$C_6$-alkylamino)carbonyl, di($C_1$-$C_6$-alkyl)aminocarbonyl, ($C_1$-$C_6$-alkylamino)sulfonyl, di($C_1$-$C_6$-alkyl)aminosulfonyl and ($C_1$-$C_6$-alkoxy)sulfonyl,
where the aliphatic and cycloaliphatic parts of the 14 aforementioned radicals are unsubstituted, partly or completely halogenated,

preferably $R^z$ is selected from H, $S(O)_2NH_2$, CN, $C_1$-$C_6$-alkyl, $C_1$-$C_6$-haloalkyl, $C_2$-$C_6$-alkenyl, $C_2$-$C_6$-alkynyl;
more preferably $R^z$ is selected from H, $S(O)_2NH_2$, CN, $C_1$-$C_4$-alkyl,$C_1$-$C_4$-haloalkyl, $C_2$-$C_4$-alkenyl, $C_2$-$C_4$-alkynyl;
most preferably $R^z$ is selected from H, $S(O)_2NH_2$, CN, $C_1$-$C_4$-alkyl,$C_1$-$C_4$-haloalkyl; especially preferably $R^z$ is selected from H, $S(O)_2NH_2$, CN, $C_1$-$C_4$-alkyl;

$R^1$ is selected from H, halogen, OH, CN, $C_1$-$C_6$-alkyl, ($C_1$-$C_6$-alkoxy)-$C_1$-$C_6$-alkyl, $C_3$-$C_6$-cycloalkyl, ($C_3$-$C_6$-cycloalkyl)-$C_1$-$C_4$-alkyl, $C_1$-$C_6$-alkoxy, $C_2$-$C_6$-alkenyloxy, $C_2$-$C_6$-alkynyloxy, $C_3$-$C_6$-cycloalkoxy, ($C_3$-$C_6$-cycloalkyl)-$C_1$-$C_4$-alkoxy,
where the aliphatic and cycloaliphatic parts of the 9 aforementioned radicals are unsubstituted, partly or completely halogenated;

preferably $R^1$ is selected from H, halogen, CN, $C_1$-$C_6$-alkyl, $C_1$-$C_6$-haloalkyl, $C_1$-$C_6$-alkoxy, and $C_1$-$C_6$-haloalkoxy;
more preferably $R^1$ is selected from H, halogen, CN, $C_1$-$C_4$-alkyl,$C_1$-$C_4$-haloalkyl, $C_1$-$C_4$-alkoxy, and $C_1$-$C_4$-haloalkoxy;
most preferably $R^1$ is selected from H, halogen, $C_1$-$C_4$-alkyl,$C_1$-$C_4$-haloalkyl, $C_1$-$C_4$-alkoxy, and $C_1$-$C_4$-haloalkoxy;
especially preferably $R^1$ is selected from H, F, $C_1$-$C_4$-alkyl,$C_1$-$C_4$-haloalkyl, $C_1$-$C_4$-alkoxy and $C_1$-$C_4$-haloalkoxy;

$R^2$ is selected from H, halogen, CN, $C_1$-$C_6$-alkyl, $C_1$-$C_6$-haloalkyl, $C_1$-$C_6$-alkoxy and $C_1$-$C_6$-haloalkoxy;

preferably $R^2$ is selected from H, halogen, CN, $C_1$-$C_4$-alkyl,$C_1$-$C_4$-haloalkyl, $C_1$-$C_4$-alkoxy, and $C_1$-$C_4$-haloalkoxy;
more preferably $R^2$ is selected from H, halogen and $C_1$-$C_4$-alkyl;
most preferably $R^2$ is selected from H, F, Cl, methyl and ethyl;

$R^3$ and $R^{3'}$ are independently of one another selected from H, $C_1$-$C_6$-alkyl, $C_3$-$C_6$-cycloalkyl, ($C_3$-$C_6$-cycloalkyl)-$C_1$-$C_4$-alkyl,
where the aliphatic and cycloaliphatic parts of the 3 aforementioned radicals are unsubstituted, partly or completely halogenated;

preferably $R^3$ and $R^{3'}$ are selected from H, $C_1$-$C_6$-alkyl, $C_1$-$C_6$-haloalkyl, $C_3$-$C_6$-cycloalkyl, $C_3$-$C_6$-halocycloalkyl, ($C_3$-$C_6$-cycloalkyl)-$C_1$-$C_4$-alkyl;

more preferably $R^3$ and $R^{3'}$ are selected from H, $C_1$-$C_4$-alkyl,$C_1$-$C_4$-haloalkyl, $C_3$-$C_6$-cycloalkyl, ($C_3$-$C_6$-cycloalkyl)-$C_1$-$C_4$-alkyl;

most preferably $R^3$ and $R^{3'}$ are selected from H, $C_1$-$C_4$-alkyl,$C_1$-$C_4$-haloalkyl;

or

$R^3$ and $R^{3'}$ together with the carbon atom to which they are attached form a moiety selected from $C_3$-$C_6$-cycloalkyl or $C_3$-$C_6$-cycloalkenyl where the carbocycle is unsubstituted, partly or completely halogenated or carry from 1 to 6 $C_1$-$C_6$-alkyl groups;

preferably $C_3$-$C_6$-cycloalkan-1,1-diyl, ipso-$C_3$-$C_6$-cycloalkendiyl, where the carbocycle are unsubstituted, partly or completely halogenated or carry from 1 to 6 $C_1$-$C_6$-alkyl groups.

$R^1$ and $R^2$ together with the carbon atom to which they are attached form a moiety selected from carbonyl, $C_3$-$C_6$-cycloalkyl, $C_3$-$C_6$-cycloalkenyl, three- to six-membered saturated or partially unsaturated heterocyclyl, where the carbocycle and the heterocycle are unsubstituted, partly or completely halogenated or carry from 1 to 6 $C_1$-$C_6$-alkyl groups;

preferably carbonyl, $C_3$-$C_6$-cycloalkan-1,1-diyl, ipso-$C_3$-$C_6$-cycloalkendiyl, three- to six-membered saturated or partially unsaturated ipso-heterocyclodiyl, where the carbocycle and the heterocycle are unsubstituted, partly or completely halogenated or carry from 1 to 6 $C_1$-$C_6$-alkyl groups;

more preferably $C_3$-$C_6$-cycloalkan-1,1-diyl, ipso-$C_3$-$C_6$-cycloalkendiyl, three- to six-membered saturated or partially unsaturated ipso-heterocyclodiyl, where the carbocycle and the heterocycle are unsubstituted, partly or completely halogenated or carry from 1 to 6 $C_1$-$C_6$-alkyl groups and where the heterocycle preferably has 1 or 2 oxygen atoms as ring members;

most preferably $C_3$-$C_6$-cycloalkan-1,1-diyl or three- to six-membered saturated ipso-heterocyclodiyl, where the carbocycle and the heterocycle are unsubstituted, partly or completely halogenated or carry from 1 to 6 $C_1$-$C_6$-alkyl groups, and where heterocyclyl preferably has 1 or 2 oxygen atoms as ring members;

or

$R^2$ and $R^3$ together with the carbon atom to which they are attached form a moiety selected from $C_3$-$C_6$-cycloalkyl, $C_3$-$C_6$-cycloalkenyl, three- to six-membered saturated or partially unsaturated heterocyclyl, where the carbocycle and the heterocycle are unsubstituted, partly or completely halogenated or carry from 1 to 6 $C_1$-$C_6$-alkyl groups;

preferably $C_3$-$C_6$-cycloalkan-1,1-diyl, ipso-$C_3$-$C_6$-cycloalkendiyl, three- to six-membered saturated or partially unsaturated ipso-heterocyclodiyl, where the carbocycle and the heterocycle are unsubstituted, partly or completely halogenated or carry from 1 to 6 $C_1$-$C_6$-alkyl groups;

more preferably $C_3$-$C_6$-cycloalkan-1,1-diyl, ipso-$C_3$-$C_6$-cycloalkendiyl, three- to six-membered saturated or partially unsaturated ipso-heterocyclodiyl, where the carbocycle and the heterocycle are unsubstituted, partly or completely halogenated or carry from 1 to 6 $C_1$-$C_6$-alkyl groups and where the heterocycle preferably has 1 or 2 oxygen atoms as ring members;

most preferably $C_3$-$C_6$-cycloalkan-1,1-diyl or three- to six-membered saturated ipso-heterocyclodiyl, where the carbocycle and the heterocycle are unsubstituted, partly or completely halogenated or carry from 1 to 6 $C_1$-$C_6$-alkyl groups, and where heterocyclyl preferably has 1 or 2 oxygen atoms as ring members;

$R^4$ is selected from H, OH, $S(O)_2NH_2$, CN, $C_1$-$C_6$-alkyl, $C_2$-$C_6$-alkenyl, $C_2$-$C_6$-alkynyl, ($C_3$-$C_6$-cycloalkyl)-$C_1$-$C_4$-alkyl, $C_3$-$C_6$-cycloalkyl)-carbonyl, $C_1$-$C_6$-alkoxy, ($C_1$-$C_6$-alkoxy)-$C_1$-$C_6$-alkyl, ($C_1$-$C_6$-alkyl)carbonyl, ($C_1$-$C_6$-alkoxy)carbonyl, ($C_1$-$C_6$-alkyl)sulfonyl, ($C_1$-$C_6$-alkylamino)carbonyl, di($C_1$-$C_6$-alkyl)aminocarbonyl, ($C_1$-$C_6$-alkylamino)sulfonyl, di($C_1$-$C_6$-alkyl)aminosulfonyl and ($C_1$-$C_6$-alkoxy)sulfonyl,

where the aliphatic and cycloaliphatic parts of the 15 aforementioned radicals are unsubstituted, partly or completely halogenated,

phenyl, phenylsulfonyl, phenylaminosulfonyl, phenylaminocarbonyl, phenyl($C_1$-$C_6$-alkyl)aminocarbonyl, phenyl-$C_1$-$C_6$ alkyl, phenylcarbonyl and phenoxycarbonyl,

where phenyl in the last 8 mentioned radicals is unsubstituted or substituted by 1, 2, 3, 4 or 5 identical or different substituents selected from halogen, CN, $NO_2$, $C_1$-$C_6$-alkyl, $C_1$-$C_6$-haloalkyl, $C_1$-$C_6$-alkoxy and $C_1$-$C_6$-haloalkoxy

preferably $R^4$ is selected from H, CN, $C_1$-$C_6$-alkyl, $C_1$-$C_6$-alkoxy, ($C_1$-$C_6$-alkoxy)-$C_1$-$C_6$-alkyl, ($C_1$-$C_6$-alkyl)carbonyl, ($C_3$-$C_6$-cycloalkyl)-carbonyl or ($C_1$-$C_6$-alkyl)sulfonyl,

where the aliphatic parts of the 6 aforementioned radicals unsubstituted partly or completely halogenated, phenyl and phenyl-$C_1$-$C_6$ alkyl,

where phenyl in the last 2 mentioned radicals is unsubstituted or substituted by 1, 2, 3, 4, or 5 identical or different substituents selected from the group consisting of halogen, CN, $NO_2$, $C_1$-$C_6$-alkyl, $C_1$-$C_6$-haloalkyl, $C_1$-$C_6$-alkoxy and $C_1$-$C_6$-haloalkoxy;

more preferably $R^4$ is selected from H, CN, $C_1$-$C_6$-alkyl, $C_1$-$C_6$-haloalkyl, $C_1$-$C_6$-alkoxy, ($C_1$-$C_6$-alkoxy)-$C_1$-$C_6$-alkyl, ($C_1$-$C_6$-alkyl)carbonyl, ($C_3$-$C_6$-cycloalkyl)-carbonyl, ($C_1$-$C_6$-haloalkyl)carbonyl, ($C_1$-$C_6$-alkyl)sulfonyl or ($C_1$-$C_6$-haloalkyl)sulfonyl;
most preferably $R^4$ is selected from H, CN, $C_1$-$C_4$-alkyl,$C_1$-$C_4$-alkoxy, ($C_1$-$C_4$-alkoxy)-$C_1$-$C_4$-alkyl, ($C_1$-$C_4$-alkyl)carbonyl, ($C_3$-$C_6$-cycloalkyl)-carbonyl, or ($C_1$-$C_4$-alkyl)sulfonyl; in particular H, CN, $CH_3$, $CH_2OCH_3$, $OCH_3$, $C(O)CH_3$, C(O)cyclopropyl or $SO_2CH_3$;
especially preferably $R^4$ is hydrogen;

$R^5$ is selected from H, OH, $S(O)_2NH_2$, CN, $C_1$-$C_6$-alkyl, $C_2$-$C_6$-alkenyl, $C_2$-$C_6$-alkynyl, ($C_3$-$C_6$-cycloalkyl)-$C_1$-$C_4$-alkyl, $C_3$-$C_6$-cycloalkyl)-carbonyl, $C_1$-$C_6$-alkoxy, ($C_1$-$C_6$-alkoxy)-$C_1$-$C_6$-alkyl, ($C_1$-$C_6$-alkyl)carbonyl, ($C_1$-$C_6$-alkoxy)carbonyl, ($C_1$-$C_6$-alkyl)sulfonyl, ($C_1$-$C_6$-alkylamino)carbonyl, di($C_1$-$C_6$-alkyl)aminocarbonyl, ($C_1$-$C_6$-alkylamino)sulfonyl, di($C_1$-$C_6$-alkyl)aminosulfonyl and ($C_1$-$C_6$-alkoxy)sulfonyl,
where the aliphatic and cycloaliphatic parts of the 15 aforementioned radicals are unsubstituted, partly or completely halogenated,

phenyl, phenylsulfonyl, phenylaminosulfonyl, phenylaminocarbonyl, phenyl($C_1$-$C_6$-alkyl)aminocarbonyl, phenyl-$C_1$-$C_6$ alkyl, phenylcarbonyl and phenoxycarbonyl,
where phenyl in the last 8 mentioned radicals is unsubstituted or substituted by 1, 2, 3, 4 or 5 identical or different substituents selected from halogen, CN, $NO_2$, $C_1$-$C_6$-alkyl, $C_1$-$C_6$-haloalkyl, $C_1$-$C_6$-alkoxy and $C_1$-$C_6$-haloalkoxy
preferably $R^5$ is selected from H, CN, $C_1$-$C_6$-alkyl, $C_1$-$C_6$-alkoxy, ($C_1$-$C_6$-alkoxy)-$C_1$-$C_6$-alkyl, ($C_1$-$C_6$-alkyl)carbonyl, ($C_3$-$C_6$-cycloalkyl)-carbonyl or ($C_1$-$C_6$-alkyl)sulfonyl,
where the aliphatic parts of the 6 aforementioned radicals unsubstituted partly or completely halogenated,
phenyl and phenyl-$C_1$-$C_6$ alkyl,
where phenyl in the last 2 mentioned radicals is unsubstituted or substituted by 1, 2, 3, 4, or 5 identical or different substituents selected from the group consisting of halogen, CN, $NO_2$, $C_1$-$C_6$-alkyl, $C_1$-$C_6$-haloalkyl, $C_1$-$C_6$-alkoxy and $C_1$-$C_6$-haloalkoxy;

more preferably $R^5$ is selected from H, CN, $C_1$-$C_6$-alkyl, $C_1$-$C_6$-haloalkyl, $C_1$-$C_6$-alkoxy, ($C_1$-$C_6$-alkoxy)-$C_1$-$C_6$-alkyl, ($C_1$-$C_6$-alkyl)carbonyl, ($C_3$-$C_6$-cycloalkyl)-carbonyl, ($C_1$-$C_6$-haloalkyl)carbonyl, ($C_1$-$C_6$-alkyl)sulfonyl or ($C_1$-$C_6$-haloalkyl)sulfonyl;
most preferably $R^5$ is selected from H, CN, $C_1$-$C_4$-alkyl,$C_1$-$C_4$-alkoxy, ($C_1$-$C_4$-alkoxy)-$C_1$-$C_4$-alkyl, ($C_1$-$C_4$-alkyl)carbonyl, ($C_3$-$C_6$-cycloalkyl)-carbonyl, or ($C_1$-$C_4$-alkyl)sulfonyl; in particular H, CN, $CH_3$, $CH_2OCH_3$, $OCH_3$, $C(O)CH_3$, C(O)cyclopropyl or $SO_2CH_3$;
especially preferably $R^5$ is hydrogen;

Particular groups of embodiments relate to the diaminotriazine compounds of formula (I), wherein P is $NR^5$. They are represented by the general formula (I.N)

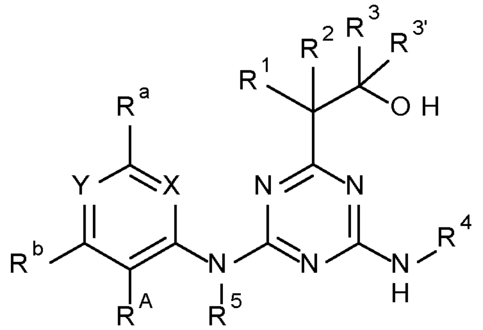

(I.N)

,

wherein $R^1$, $R^2$, $R^3$, $R^{3'}$, $R^4$, $R^5$, $R^A$, $R^a$, $R^b$, $R^c$, X and Y are as described above.
Further particular groups of embodiments relate to the diaminotriazine compounds of formula (I), wherein P is O. The are that are represented by the general formula (I.O)

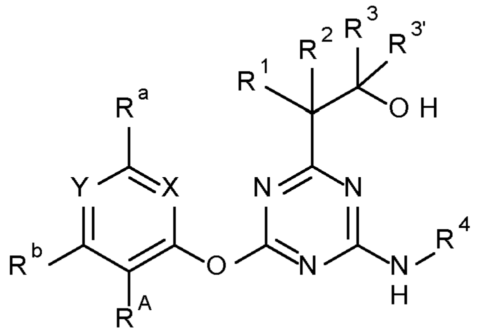

(I.O)

wherein $R^1$, $R^2$, $R^3$, $R^{3'}$, $R^4$, $R^A$, $R^a$, $R^b$, $R^c$, X and Y are as described above.

Particular groups of embodiments relate to the diaminotriazine compounds of formula (I), wherein P is $NR^5$ or O, X is $CR^c$ and Y is $CR^d$.

Further particular groups of embodiments relate to the diaminotriazine compounds of formula (I), wherein P is $NR^5$ or O, X is $CR^c$ and Y is N.

Further particular groups of embodiments relate to the diaminotriazine compounds of formula (I), wherein P is $NR^5$ or O, X is N and Y is $CR^d$.

Further particular groups of embodiments relate to the diaminotriazine compounds of formula (I), wherein P is $NR^5$ or O, X is N and Y is N.

In particularly preferred groups of embodiments the moiety (A) of diaminotriazine compounds of formula (I)

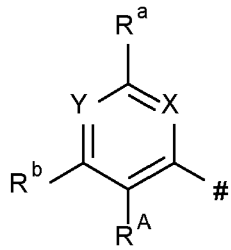

(A)

is represented by a moiety selected from (A.1), (A.2) and (A.3)

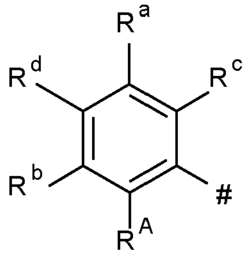

(A.1)

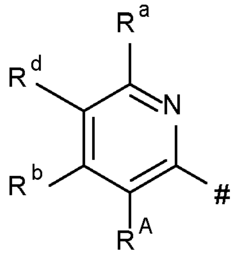

(A.2)

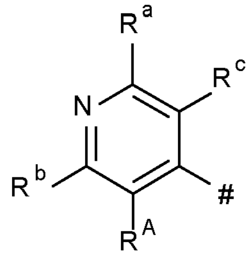

(A.3)

wherein

$R^A$ is halogen or CN, preferably F, Cl or CN;

$R^c$ is halogen, OH, CN, amino, $NO_2$, $C_1$-$C_6$-alkyl, $C_1$-$C_6$-haloalkyl, $C_1$-$C_6$-alkoxy, $C_1$-$C_6$-alkylthio, ($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino, ($C_1$-$C_6$-alkyl)carbonyl, ($C_1$-$C_6$-alkoxy)carbonyl; and

$R^a$, $R^b$ and $R^d$ independently of one another are hydrogen, halogen, OH, CN, amino, $NO_2$, $C_1$-$C_6$-alkyl, $C_1$-$C_6$-haloalkyl, $C_1$-$C_6$-alkoxy, $C_1$-$C_6$-alkylthio, ($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino,($C_1$-$C_6$-alkyl)carbonyl, ($C_1$-$C_6$-alkoxy)carbonyl;

preferably

$R^A$ is halogen or CN, preferably F, Cl or CN;

$R^c$ is halogen, CN, $C_1$-$C_6$-alkyl or $C_1$-$C_6$-alkoxy; and
$R^a$, $R^b$ and $R^d$ independently of one another are hydrogen, halogen, CN, $NO_2$, $C_1$-$C_6$-alkyl, $C_1$-$C_6$-haloalkyl or $C_1$-$C_6$-alkoxy;

more preferably

$R^A$ is halogen or CN, preferably F, Cl or CN;
$R^c$ is halogen or CN; and
$R^a$, $R^b$ and $R^d$ independently of one another are hydrogen, halogen, CN, $C_1$-$C_6$-alkyl or $C_1$-$C_6$-alkoxy;

most preferably

$R^A$ is halogen, preferably F or Cl, more preferably F;
$R^c$ is halogen; and
$R^a$, $R^b$ and $R^d$ independently of one another are hydrogen, halogen or CN;

especially preferably

$R^A$ is halogen, preferably F or Cl, more preferably F;
$R^c$ is halogen; and
$R^a$, $R^b$ and $R^d$ are hydrogen;

also especially preferably

$R^A$ is halogen, preferably F or Cl, more preferably F;
$R^a$, $R^b$ and $R^c$ are halogen; and
$R^d$ hydrogen;

also especially preferably
$R^A$, $R^a$, $R^b$, $R^c$ and $R^d$ are halogen, preferably F or Cl, more preferably F.

In especially preferred groups of embodiments the moiety (A) of diaminotriazine compounds of formula (I) is represented by the moiety (A1), wherein the variables $R^A$, $R^a$, $R^b$, $R^c$ and $R^d$ are as defined above.

In further especially preferred groups of embodiments the moiety (A) of diaminotriazine compounds of formula (I) is represented by the moiety (A2), wherein the variables $R^A$, $R^a$, $R^b$ and $R^d$ are as defined above.

In further especially preferred groups of embodiments the moiety (A) of diaminotriazine compounds of formula (I) is represented by the moiety (A3), wherein the variables $R^A$, $R^a$, $R^b$ and $R^c$ are as defined above.

Preferred are diaminotriazine compounds of formula (I), wherein the moiety (A) is represented by the moiety (A1)

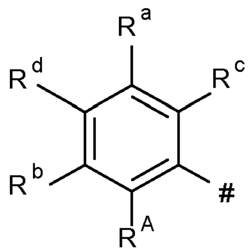

(A.1)

wherein

$R^A$ is halogen or CN, preferably F, Cl or CN;
$R^a$ and $R^b$ are H, halogen or CN;
$R^c$ is halogen, CN or $C_1$-$C_6$-alkyl;
$R^d$ is H or halogen;

preferably

$R^A$ is halogen, preferably F or Cl, more preferably F;

$R^a$, $R^b$ and $R^d$ are H or halogen;
$R^c$ is halogen or CN;

more preferably

$R^A$ is halogen, preferably F or Cl, more preferably F;
$R^a$, $R^b$ and $R^c$ are halogen;
$R^d$ is H or halogen;

most preferably

$R^A$, $R^a$, $R^b$ and $R^c$ are F;
$R^d$ is H or F;

More preferred are diaminotriazine compounds of formula (I), wherein the moiety (A) is represented by one of the moieties (A.1.1), (A.1.2) and (A.1.3);

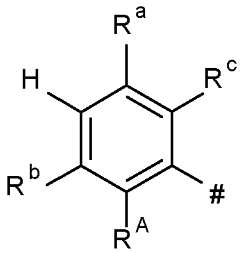
(A.1.1)

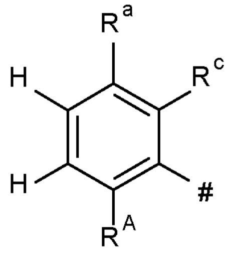
(A.1.2)

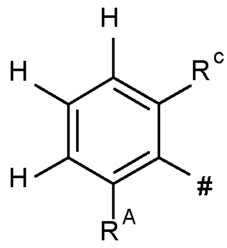
(A.1.3)

wherein

$R^A$ is halogen or CN, preferably F, Cl or CN;
$R^a$, $R^b$ and $R^c$ independently of one another are halogen, OH, CN, amino, $NO_2$, $C_1$-$C_6$-alkyl, $C_1$-$C_6$-haloalkyl, $C_1$-$C_6$-alkoxy, $C_1$-$C_6$-alkylthio, ($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino, ($C_1$-$C_6$-alkyl)carbonyl, ($C_1$-$C_6$-alkoxy)carbonyl;
preferably

$R^A$ is halogen or CN, preferably F, Cl or CN;
$R^c$ is halogen, CN, $C_1$-$C_6$-alkyl or $C_1$-$C_6$-alkoxy; and
$R^a$ and $R^b$ independently of one another are halogen, CN, $NO_2$, $C_1$-$C_6$-alkyl, $C_1$-$C_6$-haloalkyl or $C_1$-$C_6$-alkoxy;

more preferably

$R^A$ is halogen or CN, preferably F, Cl or CN;
$R^c$ is halogen or CN; and
$R^a$ and $R^b$ independently of one another are halogen, CN, $C_1$-$C_6$-alkyl or $C_1$-$C_6$-alkoxy;

most preferably

$R^A$ is halogen, preferably F or Cl, more preferably F;
$R^c$ is halogen; and
$R^a$ and $R^b$ independently of one another are halogen or CN;

especially preferably

$R^A$ is halogen, preferably F or Cl, more preferably F;
$R^a$, $R^b$ and $R^c$ are halogen.

Also preferred are diaminotriazine compounds of formula (I), wherein the moiety (A) isrepresented by (A.2)

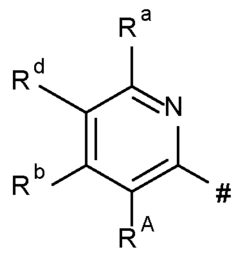

(A.2)

wherein

$R^A$ is halogen or CN, preferably F, Cl or CN;
$R^a$, $R^b$ and $R^d$ are H, halogen or CN;
preferably

$R^A$ is halogen, preferably F or Cl, more preferably F; and
$R^a$, $R^b$ and $R^d$ are H or halogen;

more preferably

$R^A$ is halogen, preferably F or Cl, more preferably F;
$R^a$ and $R^b$ are halogen; and
$R^d$ is H or halogen;

most preferably

$R^A$ is F or Cl, preferably F;
$R^a$ and $R^b$ are F or Cl; and
$R^d$ is H or F.

More preferred are diaminotriazine compounds of formula (I), wherein the moiety (A), in particular (A.2), is 4-chloro-3,5,6-trifluoro-2-pyridyl.

Also preferred are diaminotriazine compounds of formula (I), wherein the moiety (A) is represented by (A.3)

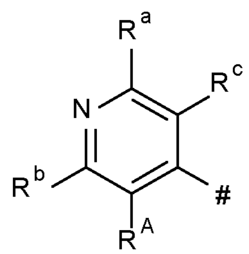

(A.3)

wherein

$R^A$ is halogen or CN, preferably F, Cl or CN;
$R^c$ is halogen or CN;
$R^a$ and $R^b$ are H, halogen or CN;
preferably

$R^A$ is halogen, preferably F or Cl, more preferably F; and
$R^c$ is halogen or CN;
$R^a$ and $R^b$ are H or halogen;

more preferably

$R^A$ is halogen, preferably F or Cl, more preferably F;
$R^a$, $R^b$ and $R^c$ are halogen; and

most preferably

$R^A$ is F or Cl, preferably F; and
$R^a$, $R^b$ and $R^c$ are F or Cl.

More preferred are diaminotriazine compounds of formula (I), wherein the moiety (A), in particular (A.3), is 2,3,5,6-tetraflouro-4-pyridyl.

In further particularly preferred groups of embodiments the moiety (A) of diaminotriazine compounds of formula (I)

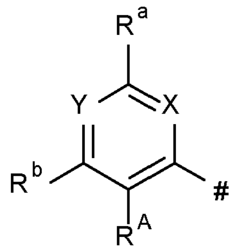

(A)

is represented by the moiety (A.4)

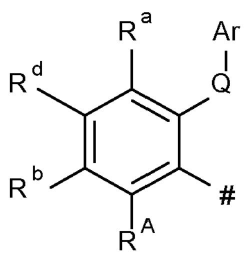

(A.4)

wherein

$R^A$ is halogen or CN, preferably F, Cl or CN;
$R^a$, $R^b$ and $R^d$ independently of one another are hydrogen, halogen, OH, CN, amino, $NO_2$, $C_1$-$C_6$-alkyl, $C_1$-$C_6$-haloalkyl, $C_1$-$C_6$-alkoxy, $C_1$-$C_6$-alkylthio, ($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino, ($C_1$-$C_6$-alkyl)carbonyl, ($C_1$-$C_6$-alkoxy)carbonyl;
Q is a chemical bond, O, $CR^{q1}R^{q2}$, S, S(O) or $S(O)_2$,
wherein $R^{q1}$ and $R^{q2}$ are identical or different selected from hydrogen, halogen, or $C_1$-$C_4$-alkyl;
Ar is phenyl, which is substituted by 1, 2 or 3 substituents $R^e$, wherein $R^e$ is selected from halogen, $C_1$-$C_6$-alkyl, $C_1$-$C_6$-haloalkyl, $C_1$-$C_6$-alkoxy, $C_1$-$C_6$-haloalkoxy;
preferably

$R^A$ is halogen or CN, preferably F, Cl or CN;
$R^a$, $R^b$ and $R^d$ independently of one another are hydrogen, halogen, CN, $NO_2$, $C_1$-$C_6$-alkyl, $C_1$-$C_6$-haloalkyl or $C_1$-$C_6$-alkoxy;
Q is a chemical bond, O, $CR^{q1}R^{q2}$, S, S(O) or $S(O)_2$,
wherein $R^{q1}$ and $R^{q2}$ are identical or different selected from hydrogen, halogen, or $C_1$-$C_4$-alkyl;
Ar is phenyl, which is substituted by 1, 2 or 3 substituents $R^e$, wherein $R^e$ is selected from halogen, $C_1$-$C_6$-alkyl, $C_1$-$C_6$-haloalkyl, $C_1$-$C_6$-alkoxy, $C_1$-$C_6$-haloalkoxy;

more preferably

$R^A$ is halogen or CN, preferably F, Cl or CN;
$R^a$, $R^b$ and $R^d$ independently of one another are hydrogen, halogen, CN, $C_1$-$C_6$-alkyl or $C_1$-$C_6$-alkoxy;
Q is a chemical bond, O, $CH_2$, S, S(O) or $S(O)_2$;

Ar is phenyl, which is substituted by 1, 2 or 3 substituents $R^e$, wherein $R^e$ is selected from halogen, $C_1$-$C_4$-alkyl, $C_1$-$C_4$-haloalkyl, and CN;

most preferably

$R^A$ is halogen, preferably F or Cl, more preferably F;
$R^a$, $R^b$ and $R^d$ independently of one another are hydrogen, halogen or CN;
Q is a chemical bond, O or $CH_2$;
Ar is phenyl, which is substituted by 1, 2 or 3 substituents $R^e$, wherein $R^e$ is selected from halogen, $C_1$-$C_4$-alkyl, $C_1$-$C_2$-haloalkyl, and CN;

especially preferably

$R^A$ is halogen, preferably F or Cl, more preferably F;
$R^a$, $R^b$ and $R^d$ are hydrogen;
Q is a O or $CH_2$;
Ar is phenyl, which is substituted by 1, 2 or 3 substituents $R^e$, wherein $R^e$ is selected from halogen, $CF_3$ and CN;

also especially preferably

$R^A$ is halogen, preferably F or Cl, more preferably F;
$R^a$ and $R^b$ are halogen;
$R^d$ is hydrogen;
Q is O or $CH_2$;
Ar is phenyl, which is substituted by 1, 2 or 3 substituents $R^e$, wherein $R^e$ is selected from halogen, $CF_3$ and CN;

also especially preferably

$R^A$, $R^a$, $R^b$ and $R^d$ are halogen, preferably F or Cl, more preferably F;
Q is O;
Ar is phenyl, which is substituted by 1, 2 or 3 substituents $R^e$, wherein $R^e$ is selected from halogen, $CF_3$ and CN.

In further particularly preferred groups of embodiments the moiety (A) of diaminotriazine compounds of formula (I)

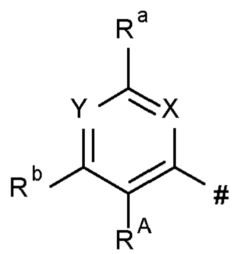

(A)

is represented by the moiety (A.5)

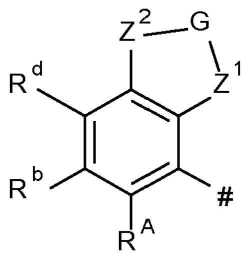

(A.5)

wherein

$R^A$ is halogen or CN, preferably F, Cl or CN;

$R^b$ and $R^d$ independently of one another are hydrogen, halogen, OH, CN, amino, $NO_2$, $C_1$-$C_6$-alkyl, $C_1$-$C_6$-haloalkyl, $C_1$-$C_6$-alkoxy, $C_1$-$C_6$-alkylthio, ($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino, ($C_1$-$C_6$-alkyl)carbonyl, ($C_1$-$C_6$-alkoxy)carbonyl;

$Z^1$ and $Z^2$ independently of one another are $CR^{f1}R^{f2}$, O, S or $NR^z$, wherein $R^z$ is selected from H, $S(O)_2NH_2$, CN, $C_1$-$C_4$-alkyl, $C_1$-$C_4$-haloalkyl, $C_2$-$C_4$-alkenyl, $C_2$-$C_4$-alkynyl; and $R^{f1}$, $R^{f2}$ are identical or different and are hydrogen or have one of the meanings given for $R^f$ defined above;

G is $CR^{f1}R^{f2}$ or $CR^{f1}R^{f2}$-$CR^{f3}R^{f4}$ wherein $R^{f1}$, $R^{f2}$, $R^{f3}$, $R^{f4}$ are identical or different and are hydrogen or have one of the meanings given for $R^f$ defined above;

preferably

$R^A$ is halogen or CN, preferably F, Cl or CN;

$R^b$ and $R^d$ independently of one another are hydrogen, halogen, CN, $NO_2$, $C_1$-$C_6$-alkyl, $C_1$-$C_6$-haloalkyl or $C_1$-$C_6$-alkoxy;

$Z^1$ and $Z^2$ independently of one another are $CR^{f1}R^{f2}$, O, S or $NR^z$, wherein $R^z$ is selected from H, $S(O)_2NH_2$, CN, $C_1$-$C_4$-alkyl, $C_1$-$C_4$-haloalkyl, $C_2$-$C_4$-alkenyl, $C_2$-$C_4$-alkynyl; and $R^{f1}$, $R^{f2}$ are identical or different and are hydrogen or have one of the meanings given for $R^f$ defined above;

G is $CR^{f1}R^{f2}$ or $CR^{f1}R^{f2}$-$CR^{f3}R^{f4}$ wherein $R^{f1}$, $R^{f2}$, $R^{f3}$, $R^{f4}$ are identical or different and are hydrogen or have one of the meanings given for $R^f$ defined above;

more preferably

$R^A$ is halogen or CN, preferably F, Cl or CN;

$R^b$ and $R^d$ independently of one another are hydrogen, halogen, CN, $C_1$-$C_6$-alkyl or $C_1$-$C_6$-alkoxy;

$Z^1$ is $CH_2$, O, S or $NR^z$, wherein $R^z$ is selected from H, $S(O)_2NH_2$, CN or $C_1$-$C_4$-alkyl;

$Z^2$ is $CH_2$, O, S or $NR^z$, wherein $R^z$ is selected from H, $S(O)_2NH_2$, CN or $C_1$-$C_4$-alkyl;

G is $CR^{f1}R^{f2}$, wherein $R^{f1}$ and $R^{f2}$ are identical or different and are hydrogen or have one of the meanings given for $R^f$ defined above;

most preferably

$R^A$ is halogen, preferably F or Cl, more preferably F;

$R^b$ and $R^d$ independently of one another are hydrogen, halogen or CN;

$Z^1$ is O or S;

$Z^2$ is O or S;

G is $CR^{f1}R^{f2}$, wherein $R^{f1}$ and $R^{f2}$ independently of one another are hydrogen, halogen, $C_1$-$C_4$-alkyl, $C_1$-$C_4$-haloalkyl, $C_1$-$C_4$-alkoxy, $C_1$-$C_4$-haloalkoxy;

especially preferably

$R^A$ is halogen, preferably F or Cl, more preferably F;

$R^b$ and $R^d$ are hydrogen;

$Z^1$ is O or S;

$Z^2$ is O or S;

G is $CR^{f1}R^{f2}$, wherein $R^{f1}$ and $R^{f2}$ independently of one another are hydrogen, halogen or $C_1$-$C_4$-alkyl;

also especially preferably

$R^A$ is halogen, preferably F or Cl, more preferably F;

$R^b$ halogen; and

Rd hydrogen;

$Z^1$ is O or S;

$Z^2$ is O or S;

G is $CR^{f1}R^{f2}$, wherein $R^{f1}$ and $R^{f2}$ independently of one another are hydrogen, halogen or $C_1$-$C_4$-alkyl;

also especially preferably

$R^A$, $R^b$ and $R^d$ are halogen, preferably F or Cl, more preferably F;

$Z^1$ is O or S;
$Z^2$ is O or S;
G is $CR^{f1}R^{f2}$, wherein $R^{f1}$ and $R^{f2}$ independently of one another are hydrogen, halogen or $C_1$-$C_4$-alkyl.

Particular groups of embodiments relate to the diaminotriazine compounds of formula (I), wherein $R^1$, $R^2$, $R^3$ and $R^{3'}$ have the following meanings:

$R^1$ H, halogen, OH, CN, $C_1$-$C_6$-alkyl, $C_1$-$C_6$-haloalkyl, ($C_1$-$C_6$-alkoxy)-$C_1$-$C_6$-alkyl, $C_3$-$C_6$-cycloalkyl or $C_1$-$C_6$-alkoxy;
$R^2$ H, halogen, CN, $C_1$-$C_6$-alkyl, $C_1$-$C_6$-haloalkyl or $C_1$-$C_6$-alkoxy;
$R^3$ H, $C_1$-$C_6$-alkyl or $C_1$-$C_6$-haloalkyl;
$R^{3'}$ H, $C_1$-$C_6$-alkyl or $C_1$-$C_6$-haloalkyl;
or
$R^1$ and $R^2$ together with the carbon atom to which they are attached form a moiety selected from the group consisting of carbonyl, $C_3$-$C_6$-cycloalkyl, $C_3$-$C_6$-cycloalkenyl and three- to six-membered heterocyclyl,
where the carbocycle and the heterocycle are unsubstituted, partly or completely halogenated or carry from 1 to 6 $C_1$-$C_6$-alkyl groups;
or
$R^2$ and $R^3$ together with the carbon atom to which they are attached form a moiety selected from the group consisting of $C_3$-$C_6$-cycloalkyl, $C_3$-$C_6$-cycloalkenyl and three-to six-membered heterocyclyl,
where the carbocycle and the heterocycle are unsubstituted, partly or completely halogenated or carry from 1 to 6 $C_1$-$C_6$-alkyl groups;

preferably

$R^1$ H, halogen, $C_1$-$C_6$-alkyl, $C_1$-$C_6$-haloalkyl or $C_1$-$C_6$-alkoxy;
$R^2$ H, halogen or $C_1$-$C_6$-alkyl;
$R^3$ H, $C_1$-$C_6$-alkyl or $C_1$-$C_6$-haloalkyl;
$R^{3'}$ H or $C_1$-$C_6$-alkyl;
or
$R^1$ and $R^2$ together with the carbon atom to which they are attached form a $C_3$-$C_6$-cycloalkyl;

more preferably

$R^1$ H, halogen, $C_1$-$C_6$-alkyl, $C_1$-$C_6$-haloalkyl or $C_1$-$C_6$-alkoxy;
$R^2$ H or $C_1$-$C_6$-alkyl;
$R^3$ H, $C_1$-$C_6$-alkyl, or $C_1$-$C_6$-haloalkyl;
$R^{3'}$ H or $C_1$-$C_6$-alkyl;
or
$R^1$ and $R^2$ together with the carbon atom to which they are attached form a $C_3$-$C_6$-cycloalkyl;

most preferably

$R^1$ H, halogen, $C_1$-$C_4$-alkyl, $C_1$-$C_4$-haloalkyl or $C_1$-$C_4$-alkoxy;
$R^2$ H or $C_1$-$C_4$-alkyl;
$R^3$ H, $C_1$-$C_4$-alkyl or $C_1$-$C_4$-haloalkyl;
$R^{3'}$ H or $C_1$-$C_4$-alkyl;
or
$R^1$ and $R^2$ together with the carbon atom to which they are attached form a $C_3$-$C_6$-cycloalkyl.

Most preferred are diaminotriazines of formula (I), wherein the moiety (A) is represented by one of the moieties (A.1), (A.1.1), (A.1.2), (A.1.3), (A.2), (A.3), (A4) and (A5) wherein
$R^A$ is F; and the remaining residue definitions are as defined above.
Examples of especially preferable combinations of $R^1$, $R^2$, $R^3$ and $R^{3'}$ are given in the following table 1:

Table 1

| # | $R^1$ | $R^2$ | $R^3$ | $R^{3'}$ |
|---|---|---|---|---|
| 1. | H | H | H | H |
| 2. | F | H | H | H |
| 3. | F | F | H | H |
| 4. | F | $CH_3$ | H | H |
| 5. | H | $CH_3$ | H | H |
| 6. | $CH_3$ | $CH_3$ | H | H |
| 7. | $CF_3$ | $CH_3$ | H | H |
| 8. | $OCH_3$ | $CH_3$ | H | H |
| 9. | $C_2H_5$ | $CH_3$ | H | H |
| 10. | $C_2H_5$ | $C_2H_5$ | H | H |
| 11. | H | H | $CH_3$ | H |
| 12. | F | H | $CH_3$ | H |
| 13. | F | F | $CH_3$ | H |
| 14. | F | $CH_3$ | $CH_3$ | H |
| 15. | H | $CH_3$ | $CH_3$ | H |
| 16. | $CH_3$ | $CH_3$ | $CH_3$ | H |
| 17. | $CF_3$ | $CH_3$ | $CH_3$ | H |
| 18. | $OCH_3$ | $CH_3$ | $CH_3$ | H |
| 19. | $C_2H_5$ | $CH_3$ | $CH_3$ | H |
| 20. | $C_2H_5$ | $C_2H_5$ | $CH_3$ | H |
| 21. | H | $OCH_3$ | $CH_3$ | H |
| 22. | F | $OCH_3$ | $CH_3$ | H |
| 23. | H | H | $C_2H_5$ | H |
| 24. | F | H | $C_2H_5$ | H |
| 25. | F | F | $C_2H_5$ | H |
| 26. | F | $CH_3$ | $C_2H_5$ | H |
| 27. | H | $CH_3$ | $C_2H_5$ | H |
| 28. | $CH_3$ | $CH_3$ | $C_2H_5$ | H |
| 29. | $CF_3$ | $CH_3$ | $C_2H_5$ | H |
| 30. | $OCH_3$ | $CH_3$ | $C_2H_5$ | H |
| 31. | $C_2H_5$ | $CH_3$ | $C_2H_5$ | H |
| 32. | $C_2H_5$ | $C_2H_5$ | $C_2H_5$ | H |
| 33. | F | $C_2H_5$ | $C_2H_5$ | H |
| 34. | H | $OCH_3$ | $C_2H_5$ | H |
| 35. | F | $OCH_3$ | $C_2H_5$ | H |
| 36. | H | H | $CH(CH_3)_2$ | H |
| 37. | F | H | $CH(CH_3)_2$ | H |

(continued)

| # | $R^1$ | $R^2$ | $R^3$ | $R^{3'}$ |
|---|---|---|---|---|
| 38. | F | F | $CH(CH_3)_2$ | H |
| 39. | H | $CH_3$ | $CH(CH_3)_2$ | H |
| 40. | H | $OCH_3$ | $CH(CH_3)_2$ | H |
| 41. | F | $CH_3$ | $CH(CH_3)_2$ | H |
| 42. | H | H | $CH_2CH_2CH_3$ | H |
| 43. | F | H | $CH_2CH_2CH_3$ | H |
| 44. | F | F | $CH_2CH_2CH_3$ | H |
| 45. | H | $CH_3$ | $CH_2CH_2CH_3$ | H |
| 46. | H | $OCH_3$ | $CH_2CH_2CH_3$ | H |
| 47. | F | $CH_3$ | $CH_2CH_2CH_3$ | H |
| 48. | H | H | $C(CH_3)_3$ | H |
| 49. | F | H | $C(CH_3)_3$ | H |
| 50. | F | F | $C(CH_3)_3$ | H |
| 51. | H | $CH_3$ | $C(CH_3)_3$ | H |
| 52. | H | $OCH_3$ | $C(CH_3)_3$ | H |
| 53. | F | $CH_3$ | $C(CH_3)_3$ | H |
| 54. | H | H | $C-C_3H_5$ | H |
| 55. | F | H | $C-C_3H_5$ | H |
| 56. | F | F | $C-C_3H_5$ | H |
| 57. | H | $CH_3$ | $C-C_3H_5$ | H |
| 58. | H | $OCH_3$ | $C-C_3H_5$ | H |
| 59. | F | $CH_3$ | $C-C_3H_5$ | H |
| 60. | H | $CH_3$ | $CF_3$ | H |
| 61. | F | $CH_3$ | $CF_3$ | H |
| 62. | $CH_3$ | $CH_3$ | $CF_3$ | H |
| 63. | $CH_2-CH_2$ | | H | H |
| 64. | $CH_2-CH_2$ | | $CH_3$ | H |
| 65. | $CH_2-CH_2-CH_2$ | | H | H |
| 66. | $CH_2-CH_2-CH_2$ | | $CH_3$ | H |
| 67. | $CH_2-CH_2-CH_2-CH_2$ | | H | H |
| 68. | $CH_2-CH_2-CH_2-CH_2$ | | $CH_3$ | H |
| 69. | $CH_2-CH_2-CH_2-CH_2-CH_2$ | | H | H |
| 70. | $CH_2-CH_2-CH_2-CH_2-CH_2$ | | $CH_3$ | H |
| 71. | $O-CH_2-CH_2-CH_2$ | | H | H |
| 72. | $O-CH_2-CH_2-CH_2$ | | $CH_3$ | H |
| 73. | $O-CH_2-CH_2-CH_2-CH_2$ | | H | H |
| 74. | $O-CH_2-CH_2-CH_2-CH_2$ | | $CH_3$ | H |
| 75. | H | H | H | $CH_3$ |

(continued)

| # | $R^1$ | $R^2$ | $R^3$ | $R^{3'}$ |
|---|---|---|---|---|
| 76. | F | H | H | $CH_3$ |
| 77. | F | F | H | $CH_3$ |
| 78. | F | $CH_3$ | H | $CH_3$ |
| 79. | H | $CH_3$ | H | $CH_3$ |
| 80. | $CH_3$ | $CH_3$ | H | $CH_3$ |
| 81. | $CF_3$ | $CH_3$ | H | $CH_3$ |
| 82. | $OCH_3$ | $CH_3$ | H | $CH_3$ |
| 83. | $C_2H_5$ | $CH_3$ | H | $CH_3$ |
| 84. | $C_2H_5$ | $C_2H_5$ | H | $CH_3$ |
| 85. | H | H | $CH_3$ | $CH_3$ |
| 86. | F | H | $CH_3$ | $CH_3$ |
| 87. | F | F | $CH_3$ | $CH_3$ |
| 88. | F | $CH_3$ | $CH_3$ | $CH_3$ |
| 89. | H | $CH_3$ | $CH_3$ | $CH_3$ |
| 90. | $CH_3$ | $CH_3$ | $CH_3$ | $CH_3$ |
| 91. | $CF_3$ | $CH_3$ | $CH_3$ | $CH_3$ |
| 92. | $OCH_3$ | $CH_3$ | $CH_3$ | $CH_3$ |
| 93. | $C_2H_5$ | $CH_3$ | $CH_3$ | $CH_3$ |
| 94. | $C_2H_5$ | $C_2H_5$ | $CH_3$ | $CH_3$ |
| 95. | H | $OCH_3$ | $CH_3$ | $CH_3$ |
| 96. | F | $OCH_3$ | $CH_3$ | $CH_3$ |
| 97. | H | H | $C_2H_5$ | $CH_3$ |
| 98. | F | H | $C_2H_5$ | $CH_3$ |
| 99. | F | F | $C_2H_5$ | $CH_3$ |
| 100. | F | $CH_3$ | $C_2H_5$ | $CH_3$ |
| 101. | H | $CH_3$ | $C_2H_5$ | $CH_3$ |
| 102. | $CH_3$ | $CH_3$ | $C_2H_5$ | $CH_3$ |
| 103. | $CF_3$ | $CH_3$ | $C_2H_5$ | $CH_3$ |
| 104. | $OCH_3$ | $CH_3$ | $C_2H_5$ | $CH_3$ |
| 105. | $C_2H_5$ | $CH_3$ | $C_2H_5$ | $CH_3$ |
| 106. | $C_2H_5$ | $C_2H_5$ | $C_2H_5$ | $CH_3$ |
| 107. | F | $C_2H_5$ | $C_2H_5$ | $CH_3$ |
| 108. | H | $OCH_3$ | $C_2H_5$ | $CH_3$ |
| 109. | F | $OCH_3$ | $C_2H_5$ | $CH_3$ |
| 110. | H | H | $CH(CH_3)_2$ | $CH_3$ |
| 111. | F | H | $CH(CH_3)_2$ | $CH_3$ |
| 112. | F | F | $CH(CH_3)_2$ | $CH_3$ |
| 113. | H | $CH_3$ | $CH(CH_3)_2$ | $CH_3$ |

(continued)

| # | $R^1$ | $R^2$ | $R^3$ | $R^{3'}$ |
|---|---|---|---|---|
| 114. | H | $OCH_3$ | $CH(CH_3)_2$ | $CH_3$ |
| 115. | F | $CH_3$ | $CH(CH_3)_2$ | $CH_3$ |
| 116. | H | H | $CH_2CH_2CH_3$ | $CH_3$ |
| 117. | F | H | $CH_2CH_2CH_3$ | $CH_3$ |
| 118. | F | F | $CH_2CH_2CH_3$ | $CH_3$ |
| 119. | H | $CH_3$ | $CH_2CH_2CH_3$ | $CH_3$ |
| 120. | H | $OCH_3$ | $CH_2CH_2CH_3$ | $CH_3$ |
| 121. | F | $CH_3$ | $CH_2CH_2CH_3$ | $CH_3$ |
| 122. | H | H | $C(CH_3)_3$ | $CH_3$ |
| 123. | F | H | $C(CH_3)_3$ | $CH_3$ |
| 124. | F | F | $C(CH_3)_3$ | $CH_3$ |
| 125. | H | $CH_3$ | $C(CH_3)_3$ | $CH_3$ |
| 126. | H | $OCH_3$ | $C(CH_3)_3$ | $CH_3$ |
| 127. | F | $CH_3$ | $C(CH_3)_3$ | $CH_3$ |
| 128. | H | H | $C-C_3H_5$ | $CH_3$ |
| 129. | F | H | $C-C_3H_5$ | $CH_3$ |
| 130. | F | F | $C-C_3H_5$ | $CH_3$ |
| 131. | H | $CH_3$ | $C-C_3H_5$ | $CH_3$ |
| 132. | H | $OCH_3$ | $C-C_3H_5$ | $CH_3$ |
| 133. | F | $CH_3$ | $C-C_3H_5$ | $CH_3$ |
| 134. | H | $CH_3$ | $CF_3$ | $CH_3$ |
| 135. | F | $CH_3$ | $CF_3$ | $CH_3$ |
| 136. | $CH_3$ | $CH_3$ | $CF_3$ | $CH_3$ |
| 137. | $CH_2-CH_2$ | | H | $CH_3$ |
| 138. | $CH_2-CH_2$ | | $CH_3$ | $CH_3$ |
| 139. | $CH_2-CH_2-CH_2$ | | H | $CH_3$ |
| 140. | $CH_2-CH_2-CH_2$ | | $CH_3$ | $CH_3$ |
| 141. | $CH_2-CH_2-CH_2-CH_2$ | | H | $CH_3$ |
| 142. | $CH_2-CH_2-CH_2-CH_2$ | | $CH_3$ | $CH_3$ |
| 143. | $CH_2-CH_2-CH_2-CH_2-CH_2$ | | H | $CH_3$ |
| 144. | $CH_2-CH_2-CH_2-CH_2-CH_2$ | | $CH_3$ | $CH_3$ |
| 145. | $O-CH_2-CH_2-CH_2$ | | H | $CH_3$ |
| 146. | $O-CH_2-CH_2-CH_2$ | | $CH_3$ | $CH_3$ |
| 147. | $O-CH_2-CH_2-CH_2-CH_2$ | | H | $CH_3$ |
| 148. | $O-CH_2-CH_2-CH_2-CH_2$ | | $CH_3$ | $CH_3$ |
| The following abbreviations were used in Table 1<br>$c-C_3H_5$: cyclopropyl | | | | |

Preferred are diaminotriazine compounds of formula (I.A.1), wherein P is NH, $R^1$ is F; $R^2$, $R^3$ and $R^{3'}$ are H; and $R^A$, $R^a$, $R^b$, $R^c$ and $R^d$ are defined in table A1-1, wherein each line of table A represents one compound of formula (I.A.1), particularly compounds A1-1.1 to A1-1.180,

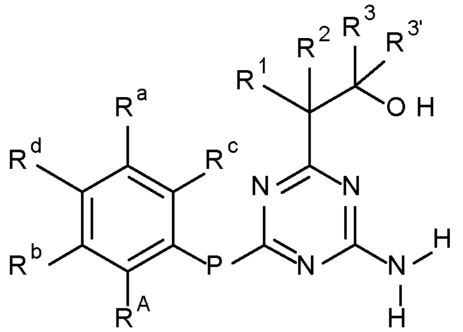

(I.A.1)

Table A1-1.

| no. | $R^A$ | $R^a$ | $R^b$ | $R^c$ | $R^d$ |
|---|---|---|---|---|---|
| A1-1.1 | F | H | H | F | H |
| A1-1.2 | F | H | H | F | F |
| A1-1.3 | F | H | H | F | Cl |
| A1-1.4 | F | H | H | F | Br |
| A1-1.5 | F | H | H | F | I |
| A1-1.6 | F | H | H | CN | H |
| A1-1.7 | F | H | H | CN | F |
| A1-1.8 | F | H | H | CN | Cl |
| A1-1.9 | F | H | H | CN | Br |
| A1-1.10 | F | H | H | CN | I |
| A1-1.11 | F | H | F | F | H |
| A1-1.12 | F | H | F | F | F |
| A1-1.13 | F | H | F | F | Cl |
| A1-1.14 | F | H | F | F | Br |
| A1-1.15 | F | H | F | F | I |
| A1-1.16 | F | H | F | CN | H |
| A1-1.17 | F | H | F | CN | F |
| A1-1.18 | F | H | F | CN | Cl |
| A1-1.19 | F | H | F | CN | Br |
| A1-1.20 | F | H | F | CN | I |
| A1-1.21 | F | H | Cl | F | H |
| A1-1.22 | F | H | Cl | F | F |
| A1-1.23 | F | H | Cl | F | Cl |
| A1-1.24 | F | H | Cl | F | Br |
| A1-1.25 | F | H | Cl | F | I |
| A1-1.26 | F | H | Cl | CN | H |
| A1-1.27 | F | H | Cl | CN | F |
| A1-1.28 | F | H | Cl | CN | Cl |
| A1-1.29 | F | H | Cl | CN | Br |
| A1-1.30 | F | H | Cl | CN | I |

| no. | $R^A$ | $R^a$ | $R^b$ | $R^c$ | $R^d$ |
|---|---|---|---|---|---|
| A1-1.31 | F | F | H | F | H |
| A1-1.32 | F | F | H | F | F |
| A1-1.33 | F | F | H | F | Cl |
| A1-1.34 | F | F | H | F | Br |
| A1-1.35 | F | F | H | F | I |
| A1-1.36 | F | F | H | CN | H |
| A1-1.37 | F | F | H | CN | F |
| A1-1.38 | F | F | H | CN | Cl |
| A1-1.39 | F | F | H | CN | Br |
| A1-1.40 | F | F | H | CN | I |
| A1-1.41 | F | F | F | F | H |
| A1-1.42 | F | F | F | F | F |
| A1-1.43 | F | F | F | F | Cl |
| A1-1.44 | F | F | F | F | Br |
| A1-1.45 | F | F | F | F | I |
| A1-1.46 | F | F | F | CN | H |
| A1-1.47 | F | F | F | CN | F |
| A1-1.48 | F | F | F | CN | Cl |
| A1-1.49 | F | F | F | CN | Br |
| A1-1.50 | F | F | F | CN | I |
| A1-1.51 | F | F | Cl | F | H |
| A1-1.52 | F | F | Cl | F | F |
| A1-1.53 | F | F | Cl | F | Cl |
| A1-1.54 | F | F | Cl | F | Br |
| A1-1.55 | F | F | Cl | F | I |
| A1-1.56 | F | F | Cl | CN | H |
| A1-1.57 | F | F | Cl | CN | F |
| A1-1.58 | F | F | Cl | CN | Cl |
| A1-1.59 | F | F | Cl | CN | Br |
| A1-1.60 | F | F | Cl | CN | I |

| no. | $R^A$ | $R^a$ | $R^b$ | $R^c$ | $R^d$ |
|---|---|---|---|---|---|
| A1-1.61 | Cl | H | H | F | H |
| A1-1.62 | Cl | H | H | F | F |
| A1-1.63 | Cl | H | H | F | Cl |
| A1-1.64 | Cl | H | H | F | Br |
| A1-1.65 | Cl | H | H | F | I |
| A1-1.66 | Cl | H | H | CN | H |
| A1-1.67 | Cl | H | H | CN | F |
| A1-1.68 | Cl | H | H | CN | Cl |
| A1-1.69 | Cl | H | H | CN | Br |
| A1-1.70 | Cl | H | H | CN | I |
| A1-1.71 | Cl | H | F | F | H |
| A1-1.72 | Cl | H | F | F | F |
| A1-1.73 | Cl | H | F | F | Cl |
| A1-1.74 | Cl | H | F | F | Br |
| A1-1.75 | Cl | H | F | F | I |
| A1-1.76 | Cl | H | F | CN | H |
| A1-1.77 | Cl | H | F | CN | F |
| A1-1.78 | Cl | H | F | CN | Cl |
| A1-1.79 | Cl | H | F | CN | Br |
| A1-1.80 | Cl | H | F | CN | I |
| A1-1.81 | Cl | H | Cl | F | H |
| A1-1.82 | Cl | H | Cl | F | F |
| A1-1.83 | Cl | H | Cl | F | Cl |
| A1-1.84 | Cl | H | Cl | F | Br |
| A1-1.85 | Cl | H | Cl | F | I |
| A1-1.86 | Cl | H | Cl | CN | H |
| A1-1.87 | Cl | H | Cl | CN | F |
| A1-1.88 | Cl | H | Cl | CN | Cl |
| A1-1.89 | Cl | H | Cl | CN | Br |
| A1-1.90 | Cl | H | Cl | CN | I |
| A1-1.91 | Cl | F | H | F | H |
| A1-1.92 | Cl | F | H | F | F |
| A1-1.93 | Cl | F | H | F | Cl |
| A1-1.94 | Cl | F | H | F | Br |
| A1-1.95 | Cl | F | H | F | I |
| A1-1.96 | Cl | F | H | CN | H |
| A1-1.97 | Cl | F | H | CN | F |
| A1-1.98 | Cl | F | H | CN | Cl |
| A1-1.99 | Cl | F | H | CN | Br |

| no. | $R^A$ | $R^a$ | $R^b$ | $R^c$ | $R^d$ |
|---|---|---|---|---|---|
| A1-1.100 | Cl | F | H | CN | I |
| A1-1.101 | Cl | F | F | F | H |
| A1-1.102 | Cl | F | F | F | F |
| A1-1.103 | Cl | F | F | F | Cl |
| A1-1.104 | Cl | F | F | F | Br |
| A1-1.105 | Cl | F | F | F | I |
| A1-1.106 | Cl | F | F | CN | H |
| A1-1.107 | Cl | F | F | CN | F |
| A1-1.108 | Cl | F | F | CN | Cl |
| A1-1.109 | Cl | F | F | CN | Br |
| A1-1.110 | Cl | F | F | CN | I |
| A1-1.111 | Cl | F | Cl | F | H |
| A1-1.112 | Cl | F | Cl | F | F |
| A1-1.113 | Cl | F | Cl | F | Cl |
| A1-1.114 | Cl | F | Cl | F | Br |
| A1-1.115 | Cl | F | Cl | F | I |
| A1-1.116 | Cl | F | Cl | CN | H |
| A1-1.117 | Cl | F | Cl | CN | F |
| A1-1.118 | Cl | F | Cl | CN | Cl |
| A1-1.119 | Cl | F | Cl | CN | Br |
| A1-1.120 | Cl | F | Cl | CN | I |
| A1-1.121 | CN | H | H | F | H |
| A1-1.122 | CN | H | H | F | F |
| A1-1.123 | CN | H | H | F | Cl |
| A1-1.124 | CN | H | H | F | Br |
| A1-1.125 | CN | H | H | F | I |
| A1-1.126 | CN | H | H | CN | H |
| A1-1.127 | CN | H | H | CN | F |
| A1-1.128 | CN | H | H | CN | Cl |
| A1-1.129 | CN | H | H | CN | Br |
| A1-1.130 | CN | H | H | CN | I |
| A1-1.131 | CN | H | F | F | H |
| A1-1.132 | CN | H | F | F | F |
| A1-1.133 | CN | H | F | F | Cl |
| A1-1.134 | CN | H | F | F | Br |
| A1-1.135 | CN | H | F | F | I |
| A1-1.136 | CN | H | F | CN | H |
| A1-1.137 | CN | H | F | CN | F |
| A1-1.138 | CN | H | F | CN | Cl |

| no. | $R^A$ | $R^a$ | $R^b$ | $R^c$ | $R^d$ |
|---|---|---|---|---|---|
| A1-1.139 | CN | H | F | CN | Br |
| A1-1.140 | CN | H | F | CN | I |
| A1-1.141 | CN | H | Cl | F | H |
| A1-1.142 | CN | H | Cl | F | F |
| A1-1.143 | CN | H | Cl | F | Cl |
| A1-1.144 | CN | H | Cl | F | Br |
| A1-1.145 | CN | H | Cl | F | I |
| A1-1.146 | CN | H | Cl | CN | H |
| A1-1.147 | CN | H | Cl | CN | F |
| A1-1.148 | CN | H | Cl | CN | Cl |
| A1-1.149 | CN | H | Cl | CN | Br |
| A1-1.150 | CN | H | Cl | CN | I |
| A1-1.151 | CN | F | H | F | H |
| A1-1.152 | CN | F | H | F | F |
| A1-1.153 | CN | F | H | F | Cl |
| A1-1.154 | CN | F | H | F | Br |
| A1-1.155 | CN | F | H | F | I |
| A1-1.156 | CN | F | H | CN | H |
| A1-1.157 | CN | F | H | CN | F |
| A1-1.158 | CN | F | H | CN | Cl |
| A1-1.159 | CN | F | H | CN | Br |
| A1-1.160 | CN | F | H | CN | I |
| A1-1.161 | CN | F | F | F | H |
| A1-1.162 | CN | F | F | F | F |
| A1-1.163 | CN | F | F | F | Cl |
| A1-1.164 | CN | F | F | F | Br |
| A1-1.165 | CN | F | F | F | I |
| A1-1.166 | CN | F | F | CN | H |
| A1-1.167 | CN | F | F | CN | F |
| A1-1.168 | CN | F | F | CN | Cl |
| A1-1.169 | CN | F | F | CN | Br |
| A1-1.170 | CN | F | F | CN | I |
| A1-1.171 | CN | F | Cl | F | H |
| A1-1.172 | CN | F | Cl | F | F |
| A1-1.173 | CN | F | Cl | F | Cl |
| A1-1.174 | CN | F | Cl | F | Br |
| A1-1.175 | CN | F | Cl | F | I |
| A1-1.176 | CN | F | Cl | CN | H |
| A1-1.177 | CN | F | Cl | CN | F |
| A1-1.178 | CN | F | Cl | CN | Cl |
| A1-1.179 | CN | F | Cl | CN | Br |
| A1-1.180 | CN | F | Cl | CN | I |

Also preferred are diaminotriazine compounds exemplified in Tables A1-2 to A1-30.

Table A1-2. Diaminotriazine compounds of formula (I.A.1), wherein P is NH, $R^1$ is F; $R^2$ is $CH_3$, $R^3$ and $R^{3'}$ are H; and $R^A$, $R^a$, $R^b$, $R^c$ and $R^d$ are defined in Table A1-1, particularly compounds of formulae A1-2.1 to A1-2.180.
Table A1-3. Diaminotriazine compounds of formula (I.A.1), wherein P is NH, $R^1$ is H; $R^2$ is $CH_3$, $R^3$ and $R^{3'}$ are H; and $R^A$, $R^a$, $R^b$, $R^e$ and $R^d$ are defined in Table A1-1, particularly compounds of formulae A1-3.1 to A1-3.180.
Table A1-4. Diaminotriazine compounds of formula (I.A.1), wherein P is NH, $R^1$ is $CH_3$; $R^2$ is $CH_3$, $R^3$ and $R^{3'}$ are H; and $R^A$, $R^a$, $R^b$, $R^e$ and $R^d$ are defined in Table A1-1, particularly compounds of formulae A1-4.1 to A1-4.180.
Table A1-5. Diaminotriazine compounds of formula (I.A.1), wherein P is NH, $R^1$ is $CF_3$; $R^2$ is $CH_3$, $R^3$ and $R^{3'}$ are H; and $R^A$, $R^a$, $R^b$, $R^e$ and $R^d$ are defined in Table A1-1, particularly compounds of formulae A1-5.1 to A1-5.180.
Table A1-6. Diaminotriazine compounds of formula (I.A.1), wherein P is NH, $R^1$ is $OCH_3$; $R^2$ is $CH_3$, $R^3$ and $R^{3'}$ are H; and $R^A$, $R^a$, $R^b$, $R^c$ and $R^d$ are defined in Table A1-1, particularly compounds of formulae A1-6.1 to A1-6.180.
Table A1-7. Diaminotriazine compounds of formula (I.A.1), wherein P is NH, $R^1$ is $C_2H_5$; $R^2$ is $CH_3$, $R^3$ and $R^{3'}$ are H; and $R^A$, $R^a$, $R^b$, $R^e$ and $R^d$ are defined in Table A1-1, particularly compounds of formulae A1-7.1 to A1-7.180.
Table A1-8. Diaminotriazine compounds of formula (I.A.1), wherein P is NH, $R^1$ is $C_2H_5$; $R^2$ is $C_2H_5$, $R^3$ and $R^{3'}$ are H; and $R^A$, $R^a$, $R^b$, $R^e$ and $R^d$ are defined in Table A1-1, particularly compounds of formulae A1-8.1 to A1-8.180.
Table A1-9. Diaminotriazine compounds of formula (I.A.1), wherein P is NH, $R^1$, $R^2$ and $R^3$ are $CH_3$, $R^{3'}$ is H; and $R^A$, $R^a$, $R^b$, $R^c$ and $R^d$ are defined in Table A1-1, particularly compounds of formulae A1-9.1 to A1 -9.180.
Table A1-10. Diaminotriazine compounds of formula (I.A.1), wherein P is NH, $R^1$ and $R^2$ are $CH_3$, $R^3$ is $CF_3$; $R^{3'}$ is H; and $R^A$, $R^a$, $R^b$, $R^e$ and $R^d$ are defined in Table A1-1, particularly compounds of formulae A1 -10.1 to A1 -10.180.
Table A1-11. Diaminotriazine compounds of formula (I.A.1), wherein P is NH, $R^1$, $R^2$, $R^3$ and $R^{3'}$ are $CH_3$; and $R^A$, $R^a$, $R^b$, $R^c$ and $R^d$ are defined in table A1-1, particularly compounds of formulae A1-11.1 to A1-11.180.
Table A1-12. Diaminotriazine compounds of formula (I.A.1), wherein P is NH, $R^1$ and $R^2$ together with the carbon

atom to which they are attached form cyclopropyl, $R^3$ and $R^{3'}$ are H; and $R^A$, $R^a$, $R^b$, $R^c$ and $R^d$ are defined in table A1-1, particularly compounds of formulae A1-12.1 to A1-12.180.

Table A1-13. Diaminotriazine compounds of formula (I.A.1), wherein P is NH, $R^1$ and $R^2$ together with the carbon atom to which they are attached form cyclobutyl, $R^3$ and $R^{3'}$ are H; and $R^A$, $R^a$, $R^b$, $R^c$ and $R^d$ are defined in table A1-1, particularly compounds of formulae A1-13.1 to A1-13.180.

Table A1-14. Diaminotriazine compounds of formula (I.A.1), wherein P is NH, $R^1$ and $R^2$ together with the carbon atom to which they are attached form cyclopentyl, $R^3$ and $R^{3'}$ are H; and $R^A$, $R^a$, $R^b$, $R^c$ and $R^d$ are defined in table A1-1, particularly compounds of formulae A1-14.1 to A1-14.180.

Table A1-15. Diaminotriazine compounds of formula (I.A.1), wherein P is NH, $R^1$ and $R^2$ together with the carbon atom to which they are attached form cyclohexyl, $R^3$ and $R^{3'}$ are H; and $R^A$, $R^a$, $R^b$, $R^c$ and $R^d$ are defined in table A1-1, particularly compounds of formulae A1-15.1 to A1-15.180.

Table A1-16. Diaminotriazine compounds of formula (I.A.1), wherein P is O, $R^1$ is F; $R^2$, $R^3$ and $R^{3'}$ are H; and $R^A$, $R^a$, $R^b$, $R^c$ and $R^d$ are defined in table A1-1, particularly compounds of formulae A1-16.1 to A1-16.180.

Table A1-17. Diaminotriazine compounds of formula (I.A.1), wherein P is O, $R^1$ is F; $R^2$ is $CH_3$, $R^3$ and $R^{3'}$ are H; and $R^A$, $R^a$, $R^b$, $R^e$ and $R^d$ are defined in Table A1-1, particularly compounds of formulae A1 -17.1 to A1 -17.180.

Table A1-18. Diaminotriazine compounds of formula (I.A.1), wherein P is O, $R^1$ is H; $R^2$ is $CH_3$, $R^3$ and $R^{3'}$ are H; and $R^A$, $R^a$, $R^b$, $R^c$ and $R^d$ are defined in Table A1-1, particularly compounds of formulae A1 -18.1 to A1-18.180.

Table A1-19. Diaminotriazine compounds of formula (I.A.1), wherein P is O, $R^1$ is $CH_3$; $R^2$ is $CH_3$, $R^3$ and $R^{3'}$ are H; and $R^A$, $R^a$, $R^b$, $R^c$ and $R^d$ are defined in Table A1-1, particularly compounds of formulae A1-19.1 to A1-19.180.

Table A1-20. Diaminotriazine compounds of formula (I.A.1), wherein P is O, $R^1$ is $CF_3$; $R^2$ is $CH_3$, $R^3$ and $R^{3'}$ are H; and $R^A$, $R^a$, $R^b$, $R^c$ and $R^d$ are defined in Table A1-1, particularly compounds of formulae A1-20.1 to A1-20.180.

Table A1-21. Diaminotriazine compounds of formula (I.A.1), wherein P is O, $R^1$ is $OCH_3$; $R^2$ is $CH_3$, $R^3$ and $R^{3'}$ are H; and $R^A$, $R^a$, $R^b$, $R^c$ and $R^d$ are defined in Table A1-1, particularly compounds of formulae A1-21.1 to A1-21.180.

Table A1-22. Diaminotriazine compounds of formula (I.A.1), wherein P is O, $R^1$ is $C_2H_5$; $R^2$ is $CH_3$, $R^3$ and $R^{3'}$ are H; and $R^A$, $R^a$, $R^b$, $R^c$ and $R^d$ are defined in Table A1-1, particularly compounds of formulae A1-22.1 to A1-22.180.

Table A1-23. Diaminotriazine compounds of formula (I.A.1), wherein P is O, $R^1$ is $C_2H_5$; $R^2$ is $C_2H_5$, $R^3$ and $R^{3'}$ are H; and $R^A$, $R^a$, $R^b$, $R^c$ and $R^d$ are defined in Table A1-1, particularly compounds of formulae A1-23.1 to A1-23.180.

Table A1-24. Diaminotriazine compounds of formula (I.A.1), wherein P is O, $R^1$, $R^2$ and $R^3$ are $CH_3$, $R^{3'}$ is H; and $R^A$, $R^a$, $R^b$, $R^c$ and $R^d$ are defined in Table A1-1, particularly compounds of formulae A1-24.1 to A1-24.180.

Table A1-25. Diaminotriazine compounds of formula (I.A.1), wherein P is O, $R^1$ and $R^2$ are $CH_3$, $R^3$ is $CF_3$; $R^{3'}$ is H; and $R^A$, $R^a$, $R^b$, $R^c$ and $R^d$ are defined in Table A1-1, particularly compounds of formulae A1-25.1 to A1-25.180.

Table A1-26. Diaminotriazine compounds of formula (I.A.1), wherein P is O, $R^1$, $R^2$, $R^3$ and $R^{3'}$ are $CH_3$; and $R^A$, $R^a$, $R^b$, $R^c$ and $R^d$ are defined in table A1-1, particularly compounds of formulae A1-26.1 to A1-26.180.

Table A1-27. Diaminotriazine compounds of formula (I.A.1), wherein P is O, $R^1$ and $R^2$ together with the carbon atom to which they are attached form cyclopropyl, $R^3$ and $R^{3'}$ are H; and $R^A$, $R^a$, $R^b$, $R^c$ and $R^d$ are defined in table A1-1, particularly compounds of formulae A1-27.1 to A1-27.180.

Table A1-28. Diaminotriazine compounds of formula (I.A.1), wherein P is O, $R^1$ and $R^2$ together with the carbon atom to which they are attached form cyclobutyl, $R^3$ and $R^{3'}$ are H; and $R^A$, $R^a$, $R^b$, $R^c$ and $R^d$ are defined in table A1-1, particularly compounds of formulae A1-28.1 to A1-28.180.

Table A1-29. Diaminotriazine compounds of formula (I.A.1), wherein P is O, $R^1$ and $R^2$ together with the carbon atom to which they are attached form cyclopentyl, $R^3$ and $R^{3'}$ are H; and $R^A$, $R^a$, $R^b$, $R^c$ and $R^d$ are defined in table A1-1, particularly compounds of formulae A1-29.1 to A1-29.180.

Table A1-30. Diaminotriazine compounds of formula (I.A.1), wherein P is O, $R^1$ and $R^2$ together with the carbon atom to which they are attached form cyclohexyl, $R^3$ and $R^{3'}$ are H; and $R^A$, $R^a$, $R^b$, $R^c$ and $R^d$ are defined in table A1-1, particularly compounds of formulae A1-30.1 to A1-30.180.

Further preferred are diaminotriazine compounds of formula (I.A.2), wherein P is NH, $R^1$ is F; $R^2$, $R^3$ and $R^{3'}$ are H; and $R^A$, $R^a$, $R^b$ and $R^d$ are defined in table A2-1, wherein each line of table A represents one compound of formula (I.A.2), particularly compounds A2-1.1 to A2-1.90,

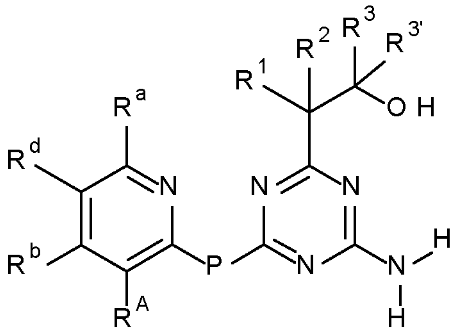

(I.A.2)

Table A2-1.

| no. | $R^A$ | $R^a$ | $R^b$ | $R^d$ |
|---|---|---|---|---|
| A2-1.1 | F | H | H | H |
| A2-1.2 | F | H | H | F |
| A2-1.3 | F | H | H | Cl |
| A2-1.4 | F | H | H | Br |
| A2-1.5 | F | H | H | I |
| A2-1.6 | F | H | F | H |
| A2-1.7 | F | H | F | F |
| A2-1.8 | F | H | F | Cl |
| A2-1.9 | F | H | F | Br |
| A2-1.10 | F | H | F | I |
| A2-1.11 | F | H | Cl | H |
| A2-1.12 | F | H | Cl | F |
| A2-1.13 | F | H | Cl | Cl |
| A2-1.14 | F | H | Cl | Br |
| A2-1.15 | F | H | Cl | I |
| A2-1.16 | F | F | H | H |
| A2-1.17 | F | F | H | F |
| A2-1.18 | F | F | H | Cl |
| A2-1.19 | F | F | H | Br |
| A2-1.20 | F | F | H | I |
| A2-1.21 | F | F | F | H |
| A2-1.22 | F | F | F | F |
| A2-1.23 | F | F | F | Cl |
| A2-1.24 | F | F | F | Br |

| no. | $R^A$ | $R^a$ | $R^b$ | $R^d$ |
|---|---|---|---|---|
| A2-1.25 | F | F | F | I |
| A2-1.26 | F | F | Cl | H |
| A2-1.27 | F | F | Cl | F |
| A2-1.28 | F | F | Cl | Cl |
| A2-1.29 | F | F | Cl | Br |
| A2-1.30 | F | F | Cl | I |
| A2-1.31 | Cl | H | H | H |
| A2-1.32 | Cl | H | H | F |
| A2-1.33 | Cl | H | H | Cl |
| A2-1.34 | Cl | H | H | Br |
| A2-1.35 | Cl | H | H | I |
| A2-1.36 | Cl | H | F | H |
| A2-1.37 | Cl | H | F | F |
| A2-1.38 | Cl | H | F | Cl |
| A2-1.39 | Cl | H | F | Br |
| A2-1.40 | Cl | H | F | I |
| A2-1.41 | Cl | H | Cl | H |
| A2-1.42 | Cl | H | Cl | F |
| A2-1.43 | Cl | H | Cl | Cl |
| A2-1.44 | Cl | H | Cl | Br |
| A2-1.45 | Cl | H | Cl | I |
| A2-1.46 | Cl | F | H | H |
| A2-1.47 | Cl | F | H | F |
| A2-1.48 | Cl | F | H | Cl |

| no. | $R^A$ | $R^a$ | $R^b$ | $R^d$ |
|---|---|---|---|---|
| A2-1.49 | Cl | F | H | Br |
| A2-1.50 | Cl | F | H | I |
| A2-1.51 | Cl | F | F | H |
| A2-1.52 | Cl | F | F | F |
| A2-1.53 | Cl | F | F | Cl |
| A2-1.54 | Cl | F | F | Br |
| A2-1.55 | Cl | F | F | I |
| A2-1.56 | Cl | F | Cl | H |
| A2-1.57 | Cl | F | Cl | F |
| A2-1.58 | Cl | F | Cl | Cl |
| A2-1.59 | Cl | F | Cl | Br |
| A2-1.60 | Cl | F | Cl | I |
| A2-1.61 | CN | H | H | H |
| A2-1.62 | CN | H | H | F |
| A2-1.63 | CN | H | H | Cl |
| A2-1.64 | CN | H | H | Br |
| A2-1.65 | CN | H | H | I |
| A2-1.66 | CN | H | F | H |
| A2-1.67 | CN | H | F | F |
| A2-1.68 | CN | H | F | Cl |
| A2-1.69 | CN | H | F | Br |

| no. | $R^A$ | $R^a$ | $R^b$ | $R^d$ |
|---|---|---|---|---|
| A2-1.70 | CN | H | F | I |
| A2-1.71 | CN | H | Cl | H |
| A2-1.72 | CN | H | Cl | F |
| A2-1.73 | CN | H | Cl | Cl |
| A2-1.74 | CN | H | Cl | Br |
| A2-1.75 | CN | H | Cl | I |
| A2-1.76 | CN | F | H | H |
| A2-1.77 | CN | F | H | F |
| A2-1.78 | CN | F | H | Cl |
| A2-1.79 | CN | F | H | Br |
| A2-1.80 | CN | F | H | I |
| A2-1.81 | CN | F | F | H |
| A2-1.82 | CN | F | F | F |
| A2-1.83 | CN | F | F | Cl |
| A2-1.84 | CN | F | F | Br |
| A2-1.85 | CN | F | F | I |
| A2-1.86 | CN | F | Cl | H |
| A2-1.87 | CN | F | Cl | F |
| A2-1.88 | CN | F | Cl | Cl |
| A2-1.89 | CN | F | Cl | Br |
| A2-1.90 | CN | F | Cl | I |

Also preferred are diaminotriazine compounds exemplified in Tables A2-2 to A2-30.

Table A2-2. Diaminotriazine compounds of formula (I.A.2), wherein P is NH, $R^1$ is F; $R^2$ is $CH_3$, $R^3$ and $R^{3'}$ are H; and $R^A$, $R^a$, $R^b$ and $R^d$ are defined in Table A2-1, particularly compounds of formulae A2-2.1 to A2-2.90.

Table A2-3. Diaminotriazine compounds of formula (I.A.2), wherein P is NH, $R^1$ is H; $R^2$ is $CH_3$, $R^3$ and $R^{3'}$ are H; and $R^A$, $R^a$, $R^b$ and $R^d$ are defined in Table A2-1, particularly compounds of formulae A2-3.1 to A2-3.90.

Table A2-4. Diaminotriazine compounds of formula (I.A.2), wherein P is NH, $R^1$ is $CH_3$; $R^2$ is $CH_3$, $R^3$ and $R^{3'}$ are H; and $R^A$, $R^a$, $R^b$ and $R^d$ are defined in Table A2-1, particularly compounds of formulae A2-4.1 to A2-4.90.

Table A2-5. Diaminotriazine compounds of formula (I.A.2), wherein P is NH, $R^1$ is $CF_3$; $R^2$ is $CH_3$, $R^3$ and $R^{3'}$ are H; and $R^A$, $R^a$, $R^b$ and $R^d$ are defined in Table A2-1, particularly compounds of formulae A2-5.1 to A2-5.90.

Table A2-6. Diaminotriazine compounds of formula (I.A.2), wherein P is NH, $R^1$ is $OCH_3$; $R^2$ is $CH_3$, $R^3$ and $R^{3'}$ are H; and $R^A$, $R^a$, $R^b$ and $R^d$ are defined in Table A2-1, particularly compounds of formulae A2-6.1 to A2-6.90.

Table A2-7. Diaminotriazine compounds of formula (I.A.2), wherein P is NH, $R^1$ is $C_2H_5$; $R^2$ is $CH_3$, $R^3$ and $R^{3'}$ are H; and $R^A$, $R^a$, $R^b$ and $R^d$ are defined in Table A2-1, particularly compounds of formulae A2-7.1 to A2-7.90.

Table A2-8. Diaminotriazine compounds of formula (I.A.2), wherein P is NH, $R^1$ is $C_2H_5$; $R^2$ is $C_2H_5$, $R^3$ and $R^{3'}$ are H; and $R^A$, $R^a$, $R^b$ and $R^d$ are defined in Table A2-1, particularly compounds of formulae A2-8.1 to A2-8.90.

Table A2-9. Diaminotriazine compounds of formula (I.A.2), wherein P is NH, $R^1$, $R^2$ and $R^3$ are $CH_3$, $R^{3'}$ is H; and $R^A$, $R^a$, $R^b$ and $R^d$ are defined in Table A2-1, particularly compounds of formulae A2-9.1 to A2-9.90.

Table A2-10. Diaminotriazine compounds of formula (I.A.2), wherein P is NH, $R^1$ and $R^2$ are $CH_3$, $R^3$ is $CF_3$; $R^{3'}$ is H; and $R^A$, $R^a$, $R^b$ and $R^d$ are defined in Table A2-1, particularly compounds of formulae A2-10.1 to A2-10.90.

Table A2-11. Diaminotriazine compounds of formula (I.A.2), wherein P is NH, $R^1$, $R^2$, $R^3$ and $R^{3'}$ are $CH_3$; and $R^A$, $R^a$, $R^b$ and $R^d$ are defined in table A2-1, particularly compounds of formulae A2-11.1 to A2-11.90.

Table A2-12. Diaminotriazine compounds of formula (I.A.2), wherein P is NH, $R^1$ and $R^2$ together with the carbon

atom to which they are attached form cyclopropyl, $R^3$ and $R^{3'}$ are H; and $R^A$, $R^a$, $R^b$ and $R^d$ are defined in table A2-1, particularly compounds of formulae A2-12.1 to A2-12.90.
Table A2-13. Diaminotriazine compounds of formula (I.A.2), wherein P is NH, $R^1$ and $R^2$ together with the carbon atom to which they are attached form cyclobutyl, $R^3$ and $R^{3'}$ are H; and $R^A$, $R^a$, $R^b$ and $R^d$ are defined in table A2-1, particularly compounds of formulae A2-13.1 to A2-13.90.
Table A2-14. Diaminotriazine compounds of formula (I.A.2), wherein P is NH, $R^1$ and $R^2$ together with the carbon atom to which they are attached form cyclopentyl, $R^3$ and $R^{3'}$ are H; and $R^A$, $R^a$, $R^b$ and $R^d$ are defined in table A2-1, particularly compounds of formulae A2-14.1 to A2-14.90.
Table A2-15. Diaminotriazine compounds of formula (I.A.2), wherein P is NH, $R^1$ and $R^2$ together with the carbon atom to which they are attached form cyclohexyl, $R^3$ and $R^{3'}$ are H; and $R^A$, $R^a$, $R^b$ and $R^d$ are defined in table A2-1, particularly compounds of formulae A2-15.1 to A2-15.90.
Table A2-16. Diaminotriazine compounds of formula (I.A.2), wherein P is O, $R^1$ is F; $R^2$, $R^3$ and $R^{3'}$ are H; and $R^A$, $R^a$, $R^b$ and $R^d$ are defined in table A2-1, particularly compounds of formulae A2-16.1 to A2-16.90.
Table A2-17. Diaminotriazine compounds of formula (I.A.2), wherein P is O, $R^1$ is F; $R^2$ is $CH_3$, $R^3$ and $R^{3'}$ are H; and $R^A$, $R^a$, $R^b$ and $R^d$ are defined in Table A2-1, particularly compounds of formulae A2-17.1 to A2-17.90.
Table A2-18. Diaminotriazine compounds of formula (I.A.2), wherein P is O, $R^1$ is H; $R^2$ is $CH_3$, $R^3$ and $R^{3'}$ are H; and $R^A$, $R^a$, $R^b$ and $R^d$ are defined in Table A2-1, particularly compounds of formulae A2-18.1 to A2-18.90.
Table A2-19. Diaminotriazine compounds of formula (I.A.2), wherein P is O, $R^1$ is $CH_3$; $R^2$ is $CH_3$, $R^3$ and $R^{3'}$ are H; and $R^A$, $R^a$, $R^b$ and $R^d$ are defined in Table A2-1, particularly compounds of formulae A2-19.1 to A2-19.90.
Table A2-20. Diaminotriazine compounds of formula (I.A.2), wherein P is O, $R^1$ is $CF_3$; $R^2$ is $CH_3$, $R^3$ and $R^{3'}$ are H; and $R^A$, $R^a$, $R^b$ and $R^d$ are defined in Table A2-1, particularly compounds of formulae A2-20.1 to A2-20.90.
Table A2-21. Diaminotriazine compounds of formula (I.A.2), wherein P is O, $R^1$ is $OCH_3$; $R^2$ is $CH_3$, $R^3$ and $R^{3'}$ are H; and $R^A$, $R^a$, $R^b$ and $R^d$ are defined in Table A2-1, particularly compounds of formulae A2-21.1 to A2-21.90.
Table A2-22. Diaminotriazine compounds of formula (I.A.2), wherein P is O, $R^1$ is $C_2H_5$; $R^2$ is $CH_3$, $R^3$ and $R^{3'}$ are H; and $R^A$, $R^a$, $R^b$ and $R^d$ are defined in Table A2-1, particularly compounds of formulae A2-22.1 to A2-22.90.
Table A2-23. Diaminotriazine compounds of formula (I.A.2), wherein P is O, $R^1$ is $C_2H_5$; $R^2$ is $C_2H_5$, $R^3$ and $R^{3'}$ are H; and $R^A$, $R^a$, $R^b$ and $R^d$ are defined in Table A2-1, particularly compounds of formulae A2-23.1 to A2-23.90.
Table A2-24. Diaminotriazine compounds of formula (I.A.2), wherein P is O, $R^1$, $R^2$ and $R^3$ are $CH_3$, $R^{3'}$ is H; and $R^A$, $R^a$, $R^b$ and $R^d$ are defined in Table A2-1, particularly compounds of formulae A2-24.1 to A2-24.90.
Table A2-25. Diaminotriazine compounds of formula (I.A.2), wherein P is O, $R^1$ and $R^2$ are $CH_3$, $R^3$ is $CF_3$; $R^{3'}$ is H; and $R^A$, $R^a$, $R^b$ and $R^d$ are defined in Table A2-1, particularly compounds of formulae A2-25.1 to A2-25.90.
Table A2-26. Diaminotriazine compounds of formula (I.A.2), wherein P is O, $R^1$, $R^2$, $R^3$ and $R^{3'}$ are $CH_3$; and $R^A$, $R^a$, $R^b$ and $R^d$ are defined in table A2-1, particularly compounds of formulae A2-26.1 to A2-26.90.
Table A2-27. Diaminotriazine compounds of formula (I.A.2), wherein P is O, $R^1$ and $R^2$ together with the carbon atom to which they are attached form cyclopropyl, $R^3$ and $R^{3'}$ are H; and $R^A$, $R^a$, $R^b$ and $R^d$ are defined in table A2-1, particularly compounds of formulae A2-27.1 to A2-27.90.
Table A2-28. Diaminotriazine compounds of formula (I.A.2), wherein P is O, $R^1$ and $R^2$ together with the carbon atom to which they are attached form cyclobutyl, $R^3$ and $R^{3'}$ are H; and $R^A$, $R^a$, $R^b$ and $R^d$ are defined in table A2-1, particularly compounds of formulae A2-28.1 to A2-28.90.
Table A2-29. Diaminotriazine compounds of formula (I.A.2), wherein P is O, $R^1$ and $R^2$ together with the carbon atom to which they are attached form cyclopentyl, $R^3$ and $R^{3'}$ are H; and $R^A$, $R^a$, $R^b$ and $R^d$ are defined in table A2-1, particularly compounds of formulae A2-29.1 to A2-29.90.
Table A2-30. Diaminotriazine compounds of formula (I.A.2), wherein P is O, $R^1$ and $R^2$ together with the carbon atom to which they are attached form cyclohexyl, $R^3$ and $R^{3'}$ are H; and $R^A$, $R^a$, $R^b$ and $R^d$ are defined in table A2-1, particularly compounds of formulae A2-30.1 to A2-30.90.

Also preferred are diaminotriazine compounds of formula (I.A.3), wherein $R^1$ is F; $R^2$, $R^3$ and $R^{3'}$ are H; and $R^A$, $R^a$, $R^b$, $R^c$ and $R^d$, are defined in table A3-1, wherein each line of table A represents one compound of formula (I.A.3), particularly compounds A3-1.1 to A3-1.36,

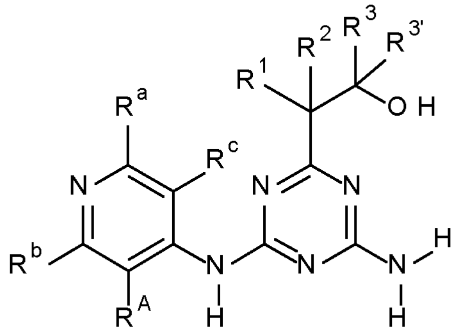

(I.A.3)

Table A3-1.

| no. | $R^A$ | $R^a$ | $R^b$ | $R^c$ |
|---|---|---|---|---|
| A3-1.1 | F | H | H | F |
| A3-1.2 | F | H | H | CN |
| A3-1.3 | F | H | F | F |
| A3-1.4 | F | H | F | CN |
| A3-1.5 | F | H | Cl | F |
| A3-1.6 | F | H | Cl | CN |
| A3-1.7 | F | F | H | F |
| A3-1.8 | F | F | H | CN |
| A3-1.9 | F | F | F | F |
| A3-1.10 | F | F | F | CN |
| A3-1.11 | F | F | Cl | F |
| A3-1.12 | F | F | Cl | CN |
| A3-1.13 | Cl | H | H | F |
| A3-1.14 | Cl | H | H | CN |
| A3-1.15 | Cl | H | F | F |
| A3-1.16 | Cl | H | F | CN |
| A3-1.17 | Cl | H | Cl | F |
| A3-1.18 | Cl | H | Cl | CN |
| A3-1.19 | Cl | F | H | F |
| A3-1.20 | Cl | F | H | CN |
| A3-1.21 | Cl | F | F | F |
| A3-1.22 | Cl | F | F | CN |
| A3-1.23 | Cl | F | Cl | F |
| A3-1.24 | Cl | F | Cl | CN |
| A3-1.25 | CN | H | H | F |
| A3-1.26 | CN | H | H | CN |
| A3-1.27 | CN | H | F | F |
| A3-1.28 | CN | H | F | CN |
| A3-1.29 | CN | H | Cl | F |

(continued)

| no. | $R^A$ | $R^a$ | $R^b$ | $R^c$ |
|---|---|---|---|---|
| A3-1.30 | CN | H | Cl | CN |
| A3-1.31 | CN | F | H | F |
| A3-1.32 | CN | F | H | CN |
| A3-1.33 | CN | F | F | F |
| A3-1.34 | CN | F | F | CN |
| A3-1.35 | CN | F | Cl | F |
| A3-1.36 | CN | F | Cl | CN |

Also preferred are diaminotriazine compounds exemplified in Tables A3-2 to A3-15.

Table A3-2. Diaminotriazine compounds of formula (I.A.3), wherein $R^1$ is F; $R^2$ is $CH_3$, $R^3$ and $R^{3'}$ are H; and $R^A$, $R^a$, $R^b$ and $R^c$ are defined in Table A3-1, particularly compounds of formulae A3-2.1 to A3-2.36.

Table A3-3. Diaminotriazine compounds of formula (I.A.3), wherein $R^1$ is H; $R^2$ is $CH_3$, $R^3$ and $R^{3'}$ are H; and $R^A$, $R^a$, $R^b$ and $R^c$ are defined in Table A3-1, particularly compounds of formulae A3-3.1 to A3-3.36.

Table A3-4. Diaminotriazine compounds of formula (I.A.3), wherein $R^1$ is $CH_3$; $R^2$ is $CH_3$, $R^3$ and $R^{3'}$ are H; and $R^A$, $R^a$, $R^b$ and $R^c$ are defined in Table A3-1, particularly compounds of formulae A3-4.1 to A3-4.36.

Table A3-5. Diaminotriazine compounds of formula (I.A.3), wherein $R^1$ is $CF_3$; $R^2$ is $CH_3$, $R^3$ and $R^{3'}$ are H; and $R^A$, $R^a$, $R^b$ and $R^c$ are defined in Table A3-1, particularly compounds of formulae A3-5.1 to A3-5.36.

Table A3-6. Diaminotriazine compounds of formula (I.A.3), wherein $R^1$ is $OCH_3$; $R^2$ is $CH_3$, $R^3$ and $R^{3'}$ are H; and $R^A$, $R^a$, $R^b$ and $R^c$ are defined in Table A3-1, particularly compounds of formulae A3-6.1 to A3-6.36.

Table A3-7. Diaminotriazine compounds of formula (I.A.3), wherein $R^1$ is $C_2H_5$; $R^2$ is $CH_3$, $R^3$ and $R^{3'}$ are H; and $R^A$, $R^a$, $R^b$ and $R^c$ are defined in Table A3-1, particularly compounds of formulae A3-7.1 to A3-7.36.

Table A3-8. Diaminotriazine compounds of formula (I.A.3), wherein $R^1$ is $C_2H_5$; $R^2$ is $C_2H_5$, $R^3$ and $R^{3'}$ are H; and $R^A$, $R^a$, $R^b$ and $R^c$ are defined in Table A3-1, particularly compounds of formulae A3-8.1 to A3-8.36.

Table A3-9. Diaminotriazine compounds of formula (I.A.3), wherein $R^1$, $R^2$ and $R^3$ are $CH_3$, $R^{3'}$ is H; and $R^A$, $R^a$, $R^b$ and $R^c$ are defined in Table A3-1, particularly compounds of formulae A3-9.1 to A3-9.36.

Table A3-10. Diaminotriazine compounds of formula (I.A.3), wherein $R^1$ and $R^2$ are $CH_3$, $R^3$ is $CF_3$; $R^{3'}$ is H; and $R^A$, $R^a$, $R^b$ and $R^c$ are defined in Table A3-1, particularly compounds of formulae A3-10.1 to A3-10.36.

Table A3-11. Diaminotriazine compounds of formula (I.A.3), wherein $R^1$, $R^2$, $R^3$ and $R^{3'}$ are $CH_3$; and $R^A$, $R^a$, $R^b$ and $R^c$ are defined in table A3-1, particularly compounds of formulae A3-11.1 to A3-11.36.

Table A3-12. Diaminotriazine compounds of formula (I.A.3), wherein $R^1$ and $R^2$ together with the carbon atom to which they are attached form cyclopropyl, $R^3$ and $R^{3'}$ are H; and $R^A$, $R^a$, $R^b$ and $R^c$ are defined in table A3-1, particularly compounds of formulae A3-12.1 to A3-12.36.

Table A3-13. Diaminotriazine compounds of formula (I.A.3), wherein $R^1$ and $R^2$ together with the carbon atom to which they are attached form cyclobutyl, $R^3$ and $R^{3'}$ are H; and $R^A$, $R^a$, $R^b$ and $R^c$ are defined in table A3-1, particularly compounds of formulae A3-13.1 to A3-13.36.

Table A3-14. Diaminotriazine compounds of formula (I.A.3), wherein $R^1$ and $R^2$ together with the carbon atom to which they are attached form cyclopentyl, $R^3$ and $R^{3'}$ are H; and $R^A$, $R^a$, $R^b$ and $R^c$ are defined in table A3-1, particularly compounds of formulae A3-14.1 to A3-14.36.

Table A3-15. Diaminotriazine compounds of formula (I.A.3), wherein $R^1$ and $R^2$ together with the carbon atom to which they are attached form cyclohexyl, $R^3$ and $R^{3'}$ are H; and $R^A$, $R^a$, $R^b$ and $R^c$ are defined in table A3-1, particularly compounds of formulae A3-15.1 to A3-15.36.

Also preferred are diaminotriazine compounds of formula (I.A.4), wherein $R^1$ is F; $R^2$, $R^3$ and $R^{3'}$ are H; and $R^A$, $R^a$, $R^b$, $R^d$, $R^{e1}$ and $R^{e2}$ are defined in table A4-1, wherein each line of table A represents one compound of formula (I.A.4), particularly compounds A4-1.1 to A4-1.288,

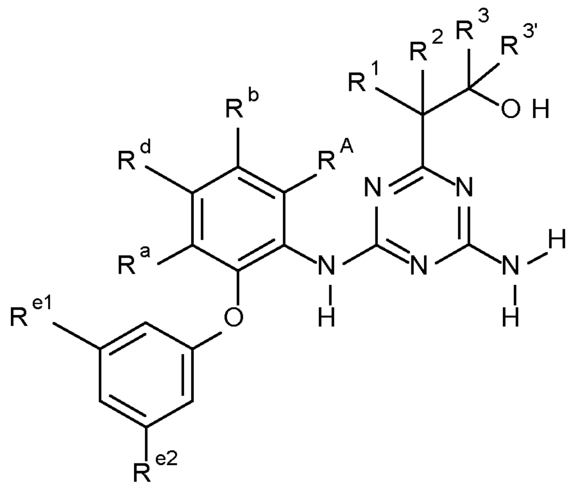

(I.A.4)

Table A4-1.

| no. | $R^A$ | $R^a$ | $R^b$ | $R^d$ | $R^{e1}$ | $R^{e2}$ |
|---|---|---|---|---|---|---|
| A4-1.1 | F | H | H | H | H | Cl |
| A4-1.2 | F | H | H | H | H | $CH_3$ |
| A4-1.3 | F | H | H | H | H | $CF_3$ |
| A4-1.4 | F | H | H | H | Cl | Cl |
| A4-1.5 | F | H | H | H | Cl | $CH_3$ |
| A4-1.6 | F | H | H | H | Cl | $CF_3$ |
| A4-1.7 | F | H | H | H | $CH_3$ | Cl |
| A4-1.8 | F | H | H | H | $CH_3$ | $CH_3$ |
| A4-1.9 | F | H | H | H | $CH_3$ | $CF_3$ |
| A4-1.10 | F | H | H | H | $CF_3$ | Cl |
| A4-1.11 | F | H | H | H | $CF_3$ | $CH_3$ |
| A4-1.12 | F | H | H | H | $CF_3$ | $CF_3$ |
| A4-1.13 | F | H | H | F | H | Cl |
| A4-1.14 | F | H | H | F | H | $CH_3$ |
| A4-1.15 | F | H | H | F | H | $CF_3$ |
| A4-1.16 | F | H | H | F | Cl | Cl |
| A4-1.17 | F | H | H | F | Cl | $CH_3$ |
| A4-1.18 | F | H | H | F | Cl | $CF_3$ |
| A4-1.19 | F | H | H | F | $CH_3$ | Cl |
| A4-1.20 | F | H | H | F | $CH_3$ | $CH_3$ |
| A4-1.21 | F | H | H | F | $CH_3$ | $CF_3$ |
| A4-1.22 | F | H | H | F | $CF_3$ | Cl |

| no. | $R^A$ | $R^a$ | $R^b$ | $R^d$ | $R^{e1}$ | $R^{e2}$ |
|---|---|---|---|---|---|---|
| A4-1.23 | F | H | H | F | $CF_3$ | $CH_3$ |
| A4-1.24 | F | H | H | F | $CF_3$ | $CF_3$ |
| A4-1.25 | F | H | F | H | H | Cl |
| A4-1.26 | F | H | F | H | H | $CH_3$ |
| A4-1.27 | F | H | F | H | H | $CF_3$ |
| A4-1.28 | F | H | F | H | Cl | Cl |
| A4-1.29 | F | H | F | H | Cl | $CH_3$ |
| A4-1.30 | F | H | F | H | Cl | $CF_3$ |
| A4-1.31 | F | H | F | H | $CH_3$ | Cl |
| A4-1.32 | F | H | F | H | $CH_3$ | $CH_3$ |
| A4-1.33 | F | H | F | H | $CH_3$ | $CF_3$ |
| A4-1.34 | F | H | F | H | $CF_3$ | Cl |
| A4-1.35 | F | H | F | H | $CF_3$ | $CH_3$ |
| A4-1.36 | F | H | F | H | $CF_3$ | $CF_3$ |
| A4-1.37 | F | H | F | F | H | Cl |
| A4-1.38 | F | H | F | F | H | $CH_3$ |
| A4-1.39 | F | H | F | F | H | $CF_3$ |
| A4-1.40 | F | H | F | F | Cl | Cl |
| A4-1.41 | F | H | F | F | Cl | $CH_3$ |
| A4-1.42 | F | H | F | F | Cl | $CF_3$ |
| A4-1.43 | F | H | F | F | $CH_3$ | Cl |
| A4-1.44 | F | H | F | F | $CH_3$ | $CH_3$ |

| no. | $R^A$ | $R^a$ | $R^b$ | $R^d$ | $R^{e1}$ | $R^{e2}$ |
|---|---|---|---|---|---|---|
| A4-1.45 | F | H | F | F | $CH_3$ | $CF_3$ |
| A4-1.46 | F | H | F | F | $CF_3$ | Cl |
| A4-1.47 | F | H | F | F | $CF_3$ | $CH_3$ |
| A4-1.48 | F | H | F | F | $CF_3$ | $CF_3$ |
| A4-1.49 | F | F | H | H | H | Cl |
| A4-1.50 | F | F | H | H | H | $CH_3$ |
| A4-1.51 | F | F | H | H | H | $CF_3$ |
| A4-1.52 | F | F | H | H | Cl | Cl |
| A4-1.53 | F | F | H | H | Cl | $CH_3$ |
| A4-1.54 | F | F | H | H | Cl | $CF_3$ |
| A4-1.55 | F | F | H | H | $CH_3$ | Cl |
| A4-1.56 | F | F | H | H | $CH_3$ | $CH_3$ |
| A4-1.57 | F | F | H | H | $CH_3$ | $CF_3$ |
| A4-1.58 | F | F | H | H | $CF_3$ | Cl |
| A4-1.59 | F | F | H | H | $CF_3$ | $CH_3$ |
| A4-1.60 | F | F | H | H | $CF_3$ | $CF_3$ |
| A4-1.61 | F | F | H | F | H | Cl |
| A4-1.62 | F | F | H | F | H | $CH_3$ |
| A4-1.63 | F | F | H | F | H | $CF_3$ |
| A4-1.64 | F | F | H | F | Cl | Cl |
| A4-1.65 | F | F | H | F | Cl | $CH_3$ |
| A4-1.66 | F | F | H | F | Cl | $CF_3$ |
| A4-1.67 | F | F | H | F | $CH_3$ | Cl |
| A4-1.68 | F | F | H | F | $CH_3$ | $CH_3$ |
| A4-1.69 | F | F | H | F | $CH_3$ | $CF_3$ |
| A4-1.70 | F | F | H | F | $CF_3$ | Cl |
| A4-1.71 | F | F | H | F | $CF_3$ | $CH_3$ |
| A4-1.72 | F | F | H | F | $CF_3$ | $CF_3$ |
| A4-1.73 | F | F | F | H | H | Cl |
| A4-1.74 | F | F | F | H | H | $CH_3$ |
| A4-1.75 | F | F | F | H | H | $CF_3$ |
| A4-1.76 | F | F | F | H | Cl | Cl |
| A4-1.77 | F | F | F | H | Cl | $CH_3$ |
| A4-1.78 | F | F | F | H | Cl | $CF_3$ |
| A4-1.79 | F | F | F | H | $CH_3$ | Cl |
| A4-1.80 | F | F | F | H | $CH_3$ | $CH_3$ |
| A4-1.81 | F | F | F | H | $CH_3$ | $CF_3$ |
| A4-1.82 | F | F | F | H | $CF_3$ | Cl |
| A4-1.83 | F | F | F | H | $CF_3$ | $CH_3$ |

| no. | $R^A$ | $R^a$ | $R^b$ | $R^d$ | $R^{e1}$ | $R^{e2}$ |
|---|---|---|---|---|---|---|
| A4-1.84 | F | F | F | H | $CF_3$ | $CF_3$ |
| A4-1.85 | F | F | F | F | H | Cl |
| A4-1.86 | F | F | F | F | H | $CH_3$ |
| A4-1.87 | F | F | F | F | H | $CF_3$ |
| A4-1.88 | F | F | F | F | Cl | Cl |
| A4-1.89 | F | F | F | F | Cl | $CH_3$ |
| A4-1.90 | F | F | F | F | Cl | $CF_3$ |
| A4-1.91 | F | F | F | F | $CH_3$ | Cl |
| A4-1.92 | F | F | F | F | $CH_3$ | $CH_3$ |
| A4-1.93 | F | F | F | F | $CH_3$ | $CF_3$ |
| A4-1.94 | F | F | F | F | $CF_3$ | Cl |
| A4-1.95 | F | F | F | F | $CF_3$ | $CH_3$ |
| A4-1.96 | F | F | F | F | $CF_3$ | $CF_3$ |
| A4-1.97 | Cl | H | H | H | H | Cl |
| A4-1.98 | Cl | H | H | H | H | $CH_3$ |
| A4-1.99 | Cl | H | H | H | H | $CF_3$ |
| A4-1.100 | Cl | H | H | H | Cl | Cl |
| A4-1.101 | Cl | H | H | H | Cl | $CH_3$ |
| A4-1.102 | Cl | H | H | H | Cl | $CF_3$ |
| A4-1.103 | Cl | H | H | H | $CH_3$ | Cl |
| A4-1.104 | Cl | H | H | H | $CH_3$ | $CH_3$ |
| A4-1.105 | Cl | H | H | H | $CH_3$ | $CF_3$ |
| A4-1.106 | Cl | H | H | H | $CF_3$ | Cl |
| A4-1.107 | Cl | H | H | H | $CF_3$ | $CH_3$ |
| A4-1.108 | Cl | H | H | H | $CF_3$ | $CF_3$ |
| A4-1.109 | Cl | H | H | F | H | Cl |
| A4-1.110 | Cl | H | H | F | H | $CH_3$ |
| A4-1.111 | Cl | H | H | F | H | $CF_3$ |
| A4-1.112 | Cl | H | H | F | Cl | Cl |
| A4-1.113 | Cl | H | H | F | Cl | $CH_3$ |
| A4-1.114 | Cl | H | H | F | Cl | $CF_3$ |
| A4-1.115 | Cl | H | H | F | $CH_3$ | Cl |
| A4-1.116 | Cl | H | H | F | $CH_3$ | $CH_3$ |
| A4-1.117 | Cl | H | H | F | $CH_3$ | $CF_3$ |
| A4-1.118 | Cl | H | H | F | $CF_3$ | Cl |
| A4-1.119 | Cl | H | H | F | $CF_3$ | $CH_3$ |
| A4-1.120 | Cl | H | H | F | $CF_3$ | $CF_3$ |
| A4-1.121 | Cl | H | F | H | H | Cl |
| A4-1.122 | Cl | H | F | H | H | $CH_3$ |

| no. | $R^A$ | $R^a$ | $R^b$ | $R^d$ | $R^{e1}$ | $R^{e2}$ |
|---|---|---|---|---|---|---|
| A4-1.123 | Cl | H | F | H | H | $CF_3$ |
| A4-1.124 | Cl | H | F | H | Cl | Cl |
| A4-1.125 | Cl | H | F | H | Cl | $CH_3$ |
| A4-1.126 | Cl | H | F | H | Cl | $CF_3$ |
| A4-1.127 | Cl | H | F | H | $CH_3$ | Cl |
| A4-1.128 | Cl | H | F | H | $CH_3$ | $CH_3$ |
| A4-1.129 | Cl | H | F | H | $CH_3$ | $CF_3$ |
| A4-1.130 | Cl | H | F | H | $CF_3$ | Cl |
| A4-1.131 | Cl | H | F | H | $CF_3$ | $CH_3$ |
| A4-1.132 | Cl | H | F | H | $CF_3$ | $CF_3$ |
| A4-1.133 | Cl | H | F | F | H | Cl |
| A4-1.134 | Cl | H | F | F | H | $CH_3$ |
| A4-1.135 | Cl | H | F | F | H | $CF_3$ |
| A4-1.136 | Cl | H | F | F | Cl | Cl |
| A4-1.137 | Cl | H | F | F | Cl | $CH_3$ |
| A4-1.138 | Cl | H | F | F | Cl | $CF_3$ |
| A4-1.139 | Cl | H | F | F | $CH_3$ | Cl |
| A4-1.140 | Cl | H | F | F | $CH_3$ | $CH_3$ |
| A4-1.141 | Cl | H | F | F | $CH_3$ | $CF_3$ |
| A4-1.142 | Cl | H | F | F | $CF_3$ | Cl |
| A4-1.143 | Cl | H | F | F | $CF_3$ | $CH_3$ |
| A4-1.144 | Cl | H | F | F | $CF_3$ | $CF_3$ |
| A4-1.145 | Cl | F | H | H | H | Cl |
| A4-1.146 | Cl | F | H | H | H | $CH_3$ |
| A4-1.147 | Cl | F | H | H | H | $CF_3$ |
| A4-1.148 | Cl | F | H | H | Cl | Cl |
| A4-1.149 | Cl | F | H | H | Cl | $CH_3$ |
| A4-1.150 | Cl | F | H | H | Cl | $CF_3$ |
| A4-1.151 | Cl | F | H | H | $CH_3$ | Cl |
| A4-1.152 | Cl | F | H | H | $CH_3$ | $CH_3$ |
| A4-1.153 | Cl | F | H | H | $CH_3$ | $CF_3$ |
| A4-1.154 | Cl | F | H | H | $CF_3$ | Cl |
| A4-1.155 | Cl | F | H | H | $CF_3$ | $CH_3$ |
| A4-1.156 | Cl | F | H | H | $CF_3$ | $CF_3$ |
| A4-1.157 | Cl | F | H | F | H | Cl |
| A4-1.158 | Cl | F | H | F | H | $CH_3$ |
| A4-1.159 | Cl | F | H | F | H | $CF_3$ |
| A4-1.160 | Cl | F | H | F | Cl | Cl |
| A4-1.161 | Cl | F | H | F | Cl | $CH_3$ |
| A4-1.162 | Cl | F | H | F | Cl | $CF_3$ |
| A4-1.163 | Cl | F | H | F | $CH_3$ | Cl |
| A4-1.164 | Cl | F | H | F | $CH_3$ | $CH_3$ |
| A4-1.165 | Cl | F | H | F | $CH_3$ | $CF_3$ |
| A4-1.166 | Cl | F | H | F | $CF_3$ | Cl |
| A4-1.167 | Cl | F | H | F | $CF_3$ | $CH_3$ |
| A4-1.168 | Cl | F | H | F | $CF_3$ | $CF_3$ |
| A4-1.169 | Cl | F | F | H | H | Cl |
| A4-1.170 | Cl | F | F | H | H | $CH_3$ |
| A4-1.171 | Cl | F | F | H | H | $CF_3$ |
| A4-1.172 | Cl | F | F | H | Cl | Cl |
| A4-1.173 | Cl | F | F | H | Cl | $CH_3$ |
| A4-1.174 | Cl | F | F | H | Cl | $CF_3$ |
| A4-1.175 | Cl | F | F | H | $CH_3$ | Cl |
| A4-1.176 | Cl | F | F | H | $CH_3$ | $CH_3$ |
| A4-1.177 | Cl | F | F | H | $CH_3$ | $CF_3$ |
| A4-1.178 | Cl | F | F | H | $CF_3$ | Cl |
| A4-1.179 | Cl | F | F | H | $CF_3$ | $CH_3$ |
| A4-1.180 | Cl | F | F | H | $CF_3$ | $CF_3$ |
| A4-1.181 | Cl | F | F | F | H | Cl |
| A4-1.182 | Cl | F | F | F | H | $CH_3$ |
| A4-1.183 | Cl | F | F | F | H | $CF_3$ |
| A4-1.184 | Cl | F | F | F | Cl | Cl |
| A4-1.185 | Cl | F | F | F | Cl | $CH_3$ |
| A4-1.186 | Cl | F | F | F | Cl | $CF_3$ |
| A4-1.187 | Cl | F | F | F | $CH_3$ | Cl |
| A4-1.188 | Cl | F | F | F | $CH_3$ | $CH_3$ |
| A4-1.189 | Cl | F | F | F | $CH_3$ | $CF_3$ |
| A4-1.190 | Cl | F | F | F | $CF_3$ | Cl |
| A4-1.191 | Cl | F | F | F | $CF_3$ | $CH_3$ |
| A4-1.192 | Cl | F | F | F | $CF_3$ | $CF_3$ |
| A4-1.193 | CN | H | H | H | H | Cl |
| A4-1.194 | CN | H | H | H | H | $CH_3$ |
| A4-1.195 | CN | H | H | H | H | $CF_3$ |
| A4-1.196 | CN | H | H | H | Cl | Cl |
| A4-1.197 | CN | H | H | H | Cl | $CH_3$ |
| A4-1.198 | CN | H | H | H | Cl | $CF_3$ |
| A4-1.199 | CN | H | H | H | $CH_3$ | Cl |
| A4-1.200 | CN | H | H | H | $CH_3$ | $CH_3$ |

| no. | $R^A$ | $R^a$ | $R^b$ | $R^d$ | $R^{e1}$ | $R^{e2}$ |
|---|---|---|---|---|---|---|
| A4-1.201 | CN | H | H | H | $CH_3$ | $CF_3$ |
| A4-1.202 | CN | H | H | H | $CF_3$ | Cl |
| A4-1.203 | CN | H | H | H | $CF_3$ | $CH_3$ |
| A4-1.204 | CN | H | H | H | $CF_3$ | $CF_3$ |
| A4-1.205 | CN | H | H | F | H | Cl |
| A4-1.206 | CN | H | H | F | H | $CH_3$ |
| A4-1.207 | CN | H | H | F | H | $CF_3$ |
| A4-1.208 | CN | H | H | F | Cl | Cl |
| A4-1.209 | CN | H | H | F | Cl | $CH_3$ |
| A4-1.210 | CN | H | H | F | Cl | $CF_3$ |
| A4-1.211 | CN | H | H | F | $CH_3$ | Cl |
| A4-1.212 | CN | H | H | F | $CH_3$ | $CH_3$ |
| A4-1.213 | CN | H | H | F | $CH_3$ | $CF_3$ |
| A4-1.214 | CN | H | H | F | $CF_3$ | Cl |
| A4-1.215 | CN | H | H | F | $CF_3$ | $CH_3$ |
| A4-1.216 | CN | H | H | F | $CF_3$ | $CF_3$ |
| A4-1.217 | CN | H | F | H | H | Cl |
| A4-1.218 | CN | H | F | H | H | $CH_3$ |
| A4-1.219 | CN | H | F | H | H | $CF_3$ |
| A4-1.220 | CN | H | F | H | Cl | Cl |
| A4-1.221 | CN | H | F | H | Cl | $CH_3$ |
| A4-1.222 | CN | H | F | H | Cl | $CF_3$ |
| A4-1.223 | CN | H | F | H | $CH_3$ | Cl |
| A4-1.224 | CN | H | F | H | $CH_3$ | $CH_3$ |
| A4-1.225 | CN | H | F | H | $CH_3$ | $CF_3$ |
| A4-1.226 | CN | H | F | H | $CF_3$ | Cl |
| A4-1.227 | CN | H | F | H | $CF_3$ | $CH_3$ |
| A4-1.228 | CN | H | F | H | $CF_3$ | $CF_3$ |
| A4-1.229 | CN | H | F | F | H | Cl |
| A4-1.230 | CN | H | F | F | H | $CH_3$ |
| A4-1.231 | CN | H | F | F | H | $CF_3$ |
| A4-1.232 | CN | H | F | F | Cl | Cl |
| A4-1.233 | CN | H | F | F | Cl | $CH_3$ |
| A4-1.234 | CN | H | F | F | Cl | $CF_3$ |
| A4-1.235 | CN | H | F | F | $CH_3$ | Cl |
| A4-1.236 | CN | H | F | F | $CH_3$ | $CH_3$ |
| A4-1.237 | CN | H | F | F | $CH_3$ | $CF_3$ |
| A4-1.238 | CN | H | F | F | $CF_3$ | Cl |
| A4-1.239 | CN | H | F | F | $CF_3$ | $CH_3$ |

| no. | $R^A$ | $R^a$ | $R^b$ | $R^d$ | $R^{e1}$ | $R^{e2}$ |
|---|---|---|---|---|---|---|
| A4-1.240 | CN | H | F | F | $CF_3$ | $CF_3$ |
| A4-1.241 | CN | F | H | H | H | Cl |
| A4-1.242 | CN | F | H | H | H | $CH_3$ |
| A4-1.243 | CN | F | H | H | H | $CF_3$ |
| A4-1.244 | CN | F | H | H | Cl | Cl |
| A4-1.245 | CN | F | H | H | Cl | $CH_3$ |
| A4-1.246 | CN | F | H | H | Cl | $CF_3$ |
| A4-1.247 | CN | F | H | H | $CH_3$ | Cl |
| A4-1.248 | CN | F | H | H | $CH_3$ | $CH_3$ |
| A4-1.249 | CN | F | H | H | $CH_3$ | $CF_3$ |
| A4-1.250 | CN | F | H | H | $CF_3$ | Cl |
| A4-1.251 | CN | F | H | H | $CF_3$ | $CH_3$ |
| A4-1.252 | CN | F | H | H | $CF_3$ | $CF_3$ |
| A4-1.253 | CN | F | H | F | H | Cl |
| A4-1.254 | CN | F | H | F | H | $CH_3$ |
| A4-1.255 | CN | F | H | F | H | $CF_3$ |
| A4-1.256 | CN | F | H | F | Cl | Cl |
| A4-1.257 | CN | F | H | F | Cl | $CH_3$ |
| A4-1.258 | CN | F | H | F | Cl | $CF_3$ |
| A4-1.259 | CN | F | H | F | $CH_3$ | Cl |
| A4-1.260 | CN | F | H | F | $CH_3$ | $CH_3$ |
| A4-1.261 | CN | F | H | F | $CH_3$ | $CF_3$ |
| A4-1.262 | CN | F | H | F | $CF_3$ | Cl |
| A4-1.263 | CN | F | H | F | $CF_3$ | $CH_3$ |
| A4-1.264 | CN | F | H | F | $CF_3$ | $CF_3$ |
| A4-1.265 | CN | F | F | H | H | Cl |
| A4-1.266 | CN | F | F | H | H | $CH_3$ |
| A4-1.267 | CN | F | F | H | H | $CF_3$ |
| A4-1.268 | CN | F | F | H | Cl | Cl |
| A4-1.269 | CN | F | F | H | Cl | $CH_3$ |
| A4-1.270 | CN | F | F | H | Cl | $CF_3$ |
| A4-1.271 | CN | F | F | H | $CH_3$ | Cl |
| A4-1.272 | CN | F | F | H | $CH_3$ | $CH_3$ |
| A4-1.273 | CN | F | F | H | $CH_3$ | $CF_3$ |
| A4-1.274 | CN | F | F | H | $CF_3$ | Cl |
| A4-1.275 | CN | F | F | H | $CF_3$ | $CH_3$ |
| A4-1.276 | CN | F | F | H | $CF_3$ | $CF_3$ |
| A4-1.277 | CN | F | F | F | H | Cl |
| A4-1.278 | CN | F | F | F | H | $CH_3$ |

| no. | $R^A$ | $R^a$ | $R^b$ | $R^d$ | $R^{e1}$ | $R^{e2}$ |
|---|---|---|---|---|---|---|
| A4-1.279 | CN | F | F | F | H | $CF_3$ |
| A4-1.280 | CN | F | F | F | Cl | Cl |
| A4-1.281 | CN | F | F | F | Cl | $CH_3$ |
| A4-1.282 | CN | F | F | F | Cl | $CF_3$ |
| A4-1.283 | CN | F | F | F | $CH_3$ | Cl |
| A4-1.284 | CN | F | F | F | $CH_3$ | $CH_3$ |
| A4-1.285 | CN | F | F | F | $CH_3$ | $CF_3$ |
| A4-1.286 | CN | F | F | F | $CF_3$ | Cl |
| A4-1.287 | CN | F | F | F | $CF_3$ | $CH_3$ |
| A4-1.288 | CN | F | F | F | $CF_3$ | $CF_3$ |

Also preferred are diaminotriazine compounds exemplified in Tables A4-2 to A4-15.

Table A4-2. Diaminotriazine compounds of formula (I.A.4), wherein $R^1$ is F; $R^2$ is $CH_3$, $R^3$ and $R^{3'}$ are H; and $R^A$, $R^a$, $R^b$, $R^d$, $R^{e1}$ and $R^{e2}$ are defined in Table A4-1, particularly compounds of formulae A4-2.1 to A4-2.288.

Table A4-3. Diaminotriazine compounds of formula (I.A.4), wherein $R^1$ is H; $R^2$ is $CH_3$, $R^3$ and $R^{3'}$ are H; and $R^A$, $R^a$, $R^b$, $R^d$, $R^{e1}$ and $R^{e2}$ are defined in Table A4-1, particularly compounds of formulae A4-3.1 to A4-3.288.

Table A4-4. Diaminotriazine compounds of formula (I.A.4), wherein $R^1$ is $CH_3$; $R^2$ is $CH_3$, $R^3$ and $R^{3'}$ are H; and $R^A$, $R^a$, $R^b$, $R^d$, $R^{e1}$ and $R^{e2}$ are defined in Table A4-1, particularly compounds of formulae A4-4.1 to A4-4.288.

Table A4-5. Diaminotriazine compounds of formula (I.A.4), wherein $R^1$ is $CF_3$; $R^2$ is $CH_3$, $R^3$ and $R^{3'}$ are H; and $R^A$, $R^a$, $R^b$, $R^d$, $R^{e1}$ and $R^{e2}$ are defined in Table A4-1, particularly compounds of formulae A4-5.1 to A4-5.288.

Table A4-6. Diaminotriazine compounds of formula (I.A.4), wherein $R^1$ is $OCH_3$; $R^2$ is $CH_3$, $R^3$ and $R^{3'}$ are H; and $R^A$, $R^a$, $R^b$, $R^d$, $R^{e1}$ and $R^{e2}$ are defined in Table A4-1, particularly compounds of formulae A4-6.1 to A4-6.288.

Table A4-7. Diaminotriazine compounds of formula (I.A.4), wherein $R^1$ is $C_2H_5$; $R^2$ is $CH_3$, $R^3$ and $R^{3'}$ are H; and $R^A$, $R^a$, $R^b$, $R^d$, $R^{e1}$ and $R^{e2}$ are defined in Table A4-1, particularly compounds of formulae A4-7.1 to A4-7.288.

Table A4-8. Diaminotriazine compounds of formula (I.A.4), wherein $R^1$ is $C_2H_5$; $R^2$ is $C_2H_5$, $R^3$ and $R^{3'}$ are H; and $R^A$, $R^a$, $R^b$, $R^d$, $R^{e1}$ and $R^{e2}$ are defined in Table A4-1, particularly compounds of formulae A4-8.1 to A4-8.288.

Table A4-9. Diaminotriazine compounds of formula (I.A.4), wherein $R^1$, $R^2$ and $R^3$ are $CH_3$, $R^{3'}$ is H; and $R^A$, $R^a$, $R^b$, $R^d$, $R^{e1}$ and $R^{e2}$ are defined in Table A4-1, particularly compounds of formulae A4-9.1 to A4-9.288.

Table A4-10. Diaminotriazine compounds of formula (I.A.4), wherein $R^1$ and $R^2$ are $CH_3$, $R^3$ is $CF_3$; $R^{3'}$ is H; and $R^A$, $R^a$, $R^b$, $R^d$, $R^{e1}$ and $R^{e2}$ are defined in Table A4-1, particularly compounds of formulae A4-10.1 to A4-10.288.

Table A4-11. Diaminotriazine compounds of formula (I.A.4), wherein $R^1$, $R^2$, $R^3$ and $R^{3'}$ are $CH_3$; and $R^A$, $R^a$, $R^b$, $R^d$, $R^{e1}$ and $R^{e2}$ are defined in table A4-1, particularly compounds of formulae A4-11.1 to A4-11.288.

Table A4-12. Diaminotriazine compounds of formula (I.A.4), wherein $R^1$ and $R^2$ together with the carbon atom to which they are attached form cyclopropyl, $R^3$ and $R^{3'}$ are H; and $R^A$, $R^a$, $R^b$, $R^d$, $R^{e1}$ and $R^{e2}$ are defined in table A4-1, particularly compounds of formulae A4-12.1 to A4-12.288.

Table A4-13. Diaminotriazine compounds of formula (I.A.4), wherein $R^1$ and $R^2$ together with the carbon atom to which they are attached form cyclobutyl, $R^3$ and $R^{3'}$ are H; and $R^A$, $R^a$, $R^b$, $R^d$, $R^{e1}$ and $R^{e2}$ are defined in table A4-1, particularly compounds of formulae A4-13.1 to A4-13.288.

Table A4-14. Diaminotriazine compounds of formula (I.A.4), wherein $R^1$ and $R^2$ together with the carbon atom to which they are attached form cyclopentyl, $R^3$ and $R^{3'}$ are H; and $R^A$, $R^a$, $R^b$, $R^d$, $R^{e1}$ and $R^{e2}$ are defined in table A4-1, particularly compounds of formulae A4-14.1 to A4-14.288.

Table A4-15. Diaminotriazine compounds of formula (I.A.4), wherein $R^1$ and $R^2$ together with the carbon atom to which they are attached form cyclohexyl, $R^3$ and $R^{3'}$ are H; and $R^A$, $R^a$, $R^b$, $R^d$, $R^{e1}$ and $R^{e2}$ are defined in table A4-1, particularly compounds of formulae A4-15.1 to A4-15.288.

Also preferred are diaminotriazine compounds of formula (I.A.5), wherein $R^1$ is F; $R^2$, $R^3$ and $R^{3'}$ are H; and $R^A$, $R^{f1}$, $R^{f2}$, $Z^1$ and $Z^2$ are defined in table A5-1, wherein each line of table A represents one compound of formula (I.A.5), particularly compounds A5-1.1 to A5-1.54,

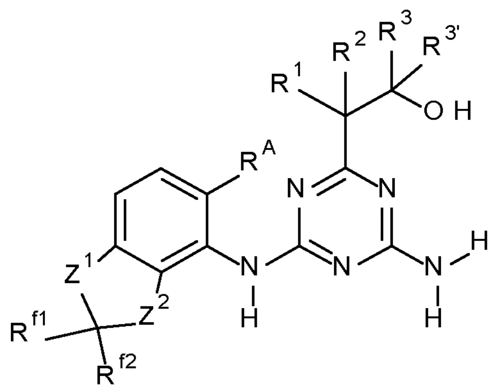

(I.A.5)

Table A5-1.

| no. | $R^A$ | Z1 | Z2 | $R^{f1}$ | $R^{f2}$ |
|---|---|---|---|---|---|
| A5-1.1 | F | $CH_2$ | $CH_2$ | H | H |
| A5-1.2 | F | $CH_2$ | $CH_2$ | H | F |
| A5-1.3 | F | $CH_2$ | $CH_2$ | H | $CH_3$ |
| A5-1.4 | F | $CH_2$ | $CH_2$ | F | H |
| A5-1.5 | F | $CH_2$ | $CH_2$ | F | F |
| A5-1.6 | F | $CH_2$ | $CH_2$ | F | $CH_3$ |
| A5-1.7 | F | $CH_2$ | $CH_2$ | $CH_3$ | H |
| A5-1.8 | F | $CH_2$ | $CH_2$ | $CH_3$ | F |
| A5-1.9 | F | $CH_2$ | $CH_2$ | $CH_3$ | $CH_3$ |

| no. | $R^A$ | Z1 | Z2 | $R^{f1}$ | $R^{f2}$ |
|---|---|---|---|---|---|
| A5-1.10 | F | O | O | H | H |
| A5-1.11 | F | O | O | H | F |
| A5-1.12 | F | O | O | H | $CH_3$ |
| A5-1.13 | F | O | O | F | H |
| A5-1.14 | F | O | O | F | F |
| A5-1.15 | F | O | O | F | $CH_3$ |
| A5-1.16 | F | O | O | $CH_3$ | H |
| A5-1.17 | F | O | O | $CH_3$ | F |
| A5-1.18 | F | O | O | $CH_3$ | $CH_3$ |

| no. | $R^A$ | Z1 | Z2 | $R^{f1}$ | $R^{f2}$ |
|---|---|---|---|---|---|
| A5-1.19 | Cl | $CH_2$ | $CH_2$ | H | H |
| A5-1.20 | Cl | $CH_2$ | $CH_2$ | H | F |
| A5-1.21 | Cl | $CH_2$ | $CH_2$ | H | $CH_3$ |
| A5-1.22 | Cl | $CH_2$ | $CH_2$ | F | H |
| A5-1.23 | Cl | $CH_2$ | $CH_2$ | F | F |
| A5-1.24 | Cl | $CH_2$ | $CH_2$ | F | $CH_3$ |
| A5-1.25 | Cl | $CH_2$ | $CH_2$ | $CH_3$ | H |
| A5-1.26 | Cl | $CH_2$ | $CH_2$ | $CH_3$ | F |
| A5-1.27 | Cl | $CH_2$ | $CH_2$ | $CH_3$ | $CH_3$ |
| A5-1.28 | Cl | O | O | H | H |
| A5-1.29 | Cl | O | O | H | F |
| A5-1.30 | Cl | O | O | H | $CH_3$ |
| A5-1.31 | Cl | O | O | F | H |
| A5-1.32 | Cl | O | O | F | F |
| A5-1.33 | Cl | O | O | F | $CH_3$ |
| A5-1.34 | Cl | O | O | $CH_3$ | H |
| A5-1.35 | Cl | O | O | $CH_3$ | F |
| A5-1.36 | Cl | O | O | $CH_3$ | $CH_3$ |

| no. | $R^A$ | Z1 | Z2 | $R^{f1}$ | $R^{f2}$ |
|---|---|---|---|---|---|
| A5-1.37 | CN | $CH_2$ | $CH_2$ | H | H |
| A5-1.38 | CN | $CH_2$ | $CH_2$ | H | F |
| A5-1.39 | CN | $CH_2$ | $CH_2$ | H | $CH_3$ |
| A5-1.40 | CN | $CH_2$ | $CH_2$ | F | H |
| A5-1.41 | CN | $CH_2$ | $CH_2$ | F | F |
| A5-1.42 | CN | $CH_2$ | $CH_2$ | F | $CH_3$ |
| A5-1.43 | CN | $CH_2$ | $CH_2$ | $CH_3$ | H |
| A5-1.44 | CN | $CH_2$ | $CH_2$ | $CH_3$ | F |
| A5-1.45 | CN | $CH_2$ | $CH_2$ | $CH_3$ | $CH_3$ |
| A5-1.46 | CN | O | O | H | H |
| A5-1.47 | CN | O | O | H | F |
| A5-1.48 | CN | O | O | H | $CH_3$ |
| A5-1.49 | CN | O | O | F | H |
| A5-1.50 | CN | O | O | F | F |
| A5-1.51 | CN | O | O | F | $CH_3$ |
| A5-1.52 | CN | O | O | $CH_3$ | H |
| A5-1.53 | CN | O | O | $CH_3$ | F |
| A5-1.54 | CN | O | O | $CH_3$ | $CH_3$ |

Also preferred are diaminotriazine compounds exemplified in Tables A5-2 to A5-15.

Table A5-2. Diaminotriazine compounds of formula (I.A.5), wherein $R^1$ is F; $R^2$ is $CH_3$, $R^3$ and $R^{3'}$ are H; and $R^A$, $R^{f1}$, $R^{f2}$, $Z^1$ and $Z^2$ are defined in Table A5-1, particularly compounds of formulae A5-2.1 to A5-2.54.

Table A5-3. Diaminotriazine compounds of formula (I.A.5), wherein $R^1$ is H; $R^2$ is $CH_3$, $R^3$ and $R^{3'}$ are H; and $R^A$, $R^{f1}$, $R^{f2}$, $Z^1$ and $Z^2$ are defined in Table A5-1, particularly compounds of formulae A5-3.1 to A5-3.54.

Table A5-4. Diaminotriazine compounds of formula (I.A.5), wherein $R^1$ is $CH_3$; $R^2$ is $CH_3$, $R^3$ and $R^{3'}$ are H; and $R^A$, $R^{f1}$, $R^{f2}$, $Z^1$ and $Z^2$ are defined in Table A5-1, particularly compounds of formulae A5-4.1 to A5-4.54.

Table A5-5. Diaminotriazine compounds of formula (I.A.5), wherein $R^1$ is $CF_3$; $R^2$ is $CH_3$, $R^3$ and $R^{3'}$ are H; and $R^A$, $R^{f1}$, $R^{f2}$, $Z^1$ and $Z^2$ are defined in Table A5-1, particularly compounds of formulae A5-5.1 to A5-5.54.

Table A5-6. Diaminotriazine compounds of formula (I.A.5), wherein $R^1$ is $OCH_3$; $R^2$ is $CH_3$, $R^3$ and $R^{3'}$ are H; and $R^A$, $R^{f1}$, $R^{f2}$, $Z^1$ and $Z^2$ are defined in Table A5-1, particularly compounds of formulae A5-6.1 to A5-6.54.

Table A5-7. Diaminotriazine compounds of formula (I.A.5), wherein $R^1$ is $C_2H_5$; $R^2$ is $CH_3$, $R^3$ and $R^{3'}$ are H; and $R^A$, $R^{f1}$, $R^{f2}$, $Z^1$ and $Z^2$ are defined in Table A5-1, particularly compounds of formulae A5-7.1 to A5-7.54.

Table A5-8. Diaminotriazine compounds of formula (I.A.5), wherein $R^1$ is $C_2H_5$; $R^2$ is $C_2H_5$, $R^3$ and $R^{3'}$ are H; and $R^A$, $R^{f1}$, $R^{f2}$, $Z^1$ and $Z^2$ are defined in Table A5-1, particularly compounds of formulae A5-8.1 to A5-8.54.

Table A5-9. Diaminotriazine compounds of formula (I.A.5), wherein $R^1$, $R^2$ and $R^3$ are $CH_3$, $R^{3'}$ is H; and $R^A$, $R^{f1}$, $R^{f2}$, $Z^1$ and $Z^2$ are defined in Table A5-1, particularly compounds of formulae A5-9.1 to A5-9.54.

Table A5-10. Diaminotriazine compounds of formula (I.A.5), wherein $R^1$ and $R^2$ are $CH_3$, $R^3$ is $CF_3$; $R^{3'}$ is H; and $R^A$, $R^{f1}$, $R^{f2}$, $Z^1$ and $Z^2$ are are defined in Table A5-1, particularly compounds of formulae A5-10.1 to A5-10.54.

Table A5-11. Diaminotriazine compounds of formula (I.A.5), wherein $R^1$, $R^2$, $R^3$ and $R^{3'}$ are $CH_3$; and $R^A$, $R^{f1}$, $R^{f2}$, $Z^1$ and $Z^2$ are defined in table A5-1, particularly compounds of formulae A5-11.1 to A5-11.54.

Table A5-12. Diaminotriazine compounds of formula (I.A.5), wherein $R^1$ and $R^2$ together with the carbon atom to which they are attached form cyclopropyl, $R^3$ and $R^{3'}$ are H; and $R^A$, $R^{f1}$, $R^{f2}$, $Z^1$ and $Z^2$ are defined in table A5-1, particularly compounds of formulae A5-12.1 to A5-12.54.

Table A5-13. Diaminotriazine compounds of formula (I.A.5), wherein $R^1$ and $R^2$ together with the carbon atom to which they are attached form cyclobutyl, $R^3$ and $R^{3'}$ are H; and $R^A$, $R^{f1}$, $R^{f2}$, $Z^1$ and $Z^2$ are defined in table A5-1,

particularly compounds of formulae A5-13.1 to A5-13.54.

Table A5-14. Diaminotriazine compounds of formula (I.A.5), wherein $R^1$ and $R^2$ together with the carbon atom to which they are attached form cyclopentyl, $R^3$ and $R^{3'}$ are H; and $R^A$, $R^{f1}$, $R^{f2}$, $Z^1$ and $Z^2$ are defined in table A5-1, particularly compounds of formulae A5-14.1 to A5-14.54.

Table A5-15. Diaminotriazine compounds of formula (I.A.5), wherein $R^1$ and $R^2$ together with the carbon atom to which they are attached form cyclohexyl, $R^3$ and $R^{3'}$ are H; and $R^A$, $R^{f1}$, $R^{f2}$, $Z^1$ and $Z^2$ are defined in table A5-1, particularly compounds of formulae A5-15.1 to A5-15.54.

The diaminotriazine compounds of formula (I) according to the invention can be prepared by standard processes of organic chemistry, for example by the following processes:

Process A)

The diaminotriazine compounds of formula (I), wherein $R^4$ and $R^5$ are independently of one another H, $C_1$-$C_6$-alkyl, $C_1$-$C_6$-alkoxy-$C_1$-$C_6$-alkyl or $C_1$-$C_6$-alkoxy can be prepared by reacting biguanidines of formula (II) with carbonyl compounds of formula (III) in the presence of a base:

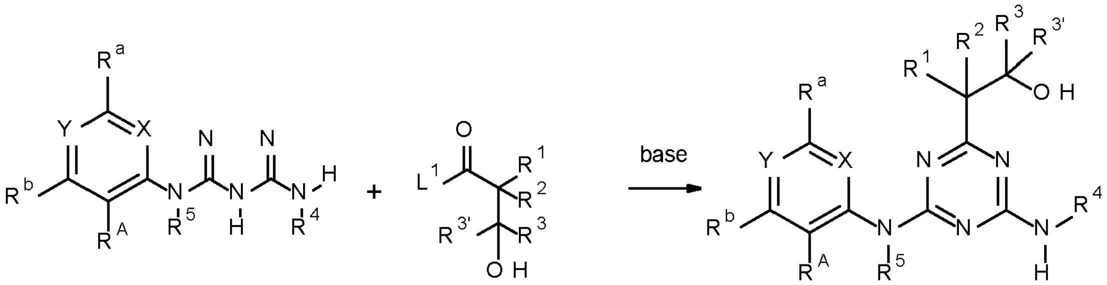


(II) (III) (I)

The variables X, Y, $R^A$, $R^a$, $R^b$, $R^1$, $R^2$, $R^3$ and $R^{3'}$ have the meanings, in particular the preferred meanings, as in formula (I) mentioned above, and

$R^4$ is H, $C_1$-$C_6$-alkyl, $C_1$-$C_6$-alkoxy-$C_1$-$C_6$-alkyl, $C_1$-$C_6$-alkoxy;
$R^5$ is H, $C_1$-$C_6$-alkyl, $C_1$-$C_6$-alkoxy-$C_1$-$C_6$-alkyl, $C_1$-$C_6$-alkoxy.
$L^1$ is a nucleophilically displaceable leaving group such as halogen, CN, $C_1$-$C_6$-alkoxy, $C_1$-$C_6$-alkoxycarbonyl, $C_1$-$C_6$-alkylcarbonyloxy or $C_1$-$C_6$-alkoxycarbonyloxy.

The reaction is carried out as described in earlier filed WO 2014/064094, Process A), page 60, line 1 to page 62, line 10. This citation is incorporated by reference herein.

The biguanidines of formula (II) required for the preparation of azines of formula (I), can be prepared by reacting guanidines of formula (IV) with amines of formula (V) in the presence of an acid:

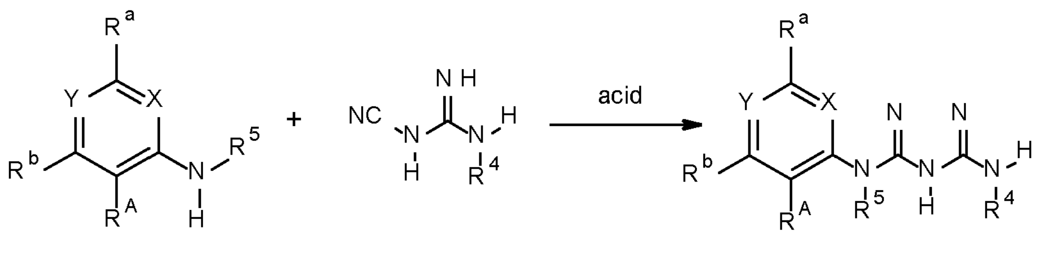


(V) (IV) (II)

X, Y, $R^A$, $R^a$ and $R^b$ have the meanings, in particular the preferred meanings, as in formula (I) mentioned above
$R^4$ is H, $C_1$-$C_6$-alkyl, $C_1$-$C_6$-alkoxy-$C_1$-$C_6$-alkyl, $C_1$-$C_6$-alkoxy;
$R^5$ is H, $C_1$-$C_6$-alkyl, $C_1$-$C_6$-alkoxy-$C_1$-$C_6$-alkyl, $C_1$-$C_6$-alkoxy.

The reaction is carried out as described in WO 2014/064094, Process A), page 62, line 35 to page 64, line 15.
The guanidines of formula (IV) required for the preparation of biguanidines of formula (II) are commercially available or can be prepared in accordance with literature procedures (e.g. J.L. LaMattina et al., J. Med. Chem. 1990, 33, 543 - 552; A. Perez-Medrano et al., J. Med. Chem. 2009, 52, 3366 - 3376).
The amines of formula (V) required for the preparation of biguanidines of formula (II) are commercially available.

Process B)

The azines of formula (I), wherein $R^4$ and $R^5$ are independently of one another H, $C_1$-$C_6$-alkyl, $C_1$-$C_6$-alkoxy-$C_1$-$C_6$-alkyl or $C_1$-$C_6$-alkoxy, can also be prepared by reacting halotriazines of formula (VI) with amines of formula (V) in the presence of a base and a catalyst:

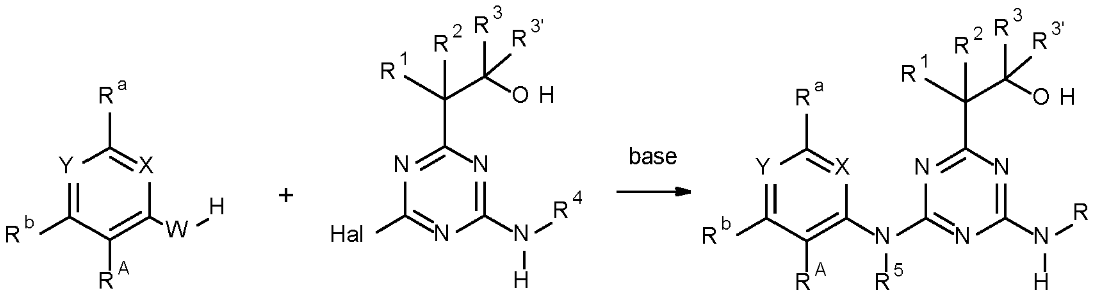


(V) (VI) (I)

The variables X, Y, $R^A$, $R^a$, $R^b$, $R^1$, $R^2$, $R^3$ and $R^{3'}$ have the meanings, in particular the preferred meanings, as in formula (I) mentioned above, and

Hal is halogen;
W is O or $NR^5$;
$R^4$ is H, $C_1$-$C_6$-alkyl, $C_1$-$C_6$-alkoxy-$C_1$-$C_6$-alkyl, $C_1$-$C_6$-alkoxy; and
$R^5$ is H, $C_1$-$C_6$-alkyl, $C_1$-$C_6$-alkoxy-$C_1$-$C_6$-alkyl, $C_1$-$C_6$-alkoxy.

The reaction of the halotriazines of formula (VI) with the compounds of formula (V) is usually carried out from 50°C to the boiling point of the reaction mixture, preferably from 50°C to 150°C, particularly preferably from 60°C to 100°C, in an inert organic solvent (e.g. P. Dao et al., Tetrahedron 2012, 68, 3856 - 3860).
The reaction can be carried out at atmospheric pressure or under elevated pressure, if appropriate, under an inert gas, continuously or batchwise.
The halotriazines of formula (VI) and the compounds of formula (V) are used in equimolar amounts or the compounds of formula (V) are used in excess with regard to the halotriazines of formula (VI). Preferably the molar ratio of the compounds of formula (V) to the halotriazines of formula (VI) is in the range from 2:1 to 1:1, preferably 1.5:1 to 1:1, especially preferred 1.2:1.
The reaction of the halotriazines of formula (VI) with the compounds of formula (V) is carried out in an organic solvent.
Suitable in principle are all solvents which are capable of dissolving the halotriazines of formula (VI) and the amines of compounds (V) at least partly and preferably fully under reaction conditions.
Examples of suitable solvents are aliphatic hydrocarbons such as pentane, hexane, cyclohexane, nitromethane and mixtures of $C_5$-$C_8$-alkanes, aromatic hydrocarbons such as benzene, chlorobenzene, toluene, cresols, o-, m- and p-xylene, halogenated hydrocarbons such as dichloromethane, 1,2-dichloroethane, chloroform, carbon tetrachloride and chlorobenzene, ethers such as diethyl ether, diisopropyl ether, tert.-butyl methylether (TBME), dioxane, anisole and tetrahydrofuran (THF), esters such as ethyl acetate and butyl acetate; nitriles such as acetonitrile and propionitrile, as well as dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethylpropylene urea (DMPU), dimethyl sulfoxide (DMSO) and 1-methyl-2 pyrrolidinone (NMP).
Preferred solvents are ethers as defined above.
The term solvent as used herein also includes mixtures of two or more of the above compounds.
The reaction of the halotriazines of formula (VI) with the compounds of formula (V) is carried out in the presence of a base.

Examples of suitable bases include metal-containing bases and nitrogen-containing bases.

Examples of suitable metal-containing bases are inorganic compounds such as alkali metal and alkaline earth metal hydroxides, and other metal hydroxides, such as lithium hydroxide, sodium hydroxide, potassium hydroxide, magnesium hydroxide, calcium hydroxide and aluminum hydroxide; alkali metal and alkaline earth metal oxide, and other metal oxides, such as lithium oxide, sodium oxide, potassium oxide, magnesium oxide, calcium oxide and magnesium oxide, iron oxide, silver oxide; alkali metal and alkaline earth metal hydrides such as lithium hydride, sodium hydride, potassium hydride and calcium hydride, alkali metal and alkaline earth metal formates, acetates and other metal salts of carboxylic acids, such as sodium formate, sodium benzoate, lithium acetate, sodium acetate, potassium acetate, magnesium acetate, and calcium acetate; alkali metal and alkaline earth metal carbonates such as lithium carbonate, sodium carbonate, potassium carbonate, magnesium carbonate, and calcium carbonate, as well as alkali metal hydrogen carbonates (bicarbonates) such as lithium hydrogen carbonate, sodium hydrogen carbonate, potassium hydrogen carbonate; alkali metal and alkaline earth metal phosphates such as sodium phosphate, potassium phosphate and calcium phosphate; alkali metal and alkaline earth metal alkoxides such as sodium methoxide, sodium ethoxide, potassium ethoxide, potassium tert-butoxide, potassium tert-pentoxide and dimethoxymagnesium; and furthermore organic bases, such as tertiary amines such as tri-$C_1$-$C_6$-alkylamines, for example triethylamine, trimethylamine, N-ethyldiisopropylamine, and N-methylpiperidine, pyridine, substituted pyridines such as collidine, lutidine, N-methylmorpholine and also bicyclic amines such as 1,8-diazabicyclo[5.4.0]undec-7-ene (DBU) or 1,5-diazabicyclo-[4.3.0]non-5-ene (DBN).

Preferred bases are alkali metal and alkaline earth metal alkoxides as defined above.

The term base as used herein also includes mixtures of two or more, preferably two of the above compounds. Particular preference is given to the use of one base.

The bases can be used in excess, preferably from 1 to 10, especially preferred from 2 to 4 base equivalents based on the halotriazines of formula (VI), and they may also be used as the solvent.

The reaction of the halotriazines of formula (VI) with the amines of formula (V), is carried out in the presence of a catalyst.

Examples of suitable catalysts include for example,
palladium based catalysts like, for example, palladium(II)acetate, tetrakis(triphenylphosphine)palladium(0), bis(triphenylphosphine)palladium(II)chloride or (1,1,-bis(diphenylphosphino)ferrocene)-dichloropalladium(II),
and optionally suitable additives such as, for example, phosphines like, for example, P(o-tolyl)$_3$, triphenylphosphine or BINAP (2,2'-Bis(diphenylphospino)-1,1'-binaphthyl).

The amount of catalyst is usually 10 to 20 mol % (0.1 to 0.2 equivalents) based on the halotriazines of formula (VI).

The end of the reaction can easily be determined by the skilled worker by means of routine methods.

The reaction mixtures are worked up in a customary manner, for example by mixing with water, separation of the phases and, if appropriate, chromatographic purification of the crude product.

The amines of formula (V) required for the preparation of azines of formula (I), wherein $R^5$ is H, $C_1$-$C_6$-alkyl, $C_1$-$C_6$-alkoxy-$C_1$-$C_6$-alkyl or $C_1$-$C_6$-alkoxy, are commercially available and/or can be prepared by analogy to known literature.

The alcohols of formula (V) required for the preparation of azines of formula (I), are commercially available and/or can be prepared by analogy to known literature.

The halotriazines of formula (VI) required for the preparation of azines of formula (I), wherein $R^4$ is H, $C_1$-$C_6$-alkyl, $C_1$-$C_6$-alkoxy-$C_1$-$C_6$-alkyl or $C_1$-$C_6$-alkoxy, are known from the literature, are commercially available and/or can be prepared by analogy (e.g. J. K.Chakrabarti et al., Tetrahedron 1975, 31, 1879 - 1882) by reacting thiotriazines of formula (VII) with a halogen:

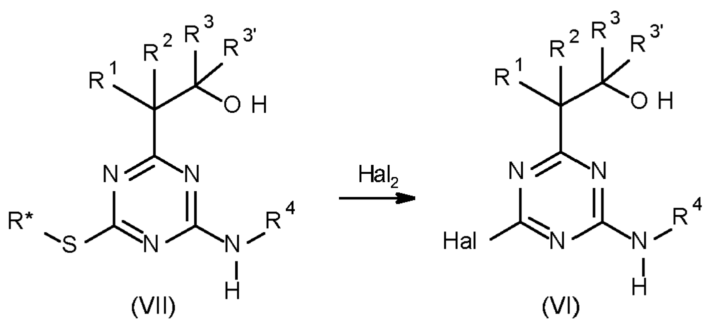


The variables $R^1$, $R^2$, $R^3$ and $R^{3'}$ have the meanings, in particular the preferred meanings, as in formula (I) mentioned above, and
Hal is halogen;

$R^*$ is $C_1$-$C_6$-alkyl, $C_2$-$C_6$-haloalkyl or phenyl;
$R^4$ is H, $C_1$-$C_6$-alkyl, $C_1$-$C_6$-alkoxy-$C_1$-$C_6$-alkyl, $C_1$-$C_6$-alkoxy.

The reaction of the thiotriazines of formula (VII) with the halogen is usually carried out from 0°C to the boiling point of the reaction mixture, preferably from 15°C to the boiling point of the reaction mixture, particularly preferably from 15°C to 40°C, in an inert organic solvent (e.g. J. K. Chakrabarti et al., Tetrahedron 1975, 31, 1879 - 1882).

The reaction can be carried out at atmospheric pressure or under elevated pressure, if appropriate under an inert gas, continuously or batchwise.

In the process according to the invention, the halogen is used in excess with regard to the thiotriazines of formula (VII).

The reaction of the thiotriazines of formula (VII) with the halogen is carried out in an organic solvent.

Suitable in principle are all solvents which are capable of dissolving the thiotriazines of formula (VII) and the halogen at least partly and preferably fully under reaction conditions.

Examples of suitable solvents are aliphatic hydrocarbons such as pentane, hexane, cyclohexane and mixtures of $C_5$-$C_8$-alkanes, halogenated hydrocarbons such as dichloromethane, 1,2-dichloroethane, chloroform and carbon tetrachloride; ethers such as diethyl ether, diisopropyl ether, tert.-butyl methylether (TBME), dioxane, anisole and tetrahydrofuran (THF), alcohols such as methanol, ethanol, n-propanol, isopropanol, n-butanol and tert.-butanol, as well as organic acids like formic acid, acetic acid, propionic acid, oxalic acid, citric acid, trifluoroacetic acid.

Preferred solvents are halogenated hydrocarbons and organic acids as defined above.

The term solvent as used herein also includes mixtures of two or more of the above compounds.

The end of the reaction can easily be determined by the skilled worker by means of routine methods.

The reaction mixtures are worked up in a customary manner, for example by mixing with water, separation of the phases and, if appropriate, chromatographic purification of the crude product.

The thiotriazines of formula (VII) required for the preparation of halotriazines of formula (VI) can be prepared in accordance by reacting guanidine-salts of formula (VIII) with carbonyl compounds of formula (III) in the presence of a base:

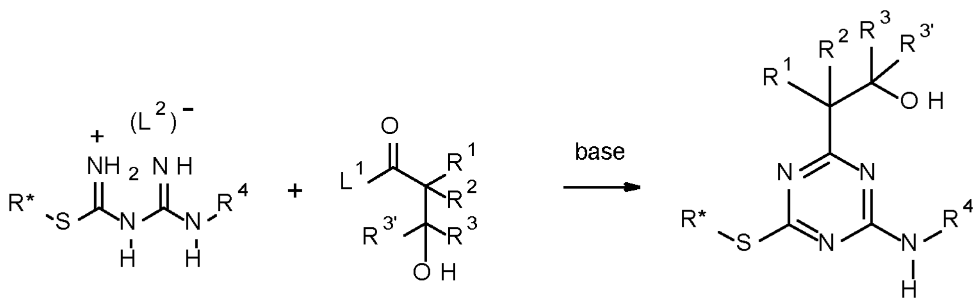


(VIII) (III) (VII)

The variables X, Y, $R^A$, $R^a$, $R^b$, $R^1$, $R^2$, $R^3$ and $R^{3'}$ have the meanings, in particular the preferred meanings, as in formula (I) mentioned above, and

$R^*$ is $C_1$-$C_6$-alkyl, $C_2$-$C_6$-haloalkyl or phenyl;
$L^1$ is a nucleophilically displaceable leaving group such as halogen, CN, $C_1$-$C_6$-alkoxy, $C_1$-$C_6$-alkoxycarbonyl, $C_1$-$C_6$-alkylcarbonyloxy or $C_1$-$C_6$-alkoxycarbonyloxy;
$L^2$ is a nucleophilically displaceable leaving group such as halogen, $C_1$-$C_6$-
$R^4$ is H, $C_1$-$C_6$-alkyl, $C_1$-$C_6$-alkoxy-$C_1$-$C_6$-alkyl, $C_1$-$C_6$-alkoxy.

The reaction of the guanidine-salt of formula (VIII) with the carbonyl compound of formula (III) is usually carried out at temperatures from 50°C to the boiling point of the reaction mixture, preferably from 50°C to 100°C.

The reaction can be carried out at atmospheric pressure or under elevated pressure, if appropriate under an inert gas, continuously or batchwise.

The guanidine-salts of formula (VIII) and the carbonyl compound of formula (III) are used in equimolar amounts or the carbonyl compound of formula (III) is used in excess with regard to the guanidine-salts of formula (VIII). Preferably the molar ratio of the carbonyl compound of formula (III) to the guanidine-salt of formula (VIII) is in the range from 1.5:1 to 1:1, preferably 1.2:1 to 1:1, especially preferred 1.2:1, also especially preferred 1:1.

The reaction of the guanidine-salt of formula (VIII) with the carbonyl compound of formula (III) is usually carried out in an organic solvent.

Suitable in principle are all solvents which are capable of dissolving the guanidine-salt of formula (VIII) and the

carbonyl compound of formula (III) at least partly and preferably fully under reaction conditions.

Examples of suitable solvents are halogenated hydrocarbons such as dichloromethane, 1,2-dichloroethane, chloroform, carbon tetrachloride and chlorobenzene, ethers such as diethyl ether, diisopropyl ether, tert.-butyl methylether (TBME), dioxane, anisole and tetrahydrofuran (THF), nitriles such as acetonitrile and propionitrile, as well as dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethylpropylene urea (DMPU), dimethyl sulfoxide (DMSO) and 1-methyl-2 pyrrolidinone (NMP).

Preferred solvents are ethers and dipolar aprotic solvents as defined above.

More preferred solvents are ethers as defined above.

The term solvent as used herein also includes mixtures of two or more of the above compounds.

The reaction of the guanidine-salts of formula (VIII) with the carbonyl compound of formula (III) is carried out in the presence of a base.

Examples of suitable bases include metal-containing bases and nitrogen-containing bases.

Examples of suitable metal-containing bases are inorganic compounds such as alkali metal and alkaline earth metal oxide, and other metal oxides, such as lithium oxide, sodium oxide, potassium oxide, magnesium oxide, calcium oxide and magnesium oxide, iron oxide, silver oxide; alkali metal and alkaline earth metal hydrides such as lithium hydride, sodium hydride, potassium hydride and calcium hydride, alkali metal and alkaline earth metal carbonates such as lithium carbonate, sodium carbonate, potassium carbonate, magnesium carbonate, and calcium carbonate, as well as alkali metal hydrogen carbonates (bicarbonates) such as lithium hydrogen carbonate, sodium hydrogen carbonate, potassium hydrogen carbonate; alkali metal and alkaline earth metal phosphates such as sodium phosphate, potassium phosphate and calcium phosphate; and furthermore organic bases, such as tertiary amines such as tri-$C_1$-$C_6$-alkylamines, for example triethylamine, trimethylamine, N-ethyldiisopropylamine, and N-methylpiperidine, pyridine, substituted pyridines such as collidine, lutidine, N-methylmorpholine, and also bicyclic amines such as 1,8-diazabicyclo[5.4.0]undec-7-ene (DBU) or 1,5-diazabicyclo[4.3.0]non-5-ene (DBN).

Preferred bases are tri-$C_1$-$C_6$-alkylamines as defined above.

The term base as used herein also includes mixtures of two or more, preferably two of the above compounds. Particular preference is given to the use of one base.

The bases are generally employed in excess; however they can also be employed in equimolar amounts, or, if appropriate, can be used as solvent.

Preferably from 1 to 5 base equivalents, particularly preferred 3 base equivalents of base are used, based on the guanidine-salts of formula (VIII).

The end of the reaction can easily be determined by the skilled worker by means of routine methods.

The reaction mixtures are worked up in a customary manner, for example by mixing with water, separation of the phases and, if appropriate, chromatographic purification of the crude product.

The carbonyl compounds of formula (III) required for the preparation of azines of formula (I) are known from the literature. They can be prepared in accordance and/or are commercially available.

The guanidine-salt of formula (VIII), wherein $L^2$ is iodine, required for the preparation of thiotriazines of formula (VII) is known from the literature (e.g. M. Freund et al., Chem. Ber. 1901, 34, 3110 - 3122; H. Eilingsfeld et al., Chem. Ber. 1967, 100, 1874 - 1891).

The guanidine-salts of formula (VIII) are commercially available and/or can be prepared in accordance with the literature cited.

Process C)

The azines of formula (I), wherein

$R^4$ is CN, ($C_1$-$C_6$-alkyl)carbonyl, ($C_1$-$C_6$-alkoxy)carbonyl, ($C_1$-$C_6$-alkyl)sulfonyl or phenylsulfonyl, wherein the phenyl is unsubstituted or substituted by one to five substituents selected from the group consisting of halogen, CN, $NO_2$, $C_1$-$C_6$-alkyl, $C_1$-$C_6$-haloalkyl and $C_1$-$C_6$-alkoxy;

can be prepared by reacting azines of formula (I), wherein $R^4$ is hydrogen with a compound of formula (IX):

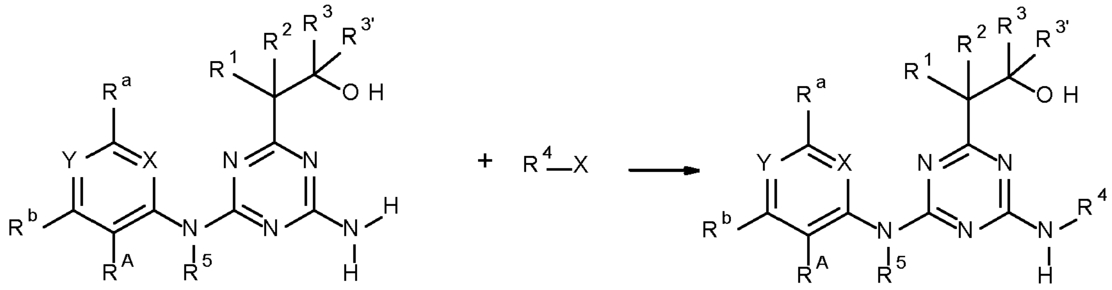

(I) wherein $R^4$ is hydrogen (IX) (I)

The variables X, Y, $R^A$, $R^a$, $R^b$, $R^1$, $R^2$, $R^3$, $R^{3'}$ and $R^5$ have the meanings, in particular the preferred meanings, as in formula (I) mentioned above, and

$R^4$ is CN, ($C_1$-$C_6$-alkyl)carbonyl, ($C_1$-$C_6$-alkoxy)carbonyl, ($C_1$-$C_6$-alkyl)sulfonyl or phenylsulfonyl, wherein the phenyl is unsubstituted or substituted by one to five substituents selected from the group consisting of halogen, CN, $NO_2$, $C_1$-$C_6$-alkyl, $C_1$-$C_6$-haloalkyl and $C_1$-$C_6$-alkoxy;
X is halogen or oxycarbonyl-$C_1$-$C_6$-alkyl;

Process D)

The azines of formula (I), wherein

$R^5$ is CN, ($C_1$-$C_6$-alkyl)carbonyl, ($C_1$-$C_6$-alkoxy)carbonyl, ($C_1$-$C_6$-alkyl)sulfonyl or phenylsulfonyl, wherein the phenyl is unsubstituted or substituted by one to five substituents selected from the group consisting of halogen, CN, $NO_2$, $C_1$-$C_6$-alkyl, $C_1$-$C_6$-haloalkyl and $C_1$-$C_6$-alkoxy;
can be prepared by reacting azines of formula (I), wherein $R^5$ is hydrogen with a compound of formula (X):

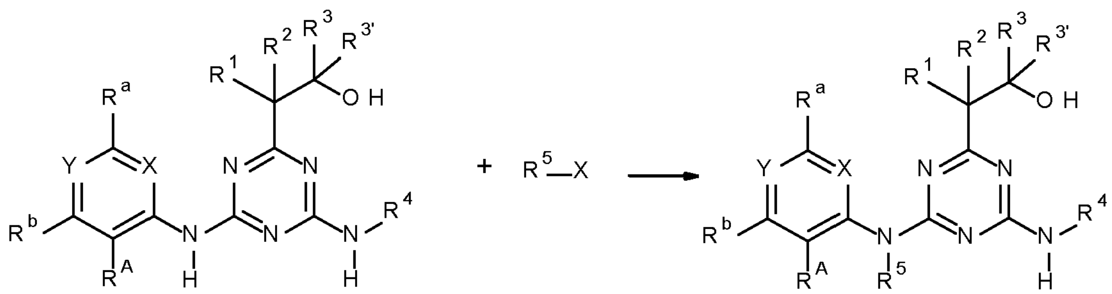

(I) wherein $R^5$ is hydrogen (X) (I)

The variables X, Y, $R^A$, $R^a$, $R^b$, $R^1$, $R^2$, $R^3$, $R^{3'}$ and $R^4$ have the meanings, in particular the preferred meanings, as in formula (I) mentioned above, and

$R^5$ is CN, ($C_1$-$C_6$-alkyl)carbonyl, ($C_1$-$C_6$-alkoxy)carbonyl, ($C_1$-$C_6$-alkyl)sulfonyl or phenylsulfonyl, wherein the phenyl is unsubstituted or substituted by one to five substituents selected from the group consisting of halogen, CN, $NO_2$, $C_1$-$C_6$-alkyl, $C_1$-$C_6$-haloalkyl and $C_1$-$C_6$-alkoxy;
X is halogen or oxycarbonyl-$C_1$-$C_6$-alkyl;

Both processes C and D independently of one another are carried out as described in WO 2014/064094, page 74, line 16 to page 76, line 25.

The diaminotriazines of formula (I) have herbicidal activity. Therefore, they can be used for controlling unwanted or undesired plants or vegetation. They can also be used in a method for controlling unwanted or undesired plants or vegetation, which method comprises allowing at least one compound of formula (I) or a salt thereof to act on plants, their environment or on seed. In order to allow the compound of formula (I) or a salt thereof to act on plants, their environment or on seed the compounds of the invention are applied to the plants, their environment or to the seed of said plants.

To widen the spectrum of action and to achieve synergistic effects, the diaminotriazine compounds of formula

(I) may be mixed with a large number of representatives of other herbicidal or growth-regulating active ingredient groups and then applied concomitantly.

Suitable components for mixtures are, for example, herbicides from the classes of the acetamides, amides, aryloxyphenoxypropionates, benzamides, benzofuran, benzoic acids, benzothiadiazinones, bipyridylium, carbamates, chloroacetamides, chlorocarboxylic acids, cyclohexanediones, dinitroanilines, dinitrophenol, diphenyl ether, glycines, imidazolinones, isoxazoles, isoxazolidinones, nitriles, N-phenylphthalimides, oxadiazoles, oxazolidinediones, oxyacetamides, phenoxycarboxylic acids, phenylcarbamates, phenylpyrazoles, phenylpyrazolines, phenylpyridazines, phosphinic acids, phosphoroamidates, phosphorodithioates, phthalamates, pyrazoles, pyridazinones, pyridines, pyridinecarboxylic acids, pyridinecarboxamides, pyrimidinediones, pyrimidinyl(thio)benzoates, quinolinecarboxylic acids, semicarbazones, sulfonylaminocarbonyltriazolinones, sulfonylureas, tetrazolinones, thiadiazoles, thiocarbamates, triazines, triazinones, triazoles, triazolinones, triazolocarboxamides, triazolopyrimidines, triketones, uracils, ureas.

The invention also relates to combinations of diaminotriazine compounds of formula (I) with at least one further herbicide B and/or at least one safener C).

The further herbicidal compound B (component B) is in particular selected from the herbicides of class b1) to b15): B) herbicides of class b1) to b15):

b1) lipid biosynthesis inhibitors;
b2) acetolactate synthase inhibitors (ALS inhibitors);
b3) photosynthesis inhibitors;
b4) protoporphyrinogen-IX oxidase inhibitors,
b5) bleacher herbicides;
b6) enolpyruvyl shikimate 3-phosphate synthase inhibitors (EPSP inhibitors);
b7) glutamine synthetase inhibitors;
b8) 7,8-dihydropteroate synthase inhibitors (DHP inhibitors);
b9) mitosis inhibitors;
b10) inhibitors of the synthesis of very long chain fatty acids (VLCFA inhibitors);
b11) cellulose biosynthesis inhibitors;
b12) decoupler herbicides;
b13) auxinic herbicides;
b14) auxin transport inhibitors; and
b15) other herbicides selected from the group consisting of bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (CAS 499223-49-3) and its salts and esters;
including their agriculturally acceptable salts or derivatives such as ethers, esters or amides.

Preference is given to those compositions according to the present invention comprising at least one herbicide B selected from herbicides of class b1, b6, b9, b10 and b11.

Examples of herbicides B which can be used in combination with the compounds of formula (I) according to the present invention are:

b1) from the group of the lipid biosynthesis inhibitors:

ACC-herbicides such as alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); and non ACC herbicides such as benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;

b2) from the group of the ALS inhibitors:

sulfonylureas such as amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl and tritosulfuron,
imidazolinones such as imazamethabenz, imazamethabenz-methyl, imazamox, imazapic, imazapyr, imazaquin and imazethapyr, triazolopyrimidine herbicides and sulfonanilides such as cloransulam, cloransulam-methyl, diclosulam, flumetsulam, florasulam, metosulam, penoxsulam, pyrimisulfan and pyroxsulam,
pyrimidinylbenzoates such as bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methylethyl ester (CAS 420138-41-6), 4-[[[2-[(4,6-dimethoxy-2-pyrimidinyl)oxy]phenyl]methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8),
sulfonylaminocarbonyl-triazolinone herbicides such as flucarbazone, flucarbazone-sodium, propoxycarbazone, propoxycarbazone-sodium, thiencarbazone and thiencarbazone-methyl; and triafamone;
among these, a preferred embodiment of the invention relates to those compositions comprising at least one imidazolinone herbicide;

b3) from the group of the photosynthesis inhibitors:
amicarbazone, inhibitors of the photosystem II, e.g. triazine herbicides, including of chlorotriazine, triazinones, triazindiones, methylthiotriazines and pyridazinones such as ametryn, atrazine, chloridazone, cyanazine, desmetryn, dimethametryn,hexazinone, metribuzin, prometon, prometryn, propazine, simazine, simetryn, terbumeton, terbuthylazin, terbutryn and trietazin, aryl urea such as chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron, phenyl carbamates such as desmedipham, karbutilat, phenmedipham, phenmedipham-ethyl, nitrile herbicides such as bromofenoxim, bromoxynil and its salts and esters, ioxynil and its salts and esters, uraciles such as bromacil, lenacil and terbacil, and bentazon and bentazon-sodium, pyridate, pyridafol, pentanochlor and propanil and inhibitors of the photosystem I such as diquat, diquat-dibromide, paraquat, paraquat-dichloride and paraquat-dimetilsulfate. Among these, a preferred embodiment of the invention relates to those compositions comprising at least one aryl urea herbicide. Among these, likewise a preferred embodiment of the invention relates to those compositions comprising at least one triazine herbicide. Among these, likewise a preferred embodiment of the invention relates to those compositions comprising at least one nitrile herbicide;
b4) from the group of the protoporphyrinogen-IX oxidase inhibitors:
acifluorfen, acifluorfen-sodium, azafenidin, bencarbazone, benzfendizone, bifenox, butafenacil, carfentrazone, carfentrazone-ethyl, chlomethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethyl-phenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0), methyl (*E*)-4-[2-chloro-5-[4-chloro-5-(difluoromethoxy)-1*H*-methyl-pyrazol-3-yl]-4-fluoro-phenoxy]-3-methoxy-but-2-enoate (CAS 948893-00-3), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4);

b5) from the group of the bleacher herbicides:

PDS inhibitors: beflubutamid, diflufenican, fluridone, flurochloridone, flurtamone, norflurazon, picolinafen, and 4-(3-trifluoromethylphenoxy)-2-(4-trifluoromethylphenyl)pyrimidine (CAS 180608-33-7), HPPD inhibitors: benzobicyclon, benzofenap, bicyclopyrone, clomazone, fenquintrione, isoxaflutole, mesotrione, pyrasulfotole, pyrazolynate, pyrazoxyfen, sulcotrione, tefuryltrione, tembotrione, tolpyralate, topramezone , bleacher, unknown target: aclonifen, amitrole and flumeturon;

b6) from the group of the EPSP synthase inhibitors:

glyphosate, glyphosate-isopropylammonium, glyposate-potassium and glyphosate-trimesium (sulfosate);

b7) from the group of the glutamine synthase inhibitors:

bilanaphos (bialaphos), bilanaphos-sodium, glufosinate, glufosinate-P and glufosinate-ammonium;

b8) from the group of the DHP synthase inhibitors:

asulam;

b9) from the group of the mitosis inhibitors:

compounds of group K1: dinitroanilines such as benfluralin, butralin, dinitramine, ethalfluralin, fluchloralin, oryzalin, pendimethalin, prodiamine and trifluralin, phosphoramidates such as amiprophos, amiprophos-methyl, and butamiphos, benzoic acid herbicides such as chlorthal, chlorthal-dimethyl, pyridines such as dithiopyr and thiazopyr, benzamides such as propyzamide and tebutam; compounds of group K2: carbetamide, chlorpropham, flamprop, flamprop-isopropyl, flamprop-methyl, flamprop-M-isopropyl, flamprop-M-methyl and propham ; among these, compounds of group K1, in particular dinitroanilines are preferred;

b10) from the group of the VLCFA inhibitors:

chloroacetamides such as acetochlor, alachlor, butachlor, dimethachlor, dimethenamid, dimethenamid-P, metazachlor, metolachlor, metolachlor-S, pethoxamid, pretilachlor, propachlor, propisochlor and thenylchlor, oxyacetanilides such as flufenacet and mefenacet, acetanilides such as diphenamid, naproanilide, napropamide and napropamide-M, tetrazolinones such fentrazamide, and other herbicides such as anilofos, cafenstrole, fenoxasulfone, ipfencarbazone, piperophos, pyroxasulfone and isoxazoline compounds of the formulae 11.1, II.2, II.3, II.4, II.5, II.6, II.7, II.8 and II.9

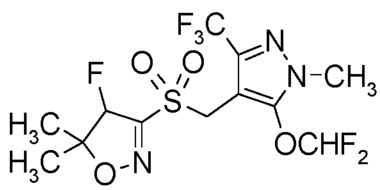

II.1

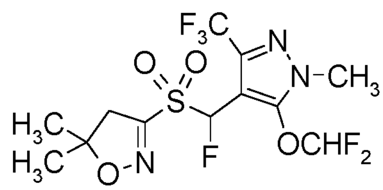

II.2

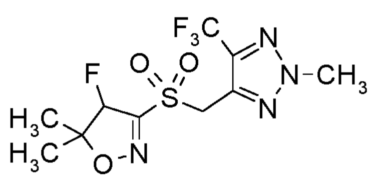

II.3

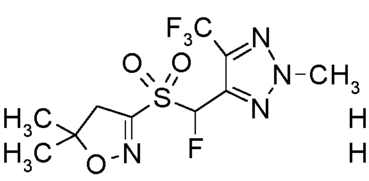

II.4

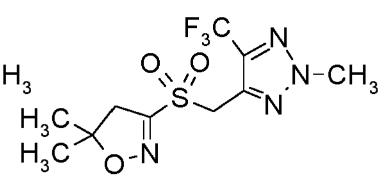

II.5

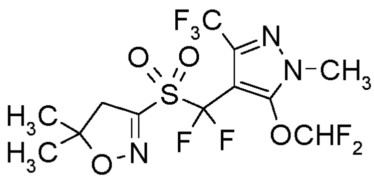

II.6

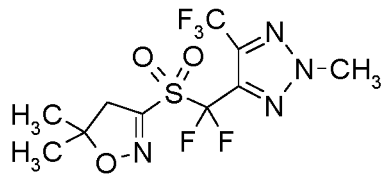

II.7

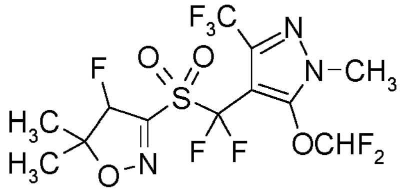

II.8

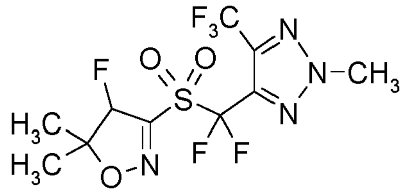

II.9

the isoxazoline compounds of the formula (I)I are known in the art, e.g. from WO 2006/024820, WO 2006/037945, WO 2007/071900 and WO 2007/096576;

among the VLCFA inhibitors, preference is given to chloroacetamides and oxyacetamides;

b11) from the group of the cellulose biosynthesis inhibitors:

chlorthiamid, dichlobenil, flupoxam, indaziflam, isoxaben, triaziflam and 1-cyclohexyl-5-pentafluorphenyloxy-$1^4$-[1,2,4,6]thiatriazin-3-ylamine (CAS 175899-01-1);

b12) from the group of the decoupler herbicides:

dinoseb, dinoterb and DNOC and its salts;

b13) from the group of the auxinic herbicides:

2,4-D and its salts and esters such as clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, fluroxypyr, fluroxypyr-butometyl, fluroxypyr-meptyl, halauxifen and its salts and esters (CAS 943832-60-8); MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, 4-amino-3-chloro-6-(4-chloro-2-fluoro-3-methoxyphenyl)-5-fluoropyridine-2-carboxylic acid and benzyl 4-amino-3-chloro-6-(4-chloro-2-fluoro-3-methoxyphenyl)-5-fluoropyridine-2-carboxylate (CAS 1390661-72-9);

b14) from the group of the auxin transport inhibitors: diflufenzopyr, diflufenzopyr-sodium, naptalam and naptalam-sodium;

b15) from the group of the other herbicides: bromobutide, chlorflurenol, chlorflurenol-methyl, cinmethylin, cumyluron, cyclopyrimorate (CAS 499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine and tridiphane.

Preferred herbicides B that can be used in combination with the compounds of the formula (I) according to the present invention are:

b1) from the group of the lipid biosynthesis inhibitors:

clethodim, clodinafop-propargyl, cycloxydim, cyhalofop-butyl, diclofop-methyl, fenoxaprop-P-ethyl, fluazifop-P-butyl, haloxyfop-P-methyl, metamifop, pinoxaden, profoxydim, propaquizafop, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl-[1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); benfuresate, dimepiperate, EPTC, esprocarb, ethofumesate, molinate, orbencarb, prosulfocarb, thiobencarb and triallate;

b2) from the group of the ALS inhibitors:

amidosulfuron, azimsulfuron, bensulfuron-methyl, bispyribac-sodium, chlorimuron-ethyl, chlorsulfuron, cloransulam-methyl, cyclosulfamuron, diclosulam, ethametsulfuron-methyl, ethoxysulfuron, flazasulfuron, florasulam, flucarbazone-sodium, flucetosulfuron, flumetsulam, flupyrsulfuron-methyl-sodium, foramsulfuron, halosulfuron-methyl, imazamethabenz-methyl, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, metosulam, metsulfuron-methyl, nicosulfuron, orthosulfamuron, oxasulfuron, penoxsulam, primisulfuron-methyl, propoxycarbazon-sodium, propyrisulfuron, prosulfuron, pyrazosulfuron-ethyl, pyribenzoxim, pyrimisulfan, pyriftalid, pyriminobac-methyl, pyrithiobac-sodium, pyroxsulam, rimsulfuron, sulfometuron-methyl, sulfosulfuron, thiencarbazone-methyl, thifensulfuron-methyl, triasulfuron, tribenuron-methyl, trifloxysulfuron, triflusulfuron-methyl, tritosulfuron and triafamone;

b3) from the group of the photosynthesis inhibitors:

ametryn, amicarbazone, atrazine, bentazone, bentazone-sodium, bromoxynil and its salts and esters, chloridazone, chlorotoluron, cyanazine, desmedipham, diquat-dibromide, diuron, fluometuron, hexazinone, ioxynil and its salts and esters, isoproturon, lenacil, linuron, metamitron, methabenzthiazuron, metribuzin, paraquat, paraquat-dichloride, phenmedipham, propanil, pyridate, simazine, terbutryn, terbuthylazine and thidiazuron;

b4) from the group of the protoporphyrinogen-IX oxidase inhibitors: acifluorfen-sodium, bencarbazone, benzfendizone, butafenacil, carfentrazone-ethyl, cinidon-ethyl, flufenpyr-ethyl, flumiclorac-pentyl, flumioxazin, fluoroglycofen-ethyl, fomesafen, lactofen, oxadiargyl, oxadiazon, oxyfluorfen, pentoxazone, pyraflufen, pyraflufen-ethyl, saflufenacil, sulfentrazone, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1*H*-pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoro-methylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H*-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0);1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4);

b5) from the group of the bleacher herbicides:

aclonifen, amitrole, beflubutamid, benzobicyclon, bicyclopyrone, clomazone, diflufenican, fenquintrione, flumeturon, flurochloridone, flurtamone, isoxaflutole, mesotrione, norflurazon, picolinafen, pyrasulfotole, pyrazolynate, sulcotrione, tefuryltrione, tembotrione, tolpyralate, topramezone and 4-(3-trifluoromethylphenoxy)-2-(4-trifluoromethylphenyl)pyrimidine (CAS 180608-33-7);

b6) from the group of the EPSP synthase inhibitors:

glyphosate, glyphosate-isopropylammonium, glyphosate-potassium and glyphosate-trimesium (sulfosate);

b7) from the group of the glutamine synthase inhibitors:

glufosinate, glufosinate-P, glufosinate-ammonium;

b8) from the group of the DHP synthase inhibitors: asulam;

b9) from the group of the mitosis inhibitors:

benfluralin, dithiopyr, ethalfluralin, flamprop, flamprop-isopropyl, flamprop-methyl, flamprop-M-isopropyl, flamprop-M-methyl, oryzalin, pendimethalin, thiazopyr and trifluralin;

b10) from the group of the VLCFA inhibitors:

acetochlor, alachlor, anilofos, butachlor, cafenstrole, dimethenamid, dimethenamid-P, fentrazamide, flufenacet, mefenacet, metazachlor, metolachlor, S-metolachlor, naproanilide, napropamide, napropamide-M, pretilachlor,

fenoxasulfone, ipfencarbazone, pyroxasulfone thenylchlor and isoxazoline-compounds of the formulae II.1, II.2, II.3, II.4, II.5, II.6, II.7, II.8 and II.9 as mentioned above;

b11) from the group of the cellulose biosynthesis inhibitors: dichlobenil, flupoxam, indaziflam, isoxaben, triaziflam and 1-cyclohexyl-5-pentafluorphenyloxy-1$^4$-[1,2,4,6]thiatriazin-3-ylamine (CAS 175899-01-1);

b13) from the group of the auxinic herbicides:

2,4-D and its salts and esters, aminocyclopyrachlor and its salts and esters, aminopyralid and its salts such as aminopyralid-dimethylammonium, aminopyralid-tris(2-hydroxypropyl)ammonium and its esters, clopyralid and its salts and esters, dicamba and its salts and esters, dichlorprop-P and its salts and esters, fluroxypyr-meptyl, halauxifen and its salts and esters (CAS 943832-60-8, MCPA and its salts and esters, MCPB and its salts and esters, mecoprop-P and its salts and esters, picloram and its salts and esters, quinclorac, quinmerac, triclopyr and its salts and esters, 4-amino-3-chloro-6-(4-chloro-2-fluoro-3-methoxyphenyl)-5-fluoropyridine-2-carboxylic acid and benzyl 4-amino-3-chloro-6-(4-chloro-2-fluoro-3-methoxyphenyl)-5-fluoropyridine-2-carboxylate (CAS 1390661-72-9);

b14) from the group of the auxin transport inhibitors: diflufenzopyr and diflufenzopyr-sodium;

b15) from the group of the other herbicides: bromobutide, cinmethylin, cumyluron, cyclopyrimorate (CAS 499223-49-3 and its salts and esters, dalapon, difenzoquat, difenzoquat-metilsulfate, DSMA, dymron (= daimuron), indanofan, metam, methylbromide, MSMA, oxaziclomefone, pyributicarb and tridiphane.

Particularly preferred herbicides B that can be used in combination with the compounds A of the formula (I) according to the present invention are:

b1) from the group of the lipid biosynthesis inhibitors: clodinafop-propargyl, cycloxydim, cyhalofop-butyl, fenoxaprop-P-ethyl, pinoxaden, profoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); esprocarb, prosulfocarb, thiobencarb and triallate;

b2) from the group of the ALS inhibitors: bensulfuron-methyl, bispyribac-sodium, cyclosulfamuron, diclosulam, flumetsulam, flupyrsulfuron-methyl-sodium, foramsulfuron, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, nicosulfuron, penoxsulam, propoxycarbazon-sodium, propyrisulfuron, pyrazosulfuron-ethyl, pyroxsulam, rimsulfuron, sulfosulfuron, thiencarbazon-methyl, tritosulfuron and triafamone;

b3) from the group of the photosynthesis inhibitors: ametryn, atrazine, diuron, fluometuron, hexazinone, isoproturon, linuron, metribuzin, paraquat, paraquat-dichloride, propanil, terbutryn and terbuthylazine;

b4) from the group of the protoporphyrinogen-IX oxidase inhibitors: flumioxazin, oxyfluorfen, pyraflufen, pyraflufen-ethyl, saflufenacil, sulfentrazone, trifludimoxazin, ethyl [3-[2-chloro-4-fluoro-5-(1-methyl-6-trifluoromethyl-2,4-dioxo-1,2,3,4-tetrahydropyrimidin-3-yl)-phenoxy]-2-pyridyloxy]acetate (CAS 353292-31-6; S-3100, 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), and 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0);

b5) from the group of the bleacher herbicides: amitrole, bicyclopyrone, clomazone, diflufenican, fenquintrione, flumeturon, flurochloridone, isoxaflutole, mesotrione, picolinafen, sulcotrione, tefuryltrione, tembotrione, tolpyralate and topramezone;

b6) from the group of the EPSP synthase inhibitors: glyphosate, glyphosate-isopropylammonium and glyphosate-trimesium (sulfosate);

b7) from the group of the glutamine synthase inhibitors: glufosinate, glufosinate-P and glufosinate-ammonium;

b9) from the group of the mitosis inhibitors: pendimethalin and trifluralin;

b10) from the group of the VLCFA inhibitors: acetochlor, cafenstrole, dimethenamid-P, fentrazamide, flufenacet, mefenacet, metazachlor, metolachlor, S-metolachlor, fenoxasulfone, ipfencarbazone and pyroxasulfone; likewise, preference is given to isoxazoline compounds of the formulae II.1, II.2, II.3, II.4, II.5, II.6, II.7, II.8 and II.9 as mentioned above;
b11) from the group of the cellulose biosynthesis inhibitors: indaziflam, isoxaben and triaziflam;
b13) from the group of the auxinic herbicides: 2,4-D and its salts and esters such as clacyfos, and aminocyclopyrachlor and its salts and esters, aminopyralid and its salts and its esters, clopyralid and its salts and esters, dicamba and its salts and esters, fluroxypyr-meptyl, halauxifen, halauxifen-methyl, quinclorac, quinmerac, 4-amino-3-chloro-6-(4-chloro-2-fluoro-3-methoxyphenyl)-5-fluoropyridine-2-carboxylic acid and benzyl 4-amino-3-chloro-6-(4-chloro-2-fluoro-3-methoxyphenyl)-5-fluoropyridine-2-carboxylate (CAS 1390661-72-9);
b14) from the group of the auxin transport inhibitors: diflufenzopyr and diflufenzopyr-sodium,
b15) from the group of the other herbicides: dymron (= daimuron), indanofan, oxaziclomefone.

Particularly preferred herbicidal compounds B are the herbicides B as defined above; in particular the herbicides B.1 - B.196 listed below in table B:

Table B:

| | Herbicide B |
|---|---|
| B.1 | clethodim |
| B.2 | clodinafop-propargyl |
| B.3 | cycloxydim |
| B.4 | cyhalofop-butyl |
| B.5 | fenoxaprop-ethyl |
| B.6 | fenoxaprop-P-ethyl |
| B.7 | metamifop |

| | Herbicide B |
|---|---|
| B.8 | pinoxaden |
| B.9 | profoxydim |
| B.10 | sethoxydim |
| B.11 | tepraloxydim |
| B.12 | tralkoxydim |
| B.13 | esprocarb |
| B.14 | ethofumesate |

| | Herbicide B |
|---|---|
| B.15 | molinate |
| B.16 | prosulfocarb |
| B.17 | thiobencarb |
| B.18 | triallate |
| B.19 | bensulfuron-methyl |
| B.20 | bispyribac-sodium |
| B.21 | cloransulam-methyl |
| B.22 | chlorsulfuron |
| B.23 | clorimuron |
| B.24 | cyclosulfamuron |
| B.25 | diclosulam |
| B.26 | florasulam |
| B.27 | flumetsulam |
| B.28 | flupyrsulfuron-methyl-sodium |
| B.29 | foramsulfuron |
| B.30 | imazamox |
| B.31 | imazamox-ammonium |
| B.32 | imazapic |
| B.33 | imazapic-ammonium |
| B.34 | imazapic-isopropylammonium |
| B.35 | imazapyr |
| B.36 | imazapyr-ammonium |
| B.37 | imazapyr-isopropylammonium |
| B.38 | imazaquin |
| B.39 | imazaquin-ammonium |
| B.40 | imazethapyr |
| B.41 | imazethapyr-ammonium |
| B.42 | imazethapyr-isopropylammonium |
| B.43 | imazosulfuron |
| B.44 | iodosulfuron-methyl-sodium |
| B.45 | iofensulfuron |
| B.46 | iofensulfuron-sodium |
| B.47 | mesosulfuron-methyl |
| B.48 | metazosulfuron |
| B.49 | metsulfuron-methyl |
| B.50 | metosulam |
| B.51 | nicosulfuron |
| B.52 | penoxsulam |
| B.53 | propoxycarbazon-sodium |

| | Herbicide B |
|---|---|
| B.54 | pyrazosulfuron-ethyl |
| B.55 | pyribenzoxim |
| B.56 | pyriftalid |
| B.57 | pyroxsulam |
| B.58 | propyrisulfuron |
| B.59 | rimsulfuron |
| B.60 | sulfosulfuron |
| B.61 | thiencarbazone-methyl |
| B.62 | thifensulfuron-methyl |
| B.63 | tribenuron-methyl |
| B.64 | tritosulfuron |
| B.65 | triafamone |
| B.66 | ametryne |
| B.67 | atrazine |
| B.68 | bentazon |
| B.69 | bromoxynil |
| B.70 | bromoxynil-octanoate |
| B.71 | bromoxynil-heptanoate |
| B.72 | bromoxynil-potassium |
| B.73 | diuron |
| B.74 | fluometuron |
| B.75 | hexazinone |
| B.76 | isoproturon |
| B.77 | linuron |
| B.78 | metamitron |
| B.79 | metribuzin |
| B.80 | propanil |
| B.81 | simazin |
| B.82 | terbuthylazine |
| B.83 | terbutryn |
| B.84 | paraquat-dichloride |
| B.85 | acifluorfen |
| B.86 | butafenacil |
| B.87 | carfentrazone-ethyl |
| B.88 | flumioxazin |
| B.89 | fomesafen |
| B.90 | oxadiargyl |
| B.91 | oxyfluorfen |
| B.92 | pyraflufen |

| | Herbicide B |
|---|---|
| B.93 | pyraflufen-ethyl |
| B.94 | saflufenacil |
| B.95 | sulfentrazone |
| B.96 | trifludimoxazin |
| B.97 | ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6) |
| B.98 | benzobicyclon |
| B.99 | bicyclopyrone |
| B.100 | clomazone |
| B.101 | diflufenican |
| B.102 | flurochloridone |
| B.103 | isoxaflutole |
| B.104 | mesotrione |
| B.105 | norflurazone |
| B.106 | picolinafen |
| B.107 | sulcotrione |
| B.108 | tefuryltrione |
| B.109 | tembotrione |
| B.110 | tolpyralate |
| B.111 | topramezone |
| B.112 | topramezone-sodium |
| B.113 | amitrole |
| B.114 | fluometuron |
| B.115 | fenquintrione |
| B.116 | glyphosate |
| B.117 | glyphosate-ammonium |
| B.118 | glyphosate-dimethylammonium |
| B.119 | glyphosate-isopropylammonium |
| B.120 | glyphosate-trimesium (sulfosate) |
| B.121 | glyphosate-potassium |
| B.122 | glufosinate |
| B.123 | glufosinate-ammonium |
| B.124 | glufosinate-P |
| B.125 | glufosinate-P-ammonium |
| B.126 | pendimethalin |
| B.127 | trifluralin |

| | Herbicide B |
|---|---|
| B.128 | acetochlor |
| B.129 | butachlor |
| B.130 | cafenstrole |
| B.131 | dimethenamid-P |
| B.132 | fentrazamide |
| B.133 | flufenacet |
| B.134 | mefenacet |
| B.135 | metazachlor |
| B.136 | metolachlor |
| B.137 | S-metolachlor |
| B.138 | pretilachlor |
| B.139 | fenoxasulfone |
| B.140 | indaziflam |
| B.141 | isoxaben |
| B.142 | triaziflam |
| B.143 | ipfencarbazone |
| B.144 | pyroxasulfone |
| B.145 | 2,4-D |
| B.146 | 2,4-D-isobutyl |
| B.147 | 2,4-D-dimethylammonium |
| B.148 | 2,4-D-N,N,N-trimethylethanolammonium |
| B.149 | aminopyralid |
| B.150 | aminopyralid-methyl |
| B.151 | aminopyralid-dimethylammonium |
| B.152 | aminopyralid-tris(2-hydroxypropyl)ammonium |
| B.153 | clopyralid |
| B.154 | clopyralid-methyl |
| B.155 | clopyralid-olamine |
| B.156 | dicamba |
| B.157 | dicamba-butotyl |
| B.158 | dicamba-diglycolamine |
| B.159 | dicamba-dimethylammonium |
| B.160 | dicamba-diolamine |
| B.161 | dicamba-isopropylammonium |
| B.162 | dicamba-potassium |
| B.163 | dicamba-sodium |
| B.164 | dicamba-trolamine |

| | Herbicide B |
|---|---|
| B.165 | dicamba-N,N-bis-(3-aminopropyl)methylamine |
| B.166 | dicamba-diethylenetriamine |
| B.167 | fluroxypyr |
| B.168 | fluroxypyr-meptyl |
| B.169 | halauxifen |
| B.170 | halauxifen-methyl |
| B.171 | MCPA |
| B.172 | MCPA-2-ethylhexyl |
| B.173 | MCPA-dimethylammonium |
| B.174 | quinclorac |
| B.175 | quinclorac-dimethylammonium |
| B.176 | quinmerac |
| B.177 | quinmerac-dimethylammonium |
| B.178 | 4-amino-3-chloro-6-(4-chloro-2-fluoro-3-methoxyphenyl)-5-fluoropyridine-2-carboxylic acid |
| B.179 | benzyl 4-amino-3-chloro-6-(4-chloro-2-fluoro-3-methoxyphenyl)-5-fluoropyridine-2-carboxylate (CAS 1390661-72-9) |

| | Herbicide B |
|---|---|
| B.180 | aminocyclopyrachlor |
| B.181 | aminocyclopyrachlor-potassium |
| B.182 | aminocyclopyrachlor-methyl |
| B.183 | diflufenzopyr |
| B.184 | diflufenzopyr-sodium |
| B.185 | dymron |
| B.186 | indanofan |
| B.187 | oxaziclomefone |
| B.188 | II.1 |
| B.189 | II.2 |
| B.190 | II.3 |
| B.191 | II.4 |
| B.192 | II.5 |
| B.193 | II.6 |
| B.194 | II.7 |
| B.195 | II.8 |
| B.196 | II.9 |

In another embodiment of the present invention the compositions according to the present invention comprise at least one diaminotriazine compound of formula (I) and at least one safener C.

Safeners are chemical compounds which prevent or reduce damage on useful plants without having a major impact on the herbicidal action of the herbicidal active components of the present compositions towards unwanted plants. They can be applied either before sowings (e.g. on seed treatments, shoots or seedlings) or in the pre-emergence application or post-emergence application of the useful plant. The safeners and the diaminotriazine compound of formula (I) and/or the herbicides B can be applied simultaneously or in succession.

Suitable safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

Examples of preferred safeners C are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Especially preferred safeners C are benoxacor, cloquintocet, cyprosulfamide, dichlormid, fenchlorazole, fenclorim, flurazole, fluxofenim, furilazole, isoxadifen, mefenpyr, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Particularly preferred safeners C are benoxacor, cloquintocet, cyprosulfamide, dichlormid, fenchlorazole, fenclorim, furilazole, isoxadifen, mefenpyr, naphtalic anhydride, 4-(dichloroacetyl)-1-oxa-4-azaspiro[4.5]decane (MON4660,

CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Particularly preferred safeners C, which, as component C, are constituent of the composition according to the invention are the safeners C as defined above; in particular the safeners C.1 - C.17 listed below in table C:

Table C:

| | Safener C |
|---|---|
| C.1 | benoxacor |
| C.2 | cloquintocet |
| C.3 | cloquintocet-mexyl |
| C.4 | cyprosulfamide |
| C.5 | dichlormid |
| C.6 | fenchlorazole |
| C.7 | fenchlorazole-ethyl |
| C.8 | fenclorim |
| C.9 | furilazole |
| C.10 | isoxadifen |
| C.11 | isoxadifen-ethyl |
| C.12 | mefenpyr |
| C.13 | mefenpyr-diethyl |
| C.14 | naphtalic acid anhydride |
| C.15 | 4-(dichloroacetyl)-1-oxa-4-azaspiro[4.5]decane (MON4660, CAS 71526-07-3) |
| C.16 | 2,2,5-trimethyl-3-(dichloroacetyl)-1,3-oxazolidine (R-29148, CAS 52836-31-4) |
| C.17 | N-(2-Methoxybenzoyl)-4-[(methylaminocarbonyl)amino] benzenesulfonamide (CAS 129531-12-0) |

The active compounds B of groups b1) to b15) and the safener compounds C are known herbicides and safeners, see, for example, The Compendium of Pesticide Common Names (http://www.alanwood.net/pesticides/); Farm Chemicals Handbook 2000 volume 86, Meister Publishing Company, 2000; B. Hock, C. Fedtke, R. R. Schmidt, Herbizide [Herbicides], Georg Thieme Verlag, Stuttgart 1995; W. H. Ahrens, Herbicide Handbook, 7th edition, Weed Science Society of America, 1994; and K. K. Hatzios, Herbicide Handbook, Supplement for the 7th edition, Weed Science Society of America, 1998. 2,2,5-Trimethyl-3-(dichloroacetyl)-1,3-oxazolidine [CAS No. 52836-31-4] is also referred to as R-29148. 4-(Dichloroacetyl)-1-oxa-4-azaspiro[4.5]decane [CAS No. 71526-07-3] is also referred to as AD-67 and MON 4660.

The assignment of the active compounds to the respective mechanisms of action is based on current knowledge. If several mechanisms of action apply to one active compound, this substance was only assigned to one mechanism of action.

Active compounds B and C having a carboxyl group can be employed in the form of the acid, in the form of an agriculturally suitable salt as mentioned above or else in the form of an agriculturally acceptable derivative in the compositions according to the invention.

In the case of dicamba, suitable salts include those, where the counterion is an agriculturally acceptable cation. For example, suitable salts of dicamba are dicamba-sodium, dicamba-potassium, dicamba-methylammonium, dicamba-dimethylammonium, dicamba-isopropylammonium, dicamba-diglycolamine, dicamba-olamine, dicamba-diolamine, dicamba-trolamine, dicamba-N,N-bis-(3-aminopropyl)methylamine and dicamba-diethylenetriamine. Examples of a suitable ester are dicamba-methyl and dicamba-butotyl.

Suitable salts of 2,4-D are 2,4-D-ammonium, 2,4-D-dimethylammonium, 2,4-D-diethylammonium, 2,4-D-diethanolammonium (2,4-D-diolamine), 2,4-D-triethanolammonium, 2,4-D-isopropylammonium, 2,4-D-triisopropanolammonium, 2,4-D-heptylammonium, 2,4-D-dodecylammonium, 2,4-D-tetradecylammonium, 2,4-D-triethylammonium, 2,4-D-tris(2-hydroxypropyl)ammonium, 2,4-D-tris(isopropyl)ammonium, 2,4-D-trolamine, 2,4-D-lithium, 2,4-D-sodium. Examples of suitable esters of 2,4-D are 2,4-D-butotyl, 2,4-D-2-butoxypropyl, 2,4-D-3-butoxypropyl, 2,4-D-butyl, 2,4-D-ethyl,

2,4-D-ethylhexyl, 2,4-D-isobutyl, 2,4-D-isooctyl, 2,4-D-isopropyl, 2,4-D-meptyl, 2,4-D-methyl, 2,4-D-octyl, 2,4-D-pentyl, 2,4-D-propyl, 2,4-D-tefuryl and clacyfos.

Suitable salts of 2,4-DB are for example 2,4-DB-sodium, 2,4-DB-potassium and 2,4-DB-dimethylammonium. Suitable esters of 2,4-DB are for example 2,4-DB-butyl and 2,4-DB-isoctyl.

Suitable salts of dichlorprop are for example dichlorprop-sodium, dichlorprop-potassium and dichlorprop-dimethylammonium. Examples of suitable esters of dichlorprop are dichlorprop-butotyl and dichlorprop-isoctyl.

Suitable salts and esters of MCPA include MCPA-butotyl, MCPA-butyl, MCPA-dimethylammonium, MCPA-diolamine, MCPA-ethyl, MCPA-thioethyl, MCPA-2-ethylhexyl, MCPA-isobutyl, MCPA-isoctyl, MCPA-isopropyl, MCPA-isopropylammonium, MCPA-methyl, MCPA-olamine, MCPA-potassium, MCPA-sodium and MCPA-trolamine.

A suitable salt of MCPB is MCPB sodium. A suitable ester of MCPB is MCPB-ethyl.

Suitable salts of clopyralid are clopyralid-potassium, clopyralid-olamine and clopyralid-tris-(2-hydroxypropyl)ammonium. Example of suitable esters of clopyralid is clopyralid-methyl.

Examples of a suitable ester of fluroxypyr are fluroxypyr-meptyl and fluroxypyr-2-butoxy-1-methylethyl, wherein fluroxypyr-meptyl is preferred.

Suitable salts of picloram are picloram-dimethylammonium, picloram-potassium, picloram-triisopropanolammonium, picloram-triisopropylammonium and picloram-trolamine. A suitable ester of picloram is picloram-isoctyl.

A suitable salt of triclopyr is triclopyr-triethylammonium. Suitable esters of triclopyr are for example triclopyr-ethyl and triclopyr-butotyl.

Suitable salts and esters of chloramben include chloramben-ammonium, chloramben-diolamine, chloramben-methyl, chloramben-methylammonium and chloramben-sodium. Suitable salts and esters of 2,3,6-TBA include 2,3,6-TBA-dimethylammonium, 2,3,6-TBA-lithium, 2,3,6-TBA-potassium and 2,3,6-TBA-sodium.

Suitable salts and esters of aminopyralid include aminopyralid-potassium, aminopyralid-dimethylammonium, and aminopyralid-tris(2-hydroxypropyl)ammonium.

Suitable salts of glyphosate are for example glyphosate-ammonium, glyphosate-diammonium, glyphoste-dimethylammonium, glyphosate-isopropylammonium, glyphosate-potassium, glyphosate-sodium, glyphosate-trimesium as well as the ethanolamine and diethanolamine salts, preferably glyphosate-diammonium, glyphosate-isopropylammonium and glyphosate-trimesium (sulfosate).

A suitable salt of glufosinate is for example glufosinate-ammonium.

A suitable salt of glufosinate-P is for example glufosinate-P-ammonium.

Suitable salts and esters of bromoxynil are for example bromoxynil-butyrate, bromoxynil-heptanoate, bromoxynil-octanoate, bromoxynil-potassium and bromoxynil-sodium.

Suitable salts and esters of ioxonil are for example ioxonil-octanoate, ioxonil-potassium and ioxonil-sodium.

Suitable salts and esters of mecoprop include mecoprop-butotyl, mecoprop-dimethylammonium, mecoprop-diolamine, mecoprop-ethadyl, mecoprop-2-ethylhexyl, mecoprop-isoctyl, mecoprop-methyl, mecoprop-potassium, mecoprop-sodium and mecoprop-trolamine.

Suitable salts of mecoprop-P are for example mecoprop-P-butotyl, mecoprop-P-dimethylammonium, mecoprop-P-2-ethylhexyl, mecoprop-P-isobutyl, mecoprop-P-potassium and mecoprop-P-sodium.

A suitable salt of diflufenzopyr is for example diflufenzopyr-sodium.

A suitable salt of naptalam is for example naptalam-sodium.

Suitable salts and esters of aminocyclopyrachlor are for example aminocyclopyrachlor-dimethylammonium, aminocyclopyrachlor-methyl, aminocyclopyrachlor-triisopropanolammonium, aminocyclopyrachlor-sodium and aminocyclopyrachlor-potassium.

A suitable salt of quinclorac is for example quinclorac-dimethylammonium.

A suitable salt of quinmerac is for example quinclorac-dimethylammonium.

A suitable salt of imazamox is for example imazamox-ammonium.

Suitable salts of imazapic are for example imazapic-ammonium and imazapic-isopropylammonium.

Suitable salts of imazapyr are for example imazapyr-ammonium and imazapyr-isopropylammonium.

A suitable salt of imazaquin is for example imazaquin-ammonium.

Suitable salts of imazethapyr are for example imazethapyr-ammonium and imazethapyr-isopropylammonium.

A suitable salt of topramezone is for example topramezone-sodium.

According to another preferred embodiment of the invention, the composition comprises at least one, preferably exactly one compound of formula (I) and as component B at least one, preferably exactly one, herbicide B.

According to another preferred embodiment of the invention, the composition comprises at least one, preferably exactly one compound of formula (I) and at least two, preferably exactly two, herbicides B different from each other.

According to another preferred embodiment of the invention, the composition comprises at least one, preferably exactly one compound of formula (I) and at least three, preferably exactly three, herbicides B different from each other.

According to another preferred embodiment of the invention, the composition comprises at least one, preferably exactly one compound of formula (I) and as component C at least one, preferably exactly one, safener C.

According to another preferred embodiment of the invention, the composition comprises at least one, preferably exactly one compound of formula (I) as component B at least one, preferably exactly one, herbicide B, and as component C at least one, preferably exactly one safener C.

According to another preferred embodiment of the invention, the composition comprises at least one, preferably exactly one compound of formula (I), at least two, preferably exactly two herbicides B different from each other, and as component C at least one, preferably exactly one, safener C.

According to another preferred embodiment of the invention, the composition comprises at least one, preferably exactly one compound of formula (I) at least three, preferably exactly three herbicides B different from each other, and as component C at least one, preferably exactly one, safener C.

Here and below, the term "binary compositions" includes compositions comprising one or more, for example 1, 2 or 3, active compounds of the formula (I) and either one or more, for example 1, 2 or 3, herbicides B or one or more safeners C.

Correspondingly, the term "ternary compositions" includes compositions comprising one or more, for example 1, 2 or 3, active compounds of the formula (I), one or more, for example 1, 2 or 3, herbicides B and one or more, for example 1, 2 or 3, safeners C.

In binary compositions comprising at least one compound of the formula (I) as component A and at least one herbicide B, the weight ratio of the active compounds A:B is generally in the range of from 1:1000 to 1000:1, preferably in the range of from 1:500 to 500:1, in particular in the range of from 1:250 to 250:1 and particularly preferably in the range of from 1:75 to 75:1.

In binary compositions comprising at least one compound of the formula (I) as component A and at least one safener C, the weight ratio of the active compounds A:C is generally in the range of from 1:1000 to 1000:1, preferably in the range of from 1:500 to 500:1, in particular in the range of from 1:250 to 250:1 and particularly preferably in the range of from 1:75 to 75:1.

In ternary compositions comprising at least one compound of formula (I) as component A, at least one herbicide B and at least one safener C, the relative proportions by weight of the components A:B are generally in the range of from 1:1000 to 1000:1, preferably in the range of from 1:500 to 500:1, in particular in the range of from 1:250 to 250:1 and particularly preferably in the range of from 1:75 to 75:1, the weight ratio of the components A:C is generally in the range of from 1:1000 to 1000:1, preferably in the range of from 1:500 to 500:1, in particular in the range of from 1:250 to 250:1 and particularly preferably in the range of from 1:75 to 75:1, and the weight ratio of the components B:C is generally in the range of from 1:1000 to 1000:1, preferably in the range of from 1:500 to 500:1, in particular in the range of from 1:250 to 250:1 and particularly preferably in the range of from 1:75 to 75:1. The weight ratio of components A + B to component C is preferably in the range of from 1:500 to 500:1, in particular in the range of from 1:250 to 250:1 and particularly preferably in the range of from 1:75 to 75:1.

The weight ratios of the individual components in the preferred mixtures mentioned below are within the limits given above, in particular within the preferred limits.

The following combinations indicated by the code AN-X.Y/M.Z, wherein

AN-X refers to the numbers of tables A1-1 to A5-15 above;
Y refers to a particular row of a table AN-X above,
M.Z refers to a particular row of table M below.

represent particular embodiments of the invention:

| | |
|---|---|
| A1-1.1/M.1 to A1-1.180/M.3545 | A1-22.1/M.1 to A1-22.180/M.3545 |
| A1-2.1/M.1 to A1-2.180/M.3545 | A1-23.1/M.1 to A1-23.180/M.3545 |
| A1-3.1/M.1 to A1-3.180/M.3545 | A1-24.1/M.1 to A1-24.180/M.3545 |
| A1-4.1/M.1 to A1-4.180/M.3545 | A1-25.1/M.1 to A1-25.180/M.3545 |
| A1-5.1/M.1 to A1-5.180/M.3545 | A1-26.1/M.1 to A1-26.180/M.3545 |
| A1-6.1/M.1 to A1-6.180/M.3545 | A1-27.1/M.1 to A1-27.180/M.3545 |
| A1-7.1/M.1 to A1-7.180/M.3545 | A1-28.1/M.1 to A1-28.180/M.3545 |
| A1-8.1/M.1 to A1-8.180/M.3545 | A1-29.1/M.1 to A1-29.180/M.3545 |
| A1-9.1/M.1 to A1-9.180/M.3545 | A1-30.1/M.1 to A1-30.180/M.3545 |
| A1-10.1/M.1 to A1-10.180/M.3545 | |
| A1-11.1/M.1 to A1-11.180/M.3545 | A2-1.1/M.1 to A2-1.90/M.3545 |
| A1-12.1/M.1 to A1-12.180/M.3545 | A2-2.1/M.1 to A2-2.90/M.3545 |
| A1-13.1/M.1 to A1-13.180/M.3545 | A2-3.1/M.1 to A2-3.90/M.3545 |

(continued)

| | |
|---|---|
| A1-14.1/M.1 to A1-14.180/M.3545 | A2-4.1/M.1 to A2-4.90/M.3545 |
| A1-15.1/M.1 to A1-15.180/M.3545 | A2-5.1/M.1 to A2-5.90/M.3545 |
| A1-16.1/M.1 to A1-16.180/M.3545 | A2-6.1/M.1 to A2-6.90/M.3545 |
| A1-17.1/M.1 to A1-17.180/M.3545 | A2-7.1/M.1 to A2-7.90/M.3545 |
| A1-18.1/M.1 to A1-18.180/M.3545 | A2-8.1/M.1 to A2-8.90/M.3545 |
| A1-19.1/M.1 to A1-19.180/M.3545 | A2-9.1/M.1 to A2-9.90/M.3545 |
| A1-20.1/M.1 to A1-20.180/M.3545 | A2-10.1/M.1 to A2-10.90/M.3545 |
| A1-21.1/M.1 to A1-21.180/M.3545 | A2-11.1/M.1 to A2-11.90/M.3545 |
| A2-12.1/M.1 to A2-12.90/M.3545 | A3-15.1/M.1 to A3-15.36/M.3545 |
| A2-13.1/M.1 to A2-13.90/M.3545 | |
| A2-14.1/M.1 to A2-14.90/M.3545 | A4-1.1/M.1 to A4-1.288/M.3545 |
| A2-15.1/M.1 to A2-15.90/M.3545 | A4-2.1/M.1 to A4-2.288/M.3545 |
| A2-16.1/M.1 to A2-16.90/M.3545 | A4-3.1/M.1 to A4-3.288/M.3545 |
| A2-17.1/M.1 to A2-17.90/M.3545 | A4-4.1/M.1 to A4-4.288/M.3545 |
| A2-18.1/M.1 to A2-18.90/M.3545 | A4-5.1/M.1 to A4-5.288/M.3545 |
| A2-19.1/M.1 to A2-19.90/M.3545 | A4-6.1/M.1 to A4-6.288/M.3545 |
| A2-20.1/M.1 to A2-20.90/M.3545 | A4-7.1/M.1 to A4-7.288/M.3545 |
| A2-21.1/M.1 to A2-21.90/M.3545 | A4-8.1/M.1 to A4-8.288/M.3545 |
| A2-22.1/M.1 to A2-22.90/M.3545 | A4-9.1/M.1 to A4-9.288/M.3545 |
| A2-23.1/M.1 to A2-23.90/M.3545 | A4-10.1/M.1 to A4-10.288/M.3545 |
| A2-24.1/M.1 to A2-24.90/M.3545 | A4-11.1/M.1 to A4-11.288/M.3545 |
| A2-25.1/M.1 to A2-25.90/M.3545 | A4-12.1/M.1 to A4-12.288/M.3545 |
| A2-26.1/M.1 to A2-26.90/M.3545 | A4-13.1/M.1 to A4-13.288/M.3545 |
| A2-27.1/M.1 to A2-27.90/M.3545 | A4-14.1/M.1 to A4-14.288/M.3545 |
| A2-28.1/M.1 to A2-28.90/M.3545 | A4-15.1/M.1 to A4-15.288/M.3545 |
| A2-29.1/M.1 to A2-29.90/M.3545 | |
| A2-30.1/M.1 to A2-30.90/M.3545 | A5-1.1/M.1 to A5-1.54/M.3545 |
| | A5-2.1/M.1 to A5-2.54/M.3545 |
| A3-1.1/M.1 to A3-1.36/M.3545 | A5-3.1/M.1 to A5-3.54/M.3545 |
| A3-2.1/M.1 to A3-2.36/M.3545 | A5-4.1/M.1 to A5-4.54/M.3545 |
| A3-3.1/M.1 to A3-3.36/M.3545 | A5-5.1/M.1 to A5-5.54/M.3545 |
| A3-4.1/M.1 to A3-4.36/M.3545 | A5-6.1/M.1 to A5-6.54/M.3545 |
| A3-5.1/M.1 to A3-5.36/M.3545 | A5-7.1/M.1 to A5-7.54/M.3545 |
| A3-6.1/M.1 to A3-6.36/M.3545 | A5-8.1/M.1 to A5-8.54/M.3545 |
| A3-7.1/M.1 to A3-7.36/M.3545 | A5-9.1/M.1 to A5-9.54/M.3545 |
| A3-8.1/M.1 to A3-8.36/M.3545 | A5-10.1/M.1 to A5-10.54/M.3545 |
| A3-9.1/M.1 to A3-9.36/M.3545 | A5-11.1/M.1 to A5-11.54/M.3545 |
| A3-10.1/M.1 to A3-10.36/M.3545 | A5-12.1/M.1 to A5-12.54/M.3545 |
| A3-11.1/M.1 to A3-11.36/M.3545 | A5-13.1/M.1 to A5-13.54/M.3545 |
| A3-12.1/M.1 to A3-12.36/M.3545 | A5-14.1/M.1 to A5-14.54/M.3545 |
| A3-13.1/M.1 to A3-13.36/M.3545 | A5-15.1/M.1 to A5-15.54/M.3545 |
| A3-14.1/M.1 to A3-14.36/M.3545 | |

Hence, the code A1-1.1/M.1 refers to the combination comprising compound A1-1.1 of table A1-1 together with herbicide B and safener C defined in row M.1 of table M, i.e.

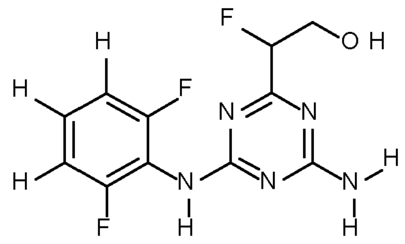

A1-1.1 + clethodim B.1

The code A3-12.2/M.35 refers to the combination comprising compound A3-12.2 of table A3-12 together with herbicide B and safener C defined in row M.32 of table M, i.e.

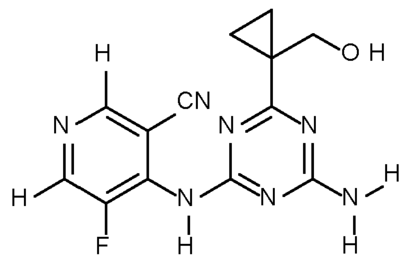

A3-12.2 + imazapic B.32

The code A4-7.280/M.2011 refers to the combination comprising compound A4-7.280 of table A4-7 together with herbicide B and safener C defined in row M.2011 of table M, i.e.

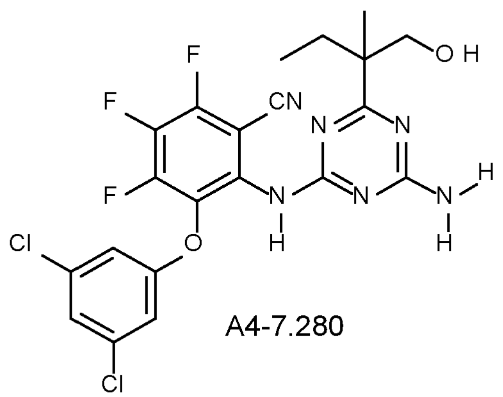

A4-7.280

+ nicosulfuron B.51 + isoxadifen C.10.

Table M (compositions M.1 to M.3545):

| comp. no. | herbicide B | safener C |
|---|---|---|
| M.1 | B.1 | -- |
| M.2 | B.2 | -- |
| M.3 | B.3 | -- |
| M.4 | B.4 | -- |
| M.5 | B.5 | -- |
| M.6 | B.6 | -- |
| M.7 | B.7 | -- |
| M.8 | B.8 | -- |

| comp. no. | herbicide B | safener C |
|---|---|---|
| M.9 | B.9 | -- |
| M.10 | B.10 | -- |
| M.11 | B.11 | -- |
| M.12 | B.12 | -- |
| M.13 | B.13 | -- |
| M.14 | B.14 | -- |
| M.15 | B.15 | -- |
| M.16 | B.16 | -- |

| comp. no. | herbicide B | safener C |
|---|---|---|
| M.17 | B.17 | -- |
| M.18 | B.18 | -- |
| M.19 | B.19 | -- |
| M.20 | B.20 | -- |
| M.21 | B.21 | -- |
| M.22 | B.22 | -- |
| M.23 | B.23 | -- |
| M.24 | B.24 | -- |
| M.25 | B.25 | -- |
| M.26 | B.26 | -- |
| M.27 | B.27 | -- |
| M.28 | B.28 | -- |
| M.29 | B.29 | -- |
| M.30 | B.30 | -- |
| M.31 | B.31 | -- |
| M.32 | B.32 | -- |
| M.33 | B.33 | -- |
| M.34 | B.34 | -- |
| M.35 | B.35 | -- |
| M.36 | B.36 | -- |
| M.37 | B.37 | -- |
| M.38 | B.38 | -- |
| M.39 | B.39 | -- |
| M.40 | B.40 | -- |
| M.41 | B.41 | -- |
| M.42 | B.42 | -- |
| M.43 | B.43 | -- |
| M.44 | B.44 | -- |
| M.45 | B.45 | -- |
| M.46 | B.46 | -- |
| M.47 | B.47 | -- |
| M.48 | B.48 | -- |
| M.49 | B.49 | -- |
| M.50 | B.50 | -- |
| M.51 | B.51 | -- |
| M.52 | B.52 | -- |
| M.53 | B.53 | -- |
| M.54 | B.54 | -- |
| M.55 | B.55 | -- |
| M.56 | B.56 | -- |

| comp. no. | herbicide B | safener C |
|---|---|---|
| M.57 | B.57 | -- |
| M.58 | B.58. | -- |
| M.59 | B.59 | -- |
| M.60 | B.60 | -- |
| M.61 | B.61 | -- |
| M.62 | B.62 | -- |
| M.63 | B.63 | -- |
| M.64 | B.64 | -- |
| M.65 | B.65 | -- |
| M.66 | B.66 | -- |
| M.67 | B.67 | -- |
| M.68 | B.68 | -- |
| M.69 | B.69 | -- |
| M.70 | B.70 | -- |
| M.71 | B.71 | -- |
| M.72 | B.72 | -- |
| M.73 | B.73 | -- |
| M.74 | B.74 | -- |
| M.75 | B.75 | -- |
| M.76 | B.76 | -- |
| M.77 | B.77 | -- |
| M.78 | B.78 | -- |
| M.79 | B.79 | -- |
| M.80 | B.80 | -- |
| M.81 | B.81 | -- |
| M.82 | B.82 | -- |
| M.83 | B.83 | -- |
| M.84 | B.84 | -- |
| M.85 | B.85 | -- |
| M.86 | B.86 | -- |
| M.87 | B.87 | -- |
| M.88 | B.88 | -- |
| M.89 | B.89 | -- |
| M.90 | B.90 | -- |
| M.91 | B.91 | -- |
| M.92 | B.92 | -- |
| M.93 | B.93 | -- |
| M.94 | B.94 | -- |
| M.95 | B.95 | -- |
| M.96 | B.96 | -- |

| comp. no. | herbicide B | safener C |
|---|---|---|
| M.97 | B.97 | -- |
| M.98 | B.98 | -- |
| M.99 | B.99 | -- |
| M.100 | B.100 | -- |
| M.101 | B.101 | -- |
| M.102 | B.102 | -- |
| M.103 | B.103 | -- |
| M.104 | B.104 | -- |
| M.105 | B.105 | -- |
| M.106 | B.106 | -- |
| M.107 | B.107 | -- |
| M.108 | B.108 | -- |
| M.109 | B.109 | -- |
| M.110 | B.110 | -- |
| M.111 | B.111 | -- |
| M.112 | B.112 | -- |
| M.113 | B.113 | -- |
| M.114 | B.114 | -- |
| M.115 | B.115 | -- |
| M.116 | B.116 | -- |
| M.117 | B.117 | -- |
| M.118 | B.118 | -- |
| M.119 | B.119 | -- |
| M.120 | B.120 | -- |
| M.121 | B.121 | -- |
| M.122 | B.122 | -- |
| M.123 | B.123 | -- |
| M.124 | B.124 | -- |
| M.125 | B.125 | -- |
| M.126 | B.126 | -- |
| M.127 | B.127 | -- |
| M.128 | B.128 | -- |
| M.129 | B.129 | -- |
| M.130 | B.130 | -- |
| M.131 | B.131 | -- |
| M.132 | B.132 | -- |
| M.133 | B.133 | -- |
| M.134 | B.134 | -- |
| M.135 | B.135 | -- |
| M.136 | B.136 | -- |

| comp. no. | herbicide B | safener C |
|---|---|---|
| M.137 | B.137 | -- |
| M.138 | B.138 | -- |
| M.139 | B.139 | -- |
| M.140 | B.140 | -- |
| M.141 | B.141 | -- |
| M.142 | B.142 | -- |
| M.143 | B.143 | -- |
| M.144 | B.144 | -- |
| M.145 | B.145 | -- |
| M.146 | B.146 | -- |
| M.147 | B.147 | -- |
| M.148 | B.148 | -- |
| M.149 | B.149 | -- |
| M.150 | B.150 | -- |
| M.151 | B.151 | -- |
| M.152 | B.152 | -- |
| M.153 | B.153 | -- |
| M.154 | B.154 | -- |
| M.155 | B.155 | -- |
| M.156 | B.156 | -- |
| M.157 | B.157 | -- |
| M.158 | B.158 | -- |
| M.159 | B.159 | -- |
| M.160 | B.160 | -- |
| M.161 | B.161 | -- |
| M.162 | B.162 | -- |
| M.163 | B.163 | -- |
| M.164 | B.164 | -- |
| M.165 | B.165 | -- |
| M.166 | B.166 | -- |
| M.167 | B.167 | -- |
| M.168 | B.168 | -- |
| M.169 | B.169 | -- |
| M.170 | B.170 | -- |
| M.171 | B.171 | -- |
| M.172 | B.172 | -- |
| M.173 | B.173 | -- |
| M.174 | B.174 | -- |
| M.175 | B.175 | -- |
| M.176 | B.176 | -- |

| comp. no. | herbicide B | safener C |
|---|---|---|
| M.177 | B.177 | -- |
| M.178 | B.178 | -- |
| M.179 | B.179 | -- |
| M.180 | B.180 | -- |
| M.181 | B.181 | -- |
| M.182 | B.182 | -- |
| M.183 | B.183 | -- |
| M.184 | B.184 | -- |
| M.185 | B.185 | -- |
| M.186 | B.186 | -- |
| M.187 | B.187 | -- |
| M.188 | B.188 | -- |
| M.189 | B.189 | -- |
| M.190 | B.190 | -- |
| M.191 | B.191 | -- |
| M.192 | B.192 | -- |
| M.193 | B.193 | -- |
| M.194 | B.194 | -- |
| M.195 | B.195 | -- |
| M.196 | B.196 | -- |
| M.197 | B.1 | C.1 |
| M.198 | B.2 | C.1 |
| M.199 | B.3 | C.1 |
| M.200 | B.4 | C.1 |
| M.201 | B.5 | C.1 |
| M.202 | B.6 | C.1 |
| M.203 | B.7 | C.1 |
| M.204 | B.8 | C.1 |
| M.205 | B.9 | C.1 |
| M.206 | B.10 | C.1 |
| M.207 | B.11 | C.1 |
| M.208 | B.12 | C.1 |
| M.209 | B.13 | C.1 |
| M.210 | B.14 | C.1 |
| M.211 | B.15 | C.1 |
| M.212 | B.16 | C.1 |
| M.213 | B.17 | C.1 |
| M.214 | B.18 | C.1 |
| M.215 | B.19 | C.1 |
| M.216 | B.20 | C.1 |

| comp. no. | herbicide B | safener C |
|---|---|---|
| M.217 | B.21 | C.1 |
| M.218 | B.22 | C.1 |
| M.219 | B.23 | C.1 |
| M.220 | B.24 | C.1 |
| M.221 | B.25 | C.1 |
| M.222 | B.26 | C.1 |
| M.223 | B.27 | C.1 |
| M.224 | B.28 | C.1 |
| M.225 | B.29 | C.1 |
| M.226 | B.30 | C.1 |
| M.227 | B.31 | C.1 |
| M.228 | B.32 | C.1 |
| M.229 | B.33 | C.1 |
| M.230 | B.34 | C.1 |
| M.231 | B.35 | C.1 |
| M.232 | B.36 | C.1 |
| M.233 | B.37 | C.1 |
| M.234 | B.38 | C.1 |
| M.235 | B.39 | C.1 |
| M.236 | B.40 | C.1 |
| M.237 | B.41 | C.1 |
| M.238 | B.42 | C.1 |
| M.239 | B.43 | C.1 |
| M.240 | B.44 | C.1 |
| M.241 | B.45 | C.1 |
| M.242 | B.46 | C.1 |
| M.243 | B.47 | C.1 |
| M.244 | B.48 | C.1 |
| M.245 | B.49 | C.1 |
| M.246 | B.50 | C.1 |
| M.247 | B.51 | C.1 |
| M.248 | B.52 | C.1 |
| M.249 | B.53 | C.1 |
| M.250 | B.54 | C.1 |
| M.251 | B.55 | C.1 |
| M.252 | B.56 | C.1 |
| M.253 | B.57 | C.1 |
| M.254 | B.58. | C.1 |
| M.255 | B.59 | C.1 |
| M.256 | B.60 | C.1 |

| comp. no. | herbicide B | safener C |
|---|---|---|
| M.257 | B.61 | C.1 |
| M.258 | B.62 | C.1 |
| M.259 | B.63 | C.1 |
| M.260 | B.64 | C.1 |
| M.261 | B.65 | C.1 |
| M.262 | B.66 | C.1 |
| M.263 | B.67 | C.1 |
| M.264 | B.68 | C.1 |
| M.265 | B.69 | C.1 |
| M.266 | B.70 | C.1 |
| M.267 | B.71 | C.1 |
| M.268 | B.72 | C.1 |
| M.269 | B.73 | C.1 |
| M.270 | B.74 | C.1 |
| M.271 | B.75 | C.1 |
| M.272 | B.76 | C.1 |
| M.273 | B.77 | C.1 |
| M.274 | B.78 | C.1 |
| M.275 | B.79 | C.1 |
| M.276 | B.80 | C.1 |
| M.277 | B.81 | C.1 |
| M.278 | B.82 | C.1 |
| M.279 | B.83 | C.1 |
| M.280 | B.84 | C.1 |
| M.281 | B.85 | C.1 |
| M.282 | B.86 | C.1 |
| M.283 | B.87 | C.1 |
| M.284 | B.88 | C.1 |
| M.285 | B.89 | C.1 |
| M.286 | B.90 | C.1 |
| M.287 | B.91 | C.1 |
| M.288 | B.92 | C.1 |
| M.289 | B.93 | C.1 |
| M.290 | B.94 | C.1 |
| M.291 | B.95 | C.1 |
| M.292 | B.96 | C.1 |
| M.293 | B.97 | C.1 |
| M.294 | B.98 | C.1 |
| M.295 | B.99 | C.1 |
| M.296 | B.100 | C.1 |

| comp. no. | herbicide B | safener C |
|---|---|---|
| M.297 | B.101 | C.1 |
| M.298 | B.102 | C.1 |
| M.299 | B.103 | C.1 |
| M.300 | B.104 | C.1 |
| M.301 | B.105 | C.1 |
| M.302 | B.106 | C.1 |
| M.303 | B.107 | C.1 |
| M.304 | B.108 | C.1 |
| M.305 | B.109 | C.1 |
| M.306 | B.110 | C.1 |
| M.307 | B.111 | C.1 |
| M.308 | B.112 | C.1 |
| M.309 | B.113 | C.1 |
| M.310 | B.114 | C.1 |
| M.311 | B.115 | C.1 |
| M.312 | B.116 | C.1 |
| M.313 | B.117 | C.1 |
| M.314 | B.118 | C.1 |
| M.315 | B.119 | C.1 |
| M.316 | B.120 | C.1 |
| M.317 | B.121 | C.1 |
| M.318 | B.122 | C.1 |
| M.319 | B.123 | C.1 |
| M.320 | B.124 | C.1 |
| M.321 | B.125 | C.1 |
| M.322 | B.126 | C.1 |
| M.323 | B.127 | C.1 |
| M.324 | B.128 | C.1 |
| M.325 | B.129 | C.1 |
| M.326 | B.130 | C.1 |
| M.327 | B.131 | C.1 |
| M.328 | B.132 | C.1 |
| M.329 | B.133 | C.1 |
| M.330 | B.134 | C.1 |
| M.331 | B.135 | C.1 |
| M.332 | B.136 | C.1 |
| M.333 | B.137 | C.1 |
| M.334 | B.138 | C.1 |
| M.335 | B.139 | C.1 |
| M.336 | B.140 | C.1 |

| comp. no. | herbicide B | safener C |
|---|---|---|
| M.337 | B.141 | C.1 |
| M.338 | B.142 | C.1 |
| M.339 | B.143 | C.1 |
| M.340 | B.144 | C.1 |
| M.341 | B.145 | C.1 |
| M.342 | B.146 | C.1 |
| M.343 | B.147 | C.1 |
| M.344 | B.148 | C.1 |
| M.345 | B.149 | C.1 |
| M.346 | B.150 | C.1 |
| M.347 | B.151 | C.1 |
| M.348 | B.152 | C.1 |
| M.349 | B.153 | C.1 |
| M.350 | B.154 | C.1 |
| M.351 | B.155 | C.1 |
| M.352 | B.156 | C.1 |
| M.353 | B.157 | C.1 |
| M.354 | B.158 | C.1 |
| M.355 | B.159 | C.1 |
| M.356 | B.160 | C.1 |
| M.357 | B.161 | C.1 |
| M.358 | B.162 | C.1 |
| M.359 | B.163 | C.1 |
| M.360 | B.164 | C.1 |
| M.361 | B.165 | C.1 |
| M.362 | B.166 | C.1 |
| M.363 | B.167 | C.1 |
| M.364 | B.168 | C.1 |
| M.365 | B.169 | C.1 |
| M.366 | B.170 | C.1 |
| M.367 | B.171 | C.1 |
| M.368 | B.172 | C.1 |
| M.369 | B.173 | C.1 |
| M.370 | B.174 | C.1 |
| M.371 | B.175 | C.1 |
| M.372 | B.176 | C.1 |
| M.373 | B.177 | C.1 |
| M.374 | B.178 | C.1 |
| M.375 | B.179 | C.1 |
| M.376 | B.180 | C.1 |

| comp. no. | herbicide B | safener C |
|---|---|---|
| M.377 | B.181 | C.1 |
| M.378 | B.182 | C.1 |
| M.379 | B.183 | C.1 |
| M.380 | B.184 | C.1 |
| M.381 | B.185 | C.1 |
| M.382 | B.186 | C.1 |
| M.383 | B.187 | C.1 |
| M.384 | B.188 | C.1 |
| M.385 | B.189 | C.1 |
| M.386 | B.190 | C.1 |
| M.387 | B.191 | C.1 |
| M.388 | B.192 | C.1 |
| M.389 | B.193 | C.1 |
| M.390 | B.194 | C.1 |
| M.391 | B.195 | C.1 |
| M.392 | B.196 | C.1 |
| M.393 | B.1 | C.2 |
| M.394 | B.2 | C.2 |
| M.395 | B.3 | C.2 |
| M.396 | B.4 | C.2 |
| M.397 | B.5 | C.2 |
| M.398 | B.6 | C.2 |
| M.399 | B.7 | C.2 |
| M.400 | B.8 | C.2 |
| M.401 | B.9 | C.2 |
| M.402 | B.10 | C.2 |
| M.403 | B.11 | C.2 |
| M.404 | B.12 | C.2 |
| M.405 | B.13 | C.2 |
| M.406 | B.14 | C.2 |
| M.407 | B.15 | C.2 |
| M.408 | B.16 | C.2 |
| M.409 | B.17 | C.2 |
| M.410 | B.18 | C.2 |
| M.411 | B.19 | C.2 |
| M.412 | B.20 | C.2 |
| M.413 | B.21 | C.2 |
| M.414 | B.22 | C.2 |
| M.415 | B.23 | C.2 |
| M.416 | B.24 | C.2 |

| comp. no. | herbicide B | safener C |
|---|---|---|
| M.417 | B.25 | C.2 |
| M.418 | B.26 | C.2 |
| M.419 | B.27 | C.2 |
| M.420 | B.28 | C.2 |
| M.421 | B.29 | C.2 |
| M.422 | B.30 | C.2 |
| M.423 | B.31 | C.2 |
| M.424 | B.32 | C.2 |
| M.425 | B.33 | C.2 |
| M.426 | B.34 | C.2 |
| M.427 | B.35 | C.2 |
| M.428 | B.36 | C.2 |
| M.429 | B.37 | C.2 |
| M.430 | B.38 | C.2 |
| M.431 | B.39 | C.2 |
| M.432 | B.40 | C.2 |
| M.433 | B.41 | C.2 |
| M.434 | B.42 | C.2 |
| M.435 | B.43 | C.2 |
| M.436 | B.44 | C.2 |
| M.437 | B.45 | C.2 |
| M.438 | B.46 | C.2 |
| M.439 | B.47 | C.2 |
| M.440 | B.48 | C.2 |
| M.441 | B.49 | C.2 |
| M.442 | B.50 | C.2 |
| M.443 | B.51 | C.2 |
| M.444 | B.52 | C.2 |
| M.445 | B.53 | C.2 |
| M.446 | B.54 | C.2 |
| M.447 | B.55 | C.2 |
| M.448 | B.56 | C.2 |
| M.449 | B.57 | C.2 |
| M.450 | B.58. | C.2 |
| M.451 | B.59 | C.2 |
| M.452 | B.60 | C.2 |
| M.453 | B.61 | C.2 |
| M.454 | B.62 | C.2 |
| M.455 | B.63 | C.2 |
| M.456 | B.64 | C.2 |

| comp. no. | herbicide B | safener C |
|---|---|---|
| M.457 | B.65 | C.2 |
| M.458 | B.66 | C.2 |
| M.459 | B.67 | C.2 |
| M.460 | B.68 | C.2 |
| M.461 | B.69 | C.2 |
| M.462 | B.70 | C.2 |
| M.463 | B.71 | C.2 |
| M.464 | B.72 | C.2 |
| M.465 | B.73 | C.2 |
| M.466 | B.74 | C.2 |
| M.467 | B.75 | C.2 |
| M.468 | B.76 | C.2 |
| M.469 | B.77 | C.2 |
| M.470 | B.78 | C.2 |
| M.471 | B.79 | C.2 |
| M.472 | B.80 | C.2 |
| M.473 | B.81 | C.2 |
| M.474 | B.82 | C.2 |
| M.475 | B.83 | C.2 |
| M.476 | B.84 | C.2 |
| M.477 | B.85 | C.2 |
| M.478 | B.86 | C.2 |
| M.479 | B.87 | C.2 |
| M.480 | B.88 | C.2 |
| M.481 | B.89 | C.2 |
| M.482 | B.90 | C.2 |
| M.483 | B.91 | C.2 |
| M.484 | B.92 | C.2 |
| M.485 | B.93 | C.2 |
| M.486 | B.94 | C.2 |
| M.487 | B.95 | C.2 |
| M.488 | B.96 | C.2 |
| M.489 | B.97 | C.2 |
| M.490 | B.98 | C.2 |
| M.491 | B.99 | C.2 |
| M.492 | B.100 | C.2 |
| M.493 | B.101 | C.2 |
| M.494 | B.102 | C.2 |
| M.495 | B.103 | C.2 |
| M.496 | B.104 | C.2 |

| comp. no. | herbicide B | safener C |
|---|---|---|
| M.497 | B.105 | C.2 |
| M.498 | B.106 | C.2 |
| M.499 | B.107 | C.2 |
| M.500 | B.108 | C.2 |
| M.501 | B.109 | C.2 |
| M.502 | B.110 | C.2 |
| M.503 | B.111 | C.2 |
| M.504 | B.112 | C.2 |
| M.505 | B.113 | C.2 |
| M.506 | B.114 | C.2 |
| M.507 | B.115 | C.2 |
| M.508 | B.116 | C.2 |
| M.509 | B.117 | C.2 |
| M.510 | B.118 | C.2 |
| M.511 | B.119 | C.2 |
| M.512 | B.120 | C.2 |
| M.513 | B.121 | C.2 |
| M.514 | B.122 | C.2 |
| M.515 | B.123 | C.2 |
| M.516 | B.124 | C.2 |
| M.517 | B.125 | C.2 |
| M.518 | B.126 | C.2 |
| M.519 | B.127 | C.2 |
| M.520 | B.128 | C.2 |
| M.521 | B.129 | C.2 |
| M.522 | B.130 | C.2 |
| M.523 | B.131 | C.2 |
| M.524 | B.132 | C.2 |
| M.525 | B.133 | C.2 |
| M.526 | B.134 | C.2 |
| M.527 | B.135 | C.2 |
| M.528 | B.136 | C.2 |
| M.529 | B.137 | C.2 |
| M.530 | B.138 | C.2 |
| M.531 | B.139 | C.2 |
| M.532 | B.140 | C.2 |
| M.533 | B.141 | C.2 |
| M.534 | B.142 | C.2 |
| M.535 | B.143 | C.2 |
| M.536 | B.144 | C.2 |

| comp. no. | herbicide B | safener C |
|---|---|---|
| M.537 | B.145 | C.2 |
| M.538 | B.146 | C.2 |
| M.539 | B.147 | C.2 |
| M.540 | B.148 | C.2 |
| M.541 | B.149 | C.2 |
| M.542 | B.150 | C.2 |
| M.543 | B.151 | C.2 |
| M.544 | B.152 | C.2 |
| M.545 | B.153 | C.2 |
| M.546 | B.154 | C.2 |
| M.547 | B.155 | C.2 |
| M.548 | B.156 | C.2 |
| M.549 | B.157 | C.2 |
| M.550 | B.158 | C.2 |
| M.551 | B.159 | C.2 |
| M.552 | B.160 | C.2 |
| M.553 | B.161 | C.2 |
| M.554 | B.162 | C.2 |
| M.555 | B.163 | C.2 |
| M.556 | B.164 | C.2 |
| M.557 | B.165 | C.2 |
| M.558 | B.166 | C.2 |
| M.559 | B.167 | C.2 |
| M.560 | B.168 | C.2 |
| M.561 | B.169 | C.2 |
| M.562 | B.170 | C.2 |
| M.563 | B.171 | C.2 |
| M.564 | B.172 | C.2 |
| M.565 | B.173 | C.2 |
| M.566 | B.174 | C.2 |
| M.567 | B.175 | C.2 |
| M.568 | B.176 | C.2 |
| M.569 | B.177 | C.2 |
| M.570 | B.178 | C.2 |
| M.571 | B.179 | C.2 |
| M.572 | B.180 | C.2 |
| M.573 | B.181 | C.2 |
| M.574 | B.182 | C.2 |
| M.575 | B.183 | C.2 |
| M.576 | B.184 | C.2 |

| comp. no. | herbicide B | safener C |
|---|---|---|
| M.577 | B.185 | C.2 |
| M.578 | B.186 | C.2 |
| M.579 | B.187 | C.2 |
| M.580 | B.188 | C.2 |
| M.581 | B.189 | C.2 |
| M.582 | B.190 | C.2 |
| M.583 | B.191 | C.2 |
| M.584 | B.192 | C.2 |
| M.585 | B.193 | C.2 |
| M.586 | B.194 | C.2 |
| M.587 | B.195 | C.2 |
| M.588 | B.196 | C.2 |
| M.589 | B.1 | C.3 |
| M.590 | B.2 | C.3 |
| M.591 | B.3 | C.3 |
| M.592 | B.4 | C.3 |
| M.593 | B.5 | C.3 |
| M.594 | B.6 | C.3 |
| M.595 | B.7 | C.3 |
| M.596 | B.8 | C.3 |
| M.597 | B.9 | C.3 |
| M.598 | B.10 | C.3 |
| M.599 | B.11 | C.3 |
| M.600 | B.12 | C.3 |
| M.601 | B.13 | C.3 |
| M.602 | B.14 | C.3 |
| M.603 | B.15 | C.3 |
| M.604 | B.16 | C.3 |
| M.605 | B.17 | C.3 |
| M.606 | B.18 | C.3 |
| M.607 | B.19 | C.3 |
| M.608 | B.20 | C.3 |
| M.609 | B.21 | C.3 |
| M.610 | B.22 | C.3 |
| M.611 | B.23 | C.3 |
| M.612 | B.24 | C.3 |
| M.613 | B.25 | C.3 |
| M.614 | B.26 | C.3 |
| M.615 | B.27 | C.3 |
| M.616 | B.28 | C.3 |

| comp. no. | herbicide B | safener C |
|---|---|---|
| M.617 | B.29 | C.3 |
| M.618 | B.30 | C.3 |
| M.619 | B.31 | C.3 |
| M.620 | B.32 | C.3 |
| M.621 | B.33 | C.3 |
| M.622 | B.34 | C.3 |
| M.623 | B.35 | C.3 |
| M.624 | B.36 | C.3 |
| M.625 | B.37 | C.3 |
| M.626 | B.38 | C.3 |
| M.627 | B.39 | C.3 |
| M.628 | B.40 | C.3 |
| M.629 | B.41 | C.3 |
| M.630 | B.42 | C.3 |
| M.631 | B.43 | C.3 |
| M.632 | B.44 | C.3 |
| M.633 | B.45 | C.3 |
| M.634 | B.46 | C.3 |
| M.635 | B.47 | C.3 |
| M.636 | B.48 | C.3 |
| M.637 | B.49 | C.3 |
| M.638 | B.50 | C.3 |
| M.639 | B.51 | C.3 |
| M.640 | B.52 | C.3 |
| M.641 | B.53 | C.3 |
| M.642 | B.54 | C.3 |
| M.643 | B.55 | C.3 |
| M.644 | B.56 | C.3 |
| M.645 | B.57 | C.3 |
| M.646 | B.58. | C.3 |
| M.647 | B.59 | C.3 |
| M.648 | B.60 | C.3 |
| M.649 | B.61 | C.3 |
| M.650 | B.62 | C.3 |
| M.651 | B.63 | C.3 |
| M.652 | B.64 | C.3 |
| M.653 | B.65 | C.3 |
| M.654 | B.66 | C.3 |
| M.655 | B.67 | C.3 |
| M.656 | B.68 | C.3 |

| comp. no. | herbicide B | safener C |
|---|---|---|
| M.657 | B.69 | C.3 |
| M.658 | B.70 | C.3 |
| M.659 | B.71 | C.3 |
| M.660 | B.72 | C.3 |
| M.661 | B.73 | C.3 |
| M.662 | B.74 | C.3 |
| M.663 | B.75 | C.3 |
| M.664 | B.76 | C.3 |
| M.665 | B.77 | C.3 |
| M.666 | B.78 | C.3 |
| M.667 | B.79 | C.3 |
| M.668 | B.80 | C.3 |
| M.669 | B.81 | C.3 |
| M.670 | B.82 | C.3 |
| M.671 | B.83 | C.3 |
| M.672 | B.84 | C.3 |
| M.673 | B.85 | C.3 |
| M.674 | B.86 | C.3 |
| M.675 | B.87 | C.3 |
| M.676 | B.88 | C.3 |
| M.677 | B.89 | C.3 |
| M.678 | B.90 | C.3 |
| M.679 | B.91 | C.3 |
| M.680 | B.92 | C.3 |
| M.681 | B.93 | C.3 |
| M.682 | B.94 | C.3 |
| M.683 | B.95 | C.3 |
| M.684 | B.96 | C.3 |
| M.685 | B.97 | C.3 |
| M.686 | B.98 | C.3 |
| M.687 | B.99 | C.3 |
| M.688 | B.100 | C.3 |
| M.689 | B.101 | C.3 |
| M.690 | B.102 | C.3 |
| M.691 | B.103 | C.3 |
| M.692 | B.104 | C.3 |
| M.693 | B.105 | C.3 |
| M.694 | B.106 | C.3 |
| M.695 | B.107 | C.3 |
| M.696 | B.108 | C.3 |

| comp. no. | herbicide B | safener C |
|---|---|---|
| M.697 | B.109 | C.3 |
| M.698 | B.110 | C.3 |
| M.699 | B.111 | C.3 |
| M.700 | B.112 | C.3 |
| M.701 | B.113 | C.3 |
| M.702 | B.114 | C.3 |
| M.703 | B.115 | C.3 |
| M.704 | B.116 | C.3 |
| M.705 | B.117 | C.3 |
| M.706 | B.118 | C.3 |
| M.707 | B.119 | C.3 |
| M.708 | B.120 | C.3 |
| M.709 | B.121 | C.3 |
| M.710 | B.122 | C.3 |
| M.711 | B.123 | C.3 |
| M.712 | B.124 | C.3 |
| M.713 | B.125 | C.3 |
| M.714 | B.126 | C.3 |
| M.715 | B.127 | C.3 |
| M.716 | B.128 | C.3 |
| M.717 | B.129 | C.3 |
| M.718 | B.130 | C.3 |
| M.719 | B.131 | C.3 |
| M.720 | B.132 | C.3 |
| M.721 | B.133 | C.3 |
| M.722 | B.134 | C.3 |
| M.723 | B.135 | C.3 |
| M.724 | B.136 | C.3 |
| M.725 | B.137 | C.3 |
| M.726 | B.138 | C.3 |
| M.727 | B.139 | C.3 |
| M.728 | B.140 | C.3 |
| M.729 | B.141 | C.3 |
| M.730 | B.142 | C.3 |
| M.731 | B.143 | C.3 |
| M.732 | B.144 | C.3 |
| M.733 | B.145 | C.3 |
| M.734 | B.146 | C.3 |
| M.735 | B.147 | C.3 |
| M.736 | B.148 | C.3 |

| comp. no. | herbicide B | safener C |
|---|---|---|
| M.737 | B.149 | C.3 |
| M.738 | B.150 | C.3 |
| M.739 | B.151 | C.3 |
| M.740 | B.152 | C.3 |
| M.741 | B.153 | C.3 |
| M.742 | B.154 | C.3 |
| M.743 | B.155 | C.3 |
| M.744 | B.156 | C.3 |
| M.745 | B.157 | C.3 |
| M.746 | B.158 | C.3 |
| M.747 | B.159 | C.3 |
| M.748 | B.160 | C.3 |
| M.749 | B.161 | C.3 |
| M.750 | B.162 | C.3 |
| M.751 | B.163 | C.3 |
| M.752 | B.164 | C.3 |
| M.753 | B.165 | C.3 |
| M.754 | B.166 | C.3 |
| M.755 | B.167 | C.3 |
| M.756 | B.168 | C.3 |
| M.757 | B.169 | C.3 |
| M.758 | B.170 | C.3 |
| M.759 | B.171 | C.3 |
| M.760 | B.172 | C.3 |
| M.761 | B.173 | C.3 |
| M.762 | B.174 | C.3 |
| M.763 | B.175 | C.3 |
| M.764 | B.176 | C.3 |
| M.765 | B.177 | C.3 |
| M.766 | B.178 | C.3 |
| M.767 | B.179 | C.3 |
| M.768 | B.180 | C.3 |
| M.769 | B.181 | C.3 |
| M.770 | B.182 | C.3 |
| M.771 | B.183 | C.3 |
| M.772 | B.184 | C.3 |
| M.773 | B.185 | C.3 |
| M.774 | B.186 | C.3 |
| M.775 | B.187 | C.3 |
| M.776 | B.188 | C.3 |

| comp. no. | herbicide B | safener C |
|---|---|---|
| M.777 | B.189 | C.3 |
| M.778 | B.190 | C.3 |
| M.779 | B.191 | C.3 |
| M.780 | B.192 | C.3 |
| M.781 | B.193 | C.3 |
| M.782 | B.194 | C.3 |
| M.783 | B.195 | C.3 |
| M.784 | B.196 | C.3 |
| M.785 | B.1 | C.4 |
| M.786 | B.2 | C.4 |
| M.787 | B.3 | C.4 |
| M.788 | B.4 | C.4 |
| M.789 | B.5 | C.4 |
| M.790 | B.6 | C.4 |
| M.791 | B.7 | C.4 |
| M.792 | B.8 | C.4 |
| M.793 | B.9 | C.4 |
| M.794 | B.10 | C.4 |
| M.795 | B.11 | C.4 |
| M.796 | B.12 | C.4 |
| M.797 | B.13 | C.4 |
| M.798 | B.14 | C.4 |
| M.799 | B.15 | C.4 |
| M.800 | B.16 | C.4 |
| M.801 | B.17 | C.4 |
| M.802 | B.18 | C.4 |
| M.803 | B.19 | C.4 |
| M.804 | B.20 | C.4 |
| M.805 | B.21 | C.4 |
| M.806 | B.22 | C.4 |
| M.807 | B.23 | C.4 |
| M.808 | B.24 | C.4 |
| M.809 | B.25 | C.4 |
| M.810 | B.26 | C.4 |
| M.811 | B.27 | C.4 |
| M.812 | B.28 | C.4 |
| M.813 | B.29 | C.4 |
| M.814 | B.30 | C.4 |
| M.815 | B.31 | C.4 |
| M.816 | B.32 | C.4 |

| comp. no. | herbicide B | safener C |
|---|---|---|
| M.817 | B.33 | C.4 |
| M.818 | B.34 | C.4 |
| M.819 | B.35 | C.4 |
| M.820 | B.36 | C.4 |
| M.821 | B.37 | C.4 |
| M.822 | B.38 | C.4 |
| M.823 | B.39 | C.4 |
| M.824 | B.40 | C.4 |
| M.825 | B.41 | C.4 |
| M.826 | B.42 | C.4 |
| M.827 | B.43 | C.4 |
| M.828 | B.44 | C.4 |
| M.829 | B.45 | C.4 |
| M.830 | B.46 | C.4 |
| M.831 | B.47 | C.4 |
| M.832 | B.48 | C.4 |
| M.833 | B.49 | C.4 |
| M.834 | B.50 | C.4 |
| M.835 | B.51 | C.4 |
| M.836 | B.52 | C.4 |
| M.837 | B.53 | C.4 |
| M.838 | B.54 | C.4 |
| M.839 | B.55 | C.4 |
| M.840 | B.56 | C.4 |
| M.841 | B.57 | C.4 |
| M.842 | B.58. | C.4 |
| M.843 | B.59 | C.4 |
| M.844 | B.60 | C.4 |
| M.845 | B.61 | C.4 |
| M.846 | B.62 | C.4 |
| M.847 | B.63 | C.4 |
| M.848 | B.64 | C.4 |
| M.849 | B.65 | C.4 |
| M.850 | B.66 | C.4 |
| M.851 | B.67 | C.4 |
| M.852 | B.68 | C.4 |
| M.853 | B.69 | C.4 |
| M.854 | B.70 | C.4 |
| M.855 | B.71 | C.4 |
| M.856 | B.72 | C.4 |

| comp. no. | herbicide B | safener C |
|---|---|---|
| M.857 | B.73 | C.4 |
| M.858 | B.74 | C.4 |
| M.859 | B.75 | C.4 |
| M.860 | B.76 | C.4 |
| M.861 | B.77 | C.4 |
| M.862 | B.78 | C.4 |
| M.863 | B.79 | C.4 |
| M.864 | B.80 | C.4 |
| M.865 | B.81 | C.4 |
| M.866 | B.82 | C.4 |
| M.867 | B.83 | C.4 |
| M.868 | B.84 | C.4 |
| M.869 | B.85 | C.4 |
| M.870 | B.86 | C.4 |
| M.871 | B.87 | C.4 |
| M.872 | B.88 | C.4 |
| M.873 | B.89 | C.4 |
| M.874 | B.90 | C.4 |
| M.875 | B.91 | C.4 |
| M.876 | B.92 | C.4 |
| M.877 | B.93 | C.4 |
| M.878 | B.94 | C.4 |
| M.879 | B.95 | C.4 |
| M.880 | B.96 | C.4 |
| M.881 | B.97 | C.4 |
| M.882 | B.98 | C.4 |
| M.883 | B.99 | C.4 |
| M.884 | B.100 | C.4 |
| M.885 | B.101 | C.4 |
| M.886 | B.102 | C.4 |
| M.887 | B.103 | C.4 |
| M.888 | B.104 | C.4 |
| M.889 | B.105 | C.4 |
| M.890 | B.106 | C.4 |
| M.891 | B.107 | C.4 |
| M.892 | B.108 | C.4 |
| M.893 | B.109 | C.4 |
| M.894 | B.110 | C.4 |
| M.895 | B.111 | C.4 |
| M.896 | B.112 | C.4 |

| comp. no. | herbicide B | safener C |
|---|---|---|
| M.897 | B.113 | C.4 |
| M.898 | B.114 | C.4 |
| M.899 | B.115 | C.4 |
| M.900 | B.116 | C.4 |
| M.901 | B.117 | C.4 |
| M.902 | B.118 | C.4 |
| M.903 | B.119 | C.4 |
| M.904 | B.120 | C.4 |
| M.905 | B.121 | C.4 |
| M.906 | B.122 | C.4 |
| M.907 | B.123 | C.4 |
| M.908 | B.124 | C.4 |
| M.909 | B.125 | C.4 |
| M.910 | B.126 | C.4 |
| M.911 | B.127 | C.4 |
| M.912 | B.128 | C.4 |
| M.913 | B.129 | C.4 |
| M.914 | B.130 | C.4 |
| M.915 | B.131 | C.4 |
| M.916 | B.132 | C.4 |
| M.917 | B.133 | C.4 |
| M.918 | B.134 | C.4 |
| M.919 | B.135 | C.4 |
| M.920 | B.136 | C.4 |
| M.921 | B.137 | C.4 |
| M.922 | B.138 | C.4 |
| M.923 | B.139 | C.4 |
| M.924 | B.140 | C.4 |
| M.925 | B.141 | C.4 |
| M.926 | B.142 | C.4 |
| M.927 | B.143 | C.4 |
| M.928 | B.144 | C.4 |
| M.929 | B.145 | C.4 |
| M.930 | B.146 | C.4 |
| M.931 | B.147 | C.4 |
| M.932 | B.148 | C.4 |
| M.933 | B.149 | C.4 |
| M.934 | B.150 | C.4 |
| M.935 | B.151 | C.4 |
| M.936 | B.152 | C.4 |

| comp. no. | herbicide B | safener C |
|---|---|---|
| M.937 | B.153 | C.4 |
| M.938 | B.154 | C.4 |
| M.939 | B.155 | C.4 |
| M.940 | B.156 | C.4 |
| M.941 | B.157 | C.4 |
| M.942 | B.158 | C.4 |
| M.943 | B.159 | C.4 |
| M.944 | B.160 | C.4 |
| M.945 | B.161 | C.4 |
| M.946 | B.162 | C.4 |
| M.947 | B.163 | C.4 |
| M.948 | B.164 | C.4 |
| M.949 | B.165 | C.4 |
| M.950 | B.166 | C.4 |
| M.951 | B.167 | C.4 |
| M.952 | B.168 | C.4 |
| M.953 | B.169 | C.4 |
| M.954 | B.170 | C.4 |
| M.955 | B.171 | C.4 |
| M.956 | B.172 | C.4 |
| M.957 | B.173 | C.4 |
| M.958 | B.174 | C.4 |
| M.959 | B.175 | C.4 |
| M.960 | B.176 | C.4 |
| M.961 | B.177 | C.4 |
| M.962 | B.178 | C.4 |
| M.963 | B.179 | C.4 |
| M.964 | B.180 | C.4 |
| M.965 | B.181 | C.4 |
| M.966 | B.182 | C.4 |
| M.967 | B.183 | C.4 |
| M.968 | B.184 | C.4 |
| M.969 | B.185 | C.4 |
| M.970 | B.186 | C.4 |
| M.971 | B.187 | C.4 |
| M.972 | B.188 | C.4 |
| M.973 | B.189 | C.4 |
| M.974 | B.190 | C.4 |
| M.975 | B.191 | C.4 |
| M.976 | B.192 | C.4 |

| comp. no. | herbicide B | safener C |
|---|---|---|
| M.977 | B.193 | C.4 |
| M.978 | B.194 | C.4 |
| M.979 | B.195 | C.4 |
| M.980 | B.196 | C.4 |
| M.981 | B.1 | C.5 |
| M.982 | B.2 | C.5 |
| M.983 | B.3 | C.5 |
| M.984 | B.4 | C.5 |
| M.985 | B.5 | C.5 |
| M.986 | B.6 | C.5 |
| M.987 | B.7 | C.5 |
| M.988 | B.8 | C.5 |
| M.989 | B.9 | C.5 |
| M.990 | B.10 | C.5 |
| M.991 | B.11 | C.5 |
| M.992 | B.12 | C.5 |
| M.993 | B.13 | C.5 |
| M.994 | B.14 | C.5 |
| M.995 | B.15 | C.5 |
| M.996 | B.16 | C.5 |
| M.997 | B.17 | C.5 |
| M.998 | B.18 | C.5 |
| M.999 | B.19 | C.5 |
| M.1000 | B.20 | C.5 |
| M.1001 | B.21 | C.5 |
| M.1002 | B.22 | C.5 |
| M.1003 | B.23 | C.5 |
| M.1004 | B.24 | C.5 |
| M.1005 | B.25 | C.5 |
| M.1006 | B.26 | C.5 |
| M.1007 | B.27 | C.5 |
| M.1008 | B.28 | C.5 |
| M.1009 | B.29 | C.5 |
| M.1010 | B.30 | C.5 |
| M.1011 | B.31 | C.5 |
| M.1012 | B.32 | C.5 |
| M.1013 | B.33 | C.5 |
| M.1014 | B.34 | C.5 |
| M.1015 | B.35 | C.5 |
| M.1016 | B.36 | C.5 |

| comp. no. | herbicide B | safener C |
|---|---|---|
| M.1017 | B.37 | C.5 |
| M.1018 | B.38 | C.5 |
| M.1019 | B.39 | C.5 |
| M.1020 | B.40 | C.5 |
| M.1021 | B.41 | C.5 |
| M.1022 | B.42 | C.5 |
| M.1023 | B.43 | C.5 |
| M.1024 | B.44 | C.5 |
| M.1025 | B.45 | C.5 |
| M.1026 | B.46 | C.5 |
| M.1027 | B.47 | C.5 |
| M.1028 | B.48 | C.5 |
| M.1029 | B.49 | C.5 |
| M.1030 | B.50 | C.5 |
| M.1031 | B.51 | C.5 |
| M.1032 | B.52 | C.5 |
| M.1033 | B.53 | C.5 |
| M.1034 | B.54 | C.5 |
| M.1035 | B.55 | C.5 |
| M.1036 | B.56 | C.5 |
| M.1037 | B.57 | C.5 |
| M.1038 | B.58. | C.5 |
| M.1039 | B.59 | C.5 |
| M.1040 | B.60 | C.5 |
| M.1041 | B.61 | C.5 |
| M.1042 | B.62 | C.5 |
| M.1043 | B.63 | C.5 |
| M.1044 | B.64 | C.5 |
| M.1045 | B.65 | C.5 |
| M.1046 | B.66 | C.5 |
| M.1047 | B.67 | C.5 |
| M.1048 | B.68 | C.5 |
| M.1049 | B.69 | C.5 |
| M.1050 | B.70 | C.5 |
| M.1051 | B.71 | C.5 |
| M.1052 | B.72 | C.5 |
| M.1053 | B.73 | C.5 |
| M.1054 | B.74 | C.5 |
| M.1055 | B.75 | C.5 |
| M.1056 | B.76 | C.5 |

| comp. no. | herbicide B | safener C |
|---|---|---|
| M.1057 | B.77 | C.5 |
| M.1058 | B.78 | C.5 |
| M.1059 | B.79 | C.5 |
| M.1060 | B.80 | C.5 |
| M.1061 | B.81 | C.5 |
| M.1062 | B.82 | C.5 |
| M.1063 | B.83 | C.5 |
| M.1064 | B.84 | C.5 |
| M.1065 | B.85 | C.5 |
| M.1066 | B.86 | C.5 |
| M.1067 | B.87 | C.5 |
| M.1068 | B.88 | C.5 |
| M.1069 | B.89 | C.5 |
| M.1070 | B.90 | C.5 |
| M.1071 | B.91 | C.5 |
| M.1072 | B.92 | C.5 |
| M.1073 | B.93 | C.5 |
| M.1074 | B.94 | C.5 |
| M.1075 | B.95 | C.5 |
| M.1076 | B.96 | C.5 |
| M.1077 | B.97 | C.5 |
| M.1078 | B.98 | C.5 |
| M.1079 | B.99 | C.5 |
| M.1080 | B.100 | C.5 |
| M.1081 | B.101 | C.5 |
| M.1082 | B.102 | C.5 |
| M.1083 | B.103 | C.5 |
| M.1084 | B.104 | C.5 |
| M.1085 | B.105 | C.5 |
| M.1086 | B.106 | C.5 |
| M.1087 | B.107 | C.5 |
| M.1088 | B.108 | C.5 |
| M.1089 | B.109 | C.5 |
| M.1090 | B.110 | C.5 |
| M.1091 | B.111 | C.5 |
| M.1092 | B.112 | C.5 |
| M.1093 | B.113 | C.5 |
| M.1094 | B.114 | C.5 |
| M.1095 | B.115 | C.5 |
| M.1096 | B.116 | C.5 |

| comp. no. | herbicide B | safener C |
|---|---|---|
| M.1097 | B.117 | C.5 |
| M.1098 | B.118 | C.5 |
| M.1099 | B.119 | C.5 |
| M.1100 | B.120 | C.5 |
| M.1101 | B.121 | C.5 |
| M.1102 | B.122 | C.5 |
| M.1103 | B.123 | C.5 |
| M.1104 | B.124 | C.5 |
| M.1105 | B.125 | C.5 |
| M.1106 | B.126 | C.5 |
| M.1107 | B.127 | C.5 |
| M.1108 | B.128 | C.5 |
| M.1109 | B.129 | C.5 |
| M.1110 | B.130 | C.5 |
| M.1111 | B.131 | C.5 |
| M.1112 | B.132 | C.5 |
| M.1113 | B.133 | C.5 |
| M.1114 | B.134 | C.5 |
| M.1115 | B.135 | C.5 |
| M.1116 | B.136 | C.5 |
| M.1117 | B.137 | C.5 |
| M.1118 | B.138 | C.5 |
| M.1119 | B.139 | C.5 |
| M.1120 | B.140 | C.5 |
| M.1121 | B.141 | C.5 |
| M.1122 | B.142 | C.5 |
| M.1123 | B.143 | C.5 |
| M.1124 | B.144 | C.5 |
| M.1125 | B.145 | C.5 |
| M.1126 | B.146 | C.5 |
| M.1127 | B.147 | C.5 |
| M.1128 | B.148 | C.5 |
| M.1129 | B.149 | C.5 |
| M.1130 | B.150 | C.5 |
| M.1131 | B.151 | C.5 |
| M.1132 | B.152 | C.5 |
| M.1133 | B.153 | C.5 |
| M.1134 | B.154 | C.5 |
| M.1135 | B.155 | C.5 |
| M.1136 | B.156 | C.5 |

| comp. no. | herbicide B | safener C |
|---|---|---|
| M.1137 | B.157 | C.5 |
| M.1138 | B.158 | C.5 |
| M.1139 | B.159 | C.5 |
| M.1140 | B.160 | C.5 |
| M.1141 | B.161 | C.5 |
| M.1142 | B.162 | C.5 |
| M.1143 | B.163 | C.5 |
| M.1144 | B.164 | C.5 |
| M.1145 | B.165 | C.5 |
| M.1146 | B.166 | C.5 |
| M.1147 | B.167 | C.5 |
| M.1148 | B.168 | C.5 |
| M.1149 | B.169 | C.5 |
| M.1150 | B.170 | C.5 |
| M.1151 | B.171 | C.5 |
| M.1152 | B.172 | C.5 |
| M.1153 | B.173 | C.5 |
| M.1154 | B.174 | C.5 |
| M.1155 | B.175 | C.5 |
| M.1156 | B.176 | C.5 |
| M.1157 | B.177 | C.5 |
| M.1158 | B.178 | C.5 |
| M.1159 | B.179 | C.5 |
| M.1160 | B.180 | C.5 |
| M.1161 | B.181 | C.5 |
| M.1162 | B.182 | C.5 |
| M.1163 | B.183 | C.5 |
| M.1164 | B.184 | C.5 |
| M.1165 | B.185 | C.5 |
| M.1166 | B.186 | C.5 |
| M.1167 | B.187 | C.5 |
| M.1168 | B.188 | C.5 |
| M.1169 | B.189 | C.5 |
| M.1170 | B.190 | C.5 |
| M.1171 | B.191 | C.5 |
| M.1172 | B.192 | C.5 |
| M.1173 | B.193 | C.5 |
| M.1174 | B.194 | C.5 |
| M.1175 | B.195 | C.5 |
| M.1176 | B.196 | C.5 |

| comp. no. | herbicide B | safener C |
|---|---|---|
| M.1177 | B.1 | C.6 |
| M.1178 | B.2 | C.6 |
| M.1179 | B.3 | C.6 |
| M.1180 | B.4 | C.6 |
| M.1181 | B.5 | C.6 |
| M.1182 | B.6 | C.6 |
| M.1183 | B.7 | C.6 |
| M.1184 | B.8 | C.6 |
| M.1185 | B.9 | C.6 |
| M.1186 | B.10 | C.6 |
| M.1187 | B.11 | C.6 |
| M.1188 | B.12 | C.6 |
| M.1189 | B.13 | C.6 |
| M.1190 | B.14 | C.6 |
| M.1191 | B.15 | C.6 |
| M.1192 | B.16 | C.6 |
| M.1193 | B.17 | C.6 |
| M.1194 | B.18 | C.6 |
| M.1195 | B.19 | C.6 |
| M.1196 | B.20 | C.6 |
| M.1197 | B.21 | C.6 |
| M.1198 | B.22 | C.6 |
| M.1199 | B.23 | C.6 |
| M.1200 | B.24 | C.6 |
| M.1201 | B.25 | C.6 |
| M.1202 | B.26 | C.6 |
| M.1203 | B.27 | C.6 |
| M.1204 | B.28 | C.6 |
| M.1205 | B.29 | C.6 |
| M.1206 | B.30 | C.6 |
| M.1207 | B.31 | C.6 |
| M.1208 | B.32 | C.6 |
| M.1209 | B.33 | C.6 |
| M.1210 | B.34 | C.6 |
| M.1211 | B.35 | C.6 |
| M.1212 | B.36 | C.6 |
| M.1213 | B.37 | C.6 |
| M.1214 | B.38 | C.6 |
| M.1215 | B.39 | C.6 |
| M.1216 | B.40 | C.6 |

| comp. no. | herbicide B | safener C |
|---|---|---|
| M.1217 | B.41 | C.6 |
| M.1218 | B.42 | C.6 |
| M.1219 | B.43 | C.6 |
| M.1220 | B.44 | C.6 |
| M.1221 | B.45 | C.6 |
| M.1222 | B.46 | C.6 |
| M.1223 | B.47 | C.6 |
| M.1224 | B.48 | C.6 |
| M.1225 | B.49 | C.6 |
| M.1226 | B.50 | C.6 |
| M.1227 | B.51 | C.6 |
| M.1228 | B.52 | C.6 |
| M.1229 | B.53 | C.6 |
| M.1230 | B.54 | C.6 |
| M.1231 | B.55 | C.6 |
| M.1232 | B.56 | C.6 |
| M.1233 | B.57 | C.6 |
| M.1234 | B.58. | C.6 |
| M.1235 | B.59 | C.6 |
| M.1236 | B.60 | C.6 |
| M.1237 | B.61 | C.6 |
| M.1238 | B.62 | C.6 |
| M.1239 | B.63 | C.6 |
| M.1240 | B.64 | C.6 |
| M.1241 | B.65 | C.6 |
| M.1242 | B.66 | C.6 |
| M.1243 | B.67 | C.6 |
| M.1244 | B.68 | C.6 |
| M.1245 | B.69 | C.6 |
| M.1246 | B.70 | C.6 |
| M.1247 | B.71 | C.6 |
| M.1248 | B.72 | C.6 |
| M.1249 | B.73 | C.6 |
| M.1250 | B.74 | C.6 |
| M.1251 | B.75 | C.6 |
| M.1252 | B.76 | C.6 |
| M.1253 | B.77 | C.6 |
| M.1254 | B.78 | C.6 |
| M.1255 | B.79 | C.6 |
| M.1256 | B.80 | C.6 |

| comp. no. | herbicide B | safener C |
|---|---|---|
| M.1257 | B.81 | C.6 |
| M.1258 | B.82 | C.6 |
| M.1259 | B.83 | C.6 |
| M.1260 | B.84 | C.6 |
| M.1261 | B.85 | C.6 |
| M.1262 | B.86 | C.6 |
| M.1263 | B.87 | C.6 |
| M.1264 | B.88 | C.6 |
| M.1265 | B.89 | C.6 |
| M.1266 | B.90 | C.6 |
| M.1267 | B.91 | C.6 |
| M.1268 | B.92 | C.6 |
| M.1269 | B.93 | C.6 |
| M.1270 | B.94 | C.6 |
| M.1271 | B.95 | C.6 |
| M.1272 | B.96 | C.6 |
| M.1273 | B.97 | C.6 |
| M.1274 | B.98 | C.6 |
| M.1275 | B.99 | C.6 |
| M.1276 | B.100 | C.6 |
| M.1277 | B.101 | C.6 |
| M.1278 | B.102 | C.6 |
| M.1279 | B.103 | C.6 |
| M.1280 | B.104 | C.6 |
| M.1281 | B.105 | C.6 |
| M.1282 | B.106 | C.6 |
| M.1283 | B.107 | C.6 |
| M.1284 | B.108 | C.6 |
| M.1285 | B.109 | C.6 |
| M.1286 | B.110 | C.6 |
| M.1287 | B.111 | C.6 |
| M.1288 | B.112 | C.6 |
| M.1289 | B.113 | C.6 |
| M.1290 | B.114 | C.6 |
| M.1291 | B.115 | C.6 |
| M.1292 | B.116 | C.6 |
| M.1293 | B.117 | C.6 |
| M.1294 | B.118 | C.6 |
| M.1295 | B.119 | C.6 |
| M.1296 | B.120 | C.6 |

| comp. no. | herbicide B | safener C |
|---|---|---|
| M.1297 | B.121 | C.6 |
| M.1298 | B.122 | C.6 |
| M.1299 | B.123 | C.6 |
| M.1300 | B.124 | C.6 |
| M.1301 | B.125 | C.6 |
| M.1302 | B.126 | C.6 |
| M.1303 | B.127 | C.6 |
| M.1304 | B.128 | C.6 |
| M.1305 | B.129 | C.6 |
| M.1306 | B.130 | C.6 |
| M.1307 | B.131 | C.6 |
| M.1308 | B.132 | C.6 |
| M.1309 | B.133 | C.6 |
| M.1310 | B.134 | C.6 |
| M.1311 | B.135 | C.6 |
| M.1312 | B.136 | C.6 |
| M.1313 | B.137 | C.6 |
| M.1314 | B.138 | C.6 |
| M.1315 | B.139 | C.6 |
| M.1316 | B.140 | C.6 |
| M.1317 | B.141 | C.6 |
| M.1318 | B.142 | C.6 |
| M.1319 | B.143 | C.6 |
| M.1320 | B.144 | C.6 |
| M.1321 | B.145 | C.6 |
| M.1322 | B.146 | C.6 |
| M.1323 | B.147 | C.6 |
| M.1324 | B.148 | C.6 |
| M.1325 | B.149 | C.6 |
| M.1326 | B.150 | C.6 |
| M.1327 | B.151 | C.6 |
| M.1328 | B.152 | C.6 |
| M.1329 | B.153 | C.6 |
| M.1330 | B.154 | C.6 |
| M.1331 | B.155 | C.6 |
| M.1332 | B.156 | C.6 |
| M.1333 | B.157 | C.6 |
| M.1334 | B.158 | C.6 |
| M.1335 | B.159 | C.6 |
| M.1336 | B.160 | C.6 |

| comp. no. | herbicide B | safener C |
|---|---|---|
| M.1337 | B.161 | C.6 |
| M.1338 | B.162 | C.6 |
| M.1339 | B.163 | C.6 |
| M.1340 | B.164 | C.6 |
| M.1341 | B.165 | C.6 |
| M.1342 | B.166 | C.6 |
| M.1343 | B.167 | C.6 |
| M.1344 | B.168 | C.6 |
| M.1345 | B.169 | C.6 |
| M.1346 | B.170 | C.6 |
| M.1347 | B.171 | C.6 |
| M.1348 | B.172 | C.6 |
| M.1349 | B.173 | C.6 |
| M.1350 | B.174 | C.6 |
| M.1351 | B.175 | C.6 |
| M.1352 | B.176 | C.6 |
| M.1353 | B.177 | C.6 |
| M.1354 | B.178 | C.6 |
| M.1355 | B.179 | C.6 |
| M.1356 | B.180 | C.6 |
| M.1357 | B.181 | C.6 |
| M.1358 | B.182 | C.6 |
| M.1359 | B.183 | C.6 |
| M.1360 | B.184 | C.6 |
| M.1361 | B.185 | C.6 |
| M.1362 | B.186 | C.6 |
| M.1363 | B.187 | C.6 |
| M.1364 | B.188 | C.6 |
| M.1365 | B.189 | C.6 |
| M.1366 | B.190 | C.6 |
| M.1367 | B.191 | C.6 |
| M.1368 | B.192 | C.6 |
| M.1369 | B.193 | C.6 |
| M.1370 | B.194 | C.6 |
| M.1371 | B.195 | C.6 |
| M.1372 | B.196 | C.6 |
| M.1373 | B.1 | C.7 |
| M.1374 | B.2 | C.7 |
| M.1375 | B.3 | C.7 |
| M.1376 | B.4 | C.7 |

| comp. no. | herbicide B | safener C |
|---|---|---|
| M.1377 | B.5 | C.7 |
| M.1378 | B.6 | C.7 |
| M.1379 | B.7 | C.7 |
| M.1380 | B.8 | C.7 |
| M.1381 | B.9 | C.7 |
| M.1382 | B.10 | C.7 |
| M.1383 | B.11 | C.7 |
| M.1384 | B.12 | C.7 |
| M.1385 | B.13 | C.7 |
| M.1386 | B.14 | C.7 |
| M.1387 | B.15 | C.7 |
| M.1388 | B.16 | C.7 |
| M.1389 | B.17 | C.7 |
| M.1390 | B.18 | C.7 |
| M.1391 | B.19 | C.7 |
| M.1392 | B.20 | C.7 |
| M.1393 | B.21 | C.7 |
| M.1394 | B.22 | C.7 |
| M.1395 | B.23 | C.7 |
| M.1396 | B.24 | C.7 |
| M.1397 | B.25 | C.7 |
| M.1398 | B.26 | C.7 |
| M.1399 | B.27 | C.7 |
| M.1400 | B.28 | C.7 |
| M.1401 | B.29 | C.7 |
| M.1402 | B.30 | C.7 |
| M.1403 | B.31 | C.7 |
| M.1404 | B.32 | C.7 |
| M.1405 | B.33 | C.7 |
| M.1406 | B.34 | C.7 |
| M.1407 | B.35 | C.7 |
| M.1408 | B.36 | C.7 |
| M.1409 | B.37 | C.7 |
| M.1410 | B.38 | C.7 |
| M.1411 | B.39 | C.7 |
| M.1412 | B.40 | C.7 |
| M.1413 | B.41 | C.7 |
| M.1414 | B.42 | C.7 |
| M.1415 | B.43 | C.7 |
| M.1416 | B.44 | C.7 |

| comp. no. | herbicide B | safener C |
|---|---|---|
| M.1417 | B.45 | C.7 |
| M.1418 | B.46 | C.7 |
| M.1419 | B.47 | C.7 |
| M.1420 | B.48 | C.7 |
| M.1421 | B.49 | C.7 |
| M.1422 | B.50 | C.7 |
| M.1423 | B.51 | C.7 |
| M.1424 | B.52 | C.7 |
| M.1425 | B.53 | C.7 |
| M.1426 | B.54 | C.7 |
| M.1427 | B.55 | C.7 |
| M.1428 | B.56 | C.7 |
| M.1429 | B.57 | C.7 |
| M.1430 | B.58. | C.7 |
| M.1431 | B.59 | C.7 |
| M.1432 | B.60 | C.7 |
| M.1433 | B.61 | C.7 |
| M.1434 | B.62 | C.7 |
| M.1435 | B.63 | C.7 |
| M.1436 | B.64 | C.7 |
| M.1437 | B.65 | C.7 |
| M.1438 | B.66 | C.7 |
| M.1439 | B.67 | C.7 |
| M.1440 | B.68 | C.7 |
| M.1441 | B.69 | C.7 |
| M.1442 | B.70 | C.7 |
| M.1443 | B.71 | C.7 |
| M.1444 | B.72 | C.7 |
| M.1445 | B.73 | C.7 |
| M.1446 | B.74 | C.7 |
| M.1447 | B.75 | C.7 |
| M.1448 | B.76 | C.7 |
| M.1449 | B.77 | C.7 |
| M.1450 | B.78 | C.7 |
| M.1451 | B.79 | C.7 |
| M.1452 | B.80 | C.7 |
| M.1453 | B.81 | C.7 |
| M.1454 | B.82 | C.7 |
| M.1455 | B.83 | C.7 |
| M.1456 | B.84 | C.7 |

| comp. no. | herbicide B | safener C |
|---|---|---|
| M.1457 | B.85 | C.7 |
| M.1458 | B.86 | C.7 |
| M.1459 | B.87 | C.7 |
| M.1460 | B.88 | C.7 |
| M.1461 | B.89 | C.7 |
| M.1462 | B.90 | C.7 |
| M.1463 | B.91 | C.7 |
| M.1464 | B.92 | C.7 |
| M.1465 | B.93 | C.7 |
| M.1466 | B.94 | C.7 |
| M.1467 | B.95 | C.7 |
| M.1468 | B.96 | C.7 |
| M.1469 | B.97 | C.7 |
| M.1470 | B.98 | C.7 |
| M.1471 | B.99 | C.7 |
| M.1472 | B.100 | C.7 |
| M.1473 | B.101 | C.7 |
| M.1474 | B.102 | C.7 |
| M.1475 | B.103 | C.7 |
| M.1476 | B.104 | C.7 |
| M.1477 | B.105 | C.7 |
| M.1478 | B.106 | C.7 |
| M.1479 | B.107 | C.7 |
| M.1480 | B.108 | C.7 |
| M.1481 | B.109 | C.7 |
| M.1482 | B.110 | C.7 |
| M.1483 | B.111 | C.7 |
| M.1484 | B.112 | C.7 |
| M.1485 | B.113 | C.7 |
| M.1486 | B.114 | C.7 |
| M.1487 | B.115 | C.7 |
| M.1488 | B.116 | C.7 |
| M.1489 | B.117 | C.7 |
| M.1490 | B.118 | C.7 |
| M.1491 | B.119 | C.7 |
| M.1492 | B.120 | C.7 |
| M.1493 | B.121 | C.7 |
| M.1494 | B.122 | C.7 |
| M.1495 | B.123 | C.7 |
| M.1496 | B.124 | C.7 |

| comp. no. | herbicide B | safener C |
|---|---|---|
| M.1497 | B.125 | C.7 |
| M.1498 | B.126 | C.7 |
| M.1499 | B.127 | C.7 |
| M.1500 | B.128 | C.7 |
| M.1501 | B.129 | C.7 |
| M.1502 | B.130 | C.7 |
| M.1503 | B.131 | C.7 |
| M.1504 | B.132 | C.7 |
| M.1505 | B.133 | C.7 |
| M.1506 | B.134 | C.7 |
| M.1507 | B.135 | C.7 |
| M.1508 | B.136 | C.7 |
| M.1509 | B.137 | C.7 |
| M.1510 | B.138 | C.7 |
| M.1511 | B.139 | C.7 |
| M.1512 | B.140 | C.7 |
| M.1513 | B.141 | C.7 |
| M.1514 | B.142 | C.7 |
| M.1515 | B.143 | C.7 |
| M.1516 | B.144 | C.7 |
| M.1517 | B.145 | C.7 |
| M.1518 | B.146 | C.7 |
| M.1519 | B.147 | C.7 |
| M.1520 | B.148 | C.7 |
| M.1521 | B.149 | C.7 |
| M.1522 | B.150 | C.7 |
| M.1523 | B.151 | C.7 |
| M.1524 | B.152 | C.7 |
| M.1525 | B.153 | C.7 |
| M.1526 | B.154 | C.7 |
| M.1527 | B.155 | C.7 |
| M.1528 | B.156 | C.7 |
| M.1529 | B.157 | C.7 |
| M.1530 | B.158 | C.7 |
| M.1531 | B.159 | C.7 |
| M.1532 | B.160 | C.7 |
| M.1533 | B.161 | C.7 |
| M.1534 | B.162 | C.7 |
| M.1535 | B.163 | C.7 |
| M.1536 | B.164 | C.7 |

| comp. no. | herbicide B | safener C |
|---|---|---|
| M.1537 | B.165 | C.7 |
| M.1538 | B.166 | C.7 |
| M.1539 | B.167 | C.7 |
| M.1540 | B.168 | C.7 |
| M.1541 | B.169 | C.7 |
| M.1542 | B.170 | C.7 |
| M.1543 | B.171 | C.7 |
| M.1544 | B.172 | C.7 |
| M.1545 | B.173 | C.7 |
| M.1546 | B.174 | C.7 |
| M.1547 | B.175 | C.7 |
| M.1548 | B.176 | C.7 |
| M.1549 | B.177 | C.7 |
| M.1550 | B.178 | C.7 |
| M.1551 | B.179 | C.7 |
| M.1552 | B.180 | C.7 |
| M.1553 | B.181 | C.7 |
| M.1554 | B.182 | C.7 |
| M.1555 | B.183 | C.7 |
| M.1556 | B.184 | C.7 |
| M.1557 | B.185 | C.7 |
| M.1558 | B.186 | C.7 |
| M.1559 | B.187 | C.7 |
| M.1560 | B.188 | C.7 |
| M.1561 | B.189 | C.7 |
| M.1562 | B.190 | C.7 |
| M.1563 | B.191 | C.7 |
| M.1564 | B.192 | C.7 |
| M.1565 | B.193 | C.7 |
| M.1566 | B.194 | C.7 |
| M.1567 | B.195 | C.7 |
| M.1568 | B.196 | C.7 |
| M.1569 | B.1 | C.8 |
| M.1570 | B.2 | C.8 |
| M.1571 | B.3 | C.8 |
| M.1572 | B.4 | C.8 |
| M.1573 | B.5 | C.8 |
| M.1574 | B.6 | C.8 |
| M.1575 | B.7 | C.8 |
| M.1576 | B.8 | C.8 |

| comp. no. | herbicide B | safener C |
|---|---|---|
| M.1577 | B.9 | C.8 |
| M.1578 | B.10 | C.8 |
| M.1579 | B.11 | C.8 |
| M.1580 | B.12 | C.8 |
| M.1581 | B.13 | C.8 |
| M.1582 | B.14 | C.8 |
| M.1583 | B.15 | C.8 |
| M.1584 | B.16 | C.8 |
| M.1585 | B.17 | C.8 |
| M.1586 | B.18 | C.8 |
| M.1587 | B.19 | C.8 |
| M.1588 | B.20 | C.8 |
| M.1589 | B.21 | C.8 |
| M.1590 | B.22 | C.8 |
| M.1591 | B.23 | C.8 |
| M.1592 | B.24 | C.8 |
| M.1593 | B.25 | C.8 |
| M.1594 | B.26 | C.8 |
| M.1595 | B.27 | C.8 |
| M.1596 | B.28 | C.8 |
| M.1597 | B.29 | C.8 |
| M.1598 | B.30 | C.8 |
| M.1599 | B.31 | C.8 |
| M.1600 | B.32 | C.8 |
| M.1601 | B.33 | C.8 |
| M.1602 | B.34 | C.8 |
| M.1603 | B.35 | C.8 |
| M.1604 | B.36 | C.8 |
| M.1605 | B.37 | C.8 |
| M.1606 | B.38 | C.8 |
| M.1607 | B.39 | C.8 |
| M.1608 | B.40 | C.8 |
| M.1609 | B.41 | C.8 |
| M.1610 | B.42 | C.8 |
| M.1611 | B.43 | C.8 |
| M.1612 | B.44 | C.8 |
| M.1613 | B.45 | C.8 |
| M.1614 | B.46 | C.8 |
| M.1615 | B.47 | C.8 |
| M.1616 | B.48 | C.8 |

| comp. no. | herbicide B | safener C |
|---|---|---|
| M.1617 | B.49 | C.8 |
| M.1618 | B.50 | C.8 |
| M.1619 | B.51 | C.8 |
| M.1620 | B.52 | C.8 |
| M.1621 | B.53 | C.8 |
| M.1622 | B.54 | C.8 |
| M.1623 | B.55 | C.8 |
| M.1624 | B.56 | C.8 |
| M.1625 | B.57 | C.8 |
| M.1626 | B.58. | C.8 |
| M.1627 | B.59 | C.8 |
| M.1628 | B.60 | C.8 |
| M.1629 | B.61 | C.8 |
| M.1630 | B.62 | C.8 |
| M.1631 | B.63 | C.8 |
| M.1632 | B.64 | C.8 |
| M.1633 | B.65 | C.8 |
| M.1634 | B.66 | C.8 |
| M.1635 | B.67 | C.8 |
| M.1636 | B.68 | C.8 |
| M.1637 | B.69 | C.8 |
| M.1638 | B.70 | C.8 |
| M.1639 | B.71 | C.8 |
| M.1640 | B.72 | C.8 |
| M.1641 | B.73 | C.8 |
| M.1642 | B.74 | C.8 |
| M.1643 | B.75 | C.8 |
| M.1644 | B.76 | C.8 |
| M.1645 | B.77 | C.8 |
| M.1646 | B.78 | C.8 |
| M.1647 | B.79 | C.8 |
| M.1648 | B.80 | C.8 |
| M.1649 | B.81 | C.8 |
| M.1650 | B.82 | C.8 |
| M.1651 | B.83 | C.8 |
| M.1652 | B.84 | C.8 |
| M.1653 | B.85 | C.8 |
| M.1654 | B.86 | C.8 |
| M.1655 | B.87 | C.8 |
| M.1656 | B.88 | C.8 |

| comp. no. | herbicide B | safener C |
|---|---|---|
| M.1657 | B.89 | C.8 |
| M.1658 | B.90 | C.8 |
| M.1659 | B.91 | C.8 |
| M.1660 | B.92 | C.8 |
| M.1661 | B.93 | C.8 |
| M.1662 | B.94 | C.8 |
| M.1663 | B.95 | C.8 |
| M.1664 | B.96 | C.8 |
| M.1665 | B.97 | C.8 |
| M.1666 | B.98 | C.8 |
| M.1667 | B.99 | C.8 |
| M.1668 | B.100 | C.8 |
| M.1669 | B.101 | C.8 |
| M.1670 | B.102 | C.8 |
| M.1671 | B.103 | C.8 |
| M.1672 | B.104 | C.8 |
| M.1673 | B.105 | C.8 |
| M.1674 | B.106 | C.8 |
| M.1675 | B.107 | C.8 |
| M.1676 | B.108 | C.8 |
| M.1677 | B.109 | C.8 |
| M.1678 | B.110 | C.8 |
| M.1679 | B.111 | C.8 |
| M.1680 | B.112 | C.8 |
| M.1681 | B.113 | C.8 |
| M.1682 | B.114 | C.8 |
| M.1683 | B.115 | C.8 |
| M.1684 | B.116 | C.8 |
| M.1685 | B.117 | C.8 |
| M.1686 | B.118 | C.8 |
| M.1687 | B.119 | C.8 |
| M.1688 | B.120 | C.8 |
| M.1689 | B.121 | C.8 |
| M.1690 | B.122 | C.8 |
| M.1691 | B.123 | C.8 |
| M.1692 | B.124 | C.8 |
| M.1693 | B.125 | C.8 |
| M.1694 | B.126 | C.8 |
| M.1695 | B.127 | C.8 |
| M.1696 | B.128 | C.8 |

| comp. no. | herbicide B | safener C |
|---|---|---|
| M.1697 | B.129 | C.8 |
| M.1698 | B.130 | C.8 |
| M.1699 | B.131 | C.8 |
| M.1700 | B.132 | C.8 |
| M.1701 | B.133 | C.8 |
| M.1702 | B.134 | C.8 |
| M.1703 | B.135 | C.8 |
| M.1704 | B.136 | C.8 |
| M.1705 | B.137 | C.8 |
| M.1706 | B.138 | C.8 |
| M.1707 | B.139 | C.8 |
| M.1708 | B.140 | C.8 |
| M.1709 | B.141 | C.8 |
| M.1710 | B.142 | C.8 |
| M.1711 | B.143 | C.8 |
| M.1712 | B.144 | C.8 |
| M.1713 | B.145 | C.8 |
| M.1714 | B.146 | C.8 |
| M.1715 | B.147 | C.8 |
| M.1716 | B.148 | C.8 |
| M.1717 | B.149 | C.8 |
| M.1718 | B.150 | C.8 |
| M.1719 | B.151 | C.8 |
| M.1720 | B.152 | C.8 |
| M.1721 | B.153 | C.8 |
| M.1722 | B.154 | C.8 |
| M.1723 | B.155 | C.8 |
| M.1724 | B.156 | C.8 |
| M.1725 | B.157 | C.8 |
| M.1726 | B.158 | C.8 |
| M.1727 | B.159 | C.8 |
| M.1728 | B.160 | C.8 |
| M.1729 | B.161 | C.8 |
| M.1730 | B.162 | C.8 |
| M.1731 | B.163 | C.8 |
| M.1732 | B.164 | C.8 |
| M.1733 | B.165 | C.8 |
| M.1734 | B.166 | C.8 |
| M.1735 | B.167 | C.8 |
| M.1736 | B.168 | C.8 |

| comp. no. | herbicide B | safener C |
|---|---|---|
| M.1737 | B.169 | C.8 |
| M.1738 | B.170 | C.8 |
| M.1739 | B.171 | C.8 |
| M.1740 | B.172 | C.8 |
| M.1741 | B.173 | C.8 |
| M.1742 | B.174 | C.8 |
| M.1743 | B.175 | C.8 |
| M.1744 | B.176 | C.8 |
| M.1745 | B.177 | C.8 |
| M.1746 | B.178 | C.8 |
| M.1747 | B.179 | C.8 |
| M.1748 | B.180 | C.8 |
| M.1749 | B.181 | C.8 |
| M.1750 | B.182 | C.8 |
| M.1751 | B.183 | C.8 |
| M.1752 | B.184 | C.8 |
| M.1753 | B.185 | C.8 |
| M.1754 | B.186 | C.8 |
| M.1755 | B.187 | C.8 |
| M.1756 | B.188 | C.8 |
| M.1757 | B.189 | C.8 |
| M.1758 | B.190 | C.8 |
| M.1759 | B.191 | C.8 |
| M.1760 | B.192 | C.8 |
| M.1761 | B.193 | C.8 |
| M.1762 | B.194 | C.8 |
| M.1763 | B.195 | C.8 |
| M.1764 | B.196 | C.8 |
| M.1765 | B.1 | C.9 |
| M.1766 | B.2 | C.9 |
| M.1767 | B.3 | C.9 |
| M.1768 | B.4 | C.9 |
| M.1769 | B.5 | C.9 |
| M.1770 | B.6 | C.9 |
| M.1771 | B.7 | C.9 |
| M.1772 | B.8 | C.9 |
| M.1773 | B.9 | C.9 |
| M.1774 | B.10 | C.9 |
| M.1775 | B.11 | C.9 |
| M.1776 | B.12 | C.9 |

| comp. no. | herbicide B | safener C |
|---|---|---|
| M.1777 | B.13 | C.9 |
| M.1778 | B.14 | C.9 |
| M.1779 | B.15 | C.9 |
| M.1780 | B.16 | C.9 |
| M.1781 | B.17 | C.9 |
| M.1782 | B.18 | C.9 |
| M.1783 | B.19 | C.9 |
| M.1784 | B.20 | C.9 |
| M.1785 | B.21 | C.9 |
| M.1786 | B.22 | C.9 |
| M.1787 | B.23 | C.9 |
| M.1788 | B.24 | C.9 |
| M.1789 | B.25 | C.9 |
| M.1790 | B.26 | C.9 |
| M.1791 | B.27 | C.9 |
| M.1792 | B.28 | C.9 |
| M.1793 | B.29 | C.9 |
| M.1794 | B.30 | C.9 |
| M.1795 | B.31 | C.9 |
| M.1796 | B.32 | C.9 |
| M.1797 | B.33 | C.9 |
| M.1798 | B.34 | C.9 |
| M.1799 | B.35 | C.9 |
| M.1800 | B.36 | C.9 |
| M.1801 | B.37 | C.9 |
| M.1802 | B.38 | C.9 |
| M.1803 | B.39 | C.9 |
| M.1804 | B.40 | C.9 |
| M.1805 | B.41 | C.9 |
| M.1806 | B.42 | C.9 |
| M.1807 | B.43 | C.9 |
| M.1808 | B.44 | C.9 |
| M.1809 | B.45 | C.9 |
| M.1810 | B.46 | C.9 |
| M.1811 | B.47 | C.9 |
| M.1812 | B.48 | C.9 |
| M.1813 | B.49 | C.9 |
| M.1814 | B.50 | C.9 |
| M.1815 | B.51 | C.9 |
| M.1816 | B.52 | C.9 |

| comp. no. | herbicide B | safener C |
|---|---|---|
| M.1817 | B.53 | C.9 |
| M.1818 | B.54 | C.9 |
| M.1819 | B.55 | C.9 |
| M.1820 | B.56 | C.9 |
| M.1821 | B.57 | C.9 |
| M.1822 | B.58. | C.9 |
| M.1823 | B.59 | C.9 |
| M.1824 | B.60 | C.9 |
| M.1825 | B.61 | C.9 |
| M.1826 | B.62 | C.9 |
| M.1827 | B.63 | C.9 |
| M.1828 | B.64 | C.9 |
| M.1829 | B.65 | C.9 |
| M.1830 | B.66 | C.9 |
| M.1831 | B.67 | C.9 |
| M.1832 | B.68 | C.9 |
| M.1833 | B.69 | C.9 |
| M.1834 | B.70 | C.9 |
| M.1835 | B.71 | C.9 |
| M.1836 | B.72 | C.9 |
| M.1837 | B.73 | C.9 |
| M.1838 | B.74 | C.9 |
| M.1839 | B.75 | C.9 |
| M.1840 | B.76 | C.9 |
| M.1841 | B.77 | C.9 |
| M.1842 | B.78 | C.9 |
| M.1843 | B.79 | C.9 |
| M.1844 | B.80 | C.9 |
| M.1845 | B.81 | C.9 |
| M.1846 | B.82 | C.9 |
| M.1847 | B.83 | C.9 |
| M.1848 | B.84 | C.9 |
| M.1849 | B.85 | C.9 |
| M.1850 | B.86 | C.9 |
| M.1851 | B.87 | C.9 |
| M.1852 | B.88 | C.9 |
| M.1853 | B.89 | C.9 |
| M.1854 | B.90 | C.9 |
| M.1855 | B.91 | C.9 |
| M.1856 | B.92 | C.9 |

| comp. no. | herbicide B | safener C |
|---|---|---|
| M.1857 | B.93 | C.9 |
| M.1858 | B.94 | C.9 |
| M.1859 | B.95 | C.9 |
| M.1860 | B.96 | C.9 |
| M.1861 | B.97 | C.9 |
| M.1862 | B.98 | C.9 |
| M.1863 | B.99 | C.9 |
| M.1864 | B.100 | C.9 |
| M.1865 | B.101 | C.9 |
| M.1866 | B.102 | C.9 |
| M.1867 | B.103 | C.9 |
| M.1868 | B.104 | C.9 |
| M.1869 | B.105 | C.9 |
| M.1870 | B.106 | C.9 |
| M.1871 | B.107 | C.9 |
| M.1872 | B.108 | C.9 |
| M.1873 | B.109 | C.9 |
| M.1874 | B.110 | C.9 |
| M.1875 | B.111 | C.9 |
| M.1876 | B.112 | C.9 |
| M.1877 | B.113 | C.9 |
| M.1878 | B.114 | C.9 |
| M.1879 | B.115 | C.9 |
| M.1880 | B.116 | C.9 |
| M.1881 | B.117 | C.9 |
| M.1882 | B.118 | C.9 |
| M.1883 | B.119 | C.9 |
| M.1884 | B.120 | C.9 |
| M.1885 | B.121 | C.9 |
| M.1886 | B.122 | C.9 |
| M.1887 | B.123 | C.9 |
| M.1888 | B.124 | C.9 |
| M.1889 | B.125 | C.9 |
| M.1890 | B.126 | C.9 |
| M.1891 | B.127 | C.9 |
| M.1892 | B.128 | C.9 |
| M.1893 | B.129 | C.9 |
| M.1894 | B.130 | C.9 |
| M.1895 | B.131 | C.9 |
| M.1896 | B.132 | C.9 |

| comp. no. | herbicide B | safener C |
|---|---|---|
| M.1897 | B.133 | C.9 |
| M.1898 | B.134 | C.9 |
| M.1899 | B.135 | C.9 |
| M.1900 | B.136 | C.9 |
| M.1901 | B.137 | C.9 |
| M.1902 | B.138 | C.9 |
| M.1903 | B.139 | C.9 |
| M.1904 | B.140 | C.9 |
| M.1905 | B.141 | C.9 |
| M.1906 | B.142 | C.9 |
| M.1907 | B.143 | C.9 |
| M.1908 | B.144 | C.9 |
| M.1909 | B.145 | C.9 |
| M.1910 | B.146 | C.9 |
| M.1911 | B.147 | C.9 |
| M.1912 | B.148 | C.9 |
| M.1913 | B.149 | C.9 |
| M.1914 | B.150 | C.9 |
| M.1915 | B.151 | C.9 |
| M.1916 | B.152 | C.9 |
| M.1917 | B.153 | C.9 |
| M.1918 | B.154 | C.9 |
| M.1919 | B.155 | C.9 |
| M.1920 | B.156 | C.9 |
| M.1921 | B.157 | C.9 |
| M.1922 | B.158 | C.9 |
| M.1923 | B.159 | C.9 |
| M.1924 | B.160 | C.9 |
| M.1925 | B.161 | C.9 |
| M.1926 | B.162 | C.9 |
| M.1927 | B.163 | C.9 |
| M.1928 | B.164 | C.9 |
| M.1929 | B.165 | C.9 |
| M.1930 | B.166 | C.9 |
| M.1931 | B.167 | C.9 |
| M.1932 | B.168 | C.9 |
| M.1933 | B.169 | C.9 |
| M.1934 | B.170 | C.9 |
| M.1935 | B.171 | C.9 |
| M.1936 | B.172 | C.9 |

| comp. no. | herbicide B | safener C |
|---|---|---|
| M.1937 | B.173 | C.9 |
| M.1938 | B.174 | C.9 |
| M.1939 | B.175 | C.9 |
| M.1940 | B.176 | C.9 |
| M.1941 | B.177 | C.9 |
| M.1942 | B.178 | C.9 |
| M.1943 | B.179 | C.9 |
| M.1944 | B.180 | C.9 |
| M.1945 | B.181 | C.9 |
| M.1946 | B.182 | C.9 |
| M.1947 | B.183 | C.9 |
| M.1948 | B.184 | C.9 |
| M.1949 | B.185 | C.9 |
| M.1950 | B.186 | C.9 |
| M.1951 | B.187 | C.9 |
| M.1952 | B.188 | C.9 |
| M.1953 | B.189 | C.9 |
| M.1954 | B.190 | C.9 |
| M.1955 | B.191 | C.9 |
| M.1956 | B.192 | C.9 |
| M.1957 | B.193 | C.9 |
| M.1958 | B.194 | C.9 |
| M.1959 | B.195 | C.9 |
| M.1960 | B.196 | C.9 |
| M.1961 | B.1 | C.10 |
| M.1962 | B.2 | C.10 |
| M.1963 | B.3 | C.10 |
| M.1964 | B.4 | C.10 |
| M.1965 | B.5 | C.10 |
| M.1966 | B.6 | C.10 |
| M.1967 | B.7 | C.10 |
| M.1968 | B.8 | C.10 |
| M.1969 | B.9 | C.10 |
| M.1970 | B.10 | C.10 |
| M.1971 | B.11 | C.10 |
| M.1972 | B.12 | C.10 |
| M.1973 | B.13 | C.10 |
| M.1974 | B.14 | C.10 |
| M.1975 | B.15 | C.10 |
| M.1976 | B.16 | C.10 |

| comp. no. | herbicide B | safener C |
|---|---|---|
| M.1977 | B.17 | C.10 |
| M.1978 | B.18 | C.10 |
| M.1979 | B.19 | C.10 |
| M.1980 | B.20 | C.10 |
| M.1981 | B.21 | C.10 |
| M.1982 | B.22 | C.10 |
| M.1983 | B.23 | C.10 |
| M.1984 | B.24 | C.10 |
| M.1985 | B.25 | C.10 |
| M.1986 | B.26 | C.10 |
| M.1987 | B.27 | C.10 |
| M.1988 | B.28 | C.10 |
| M.1989 | B.29 | C.10 |
| M.1990 | B.30 | C.10 |
| M.1991 | B.31 | C.10 |
| M.1992 | B.32 | C.10 |
| M.1993 | B.33 | C.10 |
| M.1994 | B.34 | C.10 |
| M.1995 | B.35 | C.10 |
| M.1996 | B.36 | C.10 |
| M.1997 | B.37 | C.10 |
| M.1998 | B.38 | C.10 |
| M.1999 | B.39 | C.10 |
| M.2000 | B.40 | C.10 |
| M.2001 | B.41 | C.10 |
| M.2002 | B.42 | C.10 |
| M.2003 | B.43 | C.10 |
| M.2004 | B.44 | C.10 |
| M.2005 | B.45 | C.10 |
| M.2006 | B.46 | C.10 |
| M.2007 | B.47 | C.10 |
| M.2008 | B.48 | C.10 |
| M.2009 | B.49 | C.10 |
| M.2010 | B.50 | C.10 |
| M.2011 | B.51 | C.10 |
| M.2012 | B.52 | C.10 |
| M.2013 | B.53 | C.10 |
| M.2014 | B.54 | C.10 |
| M.2015 | B.55 | C.10 |
| M.2016 | B.56 | C.10 |

| comp. no. | herbicide B | safener C |
|---|---|---|
| M.2017 | B.57 | C.10 |
| M.2018 | B.58. | C.10 |
| M.2019 | B.59 | C.10 |
| M.2020 | B.60 | C.10 |
| M.2021 | B.61 | C.10 |
| M.2022 | B.62 | C.10 |
| M.2023 | B.63 | C.10 |
| M.2024 | B.64 | C.10 |
| M.2025 | B.65 | C.10 |
| M.2026 | B.66 | C.10 |
| M.2027 | B.67 | C.10 |
| M.2028 | B.68 | C.10 |
| M.2029 | B.69 | C.10 |
| M.2030 | B.70 | C.10 |
| M.2031 | B.71 | C.10 |
| M.2032 | B.72 | C.10 |
| M.2033 | B.73 | C.10 |
| M.2034 | B.74 | C.10 |
| M.2035 | B.75 | C.10 |
| M.2036 | B.76 | C.10 |
| M.2037 | B.77 | C.10 |
| M.2038 | B.78 | C.10 |
| M.2039 | B.79 | C.10 |
| M.2040 | B.80 | C.10 |
| M.2041 | B.81 | C.10 |
| M.2042 | B.82 | C.10 |
| M.2043 | B.83 | C.10 |
| M.2044 | B.84 | C.10 |
| M.2045 | B.85 | C.10 |
| M.2046 | B.86 | C.10 |
| M.2047 | B.87 | C.10 |
| M.2048 | B.88 | C.10 |
| M.2049 | B.89 | C.10 |
| M.2050 | B.90 | C.10 |
| M.2051 | B.91 | C.10 |
| M.2052 | B.92 | C.10 |
| M.2053 | B.93 | C.10 |
| M.2054 | B.94 | C.10 |
| M.2055 | B.95 | C.10 |
| M.2056 | B.96 | C.10 |

| comp. no. | herbicide B | safener C |
|---|---|---|
| M.2057 | B.97 | C.10 |
| M.2058 | B.98 | C.10 |
| M.2059 | B.99 | C.10 |
| M.2060 | B.100 | C.10 |
| M.2061 | B.101 | C.10 |
| M.2062 | B.102 | C.10 |
| M.2063 | B.103 | C.10 |
| M.2064 | B.104 | C.10 |
| M.2065 | B.105 | C.10 |
| M.2066 | B.106 | C.10 |
| M.2067 | B.107 | C.10 |
| M.2068 | B.108 | C.10 |
| M.2069 | B.109 | C.10 |
| M.2070 | B.110 | C.10 |
| M.2071 | B.111 | C.10 |
| M.2072 | B.112 | C.10 |
| M.2073 | B.113 | C.10 |
| M.2074 | B.114 | C.10 |
| M.2075 | B.115 | C.10 |
| M.2076 | B.116 | C.10 |
| M.2077 | B.117 | C.10 |
| M.2078 | B.118 | C.10 |
| M.2079 | B.119 | C.10 |
| M.2080 | B.120 | C.10 |
| M.2081 | B.121 | C.10 |
| M.2082 | B.122 | C.10 |
| M.2083 | B.123 | C.10 |
| M.2084 | B.124 | C.10 |
| M.2085 | B.125 | C.10 |
| M.2086 | B.126 | C.10 |
| M.2087 | B.127 | C.10 |
| M.2088 | B.128 | C.10 |
| M.2089 | B.129 | C.10 |
| M.2090 | B.130 | C.10 |
| M.2091 | B.131 | C.10 |
| M.2092 | B.132 | C.10 |
| M.2093 | B.133 | C.10 |
| M.2094 | B.134 | C.10 |
| M.2095 | B.135 | C.10 |
| M.2096 | B.136 | C.10 |

| comp. no. | herbi-cide B | safe-ner C |
|---|---|---|
| M.2097 | B.137 | C.10 |
| M.2098 | B.138 | C.10 |
| M.2099 | B.139 | C.10 |
| M.2100 | B.140 | C.10 |
| M.2101 | B.141 | C.10 |
| M.2102 | B.142 | C.10 |
| M.2103 | B.143 | C.10 |
| M.2104 | B.144 | C.10 |
| M.2105 | B.145 | C.10 |
| M.2106 | B.146 | C.10 |
| M.2107 | B.147 | C.10 |
| M.2108 | B.148 | C.10 |
| M.2109 | B.149 | C.10 |
| M.2110 | B.150 | C.10 |
| M.2111 | B.151 | C.10 |
| M.2112 | B.152 | C.10 |
| M.2113 | B.153 | C.10 |
| M.2114 | B.154 | C.10 |
| M.2115 | B.155 | C.10 |
| M.2116 | B.156 | C.10 |
| M.2117 | B.157 | C.10 |
| M.2118 | B.158 | C.10 |
| M.2119 | B.159 | C.10 |
| M.2120 | B.160 | C.10 |
| M.2121 | B.161 | C.10 |
| M.2122 | B.162 | C.10 |
| M.2123 | B.163 | C.10 |
| M.2124 | B.164 | C.10 |
| M.2125 | B.165 | C.10 |
| M.2126 | B.166 | C.10 |
| M.2127 | B.167 | C.10 |
| M.2128 | B.168 | C.10 |
| M.2129 | B.169 | C.10 |
| M.2130 | B.170 | C.10 |
| M.2131 | B.171 | C.10 |
| M.2132 | B.172 | C.10 |
| M.2133 | B.173 | C.10 |
| M.2134 | B.174 | C.10 |
| M.2135 | B.175 | C.10 |
| M.2136 | B.176 | C.10 |

| comp. no. | herbi-cide B | safe-ner C |
|---|---|---|
| M.2137 | B.177 | C.10 |
| M.2138 | B.178 | C.10 |
| M.2139 | B.179 | C.10 |
| M.2140 | B.180 | C.10 |
| M.2141 | B.181 | C.10 |
| M.2142 | B.182 | C.10 |
| M.2143 | B.183 | C.10 |
| M.2144 | B.184 | C.10 |
| M.2145 | B.185 | C.10 |
| M.2146 | B.186 | C.10 |
| M.2147 | B.187 | C.10 |
| M.2148 | B.188 | C.10 |
| M.2149 | B.189 | C.10 |
| M.2150 | B.190 | C.10 |
| M.2151 | B.191 | C.10 |
| M.2152 | B.192 | C.10 |
| M.2153 | B.193 | C.10 |
| M.2154 | B.194 | C.10 |
| M.2155 | B.195 | C.10 |
| M.2156 | B.196 | C.10 |
| M.2157 | B.1 | C.11 |
| M.2158 | B.2 | C.11 |
| M.2159 | B.3 | C.11 |
| M.2160 | B.4 | C.11 |
| M.2161 | B.5 | C.11 |
| M.2162 | B.6 | C.11 |
| M.2163 | B.7 | C.11 |
| M.2164 | B.8 | C.11 |
| M.2165 | B.9 | C.11 |
| M.2166 | B.10 | C.11 |
| M.2167 | B.11 | C.11 |
| M.2168 | B.12 | C.11 |
| M.2169 | B.13 | C.11 |
| M.2170 | B.14 | C.11 |
| M.2171 | B.15 | C.11 |
| M.2172 | B.16 | C.11 |
| M.2173 | B.17 | C.11 |
| M.2174 | B.18 | C.11 |
| M.2175 | B.19 | C.11 |
| M.2176 | B.20 | C.11 |

| comp. no. | herbicide B | safener C |
|---|---|---|
| M.2177 | B.21 | C.11 |
| M.2178 | B.22 | C.11 |
| M.2179 | B.23 | C.11 |
| M.2180 | B.24 | C.11 |
| M.2181 | B.25 | C.11 |
| M.2182 | B.26 | C.11 |
| M.2183 | B.27 | C.11 |
| M.2184 | B.28 | C.11 |
| M.2185 | B.29 | C.11 |
| M.2186 | B.30 | C.11 |
| M.2187 | B.31 | C.11 |
| M.2188 | B.32 | C.11 |
| M.2189 | B.33 | C.11 |
| M.2190 | B.34 | C.11 |
| M.2191 | B.35 | C.11 |
| M.2192 | B.36 | C.11 |
| M.2193 | B.37 | C.11 |
| M.2194 | B.38 | C.11 |
| M.2195 | B.39 | C.11 |
| M.2196 | B.40 | C.11 |
| M.2197 | B.41 | C.11 |
| M.2198 | B.42 | C.11 |
| M.2199 | B.43 | C.11 |
| M.2200 | B.44 | C.11 |
| M.2201 | B.45 | C.11 |
| M.2202 | B.46 | C.11 |
| M.2203 | B.47 | C.11 |
| M.2204 | B.48 | C.11 |
| M.2205 | B.49 | C.11 |
| M.2206 | B.50 | C.11 |
| M.2207 | B.51 | C.11 |
| M.2208 | B.52 | C.11 |
| M.2209 | B.53 | C.11 |
| M.2210 | B.54 | C.11 |
| M.2211 | B.55 | C.11 |
| M.2212 | B.56 | C.11 |
| M.2213 | B.57 | C.11 |
| M.2214 | B.58. | C.11 |
| M.2215 | B.59 | C.11 |
| M.2216 | B.60 | C.11 |

| comp. no. | herbicide B | safener C |
|---|---|---|
| M.2217 | B.61 | C.11 |
| M.2218 | B.62 | C.11 |
| M.2219 | B.63 | C.11 |
| M.2220 | B.64 | C.11 |
| M.2221 | B.65 | C.11 |
| M.2222 | B.66 | C.11 |
| M.2223 | B.67 | C.11 |
| M.2224 | B.68 | C.11 |
| M.2225 | B.69 | C.11 |
| M.2226 | B.70 | C.11 |
| M.2227 | B.71 | C.11 |
| M.2228 | B.72 | C.11 |
| M.2229 | B.73 | C.11 |
| M.2230 | B.74 | C.11 |
| M.2231 | B.75 | C.11 |
| M.2232 | B.76 | C.11 |
| M.2233 | B.77 | C.11 |
| M.2234 | B.78 | C.11 |
| M.2235 | B.79 | C.11 |
| M.2236 | B.80 | C.11 |
| M.2237 | B.81 | C.11 |
| M.2238 | B.82 | C.11 |
| M.2239 | B.83 | C.11 |
| M.2240 | B.84 | C.11 |
| M.2241 | B.85 | C.11 |
| M.2242 | B.86 | C.11 |
| M.2243 | B.87 | C.11 |
| M.2244 | B.88 | C.11 |
| M.2245 | B.89 | C.11 |
| M.2246 | B.90 | C.11 |
| M.2247 | B.91 | C.11 |
| M.2248 | B.92 | C.11 |
| M.2249 | B.93 | C.11 |
| M.2250 | B.94 | C.11 |
| M.2251 | B.95 | C.11 |
| M.2252 | B.96 | C.11 |
| M.2253 | B.97 | C.11 |
| M.2254 | B.98 | C.11 |
| M.2255 | B.99 | C.11 |
| M.2256 | B.100 | C.11 |

| comp. no. | herbicide B | safener C |
|---|---|---|
| M.2257 | B.101 | C.11 |
| M.2258 | B.102 | C.11 |
| M.2259 | B.103 | C.11 |
| M.2260 | B.104 | C.11 |
| M.2261 | B.105 | C.11 |
| M.2262 | B.106 | C.11 |
| M.2263 | B.107 | C.11 |
| M.2264 | B.108 | C.11 |
| M.2265 | B.109 | C.11 |
| M.2266 | B.110 | C.11 |
| M.2267 | B.111 | C.11 |
| M.2268 | B.112 | C.11 |
| M.2269 | B.113 | C.11 |
| M.2270 | B.114 | C.11 |
| M.2271 | B.115 | C.11 |
| M.2272 | B.116 | C.11 |
| M.2273 | B.117 | C.11 |
| M.2274 | B.118 | C.11 |
| M.2275 | B.119 | C.11 |
| M.2276 | B.120 | C.11 |
| M.2277 | B.121 | C.11 |
| M.2278 | B.122 | C.11 |
| M.2279 | B.123 | C.11 |
| M.2280 | B.124 | C.11 |
| M.2281 | B.125 | C.11 |
| M.2282 | B.126 | C.11 |
| M.2283 | B.127 | C.11 |
| M.2284 | B.128 | C.11 |
| M.2285 | B.129 | C.11 |
| M.2286 | B.130 | C.11 |
| M.2287 | B.131 | C.11 |
| M.2288 | B.132 | C.11 |
| M.2289 | B.133 | C.11 |
| M.2290 | B.134 | C.11 |
| M.2291 | B.135 | C.11 |
| M.2292 | B.136 | C.11 |
| M.2293 | B.137 | C.11 |
| M.2294 | B.138 | C.11 |
| M.2295 | B.139 | C.11 |
| M.2296 | B.140 | C.11 |

| comp. no. | herbicide B | safener C |
|---|---|---|
| M.2297 | B.141 | C.11 |
| M.2298 | B.142 | C.11 |
| M.2299 | B.143 | C.11 |
| M.2300 | B.144 | C.11 |
| M.2301 | B.145 | C.11 |
| M.2302 | B.146 | C.11 |
| M.2303 | B.147 | C.11 |
| M.2304 | B.148 | C.11 |
| M.2305 | B.149 | C.11 |
| M.2306 | B.150 | C.11 |
| M.2307 | B.151 | C.11 |
| M.2308 | B.152 | C.11 |
| M.2309 | B.153 | C.11 |
| M.2310 | B.154 | C.11 |
| M.2311 | B.155 | C.11 |
| M.2312 | B.156 | C.11 |
| M.2313 | B.157 | C.11 |
| M.2314 | B.158 | C.11 |
| M.2315 | B.159 | C.11 |
| M.2316 | B.160 | C.11 |
| M.2317 | B.161 | C.11 |
| M.2318 | B.162 | C.11 |
| M.2319 | B.163 | C.11 |
| M.2320 | B.164 | C.11 |
| M.2321 | B.165 | C.11 |
| M.2322 | B.166 | C.11 |
| M.2323 | B.167 | C.11 |
| M.2324 | B.168 | C.11 |
| M.2325 | B.169 | C.11 |
| M.2326 | B.170 | C.11 |
| M.2327 | B.171 | C.11 |
| M.2328 | B.172 | C.11 |
| M.2329 | B.173 | C.11 |
| M.2330 | B.174 | C.11 |
| M.2331 | B.175 | C.11 |
| M.2332 | B.176 | C.11 |
| M.2333 | B.177 | C.11 |
| M.2334 | B.178 | C.11 |
| M.2335 | B.179 | C.11 |
| M.2336 | B.180 | C.11 |

| comp. no. | herbicide B | safener C |
|---|---|---|
| M.2337 | B.181 | C.11 |
| M.2338 | B.182 | C.11 |
| M.2339 | B.183 | C.11 |
| M.2340 | B.184 | C.11 |
| M.2341 | B.185 | C.11 |
| M.2342 | B.186 | C.11 |
| M.2343 | B.187 | C.11 |
| M.2344 | B.188 | C.11 |
| M.2345 | B.189 | C.11 |
| M.2346 | B.190 | C.11 |
| M.2347 | B.191 | C.11 |
| M.2348 | B.192 | C.11 |
| M.2349 | B.193 | C.11 |
| M.2350 | B.194 | C.11 |
| M.2351 | B.195 | C.11 |
| M.2352 | B.196 | C.11 |
| M.2353 | B.1 | C.12 |
| M.2354 | B.2 | C.12 |
| M.2355 | B.3 | C.12 |
| M.2356 | B.4 | C.12 |
| M.2357 | B.5 | C.12 |
| M.2358 | B.6 | C.12 |
| M.2359 | B.7 | C.12 |
| M.2360 | B.8 | C.12 |
| M.2361 | B.9 | C.12 |
| M.2362 | B.10 | C.12 |
| M.2363 | B.11 | C.12 |
| M.2364 | B.12 | C.12 |
| M.2365 | B.13 | C.12 |
| M.2366 | B.14 | C.12 |
| M.2367 | B.15 | C.12 |
| M.2368 | B.16 | C.12 |
| M.2369 | B.17 | C.12 |
| M.2370 | B.18 | C.12 |
| M.2371 | B.19 | C.12 |
| M.2372 | B.20 | C.12 |
| M.2373 | B.21 | C.12 |
| M.2374 | B.22 | C.12 |
| M.2375 | B.23 | C.12 |
| M.2376 | B.24 | C.12 |

| comp. no. | herbicide B | safener C |
|---|---|---|
| M.2377 | B.25 | C.12 |
| M.2378 | B.26 | C.12 |
| M.2379 | B.27 | C.12 |
| M.2380 | B.28 | C.12 |
| M.2381 | B.29 | C.12 |
| M.2382 | B.30 | C.12 |
| M.2383 | B.31 | C.12 |
| M.2384 | B.32 | C.12 |
| M.2385 | B.33 | C.12 |
| M.2386 | B.34 | C.12 |
| M.2387 | B.35 | C.12 |
| M.2388 | B.36 | C.12 |
| M.2389 | B.37 | C.12 |
| M.2390 | B.38 | C.12 |
| M.2391 | B.39 | C.12 |
| M.2392 | B.40 | C.12 |
| M.2393 | B.41 | C.12 |
| M.2394 | B.42 | C.12 |
| M.2395 | B.43 | C.12 |
| M.2396 | B.44 | C.12 |
| M.2397 | B.45 | C.12 |
| M.2398 | B.46 | C.12 |
| M.2399 | B.47 | C.12 |
| M.2400 | B.48 | C.12 |
| M.2401 | B.49 | C.12 |
| M.2402 | B.50 | C.12 |
| M.2403 | B.51 | C.12 |
| M.2404 | B.52 | C.12 |
| M.2405 | B.53 | C.12 |
| M.2406 | B.54 | C.12 |
| M.2407 | B.55 | C.12 |
| M.2408 | B.56 | C.12 |
| M.2409 | B.57 | C.12 |
| M.2410 | B.58. | C.12 |
| M.2411 | B.59 | C.12 |
| M.2412 | B.60 | C.12 |
| M.2413 | B.61 | C.12 |
| M.2414 | B.62 | C.12 |
| M.2415 | B.63 | C.12 |
| M.2416 | B.64 | C.12 |

| comp. no. | herbicide B | safener C |
|---|---|---|
| M.2417 | B.65 | C.12 |
| M.2418 | B.66 | C.12 |
| M.2419 | B.67 | C.12 |
| M.2420 | B.68 | C.12 |
| M.2421 | B.69 | C.12 |
| M.2422 | B.70 | C.12 |
| M.2423 | B.71 | C.12 |
| M.2424 | B.72 | C.12 |
| M.2425 | B.73 | C.12 |
| M.2426 | B.74 | C.12 |
| M.2427 | B.75 | C.12 |
| M.2428 | B.76 | C.12 |
| M.2429 | B.77 | C.12 |
| M.2430 | B.78 | C.12 |
| M.2431 | B.79 | C.12 |
| M.2432 | B.80 | C.12 |
| M.2433 | B.81 | C.12 |
| M.2434 | B.82 | C.12 |
| M.2435 | B.83 | C.12 |
| M.2436 | B.84 | C.12 |
| M.2437 | B.85 | C.12 |
| M.2438 | B.86 | C.12 |
| M.2439 | B.87 | C.12 |
| M.2440 | B.88 | C.12 |
| M.2441 | B.89 | C.12 |
| M.2442 | B.90 | C.12 |
| M.2443 | B.91 | C.12 |
| M.2444 | B.92 | C.12 |
| M.2445 | B.93 | C.12 |
| M.2446 | B.94 | C.12 |
| M.2447 | B.95 | C.12 |
| M.2448 | B.96 | C.12 |
| M.2449 | B.97 | C.12 |
| M.2450 | B.98 | C.12 |
| M.2451 | B.99 | C.12 |
| M.2452 | B.100 | C.12 |
| M.2453 | B.101 | C.12 |
| M.2454 | B.102 | C.12 |
| M.2455 | B.103 | C.12 |
| M.2456 | B.104 | C.12 |

| comp. no. | herbicide B | safener C |
|---|---|---|
| M.2457 | B.105 | C.12 |
| M.2458 | B.106 | C.12 |
| M.2459 | B.107 | C.12 |
| M.2460 | B.108 | C.12 |
| M.2461 | B.109 | C.12 |
| M.2462 | B.110 | C.12 |
| M.2463 | B.111 | C.12 |
| M.2464 | B.112 | C.12 |
| M.2465 | B.113 | C.12 |
| M.2466 | B.114 | C.12 |
| M.2467 | B.115 | C.12 |
| M.2468 | B.116 | C.12 |
| M.2469 | B.117 | C.12 |
| M.2470 | B.118 | C.12 |
| M.2471 | B.119 | C.12 |
| M.2472 | B.120 | C.12 |
| M.2473 | B.121 | C.12 |
| M.2474 | B.122 | C.12 |
| M.2475 | B.123 | C.12 |
| M.2476 | B.124 | C.12 |
| M.2477 | B.125 | C.12 |
| M.2478 | B.126 | C.12 |
| M.2479 | B.127 | C.12 |
| M.2480 | B.128 | C.12 |
| M.2481 | B.129 | C.12 |
| M.2482 | B.130 | C.12 |
| M.2483 | B.131 | C.12 |
| M.2484 | B.132 | C.12 |
| M.2485 | B.133 | C.12 |
| M.2486 | B.134 | C.12 |
| M.2487 | B.135 | C.12 |
| M.2488 | B.136 | C.12 |
| M.2489 | B.137 | C.12 |
| M.2490 | B.138 | C.12 |
| M.2491 | B.139 | C.12 |
| M.2492 | B.140 | C.12 |
| M.2493 | B.141 | C.12 |
| M.2494 | B.142 | C.12 |
| M.2495 | B.143 | C.12 |
| M.2496 | B.144 | C.12 |

| comp. no. | herbicide B | safener C |
|---|---|---|
| M.2497 | B.145 | C.12 |
| M.2498 | B.146 | C.12 |
| M.2499 | B.147 | C.12 |
| M.2500 | B.148 | C.12 |
| M.2501 | B.149 | C.12 |
| M.2502 | B.150 | C.12 |
| M.2503 | B.151 | C.12 |
| M.2504 | B.152 | C.12 |
| M.2505 | B.153 | C.12 |
| M.2506 | B.154 | C.12 |
| M.2507 | B.155 | C.12 |
| M.2508 | B.156 | C.12 |
| M.2509 | B.157 | C.12 |
| M.2510 | B.158 | C.12 |
| M.2511 | B.159 | C.12 |
| M.2512 | B.160 | C.12 |
| M.2513 | B.161 | C.12 |
| M.2514 | B.162 | C.12 |
| M.2515 | B.163 | C.12 |
| M.2516 | B.164 | C.12 |
| M.2517 | B.165 | C.12 |
| M.2518 | B.166 | C.12 |
| M.2519 | B.167 | C.12 |
| M.2520 | B.168 | C.12 |
| M.2521 | B.169 | C.12 |
| M.2522 | B.170 | C.12 |
| M.2523 | B.171 | C.12 |
| M.2524 | B.172 | C.12 |
| M.2525 | B.173 | C.12 |
| M.2526 | B.174 | C.12 |
| M.2527 | B.175 | C.12 |
| M.2528 | B.176 | C.12 |
| M.2529 | B.177 | C.12 |
| M.2530 | B.178 | C.12 |
| M.2531 | B.179 | C.12 |
| M.2532 | B.180 | C.12 |
| M.2533 | B.181 | C.12 |
| M.2534 | B.182 | C.12 |
| M.2535 | B.183 | C.12 |
| M.2536 | B.184 | C.12 |

| comp. no. | herbicide B | safener C |
|---|---|---|
| M.2537 | B.185 | C.12 |
| M.2538 | B.186 | C.12 |
| M.2539 | B.187 | C.12 |
| M.2540 | B.188 | C.12 |
| M.2541 | B.189 | C.12 |
| M.2542 | B.190 | C.12 |
| M.2543 | B.191 | C.12 |
| M.2544 | B.192 | C.12 |
| M.2545 | B.193 | C.12 |
| M.2546 | B.194 | C.12 |
| M.2547 | B.195 | C.12 |
| M.2548 | B.196 | C.12 |
| M.2549 | B.1 | C.13 |
| M.2550 | B.2 | C.13 |
| M.2551 | B.3 | C.13 |
| M.2552 | B.4 | C.13 |
| M.2553 | B.5 | C.13 |
| M.2554 | B.6 | C.13 |
| M.2555 | B.7 | C.13 |
| M.2556 | B.8 | C.13 |
| M.2557 | B.9 | C.13 |
| M.2558 | B.10 | C.13 |
| M.2559 | B.11 | C.13 |
| M.2560 | B.12 | C.13 |
| M.2561 | B.13 | C.13 |
| M.2562 | B.14 | C.13 |
| M.2563 | B.15 | C.13 |
| M.2564 | B.16 | C.13 |
| M.2565 | B.17 | C.13 |
| M.2566 | B.18 | C.13 |
| M.2567 | B.19 | C.13 |
| M.2568 | B.20 | C.13 |
| M.2569 | B.21 | C.13 |
| M.2570 | B.22 | C.13 |
| M.2571 | B.23 | C.13 |
| M.2572 | B.24 | C.13 |
| M.2573 | B.25 | C.13 |
| M.2574 | B.26 | C.13 |
| M.2575 | B.27 | C.13 |
| M.2576 | B.28 | C.13 |

| comp. no. | herbicide B | safener C |
|---|---|---|
| M.2577 | B.29 | C.13 |
| M.2578 | B.30 | C.13 |
| M.2579 | B.31 | C.13 |
| M.2580 | B.32 | C.13 |
| M.2581 | B.33 | C.13 |
| M.2582 | B.34 | C.13 |
| M.2583 | B.35 | C.13 |
| M.2584 | B.36 | C.13 |
| M.2585 | B.37 | C.13 |
| M.2586 | B.38 | C.13 |
| M.2587 | B.39 | C.13 |
| M.2588 | B.40 | C.13 |
| M.2589 | B.41 | C.13 |
| M.2590 | B.42 | C.13 |
| M.2591 | B.43 | C.13 |
| M.2592 | B.44 | C.13 |
| M.2593 | B.45 | C.13 |
| M.2594 | B.46 | C.13 |
| M.2595 | B.47 | C.13 |
| M.2596 | B.48 | C.13 |
| M.2597 | B.49 | C.13 |
| M.2598 | B.50 | C.13 |
| M.2599 | B.51 | C.13 |
| M.2600 | B.52 | C.13 |
| M.2601 | B.53 | C.13 |
| M.2602 | B.54 | C.13 |
| M.2603 | B.55 | C.13 |
| M.2604 | B.56 | C.13 |
| M.2605 | B.57 | C.13 |
| M.2606 | B.58. | C.13 |
| M.2607 | B.59 | C.13 |
| M.2608 | B.60 | C.13 |
| M.2609 | B.61 | C.13 |
| M.2610 | B.62 | C.13 |
| M.2611 | B.63 | C.13 |
| M.2612 | B.64 | C.13 |
| M.2613 | B.65 | C.13 |
| M.2614 | B.66 | C.13 |
| M.2615 | B.67 | C.13 |
| M.2616 | B.68 | C.13 |

| comp. no. | herbicide B | safener C |
|---|---|---|
| M.2617 | B.69 | C.13 |
| M.2618 | B.70 | C.13 |
| M.2619 | B.71 | C.13 |
| M.2620 | B.72 | C.13 |
| M.2621 | B.73 | C.13 |
| M.2622 | B.74 | C.13 |
| M.2623 | B.75 | C.13 |
| M.2624 | B.76 | C.13 |
| M.2625 | B.77 | C.13 |
| M.2626 | B.78 | C.13 |
| M.2627 | B.79 | C.13 |
| M.2628 | B.80 | C.13 |
| M.2629 | B.81 | C.13 |
| M.2630 | B.82 | C.13 |
| M.2631 | B.83 | C.13 |
| M.2632 | B.84 | C.13 |
| M.2633 | B.85 | C.13 |
| M.2634 | B.86 | C.13 |
| M.2635 | B.87 | C.13 |
| M.2636 | B.88 | C.13 |
| M.2637 | B.89 | C.13 |
| M.2638 | B.90 | C.13 |
| M.2639 | B.91 | C.13 |
| M.2640 | B.92 | C.13 |
| M.2641 | B.93 | C.13 |
| M.2642 | B.94 | C.13 |
| M.2643 | B.95 | C.13 |
| M.2644 | B.96 | C.13 |
| M.2645 | B.97 | C.13 |
| M.2646 | B.98 | C.13 |
| M.2647 | B.99 | C.13 |
| M.2648 | B.100 | C.13 |
| M.2649 | B.101 | C.13 |
| M.2650 | B.102 | C.13 |
| M.2651 | B.103 | C.13 |
| M.2652 | B.104 | C.13 |
| M.2653 | B.105 | C.13 |
| M.2654 | B.106 | C.13 |
| M.2655 | B.107 | C.13 |
| M.2656 | B.108 | C.13 |

| comp. no. | herbicide B | safener C |
|---|---|---|
| M.2657 | B.109 | C.13 |
| M.2658 | B.110 | C.13 |
| M.2659 | B.111 | C.13 |
| M.2660 | B.112 | C.13 |
| M.2661 | B.113 | C.13 |
| M.2662 | B.114 | C.13 |
| M.2663 | B.115 | C.13 |
| M.2664 | B.116 | C.13 |
| M.2665 | B.117 | C.13 |
| M.2666 | B.118 | C.13 |
| M.2667 | B.119 | C.13 |
| M.2668 | B.120 | C.13 |
| M.2669 | B.121 | C.13 |
| M.2670 | B.122 | C.13 |
| M.2671 | B.123 | C.13 |
| M.2672 | B.124 | C.13 |
| M.2673 | B.125 | C.13 |
| M.2674 | B.126 | C.13 |
| M.2675 | B.127 | C.13 |
| M.2676 | B.128 | C.13 |
| M.2677 | B.129 | C.13 |
| M.2678 | B.130 | C.13 |
| M.2679 | B.131 | C.13 |
| M.2680 | B.132 | C.13 |
| M.2681 | B.133 | C.13 |
| M.2682 | B.134 | C.13 |
| M.2683 | B.135 | C.13 |
| M.2684 | B.136 | C.13 |
| M.2685 | B.137 | C.13 |
| M.2686 | B.138 | C.13 |
| M.2687 | B.139 | C.13 |
| M.2688 | B.140 | C.13 |
| M.2689 | B.141 | C.13 |
| M.2690 | B.142 | C.13 |
| M.2691 | B.143 | C.13 |
| M.2692 | B.144 | C.13 |
| M.2693 | B.145 | C.13 |
| M.2694 | B.146 | C.13 |
| M.2695 | B.147 | C.13 |
| M.2696 | B.148 | C.13 |

| comp. no. | herbicide B | safener C |
|---|---|---|
| M.2697 | B.149 | C.13 |
| M.2698 | B.150 | C.13 |
| M.2699 | B.151 | C.13 |
| M.2700 | B.152 | C.13 |
| M.2701 | B.153 | C.13 |
| M.2702 | B.154 | C.13 |
| M.2703 | B.155 | C.13 |
| M.2704 | B.156 | C.13 |
| M.2705 | B.157 | C.13 |
| M.2706 | B.158 | C.13 |
| M.2707 | B.159 | C.13 |
| M.2708 | B.160 | C.13 |
| M.2709 | B.161 | C.13 |
| M.2710 | B.162 | C.13 |
| M.2711 | B.163 | C.13 |
| M.2712 | B.164 | C.13 |
| M.2713 | B.165 | C.13 |
| M.2714 | B.166 | C.13 |
| M.2715 | B.167 | C.13 |
| M.2716 | B.168 | C.13 |
| M.2717 | B.169 | C.13 |
| M.2718 | B.170 | C.13 |
| M.2719 | B.171 | C.13 |
| M.2720 | B.172 | C.13 |
| M.2721 | B.173 | C.13 |
| M.2722 | B.174 | C.13 |
| M.2723 | B.175 | C.13 |
| M.2724 | B.176 | C.13 |
| M.2725 | B.177 | C.13 |
| M.2726 | B.178 | C.13 |
| M.2727 | B.179 | C.13 |
| M.2728 | B.180 | C.13 |
| M.2729 | B.181 | C.13 |
| M.2730 | B.182 | C.13 |
| M.2731 | B.183 | C.13 |
| M.2732 | B.184 | C.13 |
| M.2733 | B.185 | C.13 |
| M.2734 | B.186 | C.13 |
| M.2735 | B.187 | C.13 |
| M.2736 | B.188 | C.13 |

| comp. no. | herbicide B | safener C |
|---|---|---|
| M.2737 | B.189 | C.13 |
| M.2738 | B.190 | C.13 |
| M.2739 | B.191 | C.13 |
| M.2740 | B.192 | C.13 |
| M.2741 | B.193 | C.13 |
| M.2742 | B.194 | C.13 |
| M.2743 | B.195 | C.13 |
| M.2744 | B.196 | C.13 |
| M.2745 | B.1 | C.14 |
| M.2746 | B.2 | C.14 |
| M.2747 | B.3 | C.14 |
| M.2748 | B.4 | C.14 |
| M.2749 | B.5 | C.14 |
| M.2750 | B.6 | C.14 |
| M.2751 | B.7 | C.14 |
| M.2752 | B.8 | C.14 |
| M.2753 | B.9 | C.14 |
| M.2754 | B.10 | C.14 |
| M.2755 | B.11 | C.14 |
| M.2756 | B.12 | C.14 |
| M.2757 | B.13 | C.14 |
| M.2758 | B.14 | C.14 |
| M.2759 | B.15 | C.14 |
| M.2760 | B.16 | C.14 |
| M.2761 | B.17 | C.14 |
| M.2762 | B.18 | C.14 |
| M.2763 | B.19 | C.14 |
| M.2764 | B.20 | C.14 |
| M.2765 | B.21 | C.14 |
| M.2766 | B.22 | C.14 |
| M.2767 | B.23 | C.14 |
| M.2768 | B.24 | C.14 |
| M.2769 | B.25 | C.14 |
| M.2770 | B.26 | C.14 |
| M.2771 | B.27 | C.14 |
| M.2772 | B.28 | C.14 |
| M.2773 | B.29 | C.14 |
| M.2774 | B.30 | C.14 |
| M.2775 | B.31 | C.14 |
| M.2776 | B.32 | C.14 |

| comp. no. | herbicide B | safener C |
|---|---|---|
| M.2777 | B.33 | C.14 |
| M.2778 | B.34 | C.14 |
| M.2779 | B.35 | C.14 |
| M.2780 | B.36 | C.14 |
| M.2781 | B.37 | C.14 |
| M.2782 | B.38 | C.14 |
| M.2783 | B.39 | C.14 |
| M.2784 | B.40 | C.14 |
| M.2785 | B.41 | C.14 |
| M.2786 | B.42 | C.14 |
| M.2787 | B.43 | C.14 |
| M.2788 | B.44 | C.14 |
| M.2789 | B.45 | C.14 |
| M.2790 | B.46 | C.14 |
| M.2791 | B.47 | C.14 |
| M.2792 | B.48 | C.14 |
| M.2793 | B.49 | C.14 |
| M.2794 | B.50 | C.14 |
| M.2795 | B.51 | C.14 |
| M.2796 | B.52 | C.14 |
| M.2797 | B.53 | C.14 |
| M.2798 | B.54 | C.14 |
| M.2799 | B.55 | C.14 |
| M.2800 | B.56 | C.14 |
| M.2801 | B.57 | C.14 |
| M.2802 | B.58. | C.14 |
| M.2803 | B.59 | C.14 |
| M.2804 | B.60 | C.14 |
| M.2805 | B.61 | C.14 |
| M.2806 | B.62 | C.14 |
| M.2807 | B.63 | C.14 |
| M.2808 | B.64 | C.14 |
| M.2809 | B.65 | C.14 |
| M.2810 | B.66 | C.14 |
| M.2811 | B.67 | C.14 |
| M.2812 | B.68 | C.14 |
| M.2813 | B.69 | C.14 |
| M.2814 | B.70 | C.14 |
| M.2815 | B.71 | C.14 |
| M.2816 | B.72 | C.14 |

| comp. no. | herbicide B | safener C |
|---|---|---|
| M.2817 | B.73 | C.14 |
| M.2818 | B.74 | C.14 |
| M.2819 | B.75 | C.14 |
| M.2820 | B.76 | C.14 |
| M.2821 | B.77 | C.14 |
| M.2822 | B.78 | C.14 |
| M.2823 | B.79 | C.14 |
| M.2824 | B.80 | C.14 |
| M.2825 | B.81 | C.14 |
| M.2826 | B.82 | C.14 |
| M.2827 | B.83 | C.14 |
| M.2828 | B.84 | C.14 |
| M.2829 | B.85 | C.14 |
| M.2830 | B.86 | C.14 |
| M.2831 | B.87 | C.14 |
| M.2832 | B.88 | C.14 |
| M.2833 | B.89 | C.14 |
| M.2834 | B.90 | C.14 |
| M.2835 | B.91 | C.14 |
| M.2836 | B.92 | C.14 |
| M.2837 | B.93 | C.14 |
| M.2838 | B.94 | C.14 |
| M.2839 | B.95 | C.14 |
| M.2840 | B.96 | C.14 |
| M.2841 | B.97 | C.14 |
| M.2842 | B.98 | C.14 |
| M.2843 | B.99 | C.14 |
| M.2844 | B.100 | C.14 |
| M.2845 | B.101 | C.14 |
| M.2846 | B.102 | C.14 |
| M.2847 | B.103 | C.14 |
| M.2848 | B.104 | C.14 |
| M.2849 | B.105 | C.14 |
| M.2850 | B.106 | C.14 |
| M.2851 | B.107 | C.14 |
| M.2852 | B.108 | C.14 |
| M.2853 | B.109 | C.14 |
| M.2854 | B.110 | C.14 |
| M.2855 | B.111 | C.14 |
| M.2856 | B.112 | C.14 |

| comp. no. | herbicide B | safener C |
|---|---|---|
| M.2857 | B.113 | C.14 |
| M.2858 | B.114 | C.14 |
| M.2859 | B.115 | C.14 |
| M.2860 | B.116 | C.14 |
| M.2861 | B.117 | C.14 |
| M.2862 | B.118 | C.14 |
| M.2863 | B.119 | C.14 |
| M.2864 | B.120 | C.14 |
| M.2865 | B.121 | C.14 |
| M.2866 | B.122 | C.14 |
| M.2867 | B.123 | C.14 |
| M.2868 | B.124 | C.14 |
| M.2869 | B.125 | C.14 |
| M.2870 | B.126 | C.14 |
| M.2871 | B.127 | C.14 |
| M.2872 | B.128 | C.14 |
| M.2873 | B.129 | C.14 |
| M.2874 | B.130 | C.14 |
| M.2875 | B.131 | C.14 |
| M.2876 | B.132 | C.14 |
| M.2877 | B.133 | C.14 |
| M.2878 | B.134 | C.14 |
| M.2879 | B.135 | C.14 |
| M.2880 | B.136 | C.14 |
| M.2881 | B.137 | C.14 |
| M.2882 | B.138 | C.14 |
| M.2883 | B.139 | C.14 |
| M.2884 | B.140 | C.14 |
| M.2885 | B.141 | C.14 |
| M.2886 | B.142 | C.14 |
| M.2887 | B.143 | C.14 |
| M.2888 | B.144 | C.14 |
| M.2889 | B.145 | C.14 |
| M.2890 | B.146 | C.14 |
| M.2891 | B.147 | C.14 |
| M.2892 | B.148 | C.14 |
| M.2893 | B.149 | C.14 |
| M.2894 | B.150 | C.14 |
| M.2895 | B.151 | C.14 |
| M.2896 | B.152 | C.14 |

| comp. no. | herbicide B | safener C |
|---|---|---|
| M.2897 | B.153 | C.14 |
| M.2898 | B.154 | C.14 |
| M.2899 | B.155 | C.14 |
| M.2900 | B.156 | C.14 |
| M.2901 | B.157 | C.14 |
| M.2902 | B.158 | C.14 |
| M.2903 | B.159 | C.14 |
| M.2904 | B.160 | C.14 |
| M.2905 | B.161 | C.14 |
| M.2906 | B.162 | C.14 |
| M.2907 | B.163 | C.14 |
| M.2908 | B.164 | C.14 |
| M.2909 | B.165 | C.14 |
| M.2910 | B.166 | C.14 |
| M.2911 | B.167 | C.14 |
| M.2912 | B.168 | C.14 |
| M.2913 | B.169 | C.14 |
| M.2914 | B.170 | C.14 |
| M.2915 | B.171 | C.14 |
| M.2916 | B.172 | C.14 |
| M.2917 | B.173 | C.14 |
| M.2918 | B.174 | C.14 |
| M.2919 | B.175 | C.14 |
| M.2920 | B.176 | C.14 |
| M.2921 | B.177 | C.14 |
| M.2922 | B.178 | C.14 |
| M.2923 | B.179 | C.14 |
| M.2924 | B.180 | C.14 |
| M.2925 | B.181 | C.14 |
| M.2926 | B.182 | C.14 |
| M.2927 | B.183 | C.14 |
| M.2928 | B.184 | C.14 |
| M.2929 | B.185 | C.14 |
| M.2930 | B.186 | C.14 |
| M.2931 | B.187 | C.14 |
| M.2932 | B.188 | C.14 |
| M.2933 | B.189 | C.14 |
| M.2934 | B.190 | C.14 |
| M.2935 | B.191 | C.14 |
| M.2936 | B.192 | C.14 |

| comp. no. | herbicide B | safener C |
|---|---|---|
| M.2937 | B.193 | C.14 |
| M.2938 | B.194 | C.14 |
| M.2939 | B.195 | C.14 |
| M.2940 | B.196 | C.14 |
| M.2941 | B.1 | C.15 |
| M.2942 | B.2 | C.15 |
| M.2943 | B.3 | C.15 |
| M.2944 | B.4 | C.15 |
| M.2945 | B.5 | C.15 |
| M.2946 | B.6 | C.15 |
| M.2947 | B.7 | C.15 |
| M.2948 | B.8 | C.15 |
| M.2949 | B.9 | C.15 |
| M.2950 | B.10 | C.15 |
| M.2951 | B.11 | C.15 |
| M.2952 | B.12 | C.15 |
| M.2953 | B.13 | C.15 |
| M.2954 | B.14 | C.15 |
| M.2955 | B.15 | C.15 |
| M.2956 | B.16 | C.15 |
| M.2957 | B.17 | C.15 |
| M.2958 | B.18 | C.15 |
| M.2959 | B.19 | C.15 |
| M.2960 | B.20 | C.15 |
| M.2961 | B.21 | C.15 |
| M.2962 | B.22 | C.15 |
| M.2963 | B.23 | C.15 |
| M.2964 | B.24 | C.15 |
| M.2965 | B.25 | C.15 |
| M.2966 | B.26 | C.15 |
| M.2967 | B.27 | C.15 |
| M.2968 | B.28 | C.15 |
| M.2969 | B.29 | C.15 |
| M.2970 | B.30 | C.15 |
| M.2971 | B.31 | C.15 |
| M.2972 | B.32 | C.15 |
| M.2973 | B.33 | C.15 |
| M.2974 | B.34 | C.15 |
| M.2975 | B.35 | C.15 |
| M.2976 | B.36 | C.15 |

| comp. no. | herbicide B | safener C |
|---|---|---|
| M.2977 | B.37 | C.15 |
| M.2978 | B.38 | C.15 |
| M.2979 | B.39 | C.15 |
| M.2980 | B.40 | C.15 |
| M.2981 | B.41 | C.15 |
| M.2982 | B.42 | C.15 |
| M.2983 | B.43 | C.15 |
| M.2984 | B.44 | C.15 |
| M.2985 | B.45 | C.15 |
| M.2986 | B.46 | C.15 |
| M.2987 | B.47 | C.15 |
| M.2988 | B.48 | C.15 |
| M.2989 | B.49 | C.15 |
| M.2990 | B.50 | C.15 |
| M.2991 | B.51 | C.15 |
| M.2992 | B.52 | C.15 |
| M.2993 | B.53 | C.15 |
| M.2994 | B.54 | C.15 |
| M.2995 | B.55 | C.15 |
| M.2996 | B.56 | C.15 |
| M.2997 | B.57 | C.15 |
| M.2998 | B.58. | C.15 |
| M.2999 | B.59 | C.15 |
| M.3000 | B.60 | C.15 |
| M.3001 | B.61 | C.15 |
| M.3002 | B.62 | C.15 |
| M.3003 | B.63 | C.15 |
| M.3004 | B.64 | C.15 |
| M.3005 | B.65 | C.15 |
| M.3006 | B.66 | C.15 |
| M.3007 | B.67 | C.15 |
| M.3008 | B.68 | C.15 |
| M.3009 | B.69 | C.15 |
| M.3010 | B.70 | C.15 |
| M.3011 | B.71 | C.15 |
| M.3012 | B.72 | C.15 |
| M.3013 | B.73 | C.15 |
| M.3014 | B.74 | C.15 |
| M.3015 | B.75 | C.15 |
| M.3016 | B.76 | C.15 |

| comp. no. | herbicide B | safener C |
|---|---|---|
| M.3017 | B.77 | C.15 |
| M.3018 | B.78 | C.15 |
| M.3019 | B.79 | C.15 |
| M.3020 | B.80 | C.15 |
| M.3021 | B.81 | C.15 |
| M.3022 | B.82 | C.15 |
| M.3023 | B.83 | C.15 |
| M.3024 | B.84 | C.15 |
| M.3025 | B.85 | C.15 |
| M.3026 | B.86 | C.15 |
| M.3027 | B.87 | C.15 |
| M.3028 | B.88 | C.15 |
| M.3029 | B.89 | C.15 |
| M.3030 | B.90 | C.15 |
| M.3031 | B.91 | C.15 |
| M.3032 | B.92 | C.15 |
| M.3033 | B.93 | C.15 |
| M.3034 | B.94 | C.15 |
| M.3035 | B.95 | C.15 |
| M.3036 | B.96 | C.15 |
| M.3037 | B.97 | C.15 |
| M.3038 | B.98 | C.15 |
| M.3039 | B.99 | C.15 |
| M.3040 | B.100 | C.15 |
| M.3041 | B.101 | C.15 |
| M.3042 | B.102 | C.15 |
| M.3043 | B.103 | C.15 |
| M.3044 | B.104 | C.15 |
| M.3045 | B.105 | C.15 |
| M.3046 | B.106 | C.15 |
| M.3047 | B.107 | C.15 |
| M.3048 | B.108 | C.15 |
| M.3049 | B.109 | C.15 |
| M.3050 | B.110 | C.15 |
| M.3051 | B.111 | C.15 |
| M.3052 | B.112 | C.15 |
| M.3053 | B.113 | C.15 |
| M.3054 | B.114 | C.15 |
| M.3055 | B.115 | C.15 |
| M.3056 | B.116 | C.15 |

| comp. no. | herbicide B | safener C |
|---|---|---|
| M.3057 | B.117 | C.15 |
| M.3058 | B.118 | C.15 |
| M.3059 | B.119 | C.15 |
| M.3060 | B.120 | C.15 |
| M.3061 | B.121 | C.15 |
| M.3062 | B.122 | C.15 |
| M.3063 | B.123 | C.15 |
| M.3064 | B.124 | C.15 |
| M.3065 | B.125 | C.15 |
| M.3066 | B.126 | C.15 |
| M.3067 | B.127 | C.15 |
| M.3068 | B.128 | C.15 |
| M.3069 | B.129 | C.15 |
| M.3070 | B.130 | C.15 |
| M.3071 | B.131 | C.15 |
| M.3072 | B.132 | C.15 |
| M.3073 | B.133 | C.15 |
| M.3074 | B.134 | C.15 |
| M.3075 | B.135 | C.15 |
| M.3076 | B.136 | C.15 |
| M.3077 | B.137 | C.15 |
| M.3078 | B.138 | C.15 |
| M.3079 | B.139 | C.15 |
| M.3080 | B.140 | C.15 |
| M.3081 | B.141 | C.15 |
| M.3082 | B.142 | C.15 |
| M.3083 | B.143 | C.15 |
| M.3084 | B.144 | C.15 |
| M.3085 | B.145 | C.15 |
| M.3086 | B.146 | C.15 |
| M.3087 | B.147 | C.15 |
| M.3088 | B.148 | C.15 |
| M.3089 | B.149 | C.15 |
| M.3090 | B.150 | C.15 |
| M.3091 | B.151 | C.15 |
| M.3092 | B.152 | C.15 |
| M.3093 | B.153 | C.15 |
| M.3094 | B.154 | C.15 |
| M.3095 | B.155 | C.15 |
| M.3096 | B.156 | C.15 |

| comp. no. | herbicide B | safener C |
|---|---|---|
| M.3097 | B.157 | C.15 |
| M.3098 | B.158 | C.15 |
| M.3099 | B.159 | C.15 |
| M.3100 | B.160 | C.15 |
| M.3101 | B.161 | C.15 |
| M.3102 | B.162 | C.15 |
| M.3103 | B.163 | C.15 |
| M.3104 | B.164 | C.15 |
| M.3105 | B.165 | C.15 |
| M.3106 | B.166 | C.15 |
| M.3107 | B.167 | C.15 |
| M.3108 | B.168 | C.15 |
| M.3109 | B.169 | C.15 |
| M.3110 | B.170 | C.15 |
| M.3111 | B.171 | C.15 |
| M.3112 | B.172 | C.15 |
| M.3113 | B.173 | C.15 |
| M.3114 | B.174 | C.15 |
| M.3115 | B.175 | C.15 |
| M.3116 | B.176 | C.15 |
| M.3117 | B.177 | C.15 |
| M.3118 | B.178 | C.15 |
| M.3119 | B.179 | C.15 |
| M.3120 | B.180 | C.15 |
| M.3121 | B.181 | C.15 |
| M.3122 | B.182 | C.15 |
| M.3123 | B.183 | C.15 |
| M.3124 | B.184 | C.15 |
| M.3125 | B.185 | C.15 |
| M.3126 | B.186 | C.15 |
| M.3127 | B.187 | C.15 |
| M.3128 | B.188 | C.15 |
| M.3129 | B.189 | C.15 |
| M.3130 | B.190 | C.15 |
| M.3131 | B.191 | C.15 |
| M.3132 | B.192 | C.15 |
| M.3133 | B.193 | C.15 |
| M.3134 | B.194 | C.15 |
| M.3135 | B.195 | C.15 |
| M.3136 | B.196 | C.15 |

| comp. no. | herbicide B | safener C |
|---|---|---|
| M.3137 | B.1 | C.16 |
| M.3138 | B.2 | C.16 |
| M.3139 | B.3 | C.16 |
| M.3140 | B.4 | C.16 |
| M.3141 | B.5 | C.16 |
| M.3142 | B.6 | C.16 |
| M.3143 | B.7 | C.16 |
| M.3144 | B.8 | C.16 |
| M.3145 | B.9 | C.16 |
| M.3146 | B.10 | C.16 |
| M.3147 | B.11 | C.16 |
| M.3148 | B.12 | C.16 |
| M.3149 | B.13 | C.16 |
| M.3150 | B.14 | C.16 |
| M.3151 | B.15 | C.16 |
| M.3152 | B.16 | C.16 |
| M.3153 | B.17 | C.16 |
| M.3154 | B.18 | C.16 |
| M.3155 | B.19 | C.16 |
| M.3156 | B.20 | C.16 |
| M.3157 | B.21 | C.16 |
| M.3158 | B.22 | C.16 |
| M.3159 | B.23 | C.16 |
| M.3160 | B.24 | C.16 |
| M.3161 | B.25 | C.16 |
| M.3162 | B.26 | C.16 |
| M.3163 | B.27 | C.16 |
| M.3164 | B.28 | C.16 |
| M.3165 | B.29 | C.16 |
| M.3166 | B.30 | C.16 |
| M.3167 | B.31 | C.16 |
| M.3168 | B.32 | C.16 |
| M.3169 | B.33 | C.16 |
| M.3170 | B.34 | C.16 |
| M.3171 | B.35 | C.16 |
| M.3172 | B.36 | C.16 |
| M.3173 | B.37 | C.16 |
| M.3174 | B.38 | C.16 |
| M.3175 | B.39 | C.16 |
| M.3176 | B.40 | C.16 |

| comp. no. | herbicide B | safener C |
|---|---|---|
| M.3177 | B.41 | C.16 |
| M.3178 | B.42 | C.16 |
| M.3179 | B.43 | C.16 |
| M.3180 | B.44 | C.16 |
| M.3181 | B.45 | C.16 |
| M.3182 | B.46 | C.16 |
| M.3183 | B.47 | C.16 |
| M.3184 | B.48 | C.16 |
| M.3185 | B.49 | C.16 |
| M.3186 | B.50 | C.16 |
| M.3187 | B.51 | C.16 |
| M.3188 | B.52 | C.16 |
| M.3189 | B.53 | C.16 |
| M.3190 | B.54 | C.16 |
| M.3191 | B.55 | C.16 |
| M.3192 | B.56 | C.16 |
| M.3193 | B.57 | C.16 |
| M.3194 | B.58. | C.16 |
| M.3195 | B.59 | C.16 |
| M.3196 | B.60 | C.16 |
| M.3197 | B.61 | C.16 |
| M.3198 | B.62 | C.16 |
| M.3199 | B.63 | C.16 |
| M.3200 | B.64 | C.16 |
| M.3201 | B.65 | C.16 |
| M.3202 | B.66 | C.16 |
| M.3203 | B.67 | C.16 |
| M.3204 | B.68 | C.16 |
| M.3205 | B.69 | C.16 |
| M.3206 | B.70 | C.16 |
| M.3207 | B.71 | C.16 |
| M.3208 | B.72 | C.16 |
| M.3209 | B.73 | C.16 |
| M.3210 | B.74 | C.16 |
| M.3211 | B.75 | C.16 |
| M.3212 | B.76 | C.16 |
| M.3213 | B.77 | C.16 |
| M.3214 | B.78 | C.16 |
| M.3215 | B.79 | C.16 |
| M.3216 | B.80 | C.16 |

| comp. no. | herbicide B | safener C |
|---|---|---|
| M.3217 | B.81 | C.16 |
| M.3218 | B.82 | C.16 |
| M.3219 | B.83 | C.16 |
| M.3220 | B.84 | C.16 |
| M.3221 | B.85 | C.16 |
| M.3222 | B.86 | C.16 |
| M.3223 | B.87 | C.16 |
| M.3224 | B.88 | C.16 |
| M.3225 | B.89 | C.16 |
| M.3226 | B.90 | C.16 |
| M.3227 | B.91 | C.16 |
| M.3228 | B.92 | C.16 |
| M.3229 | B.93 | C.16 |
| M.3230 | B.94 | C.16 |
| M.3231 | B.95 | C.16 |
| M.3232 | B.96 | C.16 |
| M.3233 | B.97 | C.16 |
| M.3234 | B.98 | C.16 |
| M.3235 | B.99 | C.16 |
| M.3236 | B.100 | C.16 |
| M.3237 | B.101 | C.16 |
| M.3238 | B.102 | C.16 |
| M.3239 | B.103 | C.16 |
| M.3240 | B.104 | C.16 |
| M.3241 | B.105 | C.16 |
| M.3242 | B.106 | C.16 |
| M.3243 | B.107 | C.16 |
| M.3244 | B.108 | C.16 |
| M.3245 | B.109 | C.16 |
| M.3246 | B.110 | C.16 |
| M.3247 | B.111 | C.16 |
| M.3248 | B.112 | C.16 |
| M.3249 | B.113 | C.16 |
| M.3250 | B.114 | C.16 |
| M.3251 | B.115 | C.16 |
| M.3252 | B.116 | C.16 |
| M.3253 | B.117 | C.16 |
| M.3254 | B.118 | C.16 |
| M.3255 | B.119 | C.16 |
| M.3256 | B.120 | C.16 |

| comp. no. | herbicide B | safener C |
|---|---|---|
| M.3257 | B.121 | C.16 |
| M.3258 | B.122 | C.16 |
| M.3259 | B.123 | C.16 |
| M.3260 | B.124 | C.16 |
| M.3261 | B.125 | C.16 |
| M.3262 | B.126 | C.16 |
| M.3263 | B.127 | C.16 |
| M.3264 | B.128 | C.16 |
| M.3265 | B.129 | C.16 |
| M.3266 | B.130 | C.16 |
| M.3267 | B.131 | C.16 |
| M.3268 | B.132 | C.16 |
| M.3269 | B.133 | C.16 |
| M.3270 | B.134 | C.16 |
| M.3271 | B.135 | C.16 |
| M.3272 | B.136 | C.16 |
| M.3273 | B.137 | C.16 |
| M.3274 | B.138 | C.16 |
| M.3275 | B.139 | C.16 |
| M.3276 | B.140 | C.16 |
| M.3277 | B.141 | C.16 |
| M.3278 | B.142 | C.16 |
| M.3279 | B.143 | C.16 |
| M.3280 | B.144 | C.16 |
| M.3281 | B.145 | C.16 |
| M.3282 | B.146 | C.16 |
| M.3283 | B.147 | C.16 |
| M.3284 | B.148 | C.16 |
| M.3285 | B.149 | C.16 |
| M.3286 | B.150 | C.16 |
| M.3287 | B.151 | C.16 |
| M.3288 | B.152 | C.16 |
| M.3289 | B.153 | C.16 |
| M.3290 | B.154 | C.16 |
| M.3291 | B.155 | C.16 |
| M.3292 | B.156 | C.16 |
| M.3293 | B.157 | C.16 |
| M.3294 | B.158 | C.16 |
| M.3295 | B.159 | C.16 |
| M.3296 | B.160 | C.16 |

| comp. no. | herbicide B | safener C |
|---|---|---|
| M.3297 | B.161 | C.16 |
| M.3298 | B.162 | C.16 |
| M.3299 | B.163 | C.16 |
| M.3300 | B.164 | C.16 |
| M.3301 | B.165 | C.16 |
| M.3302 | B.166 | C.16 |
| M.3303 | B.167 | C.16 |
| M.3304 | B.168 | C.16 |
| M.3305 | B.169 | C.16 |
| M.3306 | B.170 | C.16 |
| M.3307 | B.171 | C.16 |
| M.3308 | B.172 | C.16 |
| M.3309 | B.173 | C.16 |
| M.3310 | B.174 | C.16 |
| M.3311 | B.175 | C.16 |
| M.3312 | B.176 | C.16 |
| M.3313 | B.177 | C.16 |
| M.3314 | B.178 | C.16 |
| M.3315 | B.179 | C.16 |
| M.3316 | B.180 | C.16 |
| M.3317 | B.181 | C.16 |
| M.3318 | B.182 | C.16 |
| M.3319 | B.183 | C.16 |
| M.3320 | B.184 | C.16 |
| M.3321 | B.185 | C.16 |
| M.3322 | B.186 | C.16 |
| M.3323 | B.187 | C.16 |
| M.3324 | B.188 | C.16 |
| M.3325 | B.189 | C.16 |
| M.3326 | B.190 | C.16 |
| M.3327 | B.191 | C.16 |
| M.3328 | B.192 | C.16 |
| M.3329 | B.193 | C.16 |
| M.3330 | B.194 | C.16 |
| M.3331 | B.195 | C.16 |
| M.3332 | B.196 | C.16 |
| M.3333 | B.1 | C.17 |
| M.3334 | B.2 | C.17 |
| M.3335 | B.3 | C.17 |
| M.3336 | B.4 | C.17 |

| comp. no. | herbicide B | safener C |
|---|---|---|
| M.3337 | B.5 | C.17 |
| M.3338 | B.6 | C.17 |
| M.3339 | B.7 | C.17 |
| M.3340 | B.8 | C.17 |
| M.3341 | B.9 | C.17 |
| M.3342 | B.10 | C.17 |
| M.3343 | B.11 | C.17 |
| M.3344 | B.12 | C.17 |
| M.3345 | B.13 | C.17 |
| M.3346 | B.14 | C.17 |
| M.3347 | B.15 | C.17 |
| M.3348 | B.16 | C.17 |
| M.3349 | B.17 | C.17 |
| M.3350 | B.18 | C.17 |
| M.3351 | B.19 | C.17 |
| M.3352 | B.20 | C.17 |
| M.3353 | B.21 | C.17 |
| M.3354 | B.22 | C.17 |
| M.3355 | B.23 | C.17 |
| M.3356 | B.24 | C.17 |
| M.3357 | B.25 | C.17 |
| M.3358 | B.26 | C.17 |
| M.3359 | B.27 | C.17 |
| M.3360 | B.28 | C.17 |
| M.3361 | B.29 | C.17 |
| M.3362 | B.30 | C.17 |
| M.3363 | B.31 | C.17 |
| M.3364 | B.32 | C.17 |
| M.3365 | B.33 | C.17 |
| M.3366 | B.34 | C.17 |
| M.3367 | B.35 | C.17 |
| M.3368 | B.36 | C.17 |
| M.3369 | B.37 | C.17 |
| M.3370 | B.38 | C.17 |
| M.3371 | B.39 | C.17 |
| M.3372 | B.40 | C.17 |
| M.3373 | B.41 | C.17 |
| M.3374 | B.42 | C.17 |
| M.3375 | B.43 | C.17 |
| M.3376 | B.44 | C.17 |

| comp. no. | herbi-cide B | safe-ner C |
|---|---|---|
| M.3377 | B.45 | C.17 |
| M.3378 | B.46 | C.17 |
| M.3379 | B.47 | C.17 |
| M.3380 | B.48 | C.17 |
| M.3381 | B.49 | C.17 |
| M.3382 | B.50 | C.17 |
| M.3383 | B.51 | C.17 |
| M.3384 | B.52 | C.17 |
| M.3385 | B.53 | C.17 |
| M.3386 | B.54 | C.17 |
| M.3387 | B.55 | C.17 |
| M.3388 | B.56 | C.17 |
| M.3389 | B.57 | C.17 |
| M.3390 | B.58. | C.17 |
| M.3391 | B.59 | C.17 |
| M.3392 | B.60 | C.17 |
| M.3393 | B.61 | C.17 |
| M.3394 | B.62 | C.17 |
| M.3395 | B.63 | C.17 |
| M.3396 | B.64 | C.17 |
| M.3397 | B.65 | C.17 |
| M.3398 | B.66 | C.17 |
| M.3399 | B.67 | C.17 |
| M.3400 | B.68 | C.17 |
| M.3401 | B.69 | C.17 |
| M.3402 | B.70 | C.17 |
| M.3403 | B.71 | C.17 |
| M.3404 | B.72 | C.17 |
| M.3405 | B.73 | C.17 |
| M.3406 | B.74 | C.17 |
| M.3407 | B.75 | C.17 |
| M.3408 | B.76 | C.17 |
| M.3409 | B.77 | C.17 |
| M.3410 | B.78 | C.17 |
| M.3411 | B.79 | C.17 |
| M.3412 | B.80 | C.17 |
| M.3413 | B.81 | C.17 |
| M.3414 | B.82 | C.17 |
| M.3415 | B.83 | C.17 |
| M.3416 | B.84 | C.17 |

| comp. no. | herbi-cide B | safe-ner C |
|---|---|---|
| M.3417 | B.85 | C.17 |
| M.3418 | B.86 | C.17 |
| M.3419 | B.87 | C.17 |
| M.3420 | B.88 | C.17 |
| M.3421 | B.89 | C.17 |
| M.3422 | B.90 | C.17 |
| M.3423 | B.91 | C.17 |
| M.3424 | B.92 | C.17 |
| M.3425 | B.93 | C.17 |
| M.3426 | B.94 | C.17 |
| M.3427 | B.95 | C.17 |
| M.3428 | B.96 | C.17 |
| M.3429 | B.97 | C.17 |
| M.3430 | B.98 | C.17 |
| M.3431 | B.99 | C.17 |
| M.3432 | B.100 | C.17 |
| M.3433 | B.101 | C.17 |
| M.3434 | B.102 | C.17 |
| M.3435 | B.103 | C.17 |
| M.3436 | B.104 | C.17 |
| M.3437 | B.105 | C.17 |
| M.3438 | B.106 | C.17 |
| M.3439 | B.107 | C.17 |
| M.3440 | B.108 | C.17 |
| M.3441 | B.109 | C.17 |
| M.3442 | B.110 | C.17 |
| M.3443 | B.111 | C.17 |
| M.3444 | B.112 | C.17 |
| M.3445 | B.113 | C.17 |
| M.3446 | B.114 | C.17 |
| M.3447 | B.115 | C.17 |
| M.3448 | B.116 | C.17 |
| M.3449 | B.117 | C.17 |
| M.3450 | B.118 | C.17 |
| M.3451 | B.119 | C.17 |
| M.3452 | B.120 | C.17 |
| M.3453 | B.121 | C.17 |
| M.3454 | B.122 | C.17 |
| M.3455 | B.123 | C.17 |
| M.3456 | B.124 | C.17 |

| comp. no. | herbicide B | safener C |
|---|---|---|
| M.3457 | B.125 | C.17 |
| M.3458 | B.126 | C.17 |
| M.3459 | B.127 | C.17 |
| M.3460 | B.128 | C.17 |
| M.3461 | B.129 | C.17 |
| M.3462 | B.130 | C.17 |
| M.3463 | B.131 | C.17 |
| M.3464 | B.132 | C.17 |
| M.3465 | B.133 | C.17 |
| M.3466 | B.134 | C.17 |
| M.3467 | B.135 | C.17 |
| M.3468 | B.136 | C.17 |
| M.3469 | B.137 | C.17 |
| M.3470 | B.138 | C.17 |
| M.3471 | B.139 | C.17 |
| M.3472 | B.140 | C.17 |
| M.3473 | B.141 | C.17 |
| M.3474 | B.142 | C.17 |
| M.3475 | B.143 | C.17 |
| M.3476 | B.144 | C.17 |
| M.3477 | B.145 | C.17 |
| M.3478 | B.146 | C.17 |
| M.3479 | B.147 | C.17 |
| M.3480 | B.148 | C.17 |
| M.3481 | B.149 | C.17 |
| M.3482 | B.150 | C.17 |
| M.3483 | B.151 | C.17 |
| M.3484 | B.152 | C.17 |
| M.3485 | B.153 | C.17 |
| M.3486 | B.154 | C.17 |
| M.3487 | B.155 | C.17 |
| M.3488 | B.156 | C.17 |
| M.3489 | B.157 | C.17 |
| M.3490 | B.158 | C.17 |
| M.3491 | B.159 | C.17 |
| M.3492 | B.160 | C.17 |
| M.3493 | B.161 | C.17 |
| M.3494 | B.162 | C.17 |
| M.3495 | B.163 | C.17 |
| M.3496 | B.164 | C.17 |

| comp. no. | herbicide B | safener C |
|---|---|---|
| M.3497 | B.165 | C.17 |
| M.3498 | B.166 | C.17 |
| M.3499 | B.167 | C.17 |
| M.3500 | B.168 | C.17 |
| M.3501 | B.169 | C.17 |
| M.3502 | B.170 | C.17 |
| M.3503 | B.171 | C.17 |
| M.3504 | B.172 | C.17 |
| M.3505 | B.173 | C.17 |
| M.3506 | B.174 | C.17 |
| M.3507 | B.175 | C.17 |
| M.3508 | B.176 | C.17 |
| M.3509 | B.177 | C.17 |
| M.3510 | B.178 | C.17 |
| M.3511 | B.179 | C.17 |
| M.3512 | B.180 | C.17 |
| M.3513 | B.181 | C.17 |
| M.3514 | B.182 | C.17 |
| M.3515 | B.183 | C.17 |
| M.3516 | B.184 | C.17 |
| M.3517 | B.185 | C.17 |
| M.3518 | B.186 | C.17 |
| M.3519 | B.187 | C.17 |
| M.3520 | B.188 | C.17 |
| M.3521 | B.189 | C.17 |
| M.3522 | B.190 | C.17 |
| M.3523 | B.191 | C.17 |
| M.3524 | B.192 | C.17 |
| M.3525 | B.193 | C.17 |
| M.3526 | B.194 | C.17 |
| M.3527 | B.195 | C.17 |
| M.3528 | B.196 | C.17 |
| M.3529 | -- | C.1 |
| M.3530 | -- | C.2 |
| M.3531 | -- | C.3 |
| M.3532 | -- | C.4 |
| M.3533 | -- | C.5 |
| M.3534 | -- | C.6 |
| M.3535 | -- | C.7 |
| M.3536 | -- | C.8 |

| comp. no. | herbicide B | safener C |
|---|---|---|
| M.3537 | -- | C.9 |
| M.3538 | -- | C.10 |
| M.3539 | -- | C.11 |
| M.3540 | -- | C.12 |
| M.3541 | -- | C.13 |

| comp. no. | herbicide B | safener C |
|---|---|---|
| M.3542 | -- | C.14 |
| M.3543 | -- | C.15 |
| M.3544 | -- | C.16 |
| M.3545 | -- | C.17 |

It may furthermore be beneficial to apply the diaminotriazine compounds of formula (I) alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates may also be added.

The invention also relates to agrochemical compositions comprising at least an auxiliary and at least one diaminotriazine compound of formula (I) according to the invention.

An agrochemical composition comprises a pesticidally effective amount of a diaminotriazine compound of formula (I). The term "effective amount" denotes an amount of the composition or of the compounds I, which is sufficient for controlling unwanted plants, especially for controlling unwanted plants in cultivated plants and which does not result in a substantial damage to the treated plants. Such an amount can vary in a broad range and is dependent on various factors, such as the plants to be controlled, the treated cultivated plant or material, the climatic conditions and the specific diaminotriazine compound of formula (I) used.

The diaminotriazine compound of formula (I), their N-oxides or salts can be converted into customary types of agrochemical compositions, e. g. solutions, emulsions, suspensions, dusts, powders, pastes, granules, pressings, capsules, and mixtures thereof. Examples for agrochemical composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further agrochemical compositions types are defined in the "Catalogue of pesticide formulation types and international coding system", Technical Monograph No. 2, 6th Ed. May 2008, CropLife International.

The agrochemical compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of

alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidally activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for agrochemical composition types and their preparation are:

i) Water-soluble concentrates (SL, LS)

10-60 wt% of an diaminotriazine compound of formula (I) according to the invention and 5-278 wt% wetting agent (e.g. alcohol alkoxylates) are dissolved in water and/or in a water-soluble solvent (e.g. alcohols) ad 100 wt%. The active substance dissolves upon dilution with water.

ii) Dispersible concentrates (DC)

5-25 wt% of an diaminotriazine compound of formula (I) according to the invention and 1-10 wt% dispersant (e. g. polyvinylpyrrolidone) are dissolved in organic solvent (e.g. cyclohexanone) ad 100 wt%. Dilution with water gives a dispersion.

iii) Emulsifiable concentrates (EC)

278-70 wt% of an diaminotriazine compound of formula (I) according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.

iv) Emulsions (EW, EO, ES)

5-40 wt% of an diaminotriazine compound of formula (I) according to the invention and 1-10 wt% emulsifiers (e.g. calcium dodecylbenzenesulfonate and castor oil ethoxylate) are dissolved in 20-40 wt% water-insoluble organic

solvent (e.g. aromatic hydrocarbon). This mixture is introduced into water ad 100 wt% by means of an emulsifying machine and made into a homogeneous emulsion. Dilution with water gives an emulsion.

v) Suspensions (SC, OD, FS)

In an agitated ball mill, 20-60 wt% of an diaminotriazine compound of formula (I) according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.

vi) Water-dispersible granules and water-soluble granules (WG, SG)

50-80 wt% of an diaminotriazine compound of formula (I) according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.

vii) Water-dispersible powders and water-soluble powders (WP, SP, WS)

50-80 wt% of an diaminotriazine compound of formula (I) according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.

viii) Gel (GW, GF)

In an agitated ball mill, 5-25 wt% of an diaminotriazine compound of formula (I) according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.

iv) Microemulsion (ME)

5-20 wt% of an diaminotriazine compound of formula (I) according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.

iv) Microcapsules (CS)

An oil phase comprising 5-50 wt% of an diaminotriazine compound of formula (I) according to the invention, 0-40 wt% water insoluble organic solvent (e.g. aromatic hydrocarbon), 2-278 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of an diaminotriazine compound of formula (I) according to the invention, 0-40 wt% water insoluble organic solvent (e.g. aromatic hydrocarbon), and an isocyanate monomer (e.g. diphenylmethene-4,4'-diisocyanate) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.

ix) Dustable powders (DP, DS)

1-10 wt% of an diaminotriazine compound of formula (I) according to the invention are ground finely and mixed intimately with solid carrier (e.g. finely divided kaolin) ad 100 wt%.

x) Granules (GR, FG)

0.5-30 wt% of an diaminotriazine compound of formula (I) according to the invention is ground finely and

associated with solid carrier (e.g. silicate) ad 100 wt%. Granulation is achieved by extrusion, spray-drying or the fluidized bed.

xi) Ultra-low volume liquids (UL)

1-50 wt% of an diaminotriazine compound of formula (I) according to the invention are dissolved in organic solvent (e.g. aromatic hydrocarbon) ad 100 wt%.

The agrochemical compositions types i) to xi) may optionally comprise further auxiliaries, such as 0,1-1 wt% bactericides, 5-278 wt% anti-freezing agents, 0,1-1 wt% anti-foaming agents, and 0,1-1 wt% colorants.

The agrochemical compositions generally comprise between 0.01 and 95%, preferably between 0.1 and 90%, and in particular between 0.5 and 75%, by weight of the diaminotriazine compounds of formula (I). The diaminotriazine compounds of formula (I) are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The agrochemical compositions in question give, after two-to-tenfold dilution, active substance concentrations of from 0.01 to 60% by weight, preferably from 0.1 to 40% by weight, in the ready-to-use preparations. Application can be carried out before or during sowing.

Methods for applying diaminotriazine compounds of formula (I) or agrochemical compositions thereof, on to plant propagation material, especially seeds, include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

Various types of oils, wetters, adjuvants, fertilizer, or micronutrients, and further pesticides (e.g. herbicides, insecticides, fungicides, growth regulators, safeners) may be added to the diaminotriazine compounds of formula (I) or the agrochemical compositions comprising them as premix or, if appropriate not until immediately prior to use (tank mix). These agents can be admixed with the agrochemical compositions according to the invention in a weight ratio of 1:100 to 100:1, preferably 1:10 to 10:1.

The user applies the diaminotriazine compounds of formula (I) according to the invention or the agrochemical compositions comprising them usually from a pre-dosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, either individual components of the agrochemical composition according to the invention or partially premixed components, e. g. components comprising azines of formula (I) may be mixed by the user in a spray tank and further auxiliaries and additives may be added, if appropriate.

In a further embodiment, individual components of the agrochemical composition according to the invention such as parts of a kit or parts of a binary or ternary mixture may be mixed by the user himself in a spray tank and further auxiliaries may be added, if appropriate.

In a further embodiment, either individual components of the agrochemical composition according to the invention or partially premixed components, e. g components comprising diaminotriazine compounds of formula (I), can be applied jointly (e.g. after tank mix) or consecutively.

The diaminotriazine compounds of formula (I), are suitable as herbicides. They are suitable as such or as an appropriately formulated composition (agrochemical composition).

The diaminotriazine compounds of formula (I), or the agrochemical compositions comprising the azines of formula (I), control vegetation on non-crop areas very efficiently, especially at high rates of application. They act against broad-leaved weeds and grass weeds in crops such as wheat, rice, maize, soya and cotton without causing any significant damage to the crop plants. This effect is mainly observed at low rates of application.

The diaminotriazine compounds of formula (I), or the agrochemical compositions comprising them, are applied to the plants mainly by spraying the leaves or are applied to the soil in which the plant seeds have been sown. Here, the application can be carried out using, for example, water as carrier by customary spraying techniques using spray liquor amounts of from about 100 to 1000 l/ha (for example from 300 to 400 l/ha). The diaminotriazine compounds of formula (I), or the agrochemical compositions comprising them, may also be applied by the low-volume or the ultra-low-volume method, or in the form of microgranules.

Application of the diaminotriazine compounds of formula (I), or the agrochemical compositions comprising them, can be done before, during and/or after the emergence of the undesirable plants.

The diaminotriazine compounds of formula (I), or the agrochemical compositions comprising them, can be applied pre-, post-emergence or pre-plant, or together with the seed of a crop plant. It is also possible to apply the diaminotriazine compounds of formula (I), or the agrochemical compositions comprising them, by applying seed, pretreated with the diaminotriazine compounds of formula (I), or the agrochemical compositions comprising them, of a crop plant. If the active ingredients are less well tolerated by certain crop plants, application techniques may be used in which the herbicidal compositions are sprayed, with the aid of the spraying equipment, in such a way that as far as possible they do not come into contact with the leaves of the sensitive crop plants, while the active ingredients reach the leaves of undesirable plants growing underneath, or the bare soil surface (post-directed, lay-by).

In a further embodiment, the diaminotriazine compounds of formula (I), or the agrochemical compositions comprising them, can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the diaminotriazine compounds of formula (I), or the agrochemical compositions prepared therefrom. Here, the herbicidal compositions can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the useful plants mentioned above, but also the seed of transgenic plants or plants obtained by customary breeding methods.

When employed in plant protection, the amounts of active substances applied, i.e. the diaminotriazine compounds of formula (I), without formulation auxiliaries, are, depending on the kind of effect desired, from 0.001 to 2 kg per ha, preferably from 0.005 to 2 kg per ha, more preferably from 0.005 to 0.9 kg per ha and in particular from 0.05 to 0.5 kg per ha.

In another embodiment of the invention, the application rate of the diaminotriazine compounds of formula (I) is from 0.001 to 3 kg/ha, preferably from 0.005 to 2.5 kg/ha, of active substance (a.s.).

In another preferred embodiment of the invention, the rates of application of the diaminotriazine compounds of formula (I) according to the present invention (total amount of diaminotriazine compounds of formula (I)) are from 0.1 g/ha to 3000 g/ha, preferably 10 g/ha to 1000 g/ha, depending on the control target, the season, the target plants and the growth stage.

In another preferred embodiment of the invention, the application rates of the diaminotriazine compounds of formula (I) are in the range from 0.1 g/ha to 5000 g/ha and preferably in the range from 1 g/ha to 2500 g/ha or from 5 g/ha to 2000 g/ha.

In another preferred embodiment of the invention, the application rate of the diaminotriazine compounds of formula (I) is 0.1 to 1000 g/ha, preferably1 to 750 g/ha, more preferably 5 to 500 g/ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another embodiment of the invention, to treat the seed, the amounts of active substances applied, i.e. the diaminotriazine compounds of formula (I) are generally employed in amounts of from 0.001 to 10 kg per 100 kg of seed.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Depending on the application method in question, the diaminotriazine compounds of formula (I), or the agrochemical compositions comprising them, can additionally be employed in a further number of crop plants for eliminating undesirable plants. Examples of suitable crops are the following:

Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera and Zea mays.

Preferred crops are Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera and Zea mays.

Especially preferred crops are crops of cereals, corn, soybeans, rice, oilseed rape, cotton, potatoes, peanuts or permanent crops.

The diaminotriazine compounds of formula (I) according to the invention, or the agrochemical compositions comprising them, can also be used in genetically modified plants. The term "genetically modified plants" is to be understood as plants whose genetic material has been modified by the use of recombinant DNA techniques to include an inserted sequence of DNA that is not native to that plant species' genome or to exhibit a deletion of DNA that was native to that species' genome, wherein the modification(s) cannot readily be obtained by cross breeding, mutagenesis or natural recombination alone. Often, a particular genetically modified plant will be one that has obtained its genetic modification(s) by inheritance through a natural breeding or propagation process from an ancestral plant whose genome was the one directly treated by use of a recombinant DNA technique. Typically, one or more genes have been integrated into the genetic material of a genetically modified plant in order to improve certain properties of the plant. Such genetic modifications also include but are not limited to targeted post-translational modification of protein(s), oligo- or polypeptides. e. g., by inclusion therein of amino acid mutation(s) that permit, decrease, or promote glycosylation or polymer additions such as prenylation, acetylation farnesylation, or PEG moiety attachment.

Plants that have been modified by breeding, mutagenesis or genetic engineering, e.g. have been rendered tolerant to applications of specific classes of herbicides, such as auxin herbicides such as dicamba or 2,4-D; bleacher herbicides such as hydroxyphenylpyruvate dioxygenase (HPPD) inhibitors or phytoene desaturase (PDS) inhibitors; acetolactate synthase (ALS) inhibitors such as sulfonyl ureas or imidazolinones; enolpyruvyl shikimate 3-phosphate synthase (EPSP) inhibitors such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering; furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are, for example, described in Pest Management Science 61, 2005, 246; 61, 2005, 258; 61, 2005, 277; 61, 2005, 269; 61, 2005, 286; 64, 2008, 326; 64, 2008, 332; Weed Science 57, 2009, 108; Australian Journal of Agricultural Research 58, 2007, 708; Science 316, 2007, 1185; and references quoted therein. Several cultivated plants have been rendered tolerant to herbicides by mutagenesis and conventional methods of breeding, e. g., Clearfield@ summer rape (Canola, BASF SE, Germany) being tolerant to imidazolinones, e. g., imazamox, or ExpressSun® sunflowers (DuPont, USA) being tolerant to sulfonyl ureas, e. g., tribenuron. Genetic engineering methods have been used to render cultivated plants such as soybean, cotton, corn, beets and rape, tolerant to herbicides such as glyphosate, imidazolinones and glufosinate, some of which are under development or commercially available under the brands or trade names RoundupReady® (glyphosate tolerant, Monsanto, USA), Cultivance® (imidazolinone tolerant, BASF SE, Germany) and LibertyLink® (glufosinate tolerant, Bayer CropScience, Germany).

Furthermore, plants are also covered that are by the use of recombinant DNA techniques capable to synthesize one or more insecticidal proteins, especially those known from the bacterial genus Bacillus, particularly from Bacillus thuringiensis, such as delta-endotoxins, e. g., CryIA(b), CryIA(c), CryIF, CryIF(a2), CryIIA(b), CryIIIA, CryIIIB(b1) or Cry9c; vegetative insecticidal proteins (VIP), e. g., VIP1, VIP2, VIP3 or VIP3A; insecticidal proteins of bacteria colonizing nematodes, e. g., Photorhabdus spp. or Xenorhabdus spp.; toxins produced by animals, such as scorpion toxins, arachnid toxins, wasp toxins, or other insect-specific neurotoxins; toxins produced by fungi, such as Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as including pre-toxins, hybrid proteins, truncated or otherwise modified proteins. Hybrid proteins are characterized by a new combination of protein domains, (see, e. g., WO 02/0278701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g., in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications

mentioned above, and some of which are commercially available such as YieldGard® (corn cultivars producing the Cry1Ab toxin), YieldGard® Plus (corn cultivars producing CrylAb and Cry3Bb1 toxins), Starlink® (corn cultivars producing the Cry9c toxin), Herculex® RW (corn cultivars producing Cry34Ab1, Cry35Ab1 and the enzyme Phosphinothricin-N-Acetyltransferase [PAT]); NuCOTN® 33B (cotton cultivars producing the CrylAc toxin), Bollgard® I (cotton cultivars producing the Cry1Ac toxin), Bollgard® II (cotton cultivars producing CrylAc and Cry2Ab2 toxins); VIPCOT® (cotton cultivars producing a VIP-toxin); NewLeaf® (potato cultivars producing the Cry3A toxin); Bt-Xtra®, NatureGard®, Knock-Out®, BiteGard®, Protecta®, Bt11 (e. g., Agrisure® CB) and Bt176 from Syngenta Seeds SAS, France, (corn cultivars producing the CrylAb toxin and PAT enzyme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the CrylAc toxin) and 27807 from Pioneer Overseas Corporation, Belgium (corn cultivars producing the Cry1F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e.g., EP-A 392 225), plant disease resistance genes (e. g., potato culti-vars, which express resistance genes acting against Phytophthora infestans derived from the Mexican wild potato, Solanum bulbocastanum) or T4-lyso-zym (e.g., potato cultivars capable of synthesizing these proteins with increased resistance against bacteria such as Erwinia amylovora). The methods for producing such genetically modi-fied plants are generally known to the person skilled in the art and are described, e.g., in the publications mentioned above.

Furthermore, plants are also covered that are by the use of recombinant DNA techniques capable to synthesize one or more proteins to increase the productivity (e.g., bio-mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of ingredients or new ingredients, specifically to improve human or animal nutrition, e. g., oil crops that produce health-promoting long-chain omega-3 fatty acids or unsaturated omega-9 fatty acids (e. g., Nexera® rape, Dow AgroSciences, Canada).

Furthermore, plants are also covered that contain by the use of recombinant DNA techniques a modified amount of ingredients or new ingredients, specifically to improve raw material production, e.g., potatoes that produce increased amounts of amylopectin (e.g. Amflora® potato, BASF SE, Germany).

A further embodiment of the invention is a method of controlling undesired vegetation, which comprises allowing a herbicidally active amount of at least one compound of formula (I) and as defined above to act on plants, their environment or on seed.

The preparation of the diaminotriazine compounds of formula (I) is illustrated by examples.

The products shown below were characterized by the mass ([m/z]) or retention time (RT; [min.]) determined by HPLC-MS spectrometry.

HPLC-MS = high performance liquid chromatography-coupled mass spectrometry; HPLC column:
RP-18 column (Chromolith Speed ROD from Merck KgaA, Germany), 50*4.6 mm; mobile phase: acetonitrile + 0.1% trifluoroacetic acid (TFA)/water + 0.1% TFA using a gradient from 5:95 to 100:0 over 5 minutes at 40°C, flow rate 1.8 ml/min.

MS: quadrupole electrospray ionization, 80 V (positive mode).
The following abbreviations are used:

| | |
|---|---|
| CH: Cyclohexane | THF: Tetrahydrofurane |
| EtOAc: Ethyl acetate | HPLC: High pressure chromatography |
| MeOH: Methanol | LC: Liquid chromatography |
| TFA: Trifluoroacetic acid | MS: Mass spectrometry |

A Preparation examples

Example 1: Preparation of 2-[4-amino-6-(2,3,5,6-tetrafluoroanilino)-1,3,5-triazin-2-yl]-2-methyl-propan-1-ol

Step I. Preparation of 1-carbyimidoyl-3-(2,3,5,6-tetrafluorophenyl)guanidine

A suspension of 2,3,5,6-tetrafluoroaniline (2.0 g, 12.1 mmol) and 1-cyanoguanidine (1.22 g, 14.5 mmol) in a mixture of acetonitrile and aq. hydrochloride (38% w/w) were heated to 150 °C in a microwave reactor. The resulting mixture was carefully added to aq. $NaHCO_3$, ethyl acetate was added and the phases were separated. The organic

phase was dried over anhydrous $Na_2SO_4$, filtered and concentrated under reduced pressure yielding the title compound (0.83 g, 27.6% yield) as a colorless solid.
MS (ESI) m/z = 250.1 [M+H$^+$]
$^1$H NMR (400 MHz, $d_6$-DMSO): δ 7.19 - 7.10 (m, 1H), 6.75 (br s, 4H), 5.45 (s, 2H) ppm.

Step II. Preparation of 3-hydroxy-2,2-dimethyl-propanoyl fluoride

To a solution of 3-hydroxy-2,2-dimethyl-propanoic acid (1.0 g, 8.5 mmol) in DCM at -78 °C was added DAST (2.73 g, 16.9 mmol). The temperature was allowed to warm to room temperature overnight after which the mixture was used as such in the following step.

Step III. Preparation of 2-[4-amino-6-(2,3,5,6-tetrafluoroanilino)-1,3,5-triazin-2-yl]-2-methyl-propan-1-ol

3-hydroxy-2,2-dimethyl-propanoyl fluoride (1.0 g, 8.3 mmol) in DCM, as prepared in step II, was added to a solution of 1-carbyimidoyl-3-(2,3,5,6-tetrafluorophenyl)guanidine (2.32 g, 9.31 mmol) in a mixture of THF and triethylamine (2.57 g, 25.4 mmol). The reaction mixture was heated to 60 °C for 4 h, cooled to room temperature and diluted with water and ethyl acetate. The phases were separated and the organic phase was dried over $Na_2SO_4$, filtered and concentrated in vacuo. The crude product was purified using column chromatography (ISCO-Combiflash Rf, cyclohexane/ethyl acetate) to afford the title compound as a colorless solid (350 mg, 13% yield).
MS(ESI) m/z = 332.0 [M+H$^+$]
$^1$H NMR (400 MHz, $d_6$-DMSO): δ 9.37 (s, 1H), 7.82 - 7.74 (m, 1H), 7.04 - 6.93 (br, 2H), 4.64 - 4.59 (m, 1H), 3.48 (d, 2H), 1.14 (s, 3H) ppm.

Example 2: 2-[4-amino-6-(2,3,5,6-tetrafluoroanilino)-1,3,5-triazin-2-yl]-2-fluoro-ethanol

Step 1. Preparation of methyl 3-benzyloxy-2-hydroxy-propanoate

A mixture of methyl oxirane-2-carboxylate (15 g, 0.15 mol) and $Mg(ClO_4)_2$ (8.16 g, 0.037 mol) in BnOH (31.8 g, 0.29 mol) was heated to 60 °C and stirred for 24 h. Ethyl acetate (100 mL) was added, washed with water and brine, dried over $Na_2SO_4$ and concentrated to give 57 g methyl 3-benzyloxy-2-hydroxy-propanoate (crude) as a yellow oil.
1H NMR (400 MHz, chloroform-d) : δ = 7.40 - 7.28 (m, 12H), 4.69 (s, 3H), 4.59 - 4.54 (m, 1H), 4.51 - 4.46 (m, 1H), 4.42 (br s, 1H), 3.82 (s, 3H), 3.81 (br s, 1H), 3.75 (d, J=3.0 Hz, 2H), 2.66 (br s, 1H)

Step 2. Preparation of 3-benzyloxy-2-hydroxy-propanoic acid

A mixture of 3-benzyloxy-2-hydroxy-propanoate (57 g, 0.29 mol) and $LiOH.H_2O$ (25 g, 0.59 mol) in THF/$H_2O$ (250 mL/ 250 mL) was stirred for 16 h at 10 °C. After extraction with DCM (100 mL*2), the pH of the aqueous phase was adjusted to 3 with 6N HCl, extracted with DCM (100 mL*2) washed with brine, dried over $Na_2SO_4$ and concentrated to give 18 g 3-benzyloxy-2-hydroxy-propanoic acid (68%) as yellow solid.
1H NMR (400 MHz, CHLOROFORM-d) δ = 7.36 - 7.27 (m, 5H), 4.62 - 4.51 (m, 2H), 4.36 (br s, 1H), 3.85 - 3.73 (m, 3H)

Step 3. Preparation of 6-(2-benzyloxy-1-fluoro-ethyl)-N4-(2,3,5,6-tetrafluorophenyl)-1,3,5-triazine-2,4-diamine

To a solution of 3-benzyloxy-2-hydroxy-propanoic acid (1 g, 5.1 mmol) in DCM (25 mL) was added DAST (1.2 mL, 10.2 mmol) at -40 °C and the reaction was stirred for 2 h at 10 °C. The above solution was added into a mixture of 1-carbyimidoyl-3-(2,3,5,6-tetrafluorophenyl)guanidine (1.06 g, 4.25 mmol) and $Et_3N$ (1.2 mL, 8.5 mmol) in THF (25 mL) at 0 °C, heated to 60 °C and stirred for 1 h. Water (50 mL) was added, extracted with DCM (20 mL) and the organic layer was washed with brine, dried over $Na_2SO_4$ and concentrated. The crude product was purified by prep-HPLC (TFA, MeCN-$H_2O$) to give 450 mg 6-(2-benzyloxy-1-fluoro-ethyl)-N4-(2,3,5,6-tetrafluorophenyl)-1,3,5-triazine-2,4-diamine (26%) as yellow solid.
1H NMR (400 MHz, DMSO-d6) δ = 9.69 (s, 1H), 7.86 - 7.75 (m, 1H), 7.39 (br s, 1H), 7.35 - 7.28 (m, 5H), 7.23 (br s, 1H), 5.43 - 5.24 (m, 1H), 4.61 - 4.50 (m, 2H), 3.89 (br s, 1H), 3.82 (br d, J=4.5 Hz, 1H)

Step 4. Preparation of 2-[4-amino-6-(2,3,5,6-tetrafluoroanilino)-1,3,5-triazin-2-yl]-2-fluoro-ethanol

A mixture of 6-(2-benzyloxy-1-fluoro-ethyl)-N4-(2,3,5,6-tetrafluorophenyl)-1,3,5-triazine-2,4-diamine (0.8 g, 2.4 mmol) and $Pd(OH)_2$ (0.4 g) in MeOH (300 mL) was heated to 50 °C and stirred for 16 h under a hydrogen atmosphere. After filtration and concentration, the crude product was purified by prep-HPLC (TFA,MeCN-$H_2O$) to give 250 mg of 2-[4-amino-6-(2,3,5,6-tetrafluoroanilino)-1,3,5-triazin-2-yl]-2-fluoro-ethanol (40%) as a yellow solid.
MS(ESI) m/z = 321.8 [M+H$^+$]
1H NMR (400MHz, DMSO-d6) $\delta$ = 9.65 (br s, 1H), 7.86 - 7.74 (m, 1H), 7.35 (br s, 1H), 7.20 (br s, 1H), 5.21 - 4.97 (m, 2H), 3.90 - 3.66 (m, 2H)

The compounds 3 to 11 listed below in table C, D and E have been prepared by analogy to the example 1 mentioned above.

Table C:

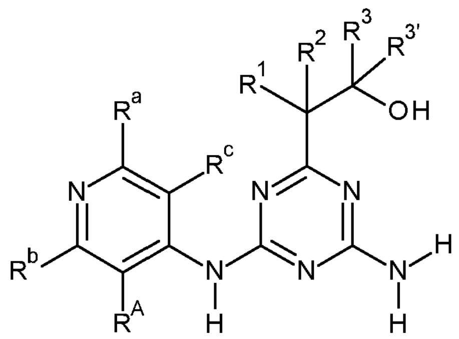

| No. | **R^a** | **R^b** | **R^c** | **R^A** | **R^1** | **R^2** | **R^3** | **R^3'** | MS1) [m/z] |
|---|---|---|---|---|---|---|---|---|---|
| 3 | F | F | F | F | F | F | $-CH_2-CH_2-CH_2-CH_2-$ | | 395.1 |
| 4 | F | F | F | F | F | F | $CH_3$ | $CH_3$ | 368.3 |
| 5 | F | F | F | F | F | H | $CH_3$ | $CH_3$ | 350.9 |
| 6 | F | F | F | F | F | $CH_3$ | $CH_3$ | $CH_3$ | 364.9 |

Table D:

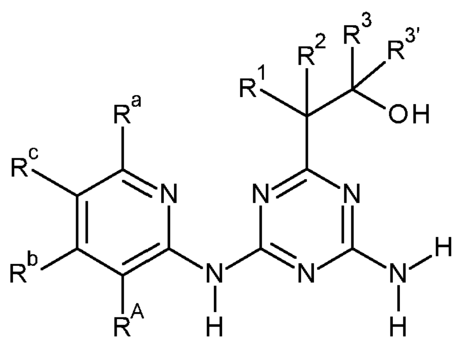

| No. | **R^a** | **R^b** | **R^c** | **R^A** | **R^1** | **R^2** | **R^3** | **R^3'** | MS1) [m/z] |
|---|---|---|---|---|---|---|---|---|---|
| 7 | F | Cl | F | F | F | F | $CH_3$ | $CH_3$ | 384.9 |
| 8 | F | Cl | F | F | F | F | $-CH_2-CH_2-CH_2-CH_2-$ | | 411.1 |
| 9 | F | Cl | F | F | F | $CH_3$ | $CH_3$ | $CH_3$ | 380.9 |

Table E:

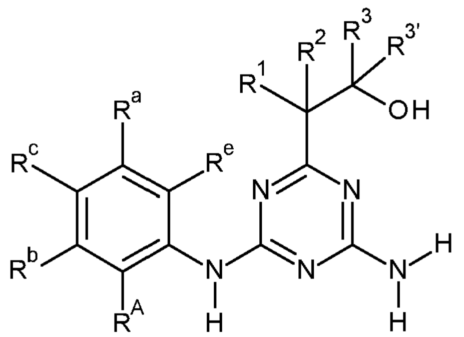

| No. | **R^a** | **R^b** | **R^c** | **R^d** | **R^A** | **R^1** | **R^2** | **R^3** | **R^3'** | MS[1)] [m/z] |
|---|---|---|---|---|---|---|---|---|---|---|
| 10 | F | F | H | F | F | F | H | $CH_3$ | $CH_3$ | 349.9 |
| 11 | F | F | H | F | F | F | $CH_3$ | $CH_3$ | $CH_3$ | 363.9 |

1) Mass Spectrum $M^+$ or $[M+H]^+$ [m/z]

B Use examples

The herbicidal activity of the azines of formula (I) was demonstrated by the following greenhouse experiments: The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This cover caused uniform germination of the test plants, unless this had been impaired by the active ingredients.

For the post-emergence treatment, the test plants were first grown to a height of 3 to 278 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.

Depending on the species, the plants were kept at 10 - 25°C or 20 - 35°C, respectively.

The test period extended over 2 to 4 weeks. During this time, the plants were tended, and their response to the individual treatments was evaluated.

Evaluation was carried out using a scale from 0 to 100. 100 means no emergence of the plants, or complete destruction of at least the aerial moieties, and 0 means no damage, or normal course of growth. A moderate herbicidal activity is given at values of at least 60, a good herbicidal activity is given at values of at least 70, and a very good herbicidal activity is given at values of at least 85.

The plants used in the greenhouse experiments were of the following species:

| Bayer code | Scientific name |
|---|---|
| ABUTH | Abutilon theophrasti |
| ALOMY | Alopecurus myosuroides |
| AMARE | Amaranthus retroflexus |
| ECHCG | Echinocloa crus-galli |
| SETFA | Setaria faberi |

The respective stated components A and B, and if appropriate, C were formulated as a 10% by weight strength emulsion concentrate and, with addition of the amount of solvent system, introduced into the spray liquor used for applying the active compound. In the examples, the solvent used was water.

In the examples below, using the method of S. R. Colby (1967) "Calculating synergistic and antagonistic responses of herbicide combinations", Weeds 278, p. 22ff., the value E, which is expected if the activity of the individual active compounds is only additive, was calculated.

$$E = X + Y - (X \cdot Y/100)$$

where

X = percent activity using active compound A at an application rate a;
Y = percent activity using active compound B at an application rate b;
E = expected activity (in %) by A + B at application rates a + b.

If the value found experimentally is higher than the value E calculated according to Colby, a synergistic effect is present.

Example 1. Compound 1 applied by pre-emergence method at an application rate of 0.125 kg/ha, showed very good herbicidal activity against ABUTH, ALOMY, AMARE, ECHCG and SETFA.
Example 2. Compound 2 applied by pre-emergence method at an application rate of 0.250 kg/ha, showed very good herbicidal activity against AMARE, ABUTH and ALOMY.
Example 3. Compound 7 applied by pre-emergence method at an application rate of 0.250 kg/ha, showed very good herbicidal activity against AMARE, ALOMY and SEFTA.
Example 4. Compound 8 applied by pre-emergence method at an application rate of 0.250 kg/ha, showed very good herbicidal activity against AMARE, ECHCG and SEFTA.
Example 5. Compound 3 applied by pre-emergence method at an application rate of 0.250 kg/ha, showed very good herbicidal activity against AMARE, ECHCG and SEFTA.
Example 6. Compound 4 applied by pre-emergence method at an application rate of 0.250 kg/ha, showed very good herbicidal activity against ABUTH, AMARE and ALOMY.
Example 7. Compound 1 applied by pre-emergence method at an application rate of 0.250 kg/ha, showed very good herbicidal activity against ABUTH, AMARE and ALOMY.
Example 8. Compound 10 applied by pre-emergence method at an application rate of 0.250 kg/ha, showed very good herbicidal activity against ABUTH, AMARE and ALOMY.
Example 9. Compound 5 applied by pre-emergence method at an application rate of 0.250 kg/ha, showed very good herbicidal activity against AMARE.
Example 10. Compound 11 applied by pre-emergence method at an application rate of 0.250 kg/ha, showed very good herbicidal activity against ABUTH, AMARE and ALOMY.
Example 11. Compound 6 applied by pre-emergence method at an application rate of 0.250 kg/ha, showed very good herbicidal activity against ABUTH, AMARE and ALOMY.
Example 12. Compound 9 applied by pre-emergence method at an application rate of 0.250 kg/ha, showed very good herbicidal activity against AMARE.

## Claims

**1.** A diaminotriazine compound of formula (I)

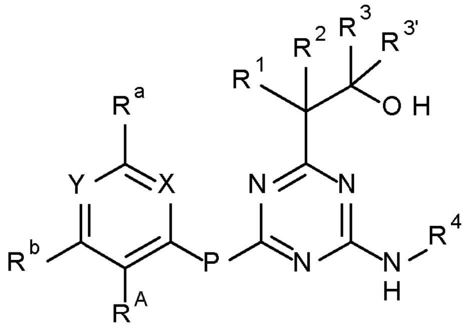

(I)

wherein

P is $NR^5$ or O;
X is $CR^c$ or N;
Y is $CR^d$ or N;
$R^A$ is halogen or CN;
$R^a$, $R^b$, $R^c$ and $R^d$ independently of one another are selected from hydrogen, halogen, OH, CN, amino, $NO_2$, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl, $C_1$-$C_6$-alkylthio, ($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino, ($C_1$-$C_6$-alkyl)-carbonyl, ($C_1$-$C_6$-alkoxy)-carbonyl, ($C_1$-$C_6$-alkyl)-carbonyloxy, $C_3$-$C_6$-cycloalkyl, C3-C6-cycloalkoxy, ($C_3$-$C_6$-cycloalkyl)-$C_1$-$C_4$-alkyl, ($C_3$-$C_6$-cycloalkyl)-$C_1$-$C_4$-alkoxy,
where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups,
or
$R^a$, $R^b$ and $R^d$ are as defined above and $R^c$ is -Q-Ar
Q is a chemical bond, O, $S(O)_m$, $CR^{q1}R^{q2}$, $NR^{q3}$, C(O), C(O)O, $CR^{q1}R^{q2}$-O, $S(O)_mNR^{q3}$ or $C(O)NR^{q3}$, wherein

m is 0, 1 or 2;
$R^{q1}$, $R^{q2}$ are independently of one another are selected from hydrogen, halogen or $C_1$-$C_4$-alkyl;
$R^{q3}$ is H, CN, $C_1$-$C_6$-alkyl, ($C_1$-$C_6$-alkoxy)-$C_1$-$C_6$-alkyl, ($C_1$-$C_6$-alkyl)-carbonyl, ($C_1$-$C_6$-alkoxy)carbonyl, ($C_1$-$C_6$-alkyl)sulfonyl, where the aliphatic parts of the 5 aforementioned radicals are unsubstituted, partly or completely halogenated;

Ar is phenyl, which is unsubstituted or carries 1, 2, 3, 4 or 5 radicals $R^e$ that are identical or different and independently of one another are selected from halogen, OH, CN, amino, $NO_2$, $C_1$-$C_6$-alkyl, $C_2$-$C_6$-alkenyl, $C_2$-$C_6$-alkynyl, $C_1$-C6-alkoxy, C2-C6-alkenyloxy, C2-C6-alkynyloxy, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl, $C_1$-$C_6$-alkylthio, ($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino, ($C_1$-$C_6$-alkyl)-carbonyl, ($C_1$-$C_6$-alkoxy)-carbonyl, ($C_1$-$C_6$-alkyl)-carbonyloxy, $C_3$-$C_6$-cycloalkyl, $C_3$-$C_6$-cycloalkoxy, ($C_3$-$C_6$-cycloalkyl)-$C_1$-$C_4$-alkyl, ($C_3$-$C_6$-cycloalkyl)-$C_1$-$C_4$-alkoxy,
where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups;
or
$R^b$ and $R^d$ are as defined above and $R^a$ and $R^c$ together with the carbon atoms to which they are attached form a saturated or unsaturated, 5- or 6-membered carbocycle or saturated or unsaturated, 5- or 6-membered heterocycle having 1 or 2 heteroatoms or heteroatom moieties, selected from O, S, S(O), $S(O)_2$, N or $NR^z$ as ring members,
where the carbocycle or the heterocycle are unsubstituted or carry 1, 2, 3 or 4 radicals $R^f$ that are identical or different and independently of one another are selected from halogen, OH, CN, amino, $NO_2$, $C_1$-$C_6$-alkyl, $C_2$-$C_6$-alkenyl, $C_2$-$C_6$-alkynyl, $C_1$-$C_6$-alkoxy, $C_2$-$C_6$-alkenyloxy, $C_2$-$C_6$-alkynyloxy, ($C_1$-$C_6$-alkoxy)-$C_1$-$C_6$-alkyl, ($C_1$-$C_6$-alkoxy)-$C_1$-$C_6$-alkoxy, ($C_1$-$C_6$-alkoxy)-$C_2$-$C_6$-alkenyl, ($C_1$-$C_6$-alkoxy)-$C_2$-$C_6$-alkynyl, $C_1$-$C_6$-alkylthio, ($C_1$-$C_6$-alkyl)sulfinyl, ($C_1$-$C_6$-alkyl)sulfonyl, ($C_1$-$C_6$-alkyl)amino, di($C_1$-$C_6$-alkyl)amino, ($C_1$-$C_6$-alkyl)-carbonyl, ($C_1$-$C_6$-alkoxy)-carbonyl, ($C_1$-$C_6$-alkyl)-carbonyloxy, $C_3$-$C_6$-cycloalkyl, C3-C6-cycloalkoxy, ($C_3$-$C_6$-cycloalkyl)-$C_1$-$C_4$-alkyl, ($C_3$-$C_6$-cycloalkyl)-$C_1$-$C_4$-alkoxy,
where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups;
$R^z$ is selected from H, OH, $S(O)_2NH_2$, CN, $C_1$-$C_6$-alkyl, $C_2$-$C_6$-alkenyl, $C_2$-$C_6$-alkynyl, ($C_3$-$C_6$-cycloalkyl)-$C_1$-$C_4$-alkyl, $C_1$-$C_6$-alkoxy, ($C_1$-$C_6$-alkoxy)-$C_1$-$C_6$-alkyl, ($C_1$-$C_6$-alkyl)-carbonyl, ($C_1$-$C_6$-alkoxy)carbonyl, ($C_1$-$C_6$-alkyl)sulfonyl, ($C_1$-$C_6$-alkylamino)carbonyl, di($C_1$-$C_6$-alkyl)aminocarbonyl, ($C_1$-$C_6$-alkylamino)sulfonyl, di($C_1$-$C_6$-alkyl)aminosulfonyl and ($C_1$-$C_6$-alkoxy)sulfonyl,
where the aliphatic and cycloaliphatic parts of the 14 aforementioned radicals are unsubstituted, partly or completely halogenated,
$R^1$ is selected from H, halogen, OH, CN, $C_1$-$C_6$-alkyl, ($C_1$-$C_6$-alkoxy)-$C_1$-$C_6$-alkyl, $C_3$-$C_6$-cycloalkyl, ($C_3$-$C_6$-cycloalkyl)-$C_1$-$C_4$-alkyl, $C_1$-$C_6$-alkoxy, $C_2$-$C_6$-alkenyloxy, $C_2$-$C_6$-alkynyloxy, $C_3$-$C_6$-cycloalkoxy, ($C_3$-$C_6$-cycloalkyl)-$C_1$-$C_4$-alkoxy,

where the aliphatic and cycloaliphatic parts of the 9 aforementioned radicals are unsubstituted, partly or completely halogenated;
$R^2$ is selected from H, halogen, CN, $C_1$-$C_6$-alkyl, $C_1$-$C_6$-haloalkyl, $C_1$-$C_6$-alkoxy and $C_1$-$C_6$-haloalkoxy;
$R^3$ and $R^{3'}$ independently of one another are selected from H, $C_1$-$C_6$-alkyl, $C_3$-$C_6$-cycloalkyl, ($C_3$-$C_6$-cycloalkyl)-$C_1$-$C_4$-alkyl,
where the aliphatic and cycloaliphatic parts of the 3 aforementioned radicals are unsubstituted, partly or completely halogenated; or
$R^3$ and $R^{3'}$ together with the carbon atom to which they are attached form a moiety selected from $C_3$-$C_6$-cycloalkyl or $C_3$-$C_6$-cycloalkenyl where the carbocycle is unsubstituted, partly or completely halogenated or carry from 1 to 6 $C_1$-$C_6$-alkyl groups;
or
$R^1$ and $R^2$ together with the carbon atom to which they are attached form a moiety selected from carbonyl, $C_3$-$C_6$-cycloalkyl, $C_3$-$C_6$-cycloalkenyl, three- to six-membered saturated or partially unsaturated heterocyclyl, where the carbocycle and the heterocycle are unsubstituted, partly or completely halogenated or carry from 1 to 6 $C_1$-$C_6$-alkyl groups;
or
$R^2$ and $R^3$ together with the carbon atom to which they are attached form a moiety selected from $C_3$-$C_6$-cycloalkyl, $C_3$-$C_6$-cycloalkenyl, three- to six-membered saturated or partially unsaturated heterocyclyl, where the carbocycle and the heterocycle are unsubstituted, partly or completely halogenated or carry from 1 to 6 $C_1$-$C_6$-alkyl groups;
$R^4$ and $R^5$ independently of one another are selected from H, OH, $S(O)_2NH_2$, CN, $C_1$-$C_6$-alkyl, $C_2$-$C_6$-alkenyl, $C_2$-$C_6$-alkynyl, ($C_3$-$C_6$-cycloalkyl)-$C_1$-$C_4$-alkyl, ($C_3$-$C_6$-cycloalkyl)-carbonyl, $C_1$-$C_6$-alkoxy, ($C_1$-$C_6$-alkoxy)-$C_1$-$C_6$-alkyl, ($C_1$-$C_6$-alkyl)carbonyl, ($C_1$-$C_6$-alkoxy)carbonyl, ($C_1$-$C_6$-alkyl)sulfonyl, ($C_1$-$C_6$-alkylamino)carbonyl, di($C_1$-$C_6$-alkyl)aminocarbonyl, ($C_1$-$C_6$-alkylamino)sulfonyl, di($C_1$-$C_6$-alkyl)aminosulfonyl and ($C_1$-$C_6$-alkoxy)sulfonyl,

where the aliphatic and cycloaliphatic parts of the 15 aforementioned radicals are unsubstituted, partly or completely halogenated,
phenyl, phenyl-$C_1$-$C_6$-alkyl, phenylsulfonyl, phenylaminosulfonyl, phenylaminocarbonyl, phenyl($C_1$-$C_6$-alkyl)aminocarbonyl, phenylcarbonyl and phenoxycarbonyl,
where phenyl in the last 8 mentioned radicals is unsubstituted or substituted by 1, 2, 3, 4 or 5 identical or different substituents selected from halogen, CN, $NO_2$, $C_1$-$C_6$-alkyl, $C_1$-$C_6$-haloalkyl, $C_1$-$C_6$-alkoxy and $C_1$-$C_6$-haloalkoxy

including their agriculturally acceptable salts.

**2.** The compound of claim 1, wherein P is $NR^5$.

**3.** The compound of any of claim 1, wherein P is O.

**4.** The compound of any of claims 1 to 3, wherein $R^A$ is halogen.

**5.** The compound of claim 4, wherein $R^A$ is F.

**6.** The compound of any of claims 1 to 5, wherein X is $CR^c$ and Y is $CR^d$.

**7.** The compound of any of claims 1 to 5, wherein X or Y are N.

**8.** The compound of any of claims 1 to 7, wherein $R^3$ and $R^{3'}$ are H.

**9.** The compound of any of claims 1 to 8, wherein $R^4$ is H.

**10.** The compound of any of claims 1 to 9, wherein $R^5$ is H.

**11.** The compound of any of claims 1 to 10, wherein Q is selected from a chemical bond, O, $CH_2$, S, S(O) and $S(O)_2$.

**12.** The compound of any of claims 1 to 11, wherein

$R^1$ is selected from halogen, $C_1$-$C_6$-alkyl, $C_1$-$C_6$-haloalkyl, $C_2$-$C_6$-alkenyl, $C_3$-$C_6$-alkynyl, $C_3$-$C_6$-cycloalkyl,

$C_3$-$C_6$-cycloalkenyl, and $C_1$-$C_6$-alkoxy-$C_1$-$C_6$-alkyl;
$R^2$ is selected from hydrogen, halogen, $C_1$-$C_4$-alkyl and $C_1$-$C_4$-alkoxy;
$R^3$and $R^{3'}$ are selected from hydrogen and $C_1$-$C_4$-alkyl;
or
$R^1$ and $R^2$ together with the carbon atom to which they are attached form a moiety selected from carbonyl, $C_3$-$C_6$-cycloalkyl, $C_3$-$C_6$-cycloalkenyl and three- to six-membered saturated or partially unsaturated heterocyclyl.
or
$R^2$ and $R^3$ together with the carbon atom to which they are attached form a moiety selected from $C_3$-$C_6$-cycloalkyl, $C_3$-$C_6$-cycloalkenyl and three- to six-membered saturated or partially unsaturated heterocyclyl.

**13.** An agrochemical composition comprising a herbicidally active amount of at least one compound as claimed in any of claims 1 to 12 and at least one inert liquid and/or solid carrier and, if appropriate, at least one surface-active substance.

**14.** A method for controlling unwanted vegetation which comprises allowing a herbicidally active amount of a herbicidal composition according to any one of claims 1 to 12 or an agrochemical composition according to claim 13 to act on plants, their environment or on seed.

**15.** Use of the compound as claimed in any of claims 1 to 12 as a herbicide or for the desiccation/defoliation of plants.

**Patentansprüche**

**1.** Diaminotriazinverbindung der Formel (I)

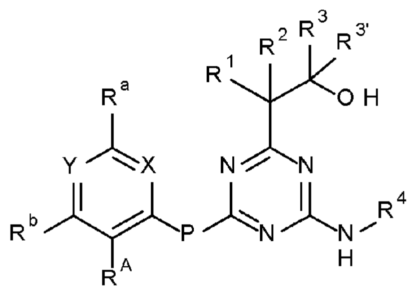

(I)

wobei

P für $NR^5$ oder O steht;
X für $CR^c$ oder N steht;
Y für $CR^d$ oder N steht;
$R^A$ für Halogen oder CN steht;
$R^a$, $R^b$, $R^c$ und $R^d$ unabhängig voneinander aus Wasserstoff, Halogen, OH, CN, Amino, $NO_2$, $C_1$-$C_6$-Alkyl, $C_2$-$C_6$-Alkenyl, $C_2$-$C_6$-Alkinyl, $C_1$-$C_6$-Alkoxy, $C_2$-$C_6$-Alkenyloxy, $C_2$-$C_6$-Alkinyloxy, ($C_1$-$C_6$-Alkoxy)-$C_1$-$C_6$-alkyl, ($C_1$-$C_6$-Alkoxy)-$C_1$-$C_6$-alkoxy, ($C_1$-$C_6$-Alkoxy)-$C_2$-$C_6$-alkenyl, ($C_1$-$C_6$-Alkoxy) -$C_2$-$C_6$-alkinyl, $C_1$-$C_6$-Alkylthio, ($C_1$-$C_6$-Alkyl)sulfinyl, ($C_1$-$C_6$-Alkyl)sulfonyl, ($C_1$-$C_6$-Alkyl) amino, Di($C_1$-$C_6$-alkyl)amino, ($C_1$-$C_6$-Alkyl)-carbonyl, ($C_1$-$C_6$-Alkoxy)carbonyl, ($C_1$-$C_6$-Alkyl)-carbonyloxy, $C_3$-$C_6$-Cycloalkyl, $C_3$-$C_6$-Cycloalkoxy, ($C_3$-$C_6$-Cycloalkyl)-$C_1$-$C_4$-alkyl und ($C_3$-$C_6$-Cycloalkyl)-$C_1$-$C_4$-alkoxy ausgewählt sind,
wobei die aliphatischen und cycloaliphatischen Teile der 22 oben genannten Reste unsubstituiert oder teilweise oder vollständig halogeniert sind und wobei die cycloaliphatischen Teile der letzten 4 genannten Reste 1, 2, 3, 4, 5 oder 6 Methylgruppen tragen können,
oder
$R^a$, $R^b$ und $R^d$ wie oben definiert sind und $R^c$ für -Q-Ar steht,
Q für eine chemische Bindung, O, $S(O)_m$, $CR^{q1}R^{q2}$, $NR^{q3}$, C(O), C(O)O, $CR^{q1}R^{q2}$-O, $S(O)_mNR^{q3}$ oder $C(O)NR^{q3}$ steht,
wobei

m für 0, 1 oder 2 steht;

$R^{q1}$, $R^{q2}$ unabhängig voneinander aus Wasserstoff, Halogen oder $C_1$-$C_4$-Alkyl ausgewählt sind;
$R^{q3}$ für H, CN, $C_1$-$C_6$-Alkyl, ($C_1$-$C_6$-Alkoxy)-$C_1$-$C_6$-alkyl, ($C_1$-$C_6$-Alkyl)carbonyl, ($C_1$-$C_6$-Alkoxy)-carbonyl oder ($C_1$-$C_6$-Alkyl)sulfonyl steht, wobei die aliphatischen Teile der 5 oben genannten Reste unsubstituiert oder teilweise oder vollständig halogeniert sind;

Ar für Phenyl steht, das unsubstituiert ist oder 1, 2, 3, 4 oder 5 Reste $R^e$ trägt, die gleich oder verschieden sind und unabhängig voneinander aus Halogen, OH, CN, Amino, $NO_2$, $C_1$-$C_6$-Alkyl, $C_2$-$C_6$-Alkenyl, $C_2$-$C_6$-Alkinyl, $C_1$-$C_6$-Alkoxy, $C_2$-$C_6$-Alkenyloxy, $C_2$-$C_6$-Alkinyloxy, ($C_1$-$C_6$-Alkoxy)-$C_1$-$C_6$-alkyl, ($C_1$-$C_6$-Alkoxy)-$C_1$-$C_6$-alkoxy, ($C_1$-$C_6$-Alkoxy)-$C_2$-$C_6$-alkenyl, ($C_1$-$C_6$-Alkoxy) -$C_2$-$C_6$-alkinyl, $C_1$-$C_6$-Alkylthio, ($C_1$-$C_6$-Alkyl)sulfinyl, ($C_1$-$C_6$-Alkyl)sulfonyl, ($C_1$-$C_6$-Alkyl) amino, Di($C_1$-$C_6$-alkyl)amino, ($C_1$-$C_6$-Alkyl)-carbonyl, ($C_1$-$C_6$-Alkoxy)carbonyl, ($C_1$-$C_6$-Alkyl)-carbonyloxy, $C_3$-$C_6$-Cycloalkyl, $C_3$-$C_6$-Cycloalkoxy, ($C_3$-$C_6$-Cycloalkyl)-$C_1$-$C_4$-alkyl und ($C_3$-$C_6$-Cycloalkyl)-$C_1$-$C_4$-alkoxy ausgewählt sind,
wobei die aliphatischen und cycloaliphatischen Teile der 22 oben genannten Reste unsubstituiert oder teilweise oder vollständig halogeniert sind und wobei die cycloaliphatischen Teile der letzten 4 genannten Reste 1, 2, 3, 4, 5 oder 6 Methylgruppen tragen können,
oder
$R^b$ und $R^d$ wie oben definiert sind und $R^a$ und $R^c$ zusammen mit den Kohlenstoffatomen, an die sie gebunden sind, einen gesättigten oder ungesättigten, 5- oder 6-gliedrigen Carbocyclus oder gesättigten oder ungesättigten, 5- oder 6-gliedrigen Heterocyclus mit 1 oder 2 Heteroatomen bzw. Heteroatomgruppierungen, die aus O, S, S(O), $S(O)_2$, N oder $NR^z$ ausgewählt sind, als Ringglieder,
wobei der Carbocyclus oder der Heterocyclus unsubstituiert sind oder 1, 2, 3 oder 4 Reste $R^f$ tragen, die gleich oder verschieden sind und unabhängig voneinander aus Halogen, OH, CN, Amino, $NO_2$, $C_1$-$C_6$-Alkyl, $C_2$-$C_6$-Alkenyl, $C_2$-$C_6$-Alkinyl, $C_1$-$C_6$-Alkoxy, $C_2$-$C_6$-Alkenyloxy, $C_2$-$C_6$-Alkinyloxy, ($C_1$-$C_6$-Alkoxy)-$C_1$-$C_6$-alkyl, ($C_1$-$C_6$-Alkoxy)-$C_1$-$C_6$-alkoxy, ($C_1$-$C_6$-Alkoxy)-$C_2$-$C_6$-alkenyl, ($C_1$-$C_6$-Alkoxy)-$C_2$-$C_6$-alkinyl, $C_1$-$C_6$-Alkylthio, ($C_1$-$C_6$-Alkyl)sulfinyl, ($C_1$-$C_6$-Alkyl)sulfonyl, ($C_1$-$C_6$-Alkyl)amino, Di($C_1$-$C_6$-alkyl)amino, ($C_1$-$C_6$-Alkyl)carbonyl, ($C_1$-$C_6$-Alkoxy) carbonyl, ($C_1$-$C_6$-Alkyl)carbonyloxy, $C_3$-$C_6$-Cycloalkyl, $C_3$-$C_6$-Cycloalkoxy, ($C_3$-$C_6$-Cycloalkyl)-$C_1$-$C_4$-alkyl und ($C_3$-$C_6$-Cycloalkyl)-$C_1$-$C_4$-alkoxy ausgewählt sind,
wobei die aliphatischen und cycloaliphatischen Teile der 22 oben genannten Reste unsubstituiert oder teilweise oder vollständig halogeniert sind und wobei die cycloaliphatischen Teile der letzten 4 genannten Reste 1, 2, 3, 4, 5 oder 6 Methylgruppen tragen können;
$R^z$ aus H, OH, $S(O)_2NH_2$, CN, $C_1$-$C_6$-Alkyl, $C_2$-$C_6$-Alkenyl, $C_2$-$C_6$-Alkinyl, ($C_3$-$C_6$-Cycloalkyl)-$C_1$-$C_4$-alkyl, $C_1$-$C_6$-Alkoxy, ($C_1$-$C_6$-Alkoxy)-$C_1$-$C_6$-alkyl, ($C_1$-$C_6$-Alkyl)carbonyl, ($C_1$-$C_6$-Alkoxy)carbonyl, ($C_1$-$C_6$-Alkyl)sulfonyl, ($C_1$-$C_6$-Alkylamino)-carbonyl, Di($C_1$-$C_6$-alkylamino)carbonyl, ($C_1$-$C_6$-Alkylamino)sulfonyl, Di($C_1$-$C_6$-alkyl)amino-sulfonyl und ($C_1$-$C_6$-Alkoxy)sulfonyl ausgewählt ist,
wobei die aliphatischen und cycloaliphatischen Teile der 14 oben genannten Reste unsubstituiert oder teilweise oder vollständig halogeniert sind,
$R^1$ aus H, Halogen, OH, CN, $C_1$-$C_6$-Alkyl, ($C_1$-$C_6$-Alkoxy)-$C_1$-$C_6$-alkyl, $C_3$-$C_6$-Cycloalkyl, ($C_3$-$C_6$-Cycloalkyl)-$C_1$-$C_4$-alkyl, $C_1$-$C_6$-Alkoxy, $C_2$-$C_6$-Alkenyloxy, $C_2$-$C_6$-Alkinyloxy, $C_3$-$C_6$-Cycloalkoxy und ($C_3$-$C_6$-Cycloalkyl) -$C_1$-$C_4$-alkoxy ausgewählt ist,
wobei die aliphatischen und cycloaliphatischen Teile der 9 oben genannten Reste unsubstituiert oder teilweise oder vollständig halogeniert sind;
$R^2$ aus H, Halogen, CN, $C_1$-$C_6$-Alkyl, $C_1$-$C_6$-Halogenalkyl, $C_1$-$C_6$-Alkoxy und $C_1$-$C_6$-Halogen-alkoxy ausgewählt ist;
$R^3$ und $R^{3'}$ unabhängig voneinander aus H, $C_1$-$C_6$-Alkyl, $C_3$-$C_6$-Cycloalkyl und ($C_3$-$C_6$-Cycloalkyl)-$C_1$-$C_4$-alkyl ausgewählt sind,
wobei die aliphatischen und cycloaliphatischen Teile der 3 oben genannten Reste unsubstituiert oder teilweise oder vollständig halogeniert sind; oder
$R^3$ und $R^{3'}$ zusammen mit dem Kohlenstoffatom, an das sie gebunden sind, eine aus $C_3$-$C_6$-Cycloalkyl oder $C_3$-$C_6$-Cycloalkenyl ausgewählte Gruppierung bilden, wobei der Carbocyclus unsubstituiert oder teilweise oder vollständig halogeniert ist und 1 bis 6 $C_1$-$C_6$-Alkylgruppen tragen kann;
oder
$R^1$ und $R^2$ zusammen mit dem Kohlenstoffatom, an das sie gebunden sind, eine aus Carbonyl, $C_3$-$C_6$-Cycloalkyl, $C_3$-$C_6$-Cycloalkenyl und drei- bis sechsgliedrigem gesättigtem oder teilweise ungesättigtem Heterocyclyl ausgewählte Gruppierung bilden, wobei der Carbocyclus und der Heterocyclus unsubstituiert oder teilweise oder vollständig halogeniert sind und 1 bis 6 $C_1$-$C_6$-Alkylgruppen tragen können;
oder
$R^2$ und $R^3$ zusammen mit dem Kohlenstoffatom, an das sie gebunden sind, eine aus $C_3$-$C_6$-Cycloalkyl, $C_3$-$C_6$-Cycloalkenyl und drei- bis sechsgliedrigem gesättigtem oder teilweise ungesättigtem Heterocyclyl ausgewählte

Gruppierung bilden, wobei der Carbocyclus und der Heterocyclus unsubstituiert oder teilweise oder vollständig halogeniert sind und 1 bis 6 $C_1$-$C_6$-Alkylgruppen tragen können;
$R^4$ und $R^5$ unabhängig voneinander aus H, OH, $S(O)_2NH_2$, CN, $C_1$-$C_6$-Alkyl, $C_2$-$C_6$-Alkenyl, $C_2$-$C_6$-Alkinyl, ($C_3$-$C_6$-Cycloalkyl) -$C_1$-$C_4$-alkyl, ($C_3$-$C_6$-Cycloalkyl) carbonyl, $C_1$-$C_6$-Alkoxy, ($C_1$-$C_6$-Alkoxy) -$C_1$-$C_6$-alkyl, ($C_1$-$C_6$-Alkyl) carbonyl, ($C_1$-$C_6$-Alkoxy) carbonyl, ($C_1$-$C_6$-Alkyl) sulfonyl, ($C_1$-$C_6$-Alkylamino)carbonyl, Di($C_1$-$C_6$-alkylamino)-carbonyl, ($C_1$-$C_6$-Alkylamino) sulfonyl, Di ($C_1$-$C_6$-alkylamino)sulfonyl und ($C_1$-$C_6$-Alkoxy)sulfonyl,

wobei die aliphatischen und cycloaliphatischen Teile der 15 oben genannten Reste unsubstituiert oder teilweise oder vollständig halogeniert sind,
Phenyl, Phenyl-$C_1$-$C_6$-alkyl, Phenylsulfonyl, Phenylaminosulfonyl, Phenylaminocarbonyl, Phenyl ($C_1$-$C_6$-alkyl) aminocarbonyl, Phenylcarbonyl und Phenoxycarbonyl,
wobei Phenyl in den letzten 8 genannten Resten unsubstituiert oder durch 1, 2, 3, 4 oder 5 gleiche oder verschiedene Substituenten, die aus Halogen, CN, $NO_2$, $C_1$-$C_6$-Alkyl, $C_1$-$C_6$-Halogenalkyl, $C_1$-$C_6$-Alkoxy und $C_1$-$C_6$-Halogenalkoxy ausgewählt sind, substituiert ist,
ausgewählt sind;

einschließlich ihrer landwirtschaftlich unbedenklichen Salze.

**2.** Verbindung nach Anspruch 1, wobei P für $NR^5$ steht.

**3.** Verbindung nach einem der Anspruch 1, wobei P für O steht.

**4.** Verbindung nach einem der Ansprüche 1 bis 3, wobei $R^A$ für Halogen steht.

**5.** Verbindung nach Anspruch 4, wobei $R^A$ für F steht.

**6.** Verbindung nach einem der Ansprüche 1 bis 5, wobei X für $CR^c$ steht und Y für $CR^d$ steht.

**7.** Verbindung nach einem der Ansprüche 1 bis 5, wobei X oder Y für N stehen.

**8.** Verbindung nach einem der Ansprüche 1 bis 7, wobei $R^3$ und $R^{3'}$ für H stehen.

**9.** Verbindung nach einem der Ansprüche 1 bis 8, wobei $R^4$ für H steht.

**10.** Verbindung nach einem der Ansprüche 1 bis 9, wobei $R^5$ für H steht.

**11.** Verbindung nach einem der Ansprüche 1 bis 10, wobei Q aus einer chemischen Bindung, O, $CH_2$, S, S(O) und $S(O)_2$ ausgewählt ist.

**12.** Verbindung nach einem der Ansprüche 1 bis 11, wobei

$R^1$ aus Halogen, $C_1$-$C_6$-Alkyl, $C_1$-$C_6$-Halogenalkyl, $C_2$-$C_6$-Alkenyl, $C_3$-$C_6$-Alkinyl, $C_3$-$C_6$-Cycloalkyl, $C_3$-$C_6$-Cycloalkenyl und $C_1$-$C_6$-Alkoxy-$C_1$-$C_6$-alkyl ausgewählt ist;
$R^2$ aus Wasserstoff, Halogen, $C_1$-$C_4$-Alkyl und $C_1$-$C_4$-Alkoxy ausgewählt ist;
$R^3$ und $R^{3'}$ aus Wasserstoff und $C_1$-$C_4$-Alkyl ausgewählt sind;
oder
$R^1$ und $R^2$ zusammen mit dem Kohlenstoffatom, an das sie gebunden sind eine aus Carbonyl, $C_3$-$C_6$-Cycloalkyl, $C_3$-$C_6$-Cycloalkenyl und drei- bis sechsgliedrigem gesättigtem oder teilweise ungesättigtem Heterocyclyl ausgewählte Gruppierung bilden;
oder
$R^2$ und $R^3$ zusammen mit dem Kohlenstoffatom, an das sie gebunden sind eine aus $C_3$-$C_6$-Cycloalkyl, $C_3$-$C_6$-Cycloalkenyl und drei- bis sechsgliedrigem gesättigtem oder teilweise ungesättigtem Heterocyclyl ausgewählte Gruppierung bilden.

**13.** Agrochemische Zusammensetzung, umfassend eine herbizid wirksame Menge mindestens einer Verbindung gemäß einem der Ansprüche 1 bis 12 und mindestens einen inerten flüssigen und/oder festen Träger und gegebenenfalls mindestens eine oberflächenaktive Substanz.

**14.** Verfahren zur Bekämpfung von unerwünschtem Pflanzenwuchs, bei dem man eine herbizid wirksame Menge mindestens einer herbiziden Zusammensetzung gemäß einem der Ansprüche 1 bis 12 oder einer agrochemischen Zusammensetzung gemäß Anspruch 13 auf Pflanzen, deren Lebensraum oder auf Saatgut einwirken lässt.

**15.** Verwendung einer Verbindung gemäß einem der Ansprüche 1 bis 12 als Herbizid oder zur Desikkation/Defoliation von Pflanzen.

**Revendications**

**1.** Composé de type diaminotriazine de formule (I)

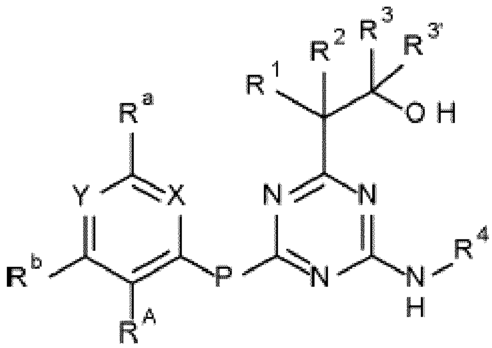

(I)

P étant $NR^5$ ou O ;
X étant $CR^c$ ou N ;
Y étant $CR^d$ ou N ;
$R^A$ étant halogène ou CN ;
$R^a$, $R^b$, $R^c$ et $R^d$ indépendamment les uns des autres étant choisis parmi hydrogène, halogène, OH, CN, amino, $NO_2$, $C_1$-$C_6$-alkyle, $C_2$-$C_6$-alcényle, $C_2$-$C_6$-alcynyle, $C_1$-$C_6$-alcoxy, $C_2$-$C_6$-alcényloxy, $C_2$-$C_6$-alcynyloxy, ($C_1$-$C_6$-alcoxy) -$C_1$-$C_6$-alkyle, ($C_1$-$C_6$-alcoxy) -$C_1$-$C_6$-alcoxy, ($C_1$-$C_6$-alcoxy) -$C_2$-$C_6$-alcényle, ($C_1$-$C_6$-alcoxy) -$C_2$-$C_6$-alcynyle, $C_1$-$C_6$-alkylthio, ($C_1$-$C_6$-alkyl) sulfinyle, ($C_1$-$C_6$-alkyl) sulfonyle, ($C_1$-$C_6$-alkyl) amino, di ($C_1$-$C_6$-alkyl)amino, ($C_1$-$C_6$-alkyl) -carbonyle, ($C_1$-$C_6$-alcoxy)-carbonyle, ($C_1$-$C_6$-alkyl)-carbonyloxy, $C_3$-$C_6$-cycloalkyle, $C_3$-$C_6$-cycloalcoxy, ($C_3$-$C_6$-cycloalkyl) -$C_1$-$C_4$-alkyle, ($C_3$-$C_6$-cycloalkyl) -$C_1$-$C_4$ -alcoxy,
où les parties aliphatiques et cycloaliphatiques des 22 radicaux mentionnés précédemment sont non substituées, partiellement ou complètement halogénées et où les parties cycloaliphatiques des 4 radicaux mentionnés en dernier peuvent porter 1, 2, 3, 4, 5 ou 6 groupes méthyle,
ou
$R^a$, $R^b$ et $R^d$ étant tels que définis ci-dessus et $R^c$ étant -Q-Ar
Q étant une liaison chimique, O, $S(O)_m$, $CR^{q1}R^{q2}$, $NR^{q3}$, C(O), C(O)O, $CR^{q1}R^{q2}$-O, $S(O)_mNR^{q3}$ ou $C(O)NR^{q3}$,

m étant 0, 1 ou 2 ;
$R^{q1}$, $R^{q2}$ étant choisis indépendamment l'un de l'autre parmi hydrogène, halogène et $C_1$-$C_4$-alkyle ;
$R^{q3}$ étant H, CN, $C_1$-$C_6$-alkyle, ($C_1$-$C_6$-alcoxy) - $C_1$-$C_6$-alkyle, ($C_1$-$C_6$-alkyl) -carbonyle, ($C_1$-$C_6$-alcoxy)carbonyle, ($C_1$-$C_6$-alkyl) sulfonyle, où les parties aliphatiques des 5 radicaux mentionnés précédemment sont non substituées, partiellement ou complètement halogénées ;

Ar étant phényle, qui est non substitué ou porte 1, 2, 3, 4 ou 5 radicaux $R^e$ qui sont identiques ou différents et sont choisis indépendamment les uns des autres parmi halogène, OH, CN, amino, $NO_2$, $C_1$-$C_6$-alkyle, $C_2$-$C_6$-alcényle, $C_2$-$C_6$-alcynyle, $C_1$-$C_6$-alcoxy, $C_2$-$C_6$-alcényloxy, $C_2$-$C_6$-alcynyloxy, ($C_1$-$C_6$-alcoxy) -$C_1$-$C_6$-alkyle, ($C_1$-$C_6$-alcoxy) -$C_1$-$C_6$-alcoxy, ($C_1$-$C_6$-alcoxy) -$C_2$-$C_6$-alcényle, ($C_1$-$C_6$-alcoxy) -$C_2$-$C_6$-alcynyle, $C_1$-$C_6$-alkylthio, ($C_1$-$C_6$-alkyl) sulfinyle, ($C_1$-$C_6$-alkyl) sulfonyle, ($C_1$-$C_6$-alkyl) amino, di ($C_1$-$C_6$-alkyl)amino, ($C_1$-$C_6$-alkyl) -carbonyle, ($C_1$-$C_6$-alcoxy)-carbonyle, ($C_1$-$C_6$-alkyl) -carbonyloxy, $C_3$-$C_6$-cycloalkyle, $C_3$-$C_6$-cycloalcoxy, ($C_3$-$C_6$-cycloalkyl) -$C_1$-$C_4$-alkyle, ($C_3$-$C_6$-cycloalkyl) -$C_1$-$C_4$ -alcoxy,
où les parties aliphatiques et cycloaliphatiques des 22 radicaux mentionnés précédemment sont non substituées, partiellement ou complètement halogénées et où les parties cycloaliphatiques des 4 radicaux mentionnés en dernier peuvent porter 1, 2, 3, 4, 5 ou 6 groupes méthyle ;

ou

$R^b$ et $R^d$ étant tels que définis ci-dessus et $R^a$ et $R^c$ formant, conjointement avec les atomes de carbone auxquels ils sont fixés, un carbocycle saturé ou insaturé à 5 ou 6 chaînons ou un hétérocycle saturé ou insaturé à 5 ou 6 chaînons possédant 1 ou 2 hétéroatomes ou groupements d'hétéroatomes, choisis parmi O, S, S(O), $S(O)_2$, N et $NR^z$ en tant qu'éléments de cycle,

où le carbocycle ou l'hétérocycle est non substitué ou porte 1, 2, 3 ou 4 radicaux $R^f$ qui sont identiques ou différents et indépendamment les uns des autres sont choisis parmi halogène, OH, CN, amino, $NO_2$, $C_1$-$C_6$-alkyle, $C_2$-$C_6$-alcényle, $C_2$-$C_6$-alcynyle, $C_1$-$C_6$-alcoxy, $C_2$-$C_6$-alcényloxy, $C_2$-$C_6$-alcynyloxy, ($C_1$-$C_6$-alcoxy) -$C_1$-$C_6$-alkyle,($C_1$-$C_6$-alcoxy) -$C_1$-$C_6$-alcoxy, ($C_1$-$C_6$-alcoxy) -$C_2$-$C_6$-alcényle, ($C_1$-$C_6$-alcoxy) -$C_2$-$C_6$-alcynyle, $C_1$-$C_6$-alkylthio, ($C_1$-$C_6$-alkyl) sulfinyle, ($C_1$-$C_6$-alkyl) sulfonyle, ($C_1$-$C_6$-alkyl) amino, di ($C_1$-$C_6$-alkyl)amino, ($C_1$-$C_6$-alkyl) -carbonyle, ($C_1$-$C_6$-alcoxy)-carbonyle, ($C_1$-$C_6$-alkyl) -carbonyloxy, $C_3$-$C_6$-cycloalkyle, $C_3$-$C_6$-cycloalcoxy, ($C_3$-$C_6$-cycloalkyl) -$C_1$-$C_4$-alkyle, ($C_3$-$C_6$-cycloalkyl) - $C_1$-$C_4$-alcoxy,

où les parties aliphatiques et cycloaliphatiques des 22 radicaux mentionnés précédemment sont non substituées, partiellement ou complètement halogénées et où les parties cycloaliphatiques des 4 radicaux mentionnés en dernier peuvent porter 1, 2, 3, 4, 5 ou 6 groupes méthyle ;

$R^z$ étant choisi parmi H, OH, $S(O)_2NH_2$, CN, $C_1$-$C_6$-alkyle, $C_2$-$C_6$-alcényle, $C_2$-$C_6$-alcynyle, ($C_3$-$C_6$-cycloalkyl) -$C_1$-$C_4$-alkyle, $C_1$-$C_6$-alcoxy, ($C_1$-$C_6$-alcoxy) -$C_1$-$C_6$-alkyle,($C_1$-$C_6$-alkyl) -carbonyle, ($C_1$-$C_6$-alcoxy) carbonyle, ($C_1$-$C_6$-alkyl) sulfonyle, ($C_1$-$C_6$-alkylamino) carbonyle, di ($C_1$-$C_6$-alkyl)aminocarbonyle, ($C_1$-$C_6$-alkylamino)sulfonyle, di ($C_1$-$C_6$-alkyl)aminosulfonyle et ($C_1$-$C_6$-alcoxy) sulfonyle,

où les parties aliphatiques et cycloaliphatiques des 14 radicaux mentionnés précédemment sont non substituées, partiellement ou complètement halogénées,

$R^1$ étant choisi parmi H, halogène, OH, CN, $C_1$-$C_6$-alkyle, ($C_1$-$C_6$-alcoxy) -$C_1$-$C_6$-alkyle, $C_3$-$C_6$-cycloalkyle, ($C_3$-$C_6$-cycloalkyl) -$C_1$-$C_4$-alkyle, $C_1$-$C_6$-alcoxy, $C_2$-$C_6$-alcényloxy, $C_2$-$C_6$-alcynyloxy, $C_3$-$C_6$-cycloalcoxy, ($C_3$-$C_6$-cycloalkyl) -$C_1$-$C_4$-alcoxy,

où les parties aliphatiques et cycloaliphatiques des 9 radicaux mentionnés précédemment sont non substituées, partiellement ou complètement halogénées ;

$R^2$ étant choisi parmi H, halogène, CN, $C_1$-$C_6$-alkyle, $C_1$-$C_6$-halogénoalkyle, $C_1$-$C_6$-alcoxy et $C_1$-$C_6$-halogénoalcoxy ;

$R^3$ et $R^{3'}$ indépendamment l'un de l'autre étant choisis parmi H, $C_1$-$C_6$-alkyle, $C_3$-$C_6$-cycloalkyle, ($C_3$-$C_6$-cycloalkyl) -$C_1$-$C_4$-alkyle,

où les parties aliphatiques et cycloaliphatiques des 3 radicaux mentionnés précédemment sont non substituées, partiellement ou complètement halogénées ; ou

$R^3$ et $R^{3'}$, conjointement avec l'atome de carbone auquel ils sont fixés, formant un groupement choisi parmi $C_3$-$C_6$-cycloalkyle et $C_3$-$C_6$-cycloalcényle où le carbocycle est non substitué, partiellement ou complètement halogéné ou porte de 1 à 6 groupes $C_1$-$C_6$-alkyle ;

ou

$R^1$ et $R^2$, conjointement avec l'atome de carbone auquel ils sont fixés, formant un groupement choisi parmi carbonyle, $C_3$-$C_6$-cycloalkyle, $C_3$-$C_6$-cycloalcényle, hétérocyclyle saturé ou partiellement insaturé à trois à six chaînons, où le carbocycle et l'hétérocycle sont non substitués, partiellement ou complètement halogénés ou portent de 1 à 6 groupes $C_1$-$C_6$-alkyle ;

ou

$R^2$ et $R^3$, conjointement avec l'atome de carbone auquel ils sont fixés, formant un groupement choisi parmi $C_3$-$C_6$-cycloalkyle, $C_3$-$C_6$-cycloalcényle, hétérocyclyle saturé ou partiellement insaturé à trois à six chaînons, où le carbocycle et l'hétérocycle sont non substitués, partiellement ou complètement halogénés ou portent de 1 à 6 groupes $C_1$-$C_6$-alkyle ;

$R^4$ et $R^5$ indépendamment l'un de l'autre étant choisis parmi H, OH, $S(O)_2NH_2$, CN, $C_1$-$C_6$-alkyle, $C_2$-$C_6$-alcényle, $C_2$-$C_6$-alcynyle, ($C_3$-$C_6$-cycloalkyl) -$C_1$-$C_4$-alkyle, ($C_3$-$C_6$-cycloalkyl) - carbonyle, $C_1$-$C_6$-alcoxy, ($C_1$-$C_6$-alcoxy) -$C_1$-$C_6$-alkyle, ($C_1$-$C_6$-alkyl) carbonyle, ($C_1$-$C_6$-alcoxy) carbonyle, ($C_1$-$C_6$-alkyl) sulfonyle, ($C_1$-$C_6$-alkylamino)carbonyle, di ($C_1$-$C_6$-alkyl)aminocarbonyle, ($C_1$-$C_6$-alkylamino)sulfonyle, di ($C_1$-$C_6$-alkyl)aminosulfonyle et ($C_1$-$C_6$-alcoxy) sulfonyle,

où les parties aliphatiques et cycloaliphatiques des 15 radicaux mentionnés précédemment sont non substituées, partiellement ou complètement halogénées,

phényle, phényl-$C_1$-$C_6$-alkyle, phénylsulfonyle, phénylaminosulfonyle, phénylaminocarbonyle, phényl ($C_1$-$C_6$-alkyl) aminocarbonyle, phénylcarbonyle et phénoxycarbonyle,

où phényle dans les 8 radicaux mentionnés en dernier est non substitué ou substitué par 1, 2, 3, 4 ou 5 substituants identiques ou différents choisis parmi halogène, CN, $NO_2$, $C_1$-$C_6$-alkyle, $C_1$-$C_6$-halogénoalkyle, $C_1$-$C_6$-alcoxy et $C_1$-$C_6$-halogénoalcoxy

y compris leurs sels acceptables sur le plan agricole.

**2.** Composé selon la revendication 1, P étant $NR^5$.

**3.** Composé selon l'une quelconque de la revendication 1, P étant O.

**4.** Composé selon l'une quelconque des revendications 1 à 3, $R^A$ étant halogène.

**5.** Composé selon la revendication 4, $R^A$ étant F.

**6.** Composé selon l'une quelconque des revendications 1 à 5, X étant $CR^c$ et Y étant $CR^d$.

**7.** Composé selon l'une quelconque des revendications 1 à 5, X ou Y étant N.

**8.** Composé selon l'une quelconque des revendications 1 à 7, $R^3$ et $R^{3'}$ étant H.

**9.** Composé selon l'une quelconque des revendications 1 à 8, $R^4$ étant H.

**10.** Composé selon l'une quelconque des revendications 1 à 9, $R^5$ étant H.

**11.** Composé selon l'une quelconque des revendications 1 à 10, Q étant choisi parmi une liaison chimique, O, $CH_2$, S, S(O) et S $(O)_2$.

**12.** Composé selon l'une quelconque des revendications 1 à 11,

$R^1$ étant choisi parmi halogène, $C_1$-$C_6$-alkyle, $C_1$-$C_6$-halogénoalkyle, $C_2$-$C_6$-alcényle, $C_3$-$C_6$-alcynyle, $C_3$-$C_6$-cycloalkyle, $C_3$-$C_6$-cycloalcényle, et $C_1$-$C_6$-alcoxy-$C_1$-$C_6$-alkyle ;
$R^2$ étant choisi parmi hydrogène, halogène, $C_1$-$C_4$-alkyle et $C_1$-$C_4$-alcoxy ;
$R^3$ et $R^{3'}$ étant choisis parmi hydrogène et $C_1$-$C_4$-alkyle ;
ou
$R^1$ et $R^2$, conjointement avec l'atome de carbone auquel ils sont fixés, formant un groupement choisi parmi carbonyle, $C_3$-$C_6$-cycloalkyle, $C_3$-$C_6$-cycloalcényle et hétérocyclyle saturé ou partiellement insaturé à trois à six chaînons,
ou
$R^2$ et $R^3$, conjointement avec l'atome de carbone auquel ils sont fixés, formant un groupement choisi parmi $C_3$-$C_6$-cycloalkyle, $C_3$-$C_6$-cycloalcényle et hétérocyclyle saturé ou partiellement insaturé à trois à six chaînons.

**13.** Composition agrochimique comprenant une quantité active sur le plan herbicide d'au moins un composé tel que revendiqué dans l'une quelconque des revendications 1 à 12 et au moins un support inerte liquide et/ou solide et, le cas échéant, au moins une substance active en surface.

**14.** Procédé pour la lutte contre de la végétation indésirable qui comprend le fait de laisser agir une quantité active sur le plan herbicide d'une composition herbicide selon l'une quelconque des revendications 1 à 12 ou d'une composition agrochimique selon la revendication 13 sur des végétaux, leur environnement ou leurs graines.

**15.** Utilisation du composé tel que revendiqué dans l'une quelconque des revendications 1 à 12 en tant qu'herbicide ou pour la dessication/défoliation de végétaux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3816419 A
- US 3932167 A
- DE 19744711
- WO 2014064094 A
- WO 2015007711 A
- WO 2015155273 A
- WO 2006024820 A
- WO 2006037945 A
- WO 2007071900 A
- WO 2007096576 A
- WO 020278701 A
- EP 374753 A
- WO 93007278 A
- WO 9534656 A
- EP 427529 A
- EP 451878 A
- WO 0318810 A
- WO 0352073 A
- WO 03018810 A
- EP 392225 A


### Non-patent literature cited in the description


- **J.L. LAMATTINA et al.** *J. Med. Chem.,* 1990, vol. 33, 543-552
- **A. PEREZ-MEDRANO et al.** *J. Med. Chem.,* 2009, vol. 52, 3366-3376
- **P. DAO et al.** *Tetrahedron,* 2012, vol. 68, 3856-3860
- **J. K.CHAKRABARTI et al.** *Tetrahedron,* 1975, vol. 31, 1879-1882
- **J. K. CHAKRABARTI et al.** *Tetrahedron,* 1975, vol. 31, 1879-1882
- **M. FREUND et al.** *Chem. Ber.,* 1901, vol. 34, 3110-3122
- **H. EILINGSFELD et al.** *Chem. Ber.,* 1967, vol. 100, 1874-1891
- *CHEMICAL ABSTRACTS,* 403640-27-7
- *CHEMICAL ABSTRACTS,* 499223-49-3
- *CHEMICAL ABSTRACTS,* 1312337-72-6
- *CHEMICAL ABSTRACTS,* 1312337-45-3
- *CHEMICAL ABSTRACTS,* 1033757-93-5
- *CHEMICAL ABSTRACTS,* 1312340-84-3
- *CHEMICAL ABSTRACTS,* 1312337-48-6
- *CHEMICAL ABSTRACTS,* 1312340-82-1
- *CHEMICAL ABSTRACTS,* 1033760-55-2
- *CHEMICAL ABSTRACTS,* 1312337-51-1
- *CHEMICAL ABSTRACTS,* 1312340-83-2
- *CHEMICAL ABSTRACTS,* 1033760-58-5
- *CHEMICAL ABSTRACTS,* 420138-41-6
- *CHEMICAL ABSTRACTS,* 420138-40-5
- *CHEMICAL ABSTRACTS,* 420138-01-8
- *CHEMICAL ABSTRACTS,* 353292-31-6
- *CHEMICAL ABSTRACTS,* 452098-92-9
- *CHEMICAL ABSTRACTS,* 915396-43-9
- *CHEMICAL ABSTRACTS,* 452099-05-7
- *CHEMICAL ABSTRACTS,* 452100-03-7
- *CHEMICAL ABSTRACTS,* 451484-50-7
- *CHEMICAL ABSTRACTS,* 1300118-96-0
- *CHEMICAL ABSTRACTS,* 1304113-05-0
- *CHEMICAL ABSTRACTS,* 948893-00-3
- *CHEMICAL ABSTRACTS,* 212754-02-4
- *CHEMICAL ABSTRACTS,* 180608-33-7
- *CHEMICAL ABSTRACTS,* 175899-01-1
- *CHEMICAL ABSTRACTS,* 943832-60-8
- *CHEMICAL ABSTRACTS,* 1390661-72-9

- *CHEMICAL ABSTRACTS,* 71526-07-3
- *CHEMICAL ABSTRACTS,* 52836-31-4
- *CHEMICAL ABSTRACTS,* 129531-12-0
- *The Compendium of Pesticide Common Names, http://www.alanwood.net/pesticides*
- Farm Chemicals Handbook 2000. Meister Publishing Company, 2000, vol. 86
- **B. HOCK ; C. FEDTKE ; R. R. SCHMIDT.** Herbizide [Herbicides. Georg Thieme Verlag, 1995
- **W. H. AHRENS.** Herbicide Handbook. Weed Science Society of America, 1994
- **K. K. HATZIOS.** Herbicide Handbook. Weed Science Society of America, 1998
- Catalogue of pesticide formulation types and international coding system. Technical Monograph. CropLife International, May 2008
- **MOLLET ; GRUBEMANN.** Formulation technology. Wiley VCH, 2001
- New developments in crop protection product formulation. **KNOWLES.** Agrow Reports DS243. T&F Informa, 2005
- International Ed. or North American Ed. McCutcheon's, Vol.1: Emulsifiers & Detergents. McCutcheon's Directories, 2008
- Adjuvants and additives. **KNOWLES.** Agrow Reports DS256. T&F Informa, 2006
- *Pest Management Science,* 2005, vol. 61, 246
- *PEST MANAGEMENT SCIENCE,* 2005, vol. 61, 258
- *PEST MANAGEMENT SCIENCE,* 2005, vol. 61, 277
- *PEST MANAGEMENT SCIENCE,* 2005, vol. 61, 269
- *PEST MANAGEMENT SCIENCE,* 2005, vol. 61, 286
- *PEST MANAGEMENT SCIENCE,* 2008, vol. 64, 326
- *PEST MANAGEMENT SCIENCE,* 2008, vol. 64, 332
- *Weed Science,* 2009, vol. 57, 108
- *Australian Journal of Agricultural Research,* 2007, vol. 58, 708
- *Science,* 2007, vol. 316, 1185
- **S. R. COLBY.** Calculating synergistic and antagonistic responses of herbicide combinations. *Weeds,* 1967, vol. 278, 22ff